# EUROPEAN PATENT APPLICATION

(11) **EP 4 588 488 A1**
(43) Date of publication of application: **23.07.2025**
(21) Application number: 24152475.0
(22) Date of filing: 17.01.2024
(51) Int. Cl.: A61K 47/36, C08B 37/02

(54) **HYDROGELS FOR CELL THERAPY**

(71) Applicant: Adocia, 69003 Lyon (FR)
(72) Inventor: ELOY, Marie-Rose, 69003, Lyon (FR); BESNARD, Romain, 69003, Lyon (FR); GAY, Gabrielle, 69003, Lyon (FR); SOULA, Olivier, 69003, Lyon (FR); JOUANNOT, Ouardane, 69003, Lyon (FR)
(74) Representative: Tripoz, Inès

(57) **Abstract**

The invention concerns a bicomponent glue comprising precursors of a hydrogel, allowing adherence between a biological tissue and another biological tissue or between a tissue and a device, in particular a hydrogel.

It also concerns therapeutic and/or surgical use in particular for improving adhesion and/or immobilizing *in vivo* an implant onto biological tissue of the recipient patient.

It also concerns a process for improving adhesion and/or immobilizing in vivo an implant onto biological tissue of the recipient patient comprising the step of applying the bicomponent glue.

## Description

The domain of the invention is therapy, in particular cell therapy. More particularly the invention is about an implant comprising a hydrogel which may incorporate:
- active principles, such as peptides, hormones or proteins, or
- secreting cells, which may be cells secreting peptides or hormones.

The aim is to prevent, treat or cure disease. In particular, this could allow the prevention and/or treatment of chronic diseases by replacing totally or in part the function of naturally occurring cells which are deficient in the patients. The invention is also about a crosslinked polymer, its precursors, a process for obtaining the crosslinked polymer and a process for obtaining a hydrogel, in particular a hydrogel containing cells.

The cells may be isolated or aggregated and may be of one type or of different types.

Hydrogels can be used in multiple systems like:
- scaffolds as controlled drug or active pharmaceutical ingredient release systems or
- scaffolds to be used as implantable device comprising cells.

Hydrogels comprise or consist of polymers that are crosslinked in a 3D network. They can either be natural or synthetic, homopolymers or copolymers. They have the ability to absorb and retain large amounts of water. This is known as the swelling of hydrogels.

In order to have a system which may be an implant able to deliver active principle on the long run, many features have to be obtained.

Among these features may be cited:
- a low degradability, in particular a low biodegradability, or no biodegradability, or a good *in vivo* stability, in order for the incorporated cells not to escape in the organism of the patient and the host cells not to penetrate in the implant,
- a good permselectivity, defined as the selective permeability toward biological elements according to their size or molecular weight, allowing a low, or even better no, immune response by isolating the incorporated cells, totally or in part, from the immune system of the host while allowing the passage of the active principle, for example a hormone, peptide or protein,
- a good mitigation of the foreign body response, or a good biocompatibility, in particular a low cytotoxicity and a good local tolerance,
- allowing the cells to have a high survival rate, such as having a good vascularisation close to the cells and sufficient flux of nutrients to the cells,
- allowing the cells to have a good functionality within the hydrogel.

In order to be used as controlled release systems or scaffolds for cells, hydrogels must have particular characteristics so as to exhibit all or part of the desired properties such as disclosed above as well as good mechanical and rheological properties.

Among the rheological and mechanical properties that are of high interest for the hydrogel may be cited:
- a good homogeneity, which can be linked to a good transparency or translucency,
- appropriate resistance and flexibility toward stress and strain mechanics, in particular when being handled and implanted, for example through laparoscopy,
- a defined mesh size to maximise oxygen and nutrients exchanges, controlled transport properties and permselectivity.
- a good stability *in vivo, i.e.* resistance to hydrolytic, enzymatic or oxidative degradation.

Among parameters which can give indications on the rheological and mechanical desired properties may be cited:
- tan δ (called loss tangent) which gives indication on mechanical properties,
- G', which gives indication on the elastic modulus (stiffness), and on the mesh size,
- compression and/or traction deformation at break, which gives indication on the elasticity and resistance of the hydrogel,
- swellability, which gives indications on the water content, dimensions and mechanical properties.

Among the problems to be solved is obtaining a hydrogel with properties allowing:
- a manipulation for implanting the hydrogel without breaking it, such as by laparoscopy, and/or
- a gel to remain in place after implantation, for example allowing the hydrogel not to get folded after implantation and/or to be immobilized relative to the tissue on which it is implanted.

A very difficult problem to solve is to obtain an implant with a small thickness (in order for the cells to be close to the tissues), a big surface (in order to have relatively large volume) and which has great mechanical characteristics (in order to allow minimally invasive surgery) and having a good biocompatibility!

Another problem to be dealt with relates to the sedimentation of the cells, or islets during the crosslinking leading to gelation.

The following prior art on hydrogel:
- Nestor Lopez Mora et al, "evaluation of dextran(ethyleneglycol) hydrogel films for giant unilamellar lipid vesicle production and their application for the encapsulation of polymersome, Soft Matters, January 2017, Vol. 13, n°33, pp 5580-5585,
- Hanwei Zhang et al., "In situ gelable interpenetrating double network hydrogel formulated from binary components: thiolated chitosan and oxidized dextran", Biomacromolecules, 2011, Vol. 12, n°5, pp 1428-1437,
- Rongsheng Zhang et al., "A novel pH and ionic strength sensitive carboxymethyl dextran hydrogsel, Biomaterials, 2005, Vol. 26, n°22, pp 4677-4683, and
- Taichi Ito et al., "Dextran-based in situ cross-linked injectable hydrogels to prevent peritoneal adhesions", Biomaterials, 2007, Vol. 28, n°23, pp 3418-3426,
disclose hydrogels which are not able to solve technical problems as the hydrogels of the invention do.

In the prior art hydrogels comprising cells which are crosslinked are very often intended for allowing the growth of a cell object, for example 3D cell culture. This kind of application needs that the hydrogels could at the same time embed the starting cells and make space for the new cells obtained by outgrowth and/or proliferation. In order to comply with these two opposite characteristics, the solution is to have degradable, for example via cleavable bonds, hydrogels which are strong enough to embed the cells and which upon degradation make sufficient space for new cells. A method of choice to obtain this degradation is to have a peptidic structure within the crosslinked hydrogel, in particular at the level of the crosslinker in between the polymeric backbone.

This type of behavior is completely incompatible with the aim of the instant invention which is to obtain a long-lasting crosslinked hydrogel encapsulating/embedding cell. In this case the hydrogel needs to have very little, or even better non-degradable, as this key feature will allow to keep the cells invisible from the immune system.

The underlying problem is solved by the provision of a gel that presents physicochemical properties to allow the manufacture of an implantable device and biocompatibility properties that allow the cells survival.

Moreover, and on the contrary to many prior arts, the invention allows the preparation of hydrogels which have tunable features, considering the precursors used and the way the crosslinking is performed. This can lead to hydrogels having a controlled incorporation and release of specific objects from the hydrogel.

The suitability of the hydrogel depends on its bulk structure, thus important parameters used to characterize the network structure of the hydrogel according to the invention are the polymer volume fraction in the swollen state, the molecular weight of the polymer chain between two neighboring crosslinking points, and the corresponding mesh size.

The problem is solved by the provision of a new cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a polyethylene glycol chain, or this at least radical being a at least divalent linear, branched or cyclic alkyl radical comprising at least a poly(oxazoline) (POx) chain..

The problem is solved by the provision of a new cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a polyethylene glycol chain.

The problem is solved by the provision of a new cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a poly(oxazoline) (POx) chain.

In an embodiment the crosslinked dextran polymer Dx bearing anionic groups wherein the at least divalent radical L(-)ᵢ is covalently bound to the dextran polymer backbone with i W radicals, wherein
- L(-)ᵢ is a linear or branched polyether
   - i is the valence of L and the number of W radicals bound to the dextran polymer and is an integer comprised from 2 to 8 (2 ≤ i ≤ 8),
   - -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment, the cross-linked dextran polymer according to the invention is not a dextran polymer bearing carboxylate groups as disclosed and described in the application PCT/EP2022/050466 filed on 11 January 2022.

In an embodiment, the cross-linked dextran polymer according to the invention is not a dextran polymer bearing carboxylate groups wherein the at least divalent radical L(-)ᵢ is covalently bound to the dextran polymer backbone with i W radicals, wherein,
- L(-)ᵢ is a linear or branched polyether bearing at its ends, heteroatoms such as oxygen, nitrogen or sulfur,
- i is the valence of L and the number of -(R₁)ₘG₁- - radicals and is an integer comprised from 2 to 8 (2 ≤ i ≤ 8),
- m is an integer equal to 0 or 1,
- W is a -(R₁)ₘG₁- radical, wherein
   - -R₁- is a linear or branched alkyl divalent radical comprising from 1 to 6 carbon atoms and optionally heteroatoms such as oxygen, nitrogen or sulfur,
   - -G₁- is a linear or branched or cyclic alkyl divalent radical comprising from 1 to 6 carbon atoms and may comprise heteroatoms such as oxygen, nitrogen or sulfur.

In an embodiment, the cross-linked dextran polymer according to the invention is not a dextran polymer bearing carboxylate groups wherein the at least divalent radical L(-)ᵢ is covalently bound to the dextran polymer backbone with i W radicals, wherein, - W is a -(R₁)ₘG₁- radical, wherein
- -G₁- is a sulfone derivative as described in the following formula: 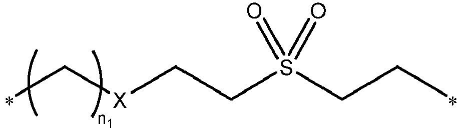 Wherein:
   - n₁ is an integer comprised from 0 to 7 (0 ≤ n₁ ≤ 7).
   - X a sulphur atom.
   - the * represent the attachment sites to the dextran backbone and divalent radical L(-)ᵢ
   - or
      - -G₁- is a succinimide derivative as described in the following formula: 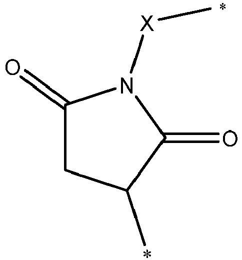 Wherein:
         - X is either a linear *-(CH₂)ₙ₁-* with m an integer comprised from 1 to 7 (1 ≤ n₁ ≤ 7),
         - the * represent the attachment sites to the dextran backbone and divalent radical L(-)ᵢ

In an embodiment, the cross-linked dextran polymer according to the invention is not a dextran polymer bearing carboxylate groups wherein the at least divalent radical L(-)ᵢ is covalently bound to the dextran polymer backbone with i W radicals, wherein,
- W is a -(R₁)ₘG₁- radical, wherein
   - -(R₁)ₘG₁- is a linear or branched alkyl divalent radical comprising less than 13 carbon atoms and optionally heteroatoms such as oxygen, nitrogen or sulfur.

In an embodiment, the dextran polymer is not a dextran polymer wherein the at least divalent radical L(-)ᵢ covalently bound to the dextran polymer backbone with i W radicals results is a radical issued from a linear or branched mercaptopolyethyleneglycol comprising at least 2 sulfur atoms and comprising at most 8 arms which
- Mn is comprised from 1000 to 25 000 g/mol (1000 ≤ Mn ≤ 25 000 g/mol) or
- polymerisation degree (DP) is comprised from 15 to 600 (15 ≤ DP ≤ 600).

In an embodiment, the dextran polymer is not a dextran polymer wherein the at least divalent radical L(-)ᵢ covalently bound to the dextran polymer backbone with i W radicals is not a radical issued from a linear or branched mercaptopolyethyleneglycol comprising at least 2 sulfur atoms and comprising at most 8 arms, which
- number-average molecular weight (Mn) is comprised from 500 to 40 000 g/mol (500 ≤ Mn ≤ 40 000 g/mol) or
- polymerisation degree (DP) is comprised from 8 to 1000 (8 ≤ DP ≤ 1000).

The properties of this family of hydrogels are tunable and tailorable to the applications by choosing and adapting the crosslinking reaction conditions, the substitution degree and molecular weight of the dextrans and cross-linkers.

The problem is solved by the provision of a new Hydrogel comprising:
- biological cells,
- a non crosslinked hyaluronate in the form of a solution, and
- a crosslinked dextran polymer Dx bearing anionic groups wherein the at least divalent radical L(-)ᵢ is covalently bound to the dextran polymer backbone with i W radicals, wherein,

- L(-)ᵢ is a linear or branched polyether
   - i is the valence of L and the number of W radicals bound to the dextran polymer and is an integer comprised from 2 to 8 (2 ≤ i ≤ 8),
   - -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment, the cross-linked dextran polymer comprised in the hydrogel according to the invention is not a dextran polymer bearing carboxylate groups wherein the at least divalent radical L(-)ᵢ is covalently bound to the dextran polymer backbone with I W radicals, wherein,
- L(-)ᵢ is a linear or branched polyether bearing at its ends, heteroatoms such as oxygen, nitrogen or sulfur,
- i is the valence of L and the number of -(R₁)ₘG₁- - radicals and is an integer comprised from 2 to 8 (2 ≤ i ≤ 8),
- m is an integer equal to 0 or 1,
- W is a -(R₁)ₘG₁- radical, wherein
   - -R₁₋ is a linear or branched alkyl divalent radical comprising from 1 to 6 carbon atoms and optionally heteroatoms such as oxygen, nitrogen or sulfur,
   - -G₁- is a linear or branched or cyclic alkyl divalent radical comprising from 1 to 6 carbon atoms and may comprise heteroatoms such as oxygen, nitrogen or sulfur.

In an embodiment, the dextran polymer comprised in the hydrogel is not a dextran polymer wherein the at least divalent radical L(-)ᵢ covalently bound to the dextran polymer backbone with i W radicals results is a radical issued from a linear or branched mercaptopolyethyleneglycol comprising at least 2 sulfur atoms and comprising at most 8 arms which
- Mn is comprised from 1000 to 25 000 g/mol (1000 ≤ Mn ≤ 25 000 g/mol) or
- polymerization degree (DP) is comprised from 15 to 600 (15 ≤ DP ≤ 600).

In an embodiment, the dextran polymer comprised in the hydrogel is not a dextran polymer wherein the at least divalent radical L(-)ᵢ covalently bound to the dextran polymer backbone with i W radicals is not a radical issued from a linear or branched mercaptopolyethyleneglycol comprising at least 2 sulfur atoms and comprising at most 8 arms, which
- number-average molecular weight (Mn) is comprised from 500 to 40 000 g/mol (500 ≤ Mn ≤ 40 000 g/mol) or
- polymerization degree (DP) is comprised from 8 to 1000 (8 ≤ DP ≤ 1000).

The cross-linked dextran polymer comprised in the hydrogel according to the invention is a dextran polymer Dx bearing anionic groups wherein the at least divalent radical L(-)ᵢ is covalently bound to the dextran polymer backbone with i -W-radicals, wherein,
- L(-)ᵢ is a linear or branched polyether, or L(-)ᵢ is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether or poly(oxazoline) derivatives and that does not comprise two or more alpha aminoacid residues in particular linked by peptidic bond(s).

The cross-linked dextran polymer comprised in the hydrogel according to the invention is a dextran polymer Dx bearing anionic groups wherein the at least divalent radical L(-)ᵢ is covalently bound to the dextran polymer backbone with i -W-radicals, wherein,
- L(-)ᵢ is a linear or branched polyether
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

The cross-linked dextran polymer comprised in the hydrogel according to the invention is a dextran polymer Dx- bearing anionic groups wherein the at least divalent radical L is covalently bound to the dextran polymer backbone with i W radicals, wherein,
- L is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment, the cross-linked dextran polymer according to the invention is not a dextran polymer bearing carboxylate groups wherein the at least divalent radical L(-)ᵢ is covalently bound to the dextran polymer backbone with i W radicals, wherein,
- L(-)ᵢ is a linear or branched polyether bearing at its ends, heteroatoms such as oxygen, nitrogen or sulfur,
- i is the valence of L and the number of -(R₁)ₘG₁- - radicals and is an integer comprised from 2 to 8 (2 ≤ i ≤ 8),
- m is an integer equal to 0 or 1,
- W is a -(R₁)ₘG₁- radical, wherein
   - -R₁₋ is a linear or branched alkyl divalent radical comprising from 1 to 6 carbon atoms and optionally heteroatoms such as oxygen, nitrogen or sulfur,
   - -G₁- is a linear or branched or cyclic alkyl divalent radical comprising from 1 to 6 carbon atoms and may comprise heteroatoms such as oxygen, nitrogen or sulfur.

In an embodiment, the dextran polymer is not a dextran polymer wherein the at least divalent radical L(-)ᵢ covalently bound to the dextran polymer backbone with i W radicals results is a radical issued from a linear or branched mercaptopolyethyleneglycol comprising at least 2 sulfur atoms and comprising at most 8 arms which
- number-average molecular weight Mn is comprised from 1000 to 25 000 g/mol (1000 ≤ Mn ≤ 25 000 g/mol) or
- polymerisation degree (DP) is comprised from 15 to 600 (15 ≤ DP ≤ 600).

In an embodiment, the dextran polymer is not a dextran polymer wherein the at least divalent radical L(-)ᵢ covalently bound to the dextran polymer backbone with i W radicals is not a radical issued from a linear or branched mercaptopolyethyleneglycol comprising at least 2 sulfur atoms and comprising at most 8 arms, which
- number-average molecular weight (Mn) is comprised from 500 to 40 000 g/mol (500 ≤ Mn ≤ 40 000 g/mol) or
- polymerisation degree (DP) is comprised from 8 to 1000 (8 ≤ DP ≤ 1000).

In an embodiment, the cross-linked dextran polymer according to the invention is not a dextran polymer bearing carboxylate groups wherein the at least divalent radical L(-)ᵢ is covalently bound to the dextran polymer backbone with i W radicals, wherein,
- W is a -(R₁)ₘG₁- radical, wherein
   - -G₁- is a sulfone derivative as described in the following formula: 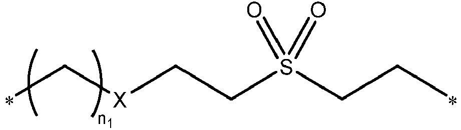 Wherein:
      - n₁ is an integer comprised from 0 to 7 (0 ≤ n₁ ≤ 7).
      - X a sulphur atom.
      - the * represent the attachment sites to the dextran backbone and divalent radical L(-)ᵢ
      - or
         - -G₁- is a succinimide derivative as described in the following formula: 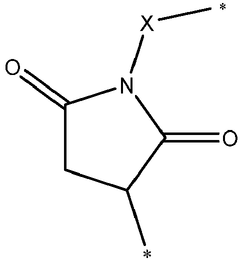 Wherein:
            - X is either a linear *-(CH₂)ₙ₁-* with n₁ an integer comprised from 1 to 7 (1 ≤ n₁ ≤ 7),
            - the * represent the attachment sites to the dextran backbone and divalent radical L(-)ᵢ

In an embodiment, the cross-linked dextran polymer according to the invention is not a dextran polymer bearing carboxylate groups wherein the at least divalent radical L(-)ᵢ is covalently bound to the dextran polymer backbone with i W radicals, wherein,
- W is a -(R₁)ₘG₁- radical, wherein
   - -(R₁)ₘG₁- is a linear or branched alkyl divalent radical comprising less than 13 carbon atoms and optionally heteroatoms such as oxygen, nitrogen or sulfur.

It also concerns and implant comprising the hydrogel of the invention.

The applicant surprisingly found that the presence of hyaluronic acid or sodium or potassium hyaluronate salts in the crosslinking mixture helps improving a homogeneous repartition of the cells or islets in the hydrogel. In other words, this decreases the effect of sedimentation of the cells or islets.

In an embodiment the hydrogel comprises hyaluronic acid or sodium or potassium hyaluronate salts.

In an embodiment the hyaluronic acid or sodium or potassium hyaluronate salts has a weight average molecular (Mw) ranging from 100 to 2 500 kg/mol.

In an embodiment the hyaluronic acid or sodium or potassium hyaluronate salts has Mw ranging from 250 to 2 500 kg/mol.

In an embodiment the hyaluronic acid or sodium or potassium hyaluronate salts has Mw ranging from 500 to 2 250 kg/mol.

In an embodiment the hyaluronic acid or sodium or potassium hyaluronate salts has a Mw ranging from 750 to 2 000 kg/mol.

In an embodiment the hyaluronic acid or sodium or potassium hyaluronate salts has a Mw ranging from 1 000 to 1 500 kg/mol.

In an embodiment the hyaluronic acid or sodium or potassium hyaluronate salts has Mw ranging from 250 to 4 000 kg/mol.

In an embodiment the hyaluronic acid or sodium or potassium hyaluronate salts has Mw ranging from 500 to 3 750 kg/mol.

In an embodiment the hyaluronic acid or sodium or potassium hyaluronate salts has a Mw ranging from 750 to 3 500 kg/mol.

In an embodiment the hyaluronic acid or sodium or potassium hyaluronate salts has a Mw ranging from 1 000 to 3 250 kg/mol.

In an embodiment the concentration of hyaluronic acid or sodium or potassium hyaluronate salts in the hydrogel ranges from 0.5 to 30 mg/ml.

In an embodiment the concentration of hyaluronic acid or sodium or potassium hyaluronate salts in the hydrogel ranges from 0.5 to 20 mg/ml.

In an embodiment the concentration of hyaluronic acid or sodium or potassium hyaluronate salts in the hydrogel ranges from 0.5 to 10 mg/ml.

In an embodiment the concentration of hyaluronic acid or sodium or potassium hyaluronate salts in the hydrogel ranges from 0.5 to 5 mg/ml.

In an embodiment the concentration of hyaluronic acid or sodium or potassium hyaluronate salts in the hydrogel ranges from 0.75 to 2.5 mg/ml.

In an embodiment the concentration of hyaluronic acid or sodium or potassium hyaluronate salts in the hydrogel ranges from 1.0 to 1.5 mg/ml.

In an embodiment the concentration of hyaluronic acid or sodium or potassium hyaluronate salts in the hydrogel ranges from 0.8 to 1.2 mg/ml.

In an embodiment with a hyaluronic acid of Mw ranging from 2 000 to 4 000 kg/mol, in particular of around 3 000 kg/mol, its concentration is ranging from 0.5 to 1.5 mg/ml.

In an embodiment with a hyaluronic acid of Mw ranging from 2 000 to 4000 kg/mol, in particular of around 3 000 kg/mol, its concentration is ranging from 0.7 to 1.2 mg/ml.

In an embodiment with a hyaluronic acid or sodium or potassium hyaluronate salts of Mw ranging from 1 000 to 2 000 kg/mol, in particular of around 1 500 kg/mol, its concentration is ranging from 0.5 to 2 mg/ml.

In an embodiment with a hyaluronic acid or sodium or potassium hyaluronate salts of 1 000 to 2 000 kg/mol, in particular around 1 500 kg/mol, its concentration is ranging from 1.0 to 1.5 mg/ml.

The cross-linked dextran polymer according to the invention is a dextran polymer Dx bearing anionic groups wherein the at least divalent radical L(-)ᵢ is covalently bound to the dextran polymer backbone with i -W- radicals, wherein,
- L(-)ᵢ is a linear or branched polyether, or L(-)ᵢ is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether or poly(oxazoline) derivatives and that does not comprise two or more alpha aminoacid residues in particular linked by peptidic bond(s).

The cross-linked dextran polymer according to the invention is a dextran polymer Dx bearing anionic groups wherein the at least divalent radical L(-)ᵢ is covalently bound to the dextran polymer backbone with i -W- radicals, wherein,
- L(-)ᵢ is a linear or branched polyether
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

The cross-linked dextran polymer according to the invention is a dextran polymer Dx- bearing anionic groups wherein the at least divalent radical L is covalently bound to the dextran polymer backbone with i W radicals, wherein,
- L is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment -W- comprises at most 60 carbon atoms.

In an embodiment -W- comprises at most 60 carbon atoms without counting any -CHz-CHzO- radicals.

In an embodiment -W- comprises at most 50 carbon atoms.

In an embodiment -W- comprises at most 50 carbon atoms without counting any -CHz-CHzO- radicals.

In an embodiment -W- comprises at most 40 carbon atoms.

In an embodiment -W- comprises at most 40 carbon atoms without counting any -CHz-CHzO- radicals.

In an embodiment -W- comprises at most 30 carbon atoms.

In an embodiment -W- comprises at most 30 carbon atoms without counting any -CHz-CHzO- radicals.

In an embodiment -W- comprises at most 20 carbon atoms.

In an embodiment -W- comprises at most 20 carbon atoms without counting any -CHz-CHzO- radicals.

In an embodiment -W- comprises at most 10 carbon atoms.

In an embodiment -W- comprises at most 10 carbon atoms without counting any -CHz-CHzO- radicals.

In an embodiment -W- comprises at most 10 oxygen atoms.

In an embodiment -W- comprises at most 10 oxygen atoms without counting any -CHz-CHzO- radicals.

In an embodiment -W- comprises at most 5 oxygen atoms.

In an embodiment -W- comprises at most 5 oxygen atoms without counting any -CH₂-CH₂O- radicals.

The crosslinked dextran hydrogel according to the invention is a dextran polymer wherein the central-linker L(-)ᵢ is a linear, or a branched polyethylene glycol (PEG) radical.

By branched PEG is meant various PEG arms connected by a linear, branched, or cyclic alkyl, or by an aromatic, comprising between 2 to 20 carbon atoms and may comprise heteroatoms such as nitrogen, oxygen, or sulphur.

In one embodiment, the crosslinked dextran hydrogel according to the invention is a dextran polymer wherein the central-linker L(-)ᵢ is a branched PEG radical which possesses at most 8 arms.

In an embodiment, the central-linker L(-)ᵢ is a PEG chosen among the PEG of formula I : 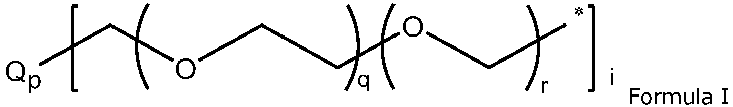 Wherein:
- i is an integer comprised from 2 to 8 (2 ≤ i ≤ 8)
- p is an integer equal to 0 or 1, and if i=2 then p=0
- q is an integer comprised from 8 to 1000 (8 ≤ q ≤ 1000)
- r is an integer equal to 0 or 1
- Q is either a carbon atom, or a linear, branched, or cyclic alkyl chain, or an aromatic, comprising 2 to 10 carbon atoms and may comprise heteroatoms such as nitrogen, oxygen, or sulphur
- the * represents the sites of f₄, which is an amine function, or an ether, or a thioether function, or an amide function, or a carbamate function or a carbon-nitrogen covalent bond, or a carbon-aromatic carbon covalent bond, or a carbon-carbon covalent bond if the crosslinking process is made by a Native Chemical Ligation (NCL).

In an embodiment q is an integer comprised from 80 to 500 (80 ≤ q ≤ 500).

In an embodiment q is an integer comprised from 100 to 300 (100 ≤ q ≤ 300).

In an embodiment, the cross-linked dextran polymer according to the invention is a dextran polymer wherein L(-)ᵢ is a radical issued from a linear or branched mercaptopolyethyleneglycol comprising at least 2 sulfur atoms and comprising at most 8 arms, which:
- number-average molecular weight (Mn) is comprised from 500 to 40 000 g/mol (500 ≤ Mn ≤ 40 000 g/mol) or
- polymerisation degree (DP) is comprised from 8 to 1000 (8 ≤ DP ≤ 1000).

In an embodiment, the cross-linked dextran polymer according to the invention is a dextran polymer wherein L(-)ᵢ is a radical issued from a linear or branched mercaptopolyethyleneglycol comprising at least 2 sulfur atoms and comprising at most 8 arms, which:
- Mn is comprised from 1000 to 25 000 g/mol (1000 ≤ Mn ≤ 25 000 g/mol) or
- polymerisation degree (DP) is comprised from 15 to 600 (15 ≤ DP ≤ 600).

In an embodiment, the cross-linked dextran polymer according to the invention is a dextran polymer wherein L(-)ᵢ is a radical according to formula I, issued from the thiol polyethylene glycols or mercaptopoly(oxyethylenes) cited in the following table:

| Chemical Name | i | Mn (kg/mol) |
|---|---|---|
| Poly(ethylene glycol) dithiol | 2 | 10 |
| Poly(ethylene glycol) dithiol | 2 | 3.4 |
| Poly(ethylene glycol) dithiol | 2 | 1 |
| Pentaerythritol tetra(mercaptoethyl) polyoxyethylene | 4 | 5.2 |
| Pentaerythritol tetra(mercaptoethyl) polyoxyethylene | 4 | 20 |
| Pentaerythritol tetra(mercaptoethyl) polyoxyethylene | 4 | 40 |
| tripentaerythritol octa(mercaptoethyl) polyoxyethylene | 8 | 20 |

In an embodiment, the cross-linked dextran polymer according to the invention is a dextran polymer wherein L(-)ᵢ is a radical according to formula I issued from a pentaerythritol tetra(mercaptoethyl) polyoxyethylene, CAS# 188492-68-4.

In an embodiment, the cross-linked dextran polymer according to the invention is a dextran polymer wherein L(-)ᵢ is a radical according to formula I issued from a linear (mercaptoethyl)polyoxyethylene, CAS# 68865-60-1.

In an embodiment, the cross-linked dextran polymer according to the invention is a dextran polymer wherein L(-)ᵢ is a radical according to formula I, issued from a pentaerythritol poly(oxyethylene) azide cited in the following table:

| Chemical Name | i | Mn (kg/mol) |
|---|---|---|
| 2-arm PEG azide (Polyoxyethylene bis(azide)) | 2 | 5 |
| 2-arm PEG azide | 2 | 10 |
| 4-arm PEG azide, pentaerythritol core | 4 | 20 |
| 4-arm PEG azide, pentaerythritol core | 4 | 40 |
| 8-arm PEG azide, tripentaerythritol core | 8 | 20 |

In an embodiment, the cross-linked dextran polymer according to the invention is a dextran polymer wherein L(-)i is a radical according to formula I issued from a pentaerythritol 4-arm PEG azide, CAS# 225531-50-0.

In an embodiment, the cross-linked dextran polymer according to the invention is a dextran polymer wherein L(-)ᵢ is a radical according to formula I, issued from a pentaerythritol poly(dibenzocyclooctyne) polyoxyethylene cited in the following table:

| Chemical Name | i | Mn (kg/mol) |
|---|---|---|
| 2-arm PEG DBCO Dibenzocycloctyne-PEG-Dibenzocycclotyne | 2 | 5 |
| 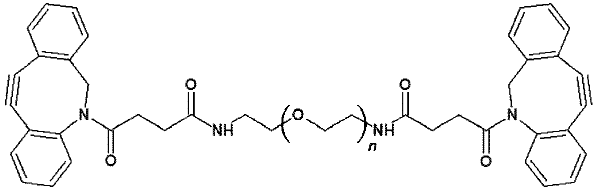 | | |
| 2-arm PEG DBCO | 2 | 10 |
| 4-arm PEG DBCO, pentaerythritol core | 4 | 20 |
| 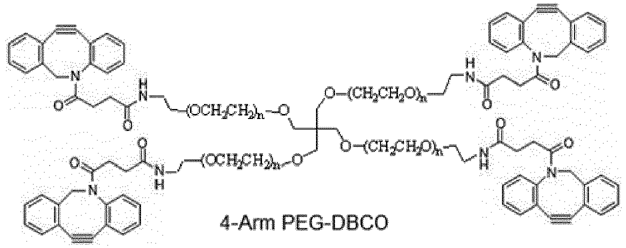 | | |
| 4-arm PEG DBCO, pentaerythritol core | 4 | 40 |
| 8-arm PEG DBCO, tripentaerythritol core | 8 | 20 |
| 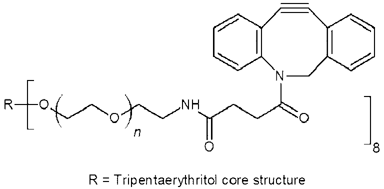 | | |

In an embodiment, the cross-linked dextran polymer according to the invention is a dextran polymer wherein L(-)i is a radical according to formula I issued from a pentaerythritol 4-arm PEG DBCO.

In an embodiment, the cross-linked dextran polymer according to the invention is a dextran polymer wherein L(-)ᵢ is a radical according to formula I, issued from the maleimide polyethylene glycols cited in the following table:

| Chemical Name | i | Mn (kg/mol) |
|---|---|---|
| 2-arm PEG Mal, Maleimide-PEG-Maleimide | 2 | 5 |
| 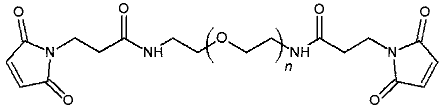 | | |
| 2-arm PEG Mal | 2 | 10 |
| 4-arm PEG Mal, pentaerythritol core | 4 | 20 |
| 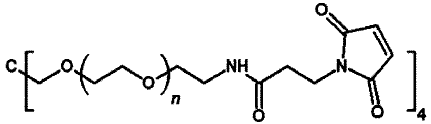 | | |
| 4-arm PEG Mal, pentaerythritol core | 4 | 40 |
| 8-arm PEG Mal, tripentaerythritol core | 8 | 20 |
| 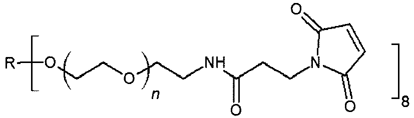 R = Tripentaerythritol core structure | | |

In an embodiment, the cross-linked dextran polymer according to the invention is a dextran polymer wherein L(-)ᵢ is a radical according to formula I issued from a 4-arm poly(ethylene glycol) maleimide.

In an embodiment, the cross-linked dextran polymer according to the invention is a dextran polymer wherein L(-)ᵢ is a radical according to formula I, issued from the norbornene polyethylene glycols cited in the following table:

| Chemical Name | i | Mn (kg/mol) |
|---|---|---|
| 2-arm PEG Nb, Norbornene-PEG-Norbornene | 2 | 5 |
| 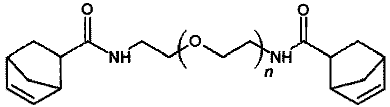 | | |
| 2-arm PEG Nb | 2 | 10 |
| 4-arm PEG Nb, pentaerythritol core | 4 | 20 |
| 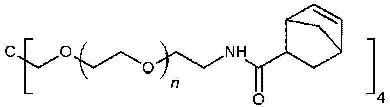 | | |
| 4-arm PEG Nb, pentaerythritol core | 4 | 40 |
| 8-arm PEG Nb, tripentaerythritol core | 8 | 20 |
| 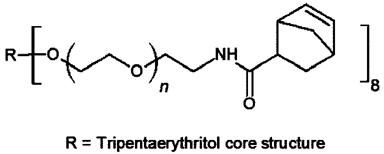 | | |

In an embodiment, the cross-linked dextran polymer according to the invention is a dextran polymer wherein L(-)ᵢ is a radical according to formula I issued from a 4-arm poly(ethylene glycol) norbornene.

In one embodiment, the crosslinked dextran hydrogel according to the invention is a dextran polymer wherein the central-linker L is a linear, or a branched POx radical.

In an embodiment, the cross-linked dextran polymer according to the invention is a dextran polymer wherein L is a linear or branched POx radical comprising at most 8 arms, which number-average molecular weight (Mn) is comprised from 500 to 40 000 g/mol (500 ≤ Mn ≤ 40 000 g/mol).

In an embodiment, the cross-linked dextran polymer according to the invention is a dextran polymer wherein L is a linear or branched POx radical comprising at most 8 arms, which number-average molecular weight (Mn) is comprised from 1000 to 25 000 g/mol (1 000 ≤ Mn ≤ 25 000 g/mol).

In one embodiment, the POx central linker is a 2-arm POx, chosen among the linkers of formula XII. 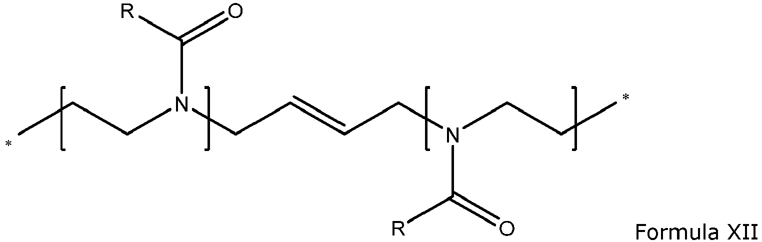 Wherein:
- The radical -R is a linear, -(CH₂)ₙ₁-CH₃ with m an integer comprised from 0 to 4 (0 ≤ n₁ ≤ 4), branched, or cyclic alkyl derivative.
- The * represents the sites of f₃, which is an amine function, or an ether function, or a thioether function, or an amide function, or a carbamate function, or a carbon-nitrogen covalent bond, or a carbon-aromatic carbon covalent bond.

In one embodiment, the POx central linker is a 2-arm POx, chosen among the linkers of formula XIIbis. 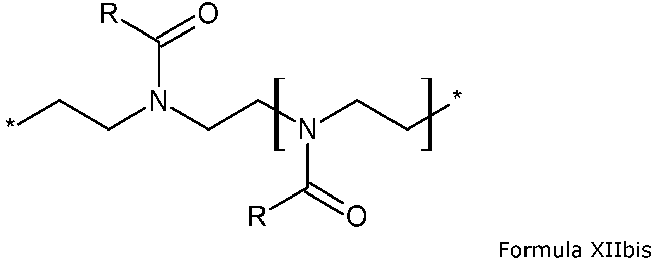 Wherein:
- The radical -R is a linear, -(CH₂)ₙ₁-CH₃ with m an integer comprised from 0 to 4 (0 ≤ n₁ ≤ 4), branched, or cyclic alkyl derivative.
- The * represents the sites of f₃, which is an amine function, or an ether function, or a thioether function, or an amide function, or a carbamate function, or a carbon-nitrogen covalent bond, or a carbon-aromatic carbon covalent bond.

In one embodiment, the POx central linker is a 4-arm POx, chosen among the linkers of formula XIII. 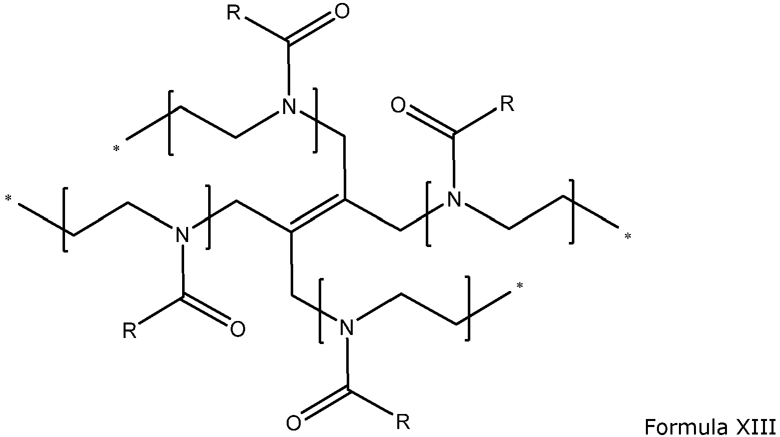 Wherein:
- The radical -R is a linear, -(CH₂)ₙ₁-CH₃ with n₁ an integer comprised from 0 to 4 (0 ≤ n₁ ≤ 4), branched, or cyclic alkyl derivative.
- The * represents the sites of f₃, which is an amine function, or an ether function, or a thioether function, or an amide function, or a carbamate function, or a carbon-nitrogen covalent bond, or a carbon-aromatic carbon covalent bond.

In another embodiment, the POx central linker is a 4-arm POx, chosen among the linkers of formula XIV: 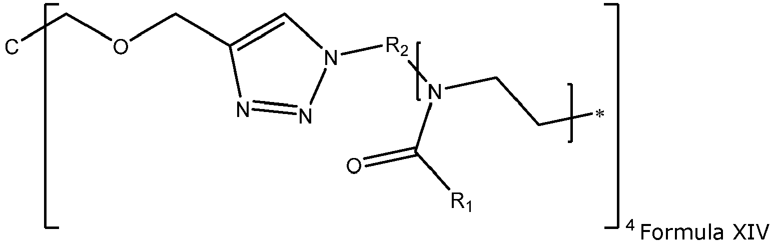 Wherein:
- The radical -R₁ is a linear, -(CH₂)ₙ₂-CH₃ with n₁ an integer comprised from 0 to 4 (0 ≤ n₁ ≤ 4), branched, or cyclic alkyl derivative.
- The divalent radical -R₂- is a linear, -(CH₂)ₙ₂- with n₂ an integer comprised from 2 to 6 (2 ≤ n₂ ≤ 6).
- The * represents the sites of f₃, which is an amine function, or an ether function, or a thioether function, or an amide function, or a carbamate function, or a carbon-nitrogen covalent bond, or a carbon-aromatic carbon covalent bond.

In another embodiment, the POx central linker is a 4-arm POx, chosen among the linkers of formula XV: 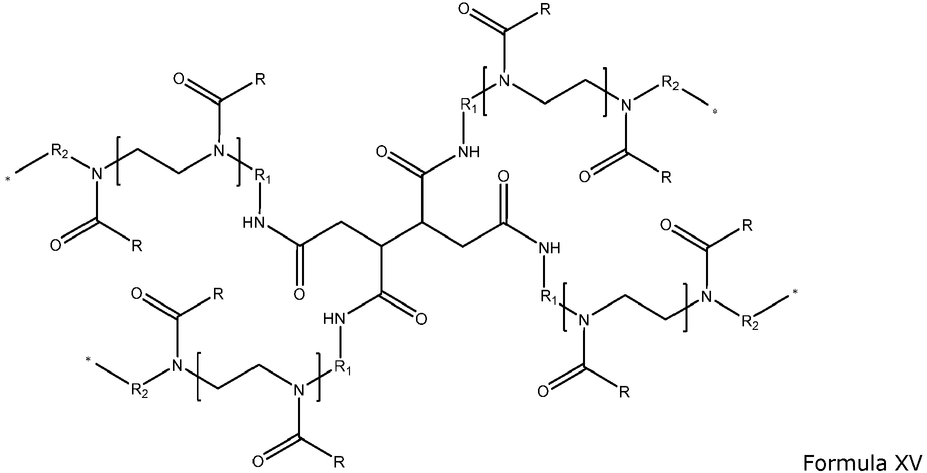 Wherein:
- The radical -R is a linear, -(CH₂)ₙ₁-CH₃ with m an integer comprised from 0 to 4 (0 ≤ n₁ ≤ 4), branched, or cyclic alkyl derivative.
- In one embodiment, R₁ = -CH₂-CH₂- and R₂ is a linear, -(CH₂)ₙ₂- with n₂ an integer comprised from 2 to 6 (2 ≤ n₂ ≤ 6)
- In another embodiment, R₂ = -CH₂-CH₂- and R₁ is a linear, *-(CH₂)ₙ₂-* with nz an integer comprised from 2 to 6 (2 ≤ n₂ ≤ 6)
- The * represents the sites of f₃, which is an amine function, or an ether function, or a thioether function, or an amide function, or a carbamate function, or a carbon-nitrogen covalent bond, or a carbon-aromatic carbon covalent bond.

The hydroxyl functions of the dextran polymer Dx- can be functionalised by at least one specific anionic group such as: alkyl carboxylate, sulphate anions, or sulfonate anions, or phosphate anions, or phosphonate anions.

In one embodiment, the hydroxyl functions of the dextran polymer Dx- can be functionalised by sulphate anions in salified form, and optionally by alkyl carboxylate derivatives in salified form.

In another embodiment, the hydroxyl functions of the dextran polymer Dx-can be functionalised by sulfonate anions in salified form, and optionally by alkyl carboxylate derivatives in salified form.

In another embodiment, the hydroxyl functions of the dextran polymer backbone Dx-, can be functionalized by phosphate anions in salified form, and optionally by alkyl carboxylate derivatives in salified form.

In another embodiment, the hydroxyl functions of the dextran polymer Dx-can be functionalised by phosphonate anions in salified form, and optionally by alkyl carboxylate derivatives in salified form.

In another embodiment, the hydroxyl functions of the dextran polymer Dx-can be functionalised by alkyl carboxylate derivatives in salified form.

In an embodiment, the hydroxyl functions of the dextran polymer Dx- are functionalised by one specific anionic group: alkyl carboxylates anions.

In an embodiment, the hydroxyl functions of the dextran polymer Dx- are functionalised only by one specific anionic group: alkyl carboxylates anions.

The specific anionic groups defined previously are chosen among the groups of formula II: 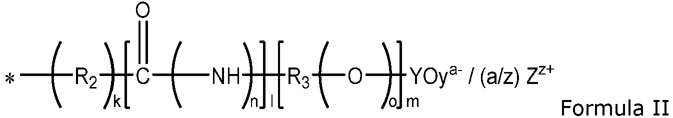 Wherein:
- * represents the link to the O atoms of the dextran to form an ether function.
- y=2 or 3.
- When y=2, alkyl carboxylate derivatives, then:
   ∘ Y=C and a=1.
   ∘ k=1, l=0 and m=0.
   ∘ R₂=Alkyl.
- When y=3, anionic group, then:
   ∘ Y=S and a=1, or Y=P and a=2.
   ∘ k=0 or 1.
   ∘ l=0 or 1.
   ∘ m=0 or 1.
   ∘ n=1 or 2. In particular n = 1,
   ∘ o=0 or 1.
   ∘ if l=1 then m=1.
   ∘ R₃=linear, branched, or cyclic alkyl which may contain one heteroatom such as nitrogen, or aromatic, or PEG.
   ∘ R₂=Alkyl.
- And, Z is a counter ion, which can be an alkali metal and z=1, or which can be an alkaline earth metal and z=2.

In a preferred embodiment, the dextran backbone, Dx-, can be functionalised by sulphate anions in salified form, and optionally by alkyl carboxylate derivatives in salified form.

In another preferred embodiment, the dextran backbone, Dx-, can be functionalised by alkyl sulfonate anions in salified form, and optionally by alkyl carboxylate derivatives in salified form.

In another preferred embodiment, the dextran backbone, Dx-, can be functionalised by sulfonate anions in salified form, supported by an alkyl chain comprising a dimethyl-ammonium cation, and optionally by alkyl carboxylate derivatives in salified form.

In another preferred embodiment, the dextran backbone, Dx-, can be functionalised by alkyl carboxylate derivatives in salified form.

In an embodiment, the cross-linked dextran polymer bearing anionic groups according to the invention is a dextran polymer wherein the dextran polymer backbone is according to formula III, 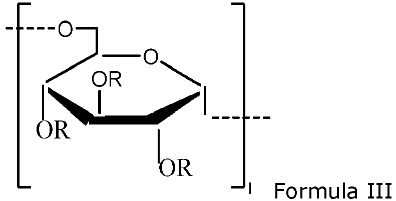 wherein R is chosen among
- -H, a anionic group of formula II, or a -W- radical bearing a L(-)ᵢ crosslinker,
- i is comprised from 20 to 5000 (20 ≤ i ≤ 5000),
- -W- and L(-)ᵢ radicals having the previously defined meanings.

In an embodiment, the cross-linked dextran polymer bearing anionic groups according to the invention is a dextran polymer wherein the dextran polymer backbone is according to formula XI, 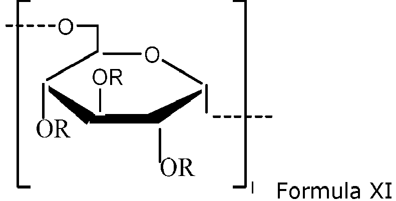 wherein R is chosen among
- -H, a anionic group of formula II, or a -(A-f₂)ₐ-G₁- radical bearing a L(-)ᵢ crosslinker,
- l is comprised from 20 to 5000 (20 ≤ l ≤ 5000),
- -(A-f₂)ₐ-G₁- and L(-)ᵢ radicals having the previously defined meanings.

In an embodiment, the cross-linked dextran polymer according to the invention is a dextran polymer wherein the dextran polymer backbone is a dextran having a weight average molecular weight (Mw) comprised from 5 to 1000 kDa before crosslinking and substitution.

In other words, the cross-linked dextran polymer according to the invention is obtained after substitution and crosslinking of a native dextran polymer having a weight average molecular weight (Mw) comprised from 5 to 1000 kDa.

In an embodiment, the cross-linked dextran polymer according to the invention is a dextran polymer wherein the dextran polymer backbone is a dextran having a weight average molecular weight (Mw) comprised from 5 to 250 kDa before crosslinking and substitution.

In an embodiment, the cross-linked dextran polymer according to the invention is a dextran polymer wherein the dextran polymer backbone is a dextran having a weight average molecular weight (Mw) comprised from 5 to 100 kDa before crosslinking and substitution.

In an embodiment, the cross-linked dextran polymer according to the invention is a dextran polymer wherein the dextran polymer backbone is a dextran having a weight average molecular weight (Mw) comprised from 5 to 50 kDa before crosslinking and substitution.

In an embodiment, the cross-linked dextran polymer according to the invention is a dextran polymer wherein the dextran polymer backbone is a dextran having a weight average molecular weight (Mw) comprised from 5 to 25 kDa before crosslinkingcrosslinking and substitution.

In an embodiment, the cross-linked dextran polymer according to the invention is a dextran polymer wherein the dextran polymer backbone is a dextran having a weight average molecular weight (Mw) comprised from 250 to 1000 kDa before crosslinking and substitution.

In an embodiment, the cross-linked dextran polymer according to the invention is a dextran polymer wherein the dextran polymer backbone is a dextran having a weight average molecular weight (Mw) comprised from 10 to 500 kDa before crosslinking and substitution.

In an embodiment, the cross-linked dextran polymer according to the invention is a dextran polymer wherein the dextran polymer backbone is a dextran having a weight average molecular weight (Mw) comprised from 20 to 500 kDa before crosslinking and substitution.

In an embodiment, the cross-linked dextran polymer according to the invention is a dextran polymer wherein the dextran polymer backbone is a dextran having a weight average molecular weight (Mw) comprised from 20 to 100 kDa before crosslinking and substitution.

In an embodiment, the cross-linked dextran polymer according to the invention is a dextran polymer wherein the dextran polymer backbone is a dextran having a weight average molecular weight (Mw) comprised from 20 to 50 kDa before crosslinking and substitution.

In an embodiment, the cross-linked dextran polymer according to the invention is a dextran polymer wherein the dextran polymer backbone is a dextran having a weight average molecular weight (Mw) comprised from 40 to 250 kDa before crosslinking and substitution.

In an embodiment, the cross-linked dextran polymer according to the invention is a dextran polymer wherein the dextran polymer backbone is a dextran having a weight average molecular weight (Mw) comprised from 40 to 100 kDa before crosslinking and substitution.

In an embodiment, the cross-linked dextran polymer according to the invention is a cross-linked dextran polymer wherein the degree of substitution (DS₁) of the dextran backbone with the a -W- radical or a -(A-f₂)ₐ-G₁- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the degree of substitution (DS₁) of the dextran backbone with the a -W- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the degree of substitution (DS₁) of the dextran backbone with a -(A-f₂)ₐ-G₁- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the degree of substitution (DS₁) of the dextran backbone with the -W- radical or the -(A-f₂)ₐ-G₁- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the degree of substitution (DS₁) of the dextran backbone with the -W- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the degree of substitution (DS₁) of the dextran backbone with the -(A-f₂)ₐ-G₁- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the degree of substitution (DS₁) of the dextran backbone with the -W- radical or the -(A-f₂)ₐ-G₁- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the degree of substitution (DS₁) of the dextran backbone with the -W- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the degree of substitution (DS₁) of the dextran backbone with the -(A-f₂)ₐ-G₁- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the degree of substitution (DS₁) of the dextran backbone with the -W- radical or the -(A-f₂)ₐ-G₁- radical bearing a L(-)ᵢ crosslinker is comprised in the range from 0.1 to 0.4 (0.1 ≤ DS₁ ≤ 0.4).

In an embodiment, the cross-linked dextran polymer according to the invention is a cross-linked dextran polymer wherein the degree of substitution (DS₁) of the dextran backbone with the -W- radical bearing a L(-)ᵢ crosslinker is comprised in the range from 0.1 to 0.4 (0.1 ≤ DS₁ ≤ 0.4).

In an embodiment, the cross-linked dextran polymer according to the invention is a cross-linked dextran polymer wherein the degree of substitution (DS₁) of the dextran backbone with the -(A-f₂)ₐ-G₁- radical bearing a L(-)ᵢ crosslinker is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 5 to 250 kDa before crosslinking and substitution and the degree of substitution (DS₁) of the dextran backbone with the -W- radical or the -(A-f₂)ₐ-G₁- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 5 to 250 kDa before crosslinking and substitution and the degree of substitution (DS₁) of the dextran backbone with the -W- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 5 to 250 kDa before crosslinking and substitution and the degree of substitution (DS₁) of the dextran backbone with the -(A-f₂)ₐ-G₁- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 20 to 100 kDa before crosslinking and substitution and the degree of substitution (DS₁) of the dextran backbone with the -W- radical or the -(A-f₂)ₐ-G₁- radical bearing a L(-)ᵢ crosslinker groups is comprised in the range from 0.2 to 0.4 (0.2 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 20 to 100 kDa before crosslinking and substitution and the degree of substitution (DS₁) of the dextran backbone with the -W- radical bearing a L(-)ᵢ crosslinker groups is comprised in the range from 0.2 to 0.4 (0.2 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 20 to 100 kDa before crosslinking and substitution and the degree of substitution (DS₁) of the dextran backbone with the -(A-f₂)ₐ-G₁- radical bearing a L(-)ᵢ crosslinker groups is comprised in the range from 0.2 to 0.4 (0.2 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 20 to 100 kDa before crosslinking and substitution and the degree of substitution (DS₁) of the dextran backbone with the -W- radical or the -(A-f₂)ₐ-G₁- radical bearing a L(-)ᵢ crosslinker is comprised in the range from 0.2 to 0.3 (0.2 ≤ DS₁ ≤ 0.3).

In an embodiment, the cross-linked dextran polymer according to the invention is a cross-linked dextran polymer wherein the dextran polymer backbone is a dextran having a weight average molecular weight (Mw) from 20 to 100 kDa before crosslinking and substitution and the degree of substitution (DS₁) of the dextran backbone with the -W- radical bearing a L(-)ᵢ crosslinker is comprised in the range from 0.2 to 0.3 (0.2 ≤ DS₁ ≤ 0.3).

In an embodiment, the cross-linked dextran polymer according to the invention is a cross-linked dextran polymer wherein the dextran polymer backbone is a dextran having a weight average molecular weight (Mw) from 20 to 100 kDa before crosslinking and substitution and the degree of substitution (DS₁) of the dextran backbone with the -(A-f₂)ₐ-G₁- radical bearing a L(-)ᵢ crosslinker is comprised in the range from 0.2 to 0.3 (0.2 ≤ DS₁ ≤ 0.3).

In an embodiment, the cross-linked dextran polymer according to the invention is a cross-linked dextran polymer wherein the dextran polymer backbone is a dextran having a weight average molecular weight (Mw) is from 250 to 1000 kDa before crosslinking and substitution and the degree of substitution (DS₁) of the dextran backbone with the -W- radical or the -(A-f₂)ₐ-G₁- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 250 to 1000 kDa before crosslinking and substitution and the degree of substitution (DS₁) of the dextran backbone with the -W- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 250 to 1000 kDa before crosslinking and substitution and the degree of substitution (DS₁) of the dextran backbone with the -(A-f₂)ₐ-G₁- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -W- radical or the -(A-f₂)ₐ-G₁- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -W- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -(A-f₂)ₐ-G₁- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -W- radical or the -(A-f₂)ₐ-G₁- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -W- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -(A-f₂)ₐ-G₁- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -W- radical or a -(A-f₂)ₐ-G₁- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -W- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with a -(A-f₂)ₐ-G₁- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the degree of substitution (DS₂) of the dextran backbone with the total methylcarboxylates grafted on the dextran is comprised in the range from 0.3 to 2.5 (0.3 ≤ DS₂ ≤ 2.5).

In an embodiment, the cross-linked dextran polymer according to the invention is a cross-linked dextran polymer wherein the degree of substitution (DS₂) of the dextran backbone with the total methylcarboxylates grafted on the dextran is comprised in the range from 0.5 to 2.3 (0.5 ≤ DS₂ ≤ 2.3).

In an embodiment, the cross-linked dextran polymer according to the invention is a cross-linked dextran polymer wherein the degree of substitution (DS₂) of the dextran backbone with the total methylcarboxylates grafted on the dextran is comprised in the range from 1.5 to 2.5 (1.5 ≤ DS₂ ≤ 2.5).

In an embodiment, the cross-linked dextran polymer according to the invention is a cross-linked dextran polymer wherein the degree of substitution (DS₂) of the dextran backbone with the total methylcarboxylates grafted on the dextran is comprised in the range from 1.7 to 2.3 (1.7 ≤ DS₂ ≤ 2.3).

In an embodiment, the cross-linked dextran polymer according to the invention is a cross-linked dextran polymer wherein the degree of substitution (DS₂) of the dextran backbone with the total methylcarboxylates grafted on the dextran is comprised in the range from 1.8 to 2.2 (1.8 ≤ DS₂ ≤ 2.5).

In an embodiment, the cross-linked dextran polymer according to the invention is a cross-linked dextran polymer wherein the degree of substitution (DS₂) of the dextran backbone with the total methylcarboxylates grafted on the dextran is comprised in the range from 0.3 to 1.5 (0.3 ≤ DS₂ ≤ 1.5).

In an embodiment, the cross-linked dextran polymer according to the invention is a cross-linked dextran polymer wherein the degree of substitution (DS₂) of the dextran backbone with the total methylcarboxylates grafted on the dextran is comprised in the range from 0.3 to 0.8 (0.3 ≤ DS₂ ≤ 0.8).

In an embodiment, the cross-linked dextran polymer according to the invention is a cross-linked dextran polymer wherein the degree of substitution (DS₄) of the dextran backbone with a radical of formula I or a radical of formula XII, XIIbis, XIII or XIV, is comprised in the range from 0.5 to 3 (0.5 ≤ DS₄ ≤ 3).

In an embodiment, the cross-linked dextran polymer according to the invention is a cross-linked dextran polymer wherein the degree of substitution (DS₄) of the dextran backbone with a radical of formula I is comprised in the range from 0.5 to 3 (0.5 ≤ DS₄ ≤ 3).

In an embodiment, the cross-linked dextran polymer according to the invention is a cross-linked dextran polymer wherein the degree of substitution (DS₄) of the dextran backbone with a radical of formula XII, XIIbis, XIII or XIV, is comprised in the range from 0.5 to 3 (0.5 ≤ DS₄ ≤ 3).

In an embodiment, the cross-linked dextran polymer according to the invention is a cross-linked dextran polymer wherein the degree of substitution (DS₄) of the dextran backbone with a radical of formula I or a radical of formula XII, XIIbis, XIII or XIV, is comprised in the range from 1 to 2.75 (1 ≤ DS₄ ≤ 2.75).

In an embodiment, the cross-linked dextran polymer according to the invention is a cross-linked dextran polymer wherein the degree of substitution (DS₄) of the dextran backbone with a radical of formula I is comprised in the range from 1 to 2.75 (1 ≤ DS₄ ≤ 2.75).

In an embodiment, the cross-linked dextran polymer according to the invention is a cross-linked dextran polymer wherein the degree of substitution (DS₄) of the dextran backbone with a radical of formula XII, XIIbis, XIII or XIV, is comprised in the range from 1 to 2.75 (1 ≤ DS₄ ≤ 2.75).

In an embodiment, the cross-linked dextran polymer according to the invention is a cross-linked dextran polymer wherein the degree of substitution (DS₄) of the dextran backbone with a radical of formula I or a radical of formula XII, XIIbis, XIII or XIV, is comprised in the range from 1.5 to 2.5 (1.5 ≤ DS₄ ≤ 2.5).

In an embodiment, the cross-linked dextran polymer according to the invention is a cross-linked dextran polymer wherein the degree of substitution (DS₄) of the dextran backbone with a radical of formula I is comprised in the range from 1.5 to 2.5 (1.5 ≤ DS₄ ≤ 2.5).

In an embodiment, the cross-linked dextran polymer according to the invention is a cross-linked dextran polymer wherein the degree of substitution (DS₄) of the dextran backbone with a radical of formula XII, XIIbis, XIII or XIV, is comprised in the range from 1.5 to 2.5 (1.5 ≤ DS₄ ≤ 2.5).

In an embodiment, the cross-linked dextran polymer according to the invention is a cross-linked dextran polymer wherein the degree of substitution (DS₄) of the dextran backbone with a radical of formula I or a radical of formula XII, XIIbis, XIII or XIV, is comprised in the range from 1.75 to 2.25 (1.75 ≤ DS₄ ≤ 2.25).

In an embodiment, the cross-linked dextran polymer according to the invention is a cross-linked dextran polymer wherein the degree of substitution (DS₄) of the dextran backbone with a radical of formula I is comprised in the range from 1.75 to 2.25 (1.75 ≤ DS₄ ≤ 2.25).

In an embodiment, the cross-linked dextran polymer according to the invention is a cross-linked dextran polymer wherein the degree of substitution (DS₄) of the dextran backbone with a radical of formula XII, XIIbis, XIII or XIV, is comprised in the range from 1.75 to 2.25 (1.75 ≤ DS₄ ≤ 2.25).

In an embodiment, the cross-linked dextran polymer according to the invention is a cross-linked dextran polymer wherein the degree of substitution of carboxylate (DS_{c}) of the dextran backbone is comprised in the range from 0.2 to 3 (0.2 ≤ DS_{c} ≤ 3).

In an embodiment, the cross-linked dextran polymer according to the invention is a cross-linked dextran polymer wherein the degree of substitution of carboxylate (DS_{c}) of the dextran backbone is comprised in the range from 0.3 to 2.5 (0.3 ≤ DS_{c} ≤ 2.5).

In an embodiment, the cross-linked dextran polymer according to the invention is a cross-linked dextran polymer wherein the degree of substitution of sulfate, sulfonate, phosphate, phosphonate (DS₃) of the dextran backbone is comprised in the range from 0.2 to 2.5 (0.2 ≤ DS₃ ≤ 2.5).

In an embodiment, the cross-linked dextran polymer according to the invention is a cross-linked dextran polymer wherein the degree of substitution of sulfate, sulfonate, phosphate, phosphonate (DS₃) of the dextran backbone is comprised in the range from 0.3 to 2.0 (0.3 ≤ DS₃ ≤ 2.0).

In an embodiment, the cross-linked dextran polymer according to the invention is a cross-linked dextran polymer having a molar ratio (DC) between the molar concentration of the -W- radical or a -(A-f₂)ₐ-G₁- radical and the molar concentration of the reactive functions of the cross-linker L(-)ᵢ comprised in a range from 0.5 to 1.5 (0.5 ≤ DC ≤ 1.5).

In an embodiment, the cross-linked dextran polymer according to the invention is a cross-linked dextran polymer having a molar ratio (DC) between the molar concentration of the -W- radical and the molar concentration of the reactive functions of the cross-linker L(-)ᵢ comprised in a range from 0.5 to 1.5 (0.5 ≤ DC ≤ 1.5).

In an embodiment, the cross-linked dextran polymer according to the invention is a cross-linked dextran polymer having a molar ratio (DC) between the molar concentration of a -(A-f₂)ₐ-G₁- radical and the molar concentration of the reactive functions of the cross-linker L(-)ᵢ comprised in a range from 0.5 to 1.5 (0.5 ≤ DC ≤ 1.5).

In an embodiment, the cross-linked dextran polymer according to the invention is a cross-linked dextran polymer having a molar ratio between the molar concentration of the -W- radical or a -(A-f₂)ₐ-G₁- radical and the molar concentration of the reactive functions of the cross-linker L(-)ᵢ is comprised in a range from 0.8 to 1.2 (0.8 ≤ DC ≤ 1.2).

In an embodiment, the cross-linked dextran polymer according to the invention is a cross-linked dextran polymer having a molar ratio between the molar concentration of the -W- radical and the molar concentration of the reactive functions of the cross-linker L(-)ᵢ is comprised in a range from 0.8 to 1.2 (0.8 ≤ DC ≤ 1.2).

In an embodiment, the cross-linked dextran polymer according to the invention is a cross-linked dextran polymer having a molar ratio between the molar concentration of a -(A-f₂)ₐ-G₁- radical and the molar concentration of the reactive functions of the cross-linker L(-)ᵢ is comprised in a range from 0.8 to 1.2 (0.8 ≤ DC ≤ 1.2).

In an embodiment, the cross-linked dextran polymer according to the invention is a cross-linked dextran polymer having a molar ratio between the molar concentration of the -W- radical or a -(A-f₂)ₐ-G₁- radical and the molar concentration of the reactive functions of the cross-linker L(-)ᵢ is comprised in a range from 0.9 to 1.1 (0.9 ≤ DC ≤ 1.1).

In an embodiment, the cross-linked dextran polymer according to the invention is a cross-linked dextran polymer having a molar ratio between the molar concentration of the -W- radical and the molar concentration of the reactive functions of the cross-linker L(-)ᵢ is comprised in a range from 0.9 to 1.1 (0.9 ≤ DC ≤ 1.1).

In an embodiment, the cross-linked dextran polymer according to the invention is a cross-linked dextran polymer having a molar ratio between the molar concentration of a -(A-f₂)ₐ-G₁- radical and the molar concentration of the reactive functions of the cross-linker L(-)ᵢ is comprised in a range from 0.9 to 1.1 (0.9 ≤ DC ≤ 1.1).

In an embodiment, the cross-linked dextran polymer according to the invention is a cross-linked dextran polymer having a molar ratio between the molar concentration of the -W- radical or a -(A-f₂)ₐ-G₁- radical and the molar concentration of the reactive functions of the cross-linker L(-)ᵢ is comprised in a range from 0.95 to 1.05 (0.95 ≤ DC ≤ 1.05).

In an embodiment, the cross-linked dextran polymer according to the invention is a cross-linked dextran polymer having a molar ratio between the molar concentration of the -W- radical and the molar concentration of the reactive functions of the cross-linker L(-)ᵢ is comprised in a range from 0.95 to 1.05 (0.95 ≤ DC ≤ 1.05).

In an embodiment, the cross-linked dextran polymer according to the invention is a cross-linked dextran polymer having a molar ratio between the molar concentration of a -(A-f₂)ₐ-G₁- radical and the molar concentration of the reactive functions of the cross-linker L(-)ᵢ is comprised in a range from 0.95 to 1.05 (0.95 ≤ DC ≤ 1.05).

In an embodiment, the cross-linked dextran polymer according to the invention is a cross-linked dextran polymer having a molar ratio between the molar concentration of the -W- radical or a -(A-f₂)ₐ-G₁- radical and the molar concentration of the reactive functions of the cross-linker L(-)ᵢ is 1 (DC = 1).

In an embodiment, the cross-linked dextran polymer according to the invention is a cross-linked dextran polymer having a molar ratio between the molar concentration of the -W- radical and the molar concentration of the reactive functions of the cross-linker L(-)ᵢ is 1 (DC = 1).

In an embodiment, the cross-linked dextran polymer according to the invention is a cross-linked dextran polymer having a molar ratio between the molar concentration of a -(A-f₂)ₐ-G₁- radical and the molar concentration of the reactive functions of the cross-linker L(-)ᵢ is 1 (DC = 1).

In an embodiment, the cross-linked dextran polymer according to the invention is a cross-linked dextran polymer obtained from a reaction between the reactive function of the -W- precursor and the reactive function of the L(-)i precursor where reactive functions are present in the same concentration (DC = 1) and are comprised in a range going from 5 to 25 mM.

In an embodiment they are comprised in the range of 5 to 10 mM.

In an embodiment they are comprised in the range of 10 to 15 mM.

In an embodiment they are comprised in the range of 15 to 20 mM.

In an embodiment they are comprised in the range of 20 to 25 mM.

In an embodiment -W- is chosen among the radicals of formula IV. 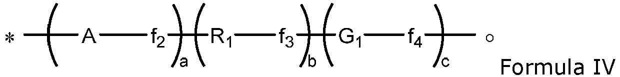 Wherein
- * represents the site of f₁ and ° represents the site of attachment with L.
- a is an integer equal to 0 or 1.
- b is an integer equal to 0 or 1.
- c is an integer equal to 0 or 1.
- In one embodiment a=0, f₁ is an ether function, or a carbamate function.
- In one embodiment a=1,
   ∘ the divalent radical -A- is a linear, -(CH₂)ₙ₁- with m an integer comprised from 1 to 7 (1 ≤ n₁ ≤ 7), branched, or cyclic alkyl derivative. It may also be branched by at least one hydroxyl group, -CH₂-CH(OH)-(CH₂)ₙ₂- with n₂ an integer comprised from 1 to 5 (1 ≤ n₂ ≤ 5); f₁ is an ether function, or a carbamate function, and f₂ is an amide function.
      Or,
   ∘ the divalent radical -A- is a linear polyether (PEG) derivative; f₁ is an ether function, or a carbamate function, and f₂ is an amide function. Or,
   ∘ in another embodiment the divalent radical -A- is a 4-Alkyl-1,4-triazole derivative or a 4-PEG-1,4-triazole derivative; f₁ is an ether function, or a carbamate function, and f₂ is a carbon-nitrogen covalent bond.
      Or,
   ∘ in another embodiment the divalent radical -A- is a 1-Alkyl-1,4-triazole derivative or a 1-PEG-1,4-triazole derivative; f₁ is an ether function, or a carbamate function, and f₂ is a carbon-aromatic carbon covalent bond.
- The divalent radical -R₁- is a linear, branched, or cyclic alkyl derivative, and/or an aromatic derivative, and/or a polyether (PEG) derivative, which can contain heteroatoms such as nitrogen, oxygen, or sulphur.
   ∘ If b=0, then f₁ is an ether function, or a carbamate function.
   ∘ If b=1, then f₁ is an ether function, or a carbamate function, and f₃ is an amide function, or an amine function, or an ether function, or a thioether function, or a carbamate function, or a carbon-nitrogen covalent bond, or a carbon-aromatic carbon covalent bond or a carbon-carbon covalent bond if the crosslinking process is made by a Native Chemical Ligation (NCL).
- The divalent radical -G₁- is a linear, branched, or cyclic alkyl derivative, or an aromatic derivative, which can contain heteroatoms such as: at most 5 nitrogen atoms, at most 10 oxygen atoms, at most 5 sulphur atoms, or at most one phosphorus atom. In a preferred embodiment, -G₁- is a succinimide derivative, or an alkyl sulfone derivative which can contain one heteroatom such as oxygen or sulphur, or an ethyl amide derivative, or a 1,4-triazole derivative, or a multicycle derivative from a Diels-Alder reaction, or an aromatic phosphine derivative created by a Staudinger ligation, or a cysteine derivative coming from a Native Chemical Ligation.
   ∘ If c=0, then f₁, is an ether function, or a carbamate function.
   ∘ If c=1, then f₁, is an ether function, or a carbamate function, and f₄ is an amine function, or an amide function, or a carbamate function, or a thioether function, or an ether function, or a carbon-nitrogen covalent bond, or carbon-aromatic carbon covalent bond, or a carbon-carbon covalent bond if the crosslinking process is made by a Native Chemical Ligation (NCL)

The cross-linked dextran polymer according to the invention is a dextran polymer Dx- bearing anionic groups wherein an at least divalent radical L(-)ᵢ is covalently bound to the dextran polymer backbone with i radicals, chosen among the dextrans of formula X, 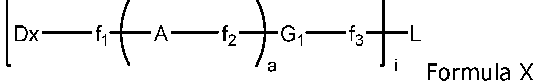 Wherein :
- a is an integer equal to 0 or 1.
- i is an integer comprised from 2 to 8, (2 ≤ i ≤ 8).
- L can be linked to the same [Dx-f₁-(A-f₂)ₐ-G₁-f₃] radicals, or to different ones.
- Dx- is a dextran moiety, which can be substituted by specific anionic groups in salified form, and optionally by alkyl carboxylate derivatives in salified form.
- f₁ is an ether function.
- The divalent radical -A- is a linear, -(CH₂)ₙ₁- with n₁ an integer comprised from 1 to 7 (1 ≤ n₁ ≤ 7), branched, or cyclic alkyl derivative. It may also be branched by at least one hydroxyl group, -CH₂-CH(OH)-(CH₂)ₙ₂- with n₂ an integer comprised from 1 to 5 (1 ≤ n₂ ≤ 5)
- f₂ is an amide function.
- The divalent radical -G₁- is a linear, branched, or cyclic alkyl derivative, or an aromatic derivative, which can contain heteroatoms such as: at most 5 nitrogen atoms, at most 10 oxygen atoms, at most 5 sulphur atoms. In a preferred embodiment, -G₁- is a succinimide derivative, or an alkyl sulfone derivative which can contain one heteroatom such as oxygen or sulphur, or a 1,4-triazole derivative.
- The integer i is the valence of the central-linker L, and the number of, identical or different, [Dx-f₁-(A-f₂)ₐ-G₁-f₃] radicals connected to L.
- f₃ is an amine function, or a thioether function, or an ether function, or an amide function, or a carbamate function, or a carbon-nitrogen covalent bond, or carbon-aromatic carbon covalent bond.
- The central-linker L is a poly(oxazoline) (POx) derivative, which can be linear or branched.

In this embodiment, the cross-linked dextran polymer according to the invention is chosen among the dextran polymers of formula V. 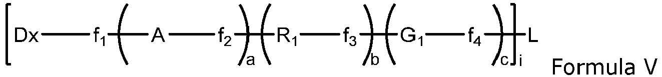 Wherein
- f₁, f₂, f₃, f₄, -A-, -R₁₋, -G₁₋ are defined as above in Formula IV, and
- Dx- is a dextran moiety, which can be substituted by specific anionic groups in salified form, and optionally by alkyl carboxylate derivatives in salified form as previously defined.
- The integer i is the valence of the central-linker L, and the number of, identical or different, [Dx-f₁-(A-f₂)ₐ-(R₁-f₃)_{b}-(G₁-f₄)_{c}] radicals connected to L.
- The central-linker L is a polyether (PEG) derivative, which can be linear or branched.
- In one embodiment, if b=0 and c=1, then the central-linker L can be a poly(oxazoline) (POx) derivative, which can be linear or branched

In this embodiment, the cross-linked dextran polymer according to the invention is chosen among the dextran polymers of formula V. 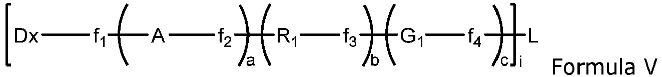 Wherein
- f₁, f₂, f₃, f₄, -A-, -R₁₋, -G₁₋ are defined as above in Formula IV, and
- Dx- is a dextran moiety, which can be substituted by specific anionic groups in salified form, and optionally by alkyl carboxylate derivatives in salified form as previously defined.
- The integer i is the valence of the central-linker L, and the number of, identical or different, [Dx-f₁-(A-f₂)ₐ-(R₁-f₃)_{b}-(G₁-f₄)_{c}] radicals connected to L.
- The central-linker L is a polyether (PEG) derivative, which can be linear or branched.

In one embodiment L is linked to the same [Dx-f₁-(A-f₂)ₐ-(R₁-f₃)_{b}-(G₁-f₄)_{c}] radicals.

In one embodiment L is linked to different [Dx-f₁-(A-f₂)ₐ-(R₁-f₃)_{b}-(G₁-f₄)_{c}] radicals.

If a=0, then:
- In one embodiment, f₁ is an ether function.
- In another embodiment, f₁ is a carbamate function.

If a=1, then:
- In one embodiment, f₁ is an ether function.
- In another embodiment, f₁ is a carbamate function.

If a=0 and b=0, then:
- In one embodiment, f₁ is an ether function.
- In another embodiment, f₁ is a carbamate function.

If a=0 and b=1, then:
- In one embodiment, f₁ is an ether function.
- In another embodiment, f₁ is a carbamate function.

If a=b=c=0, then:
- In one embodiment, f₁ is an ether function.
- In another embodiment, f₁ is a carbamate function.

In one embodiment, respective to formula V, the divalent radical -A- is a linear polyether (PEG) derivative chosen among the PEG of following formula:

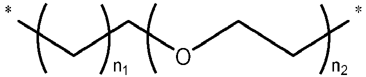

Wherein:
- n₁ is an integer equal to 0 or 1.
- n₂ is an integer comprised from 1 to 7 (1 ≤ n₁ ≤ 7).
- The * represent the sites of f₁ and f₂.
- In a preferred embodiment, the * represent the sites of f₁ and f₂, which are respectively ether and amide functions.

In another embodiment, respective to formula V, the divalent radical -A- is a 1-Alkyl-1,4-triazole derivative, or a 1-PEG-1,4-triazole derivative, chosen among the triazole derivative of following formula:

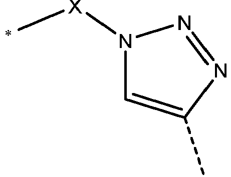

Wherein:
- X is either a linear *-(CH₂)ₙ₁-* with n₁ an integer comprised from 1 to 7 (1 ≤ n₁ ≤ 7), branched, or cyclic alkyl derivative, or X is a PEG derivative.
- The * represents the site of f₁ and the dotted bond represents f₂.

In another embodiment, respective to formula V, the divalent radical -A- is a 4-Alkyl-1,4-triazole derivative, or a 4-PEG-1,4-triazole derivative, chosen among the triazole derivative of following formula:

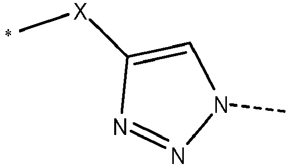

Wherein:
- X is either a linear *-(CH₂)ₙ₁-* with n₁ an integer comprised from 1 to 7 (1 ≤ n₁ ≤ 7), branched, or cyclic alkyl derivative, or X is a PEG derivative.
- The * represents the site of f₁ and the dotted bond represents f₂.

If a= 1, then:
- In one embodiment, f₂ is an amide function.
- In another embodiment, f₂ is a carbon-nitrogen covalent bond.
- In another embodiment, f₂ is a carbon-aromatic carbon covalent bond.

In one embodiment -A-, respective to formula X, is a linear, -(CH₂)ₙ₁- with ni an integer comprised from 1 to 7 (1 ≤ n₁ ≤ 7), branched, or cyclic alkyl derivative. It may also be branched by at least one hydroxyl group, -CH₂-CH(OH)-(CH₂)ₙ₂- with n₂ an integer comprised from 1 to 5 (1 ≤ n₂ ≤ 5).

In one embodiment, respective to formula V, the divalent radical -R₁- is a linear alkyl derivative, according to the following formula: 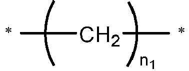 Wherein:
- n₁ is an integer comprised from 1 to 7 (1 ≤ n₁ ≤ 7).
- In one embodiment, if a=1, then the * represent the sites of f₂ and f₃.
- In another embodiment, if a=0, the * represent the sites of f₁ and f₃.

In another embodiment, respective to formula V, the divalent radical -R₁-is a polyether (PEG) derivative, according to the following formula: 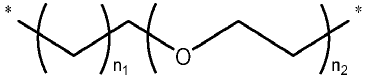 Wherein:
- n₁ is an integer equal to 0 or 1.
- n₂ is an integer comprised from 1 to 7 (1 ≤ n₂ ≤ 7).
- In one embodiment, if a=1, then the * represent the sites of f₂ and f₃.
- In another embodiment, if a=0, the * represent the sites of f₁ and f₃.

In another embodiment, respective to formula V, if a=0, then the divalent radical -R₁- may be a branched alkyl, wherein at least one hydroxyl group is attached to the alkyl chain in β position from f₁, which is an ether function. 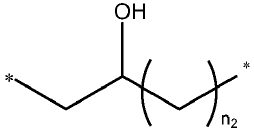 Wherein:
- n₂ is an integer comprised from 1 to 5 (1 ≤ n₂ ≤ 5).
- In another embodiment if a=0, the * represent the sites of f₁ and f₃.

In a preferred embodiment, respective to formula V, the divalent radical - R₁- is a linear alkyl derivative, according to the following formula: 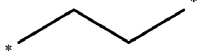 Wherein:
the * represent the sites of f₂, which is an amide function, and f₃, which is an amide function.

In a preferred embodiment, respective to formula V, the divalent radical - R₁- is a PEG derivative, according to the following formula: 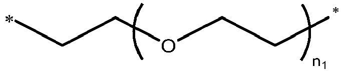 Wherein:
- n₁ is an integer comprised from 1 to 7 (1 ≤ n₁ ≤ 7).
- The * represent the sites of f₂ and f₃, which are two amide functions.

If b=1, then:
- In one embodiment, f₃ is an amine function.
- In another embodiment, f₃ is an ether function.
- In another embodiment, f₃ is a thioether function.
- In another embodiment, f₃ is an amide function.
- In another embodiment, f₃ is a carbamate function.
- In another embodiment, f₃ is a carbon-nitrogen covalent bond.
- In another embodiment, f₃ is a carbon-aromatic carbon covalent bond.
- In another embodiment, if the crosslinking process is made by a Native Chemical Ligation (NCL), then f₃ is a carbon-carbon covalent bond.

The nature of radical G1 depends on the crosslinking process, below are described the different crosslinking process together with the G1 radicals.

In one embodiment, the crosslinking process is realized with a Michael addition with maleimide derivatives, or vinyl sulfone derivatives, or acrylamide derivatives.

In one embodiment, respective to formula X, the integer a=1, the crosslinking process is realised with a Michael addition with maleimide derivatives, or vinyl sulfone derivatives.

In one embodiment, respective to formula V, the integer a=c=1 and L is a POx derivative, the crosslinking process is realised with a Michael addition with maleimide derivatives, or vinyl sulfone derivatives.

In one embodiment, respective to formula V, the divalent radical -G₁- is a succinimide derivative according to the following formula: 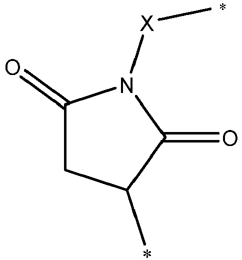 Wherein:
- X is either a linear *-(CH₂)ₙ₁-* with m an integer comprised from 1 to 7 (1 ≤ n₁ ≤ 7), branched, or cyclic alkyl derivative, or X is an aromatic, or X is a PEG derivative.
- In one embodiment if b=1, then the * represent the sites of f₃ and f₄.
- In another embodiment if a=b=0, then the * represent the sites of f₁ and f₄.
- In another embodiment if a=1 and b=0, then the * represent the sites of f₂ and f₄.
- In a preferred embodiment, X is an ethyl group and the * represent the sites of f₂, which is an amide function, and f₄, which is a thioether function.

In one embodiment, respective to formula X, the divalent radical -G₁- is a succinimide derivative according to the following formula: 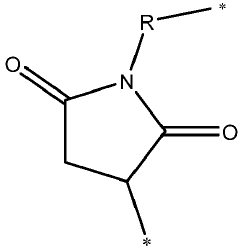 Wherein:
- R is a linear, branched, or cyclic alkyl derivative, or R is an aromatic, or R is a PEG derivative.

The * represent the sites of f₂, which is an amide function, and f₃, which is an amine function, or an ether function, or a thioether function.

In one embodiment, respective to formula V, if b=0, c=1 and L is a POx derivative, the divalent radical -G₁- is a succinimide derivative according to the following formula: 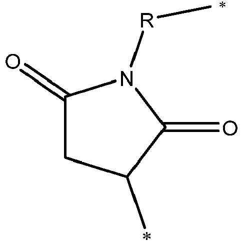 Wherein:
- R is a linear, branched, or cyclic alkyl derivative, or R is an aromatic, or R is a PEG derivative.
- The * represent the sites of f₂, which is an amide function, and f₃, which is an amine function, or an ether function, or a thioether function.

In another embodiment, respective to formula X, the divalent radical -G₁-is a succinimide derivative according to the following formula: 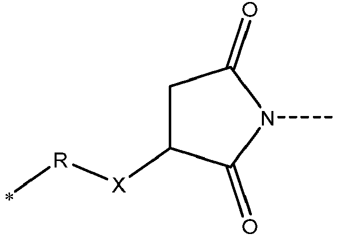 Wherein:
- X is an oxygen atom, or a sulphur atom, or a nitrogen atom.
- R is a linear, branched, or cyclic alkyl derivative, or R is a PEG derivative.
- The * represents the site of f₂, which is an amide function, and the dotted bond represents f₃, which is a carbon-nitrogen covalent bond.

In another embodiment, respective to formula V, if b=0, c=1 and L is a POx derivative, the divalent radical -G₁- is a succinimide derivative according to the following formula: 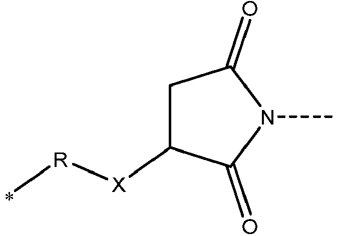 Wherein:
- X is an oxygen atom, or a sulphur atom, or a nitrogen atom.
- R is a linear, branched, or cyclic alkyl derivative, or R is a PEG derivative.
- The * represents the site of f₂, which is an amide function, and the dotted bond represents f₃, which is a carbon-nitrogen covalent bond.

In another embodiment, respective to formula V, the divalent radical -G₁-is a diethyl sulfone derivative according to the following formula: 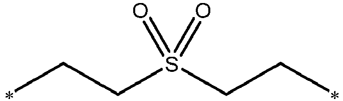 Wherein:
- In one embodiment if b=1, the * represent the sites of f₃ and f₄.
- In another embodiment if a=b=0, the * represent the sites of f₁ and f₄.
- In another embodiment if b=0, the * represent the sites of f₂ and f₄.
- In a preferred embodiment, the * represent the sites of f₃ and f₄, which are thioether functions.

In another embodiment, respective to formula V, the divalent radical -G₁-is a sulfone derivative according to the following formula: 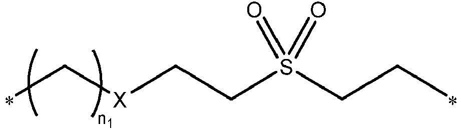 Wherein:
- n₁ is an integer comprised from 0 to 7 (0 ≤ n₁ ≤ 7).
- X is either an oxygen atom, or a sulphur atom, or a CH₂ group.
- In one embodiment if b=1, the * represent the sites of f₃ and f₄.
- In another embodiment if a=b=0, the * represent the sites of f₁ and f₄.
- In another embodiment if a=1 and b=0, the * represent the sites of f₂ and f₄.
- In preferred embodiment a=1, b=0, X is a sulphur atom, n₁=2, f₂ is an amide function, and f₄ is a thioether function.

In another embodiment, respective to formula X, the divalent radical -G₁-is a sulfone derivative according to the following formula: 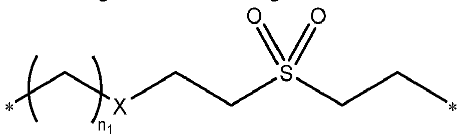 Wherein:
- n₁ is an integer comprised from 0 to 7 (0 ≤ n₁ ≤ 7).
- X is an oxygen atom, or a sulphur atom, or a CH₂ group.
- The * represent the sites of f₂, which is an amide function, and f₃, which is an amine function, or an ether function or a thioether function.

In another embodiment, respective to formula V, if b=0, c=1 and L is a POx derivative, the divalent radical -G₁- is a sulfone derivative according to the following formula: 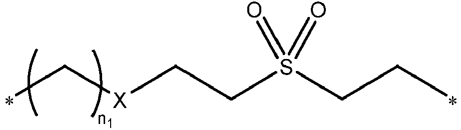 Wherein:
- n₁ is an integer comprised from 0 to 7 (0 ≤ n₁ ≤ 7).
- X is an oxygen atom, or a sulphur atom, or a CH₂ group.
- The * represent the sites of f₂, which is an amide function, and f₃, which is an amine function, or an ether function or a thioether function.

In another embodiment, respective to formula V, the divalent radical -G₁-is an acrylamide derivative according to the following formula: 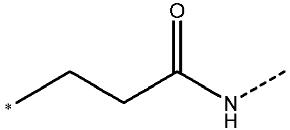 Wherein:
- In one embodiment, the * represents the site of f₃, which is an amine function, or an ether function, or a thioether function, and the dotted bond represents f₄, which is a carbon-nitrogen covalent bond.
- In another embodiment, the dotted bond represents f₃, which is a carbon-nitrogen covalent bond, and the * represents the site of f₄, which is an amine function, or an ether function, or a thioether function.

In one embodiment, the crosslinking process is realised with a 1,3-cycloaddition between alkyne and azide derivatives, known as 1,3-dipolar cycloaddition or Huisgen reaction.

In one embodiment, respective to formula V, the divalent radical -G₁- is a 1,4-triazole derivative according to the following formula: 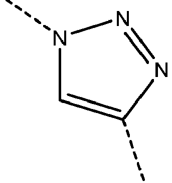 Wherein the two dotted bonds represent f₃ and f₄ which are covalent bonds or chemical functions defined previously and after.

In one embodiment, respective to formula X, the integer a=1 , the divalent radical -G₁- is a 1,4-triazole derivative according to the following formula: 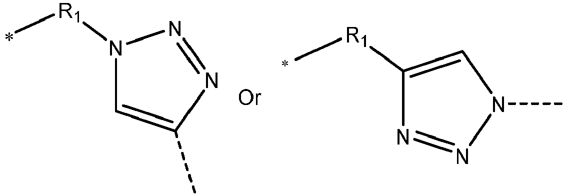 Wherein:
- R₁ is a linear, branched, or cyclic alkyl derivative, which can contain heteroatom such as oxygen, or R₁ is an aromatic derivative, or R₁ is a PEG derivative.
- The * represents the site of f₂, which is an amide function, and the dotted bond represents f₃, which is either a carbon-nitrogen covalent bond or a carbon-aromatic carbon covalent bond.

In one embodiment, respective to formula V, the integer a=c=1, and L is a Pox derivative, the divalent radical -G₁- is a 1,4-triazole derivative according to the following formula: 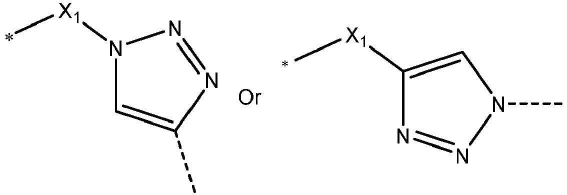 Wherein:
- X₁ is a linear, branched, or cyclic alkyl derivative, which can contain heteroatom such as oxygen, or X₁ is an aromatic derivative, or X₁ is a PEG derivative.
- The * represents the site of f₂, which is an amide function, and the dotted bond represents f₄, which is either a carbon-nitrogen covalent bond or a carbon-aromatic carbon covalent bond.

In another embodiment, respective to formula X, the integer a=0, the divalent radical -G₁- is a 1,4-triazole derivative according to the following formula: 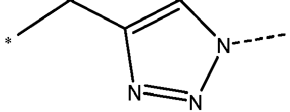 Wherein:
- The * represents the site of f₁, which is an ether function, and the dotted bond represents f₃, which is a carbon-nitrogen covalent bond.

In another embodiment, respective to formula V, the integer a=0, b=0, c=1 and L is a Pox derivative, the divalent radical -G₁- is a 1,4-triazole derivative according to the following formula: 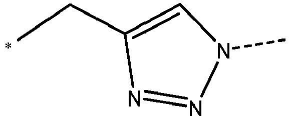 Wherein:
- The * represents the site of f₁, which is an ether function, and the dotted bond represents f₄, which is a carbon-nitrogen covalent bond.

In one embodiment, the crosslinking process is realised with a 1,3-cycloaddition between strain alkyne and azide derivatives, known as Strain-promoted azide-alkyne cycloaddition, or SPAAC.

In one embodiment, respective to formula X, the integer a=1, the crosslinking process is realised with a 1,3-cycloaddition between strain alkyne and azide derivatives, known as Strain-promoted azide-alkyne cycloaddition or SPAAC.

In one embodiment, respective to formula V, the integer a=c=1, b=0 and L is a POx derivative, the crosslinking process is realised with a 1,3-cycloaddition between strain alkyne and azide derivatives, known as Strain-promoted azide-alkyne cycloaddition or SPAAC.

In one embodiment, respective to formula V, the divalent radical -G₁- is a triazole derivative according to the following formula: 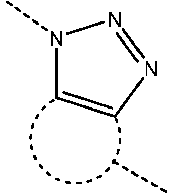
- Wherein:
   - The dotted circle represents a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.
   - The two dotted bonds represent f₃ and f₄, which are covalent bonds or chemical functions defined previously and after.
   - In another embodiment if a=b=0, the two dotted bonds represent f₁ and f₄.
   - In another embodiment if a = 1 and b=0, the two dotted bonds represent f₂ and f₄.
   - In a preferred embodiment, the dotted bonds represent f₂, which is an amide function, and f₄, which is a carbon-nitrogen covalent bond.

In another embodiment, respective to formula V, the divalent radical -G₁-is a triazole derivative according to the following formula: 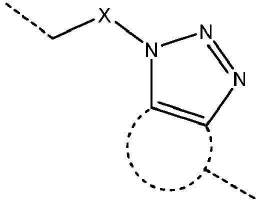 Wherein:
- The dotted circle represents a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.
- X is either a linear *-(CH₂)ₙ₁-* with n₁ an integer comprised from 1 to 7 (1 ≤ n₁ ≤ 7), branched, or cyclic alkyl derivative, or X is a PEG derivative.
- The two dotted bonds represent f₃ and f₄, which are covalent bonds or chemical functions defined previously and after.
- In another embodiment if a=b=0, the * represent the sites of f₁, and the dotted bond represents f₄.
- In another embodiment if a=1 and b=0, the * represent the sites of f₁, and the dotted bond represents f₄.
- In a preferred embodiment, X is a PEG derivative, the dotted bonds represent f₂ and f₄, which are amide functions.

In another embodiment, respective to formula X, the divalent radical -G₁-is a triazole derivative according to the following formula: 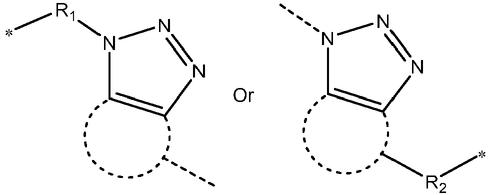 Wherein:
- The dotted circle represents a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.
- R₁ is a linear, branched, or cyclic alkyl derivative, which can contain heteroatom such as oxygen, or R₁ is an aromatic derivative, or R₁ is a PEG derivative.
- R₂ is a linear, branched, or cyclic alkyl derivative, which can contain heteroatom such as oxygen, or R₂ is an aromatic derivative, or R₂ is a PEG derivative.
- The * represents the site of f₂, which is an amide function, and the dotted bond represents f₃, which is a carbon-nitrogen covalent bond, or an amide function, or a carbamate function.

In another embodiment, respective to formula V, b=0 and c=1, the divalent radical -G₁- is a triazole derivative according to the following formula: 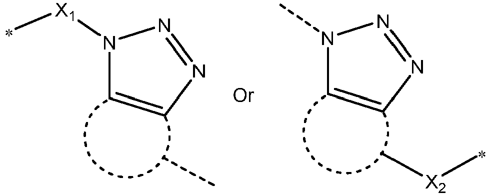 Wherein:
- The dotted circle represents a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.
- X₁ is a linear, branched, or cyclic alkyl derivative, which can contain heteroatom such as oxygen, or Xiis an aromatic derivative, or Xiis a PEG derivative.
- X₂is a linear, branched, or cyclic alkyl derivative, which can contain heteroatom such as oxygen, or X₂is an aromatic derivative, or X₂is a PEG derivative.
- The * represents the site of fz, which is an amide function, and the dotted bond represents f₄, which is a carbon-nitrogen covalent bond, or an amide function, or a carbamate function.

In a preferred embodiment, respective to formula V, the divalent radical - G₁- is a triazole derivative according to the following formula: 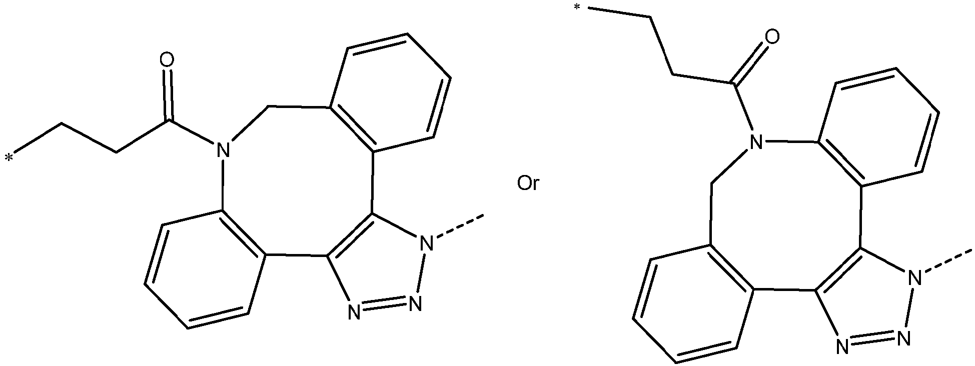 Wherein: the * represents the site of fz, which is an amide function, and the dotted bond represents f₄, which is a carbon-nitrogen bond.

In a preferred embodiment, respective to formula X, the divalent radical - G₁- is a triazole derivative according to the following formula: 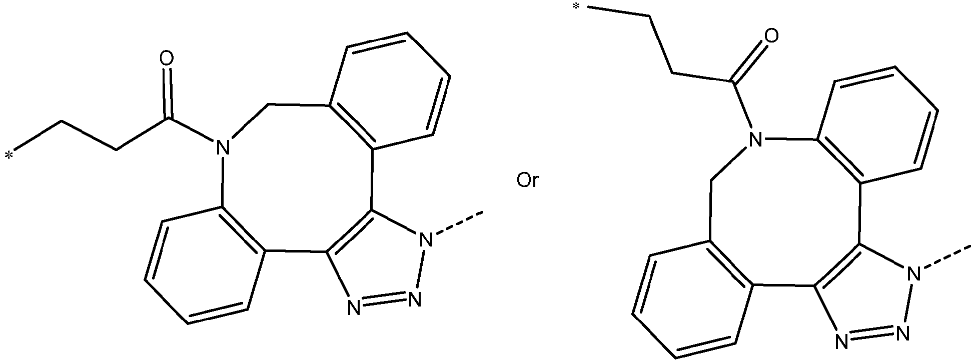 Wherein: The * represents the site of f₂, which is an amide function, and the dotted bond represents f₃, which is a carbon-nitrogen covalent bond.

In another preferred embodiment, respective to formula V, the divalent radical -G₁- is a triazole derivative according to the following formula: 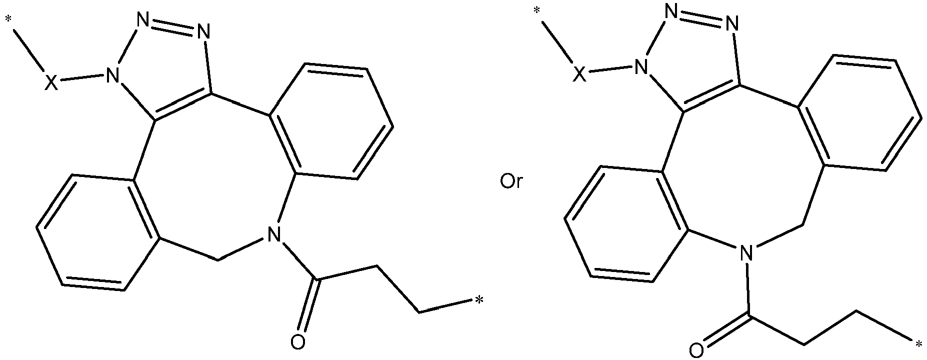 Wherein:
- X is a PEG derivative.
- The * represent the sites of f₂ and f₄, which are amide functions.

In another preferred embodiment, respective to formula X, the divalent radical -G₁- is a triazole derivative according to the following formula: 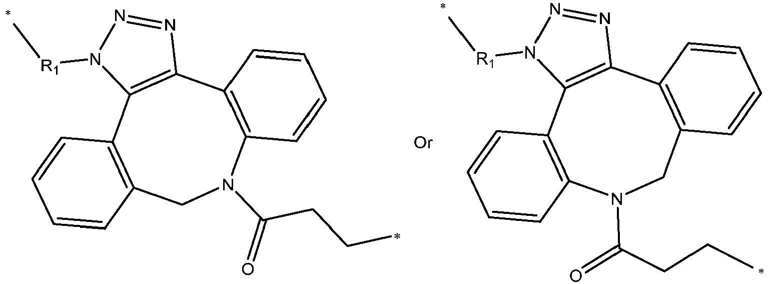 Wherein:
- R₁ is a linear, branched, or cyclic alkyl derivative, which can contain heteroatom such as oxygen, or R₁ is an aromatic derivative, or R₁ is a PEG derivative.
- The * represent the sites of f₂ and f₃, which are amide functions.

In another preferred embodiment, respective to formula V, the divalent radical -G₁- is a triazole derivative according to the following formula: 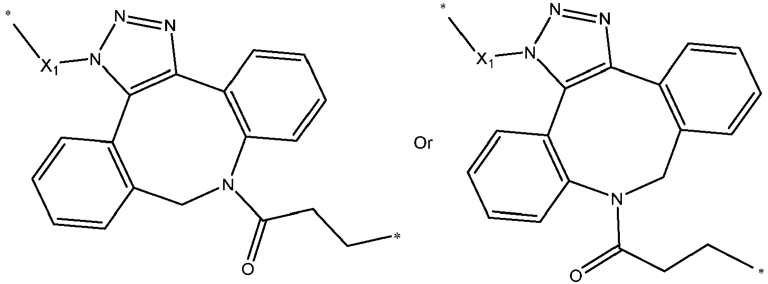 Wherein:
- X₁ is a linear, branched, or cyclic alkyl derivative, which can contain heteroatom such as oxygen, or X₁ is an aromatic derivative, or X₁ is a PEG derivative.
- The * represent the sites of f₂ and f₄, which are amide functions.

In another embodiment, respective to formula V, the divalent radical -G₁-is a triazole derivative according to the following formula: 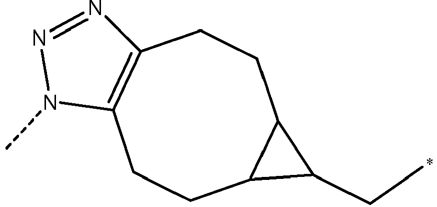 Wherein: the * represents the site of f₃, and the dotted bond represents f₄.

In another embodiment, respective to formula V, the divalent radical -G₁-is a triazole derivative according to the following formula: 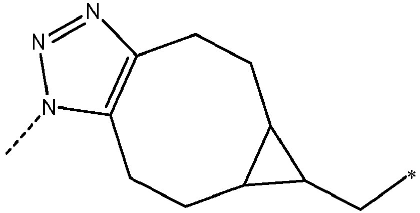 Wherein, the dotted bond represents f₃, and the * represents the site of f₄.

In one embodiment, the crosslinking process is realized with a Diels-Alder cycloaddition between maleimide and furane derivatives.

In one embodiment, respective to formula V, the divalent radical -G₁- is a multiple cycle derivative, composed of one succinimide moiety, according to the following formula: 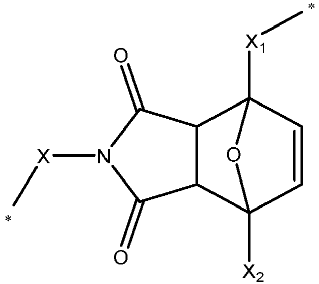 Wherein:
- X is either a linear *-(CH₂)ₙ₁-* with m an integer comprised from 1 to 7 (1 ≤ n₁ ≤ 7), branched, or cyclic alkyl derivative, or X is a PEG derivative.
- X₁ is either a linear *-(CH₂)ₙ₁-* with m an integer comprised from 0 to 7 (0 ≤ n₁ ≤ 7), branched, or cyclic alkyl derivative.
- X₂ is either -H or -Me.
- In one embodiment if a=b=1, the * represent the sites of f₃ and f₄.
- In another embodiment if a=b=0, the * represent the sites of f₁ and f₄.
- In another embodiment if a=1 and b=0, the * represent the sites of f₂ and f₄.

In one embodiment, the crosslinking process is realised with an inverse electron-demand Diels-Alder reaction or IEDDA, between tetrazine and norbornene derivatives.

In one embodiment, respective to formula V, the divalent radical -G₁- is a multiple cycle derivative, composed of one pyridazine moiety, according to the following formula: 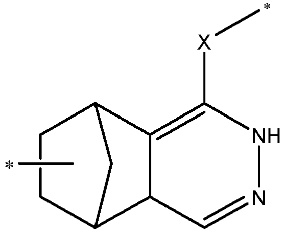 Wherein:
- X is either a linear *-(CH₂)ₙ₁-* with m an integer comprised from 1 to 7 (1 ≤ n₁ ≤ 7), branched, or cyclic alkyl derivative, or X is an aromatic derivative, or X is a PEG derivative.
- In one embodiment if a=b=1, the * represent the sites of f₃ and f₄.
- In another embodiment if a=b=0, the * represent the sites of f₁ and f₄.
- In another embodiment if a=1 and b=0, the * represent the sites of f₂ and f₄.

In another embodiment, respective to formula V, the divalent radical -G₁-is a multiple cycle derivative, composed of one pyridazine moiety, according to the following formula: 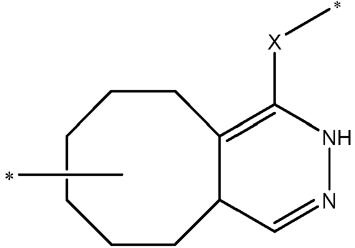 Wherein:
- X is either a linear *-(CH₂)ₙ₁-* with m an integer comprised from 1 to 7 (1 ≤ n₁ ≤ 7), branched, or cyclic alkyl derivative, or X is an aromatic derivative, or X is a PEG derivative.
- In one embodiment if a=b=1, the * represent the sites of f₃ and f₄.
- In another embodiment if a=b=0, the * represent the sites of f₁ and f₄.
- In another embodiment if a=1 and b=0, the * represent the sites of f₂ and f₄.

In one embodiment, the crosslinking process is realised with a Staudinger ligation, between an aromatic phosphine and an azide derivative.

In one embodiment, respective to formula V, the divalent radical -G₁- is an aromatic derivative, according to the following formula: 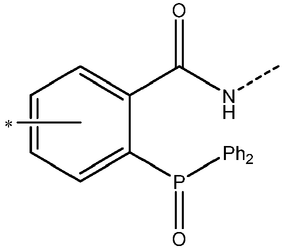 Wherein:
- In one embodiment if a=b=1, the * represent the sites of f₃ and f₄.
- In another embodiment if a=b=0, the * represent the sites of f₁ and f₄.
- In another embodiment if a= 1 and b=0, the * represent the sites of f₂ and f₄.

In one embodiment, respective to formula V, the crosslinking process is realised with a Native Chemical Ligation (NCL), between thioester and N-terminal cysteine derivatives.

In one embodiment, the divalent radical -G₁- can be formalised according to the following formula: 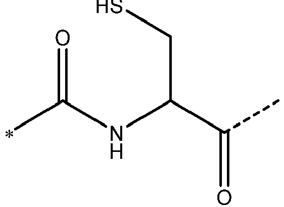 Wherein:
- The dotted line represents a carbon-nitrogen covalent bond.
- In one embodiment if a=b=1, the * and the dotted line represent the sites of f₃ and f₄.
- In another embodiment if a=b=0, the * and the dotted line represent the sites of f₁ and f₄.
- In another embodiment if a=1 and b=0, the * and the dotted line represent the sites of f₂ and f₄.
If c=1, then:
- In one embodiment, f₄ is an amine function.
- In another embodiment, f₄ is an ether function.
- In another embodiment, f₄ is a thioether function.
- In another embodiment, f₄ is an amide function.
- In another embodiment, f₄ is a carbamate function.
- In another embodiment, f₄ is a carbon-nitrogen covalent bond.
- In another embodiment, f₄ is a carbon-aromatic carbon covalent bond.
- In another embodiment, if the crosslinking process is made by a Native Chemical Ligation (NCL), then f₄ is a carbon-carbon covalent bond.

In one embodiment, f₃ is an amine function.

In another embodiment, f₃ is an ether function.

In another embodiment, f₃ is a thioether function.

In another embodiment, f₃ is an amide function.

In another embodiment, f₃ is a carbamate function.

In another embodiment, f₃ is a carbon-nitrogen covalent bond.

In another embodiment, f₃ is a carbon-aromatic carbon covalent bond.

Cross-linked dextran polymer according to the invention is chosen among the dextran polymers of formula V. 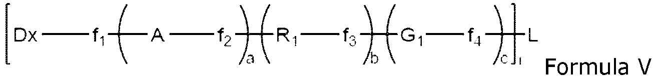 Wherein:
- i is an integer comprised from 2 to 8 (2 ≤ i ≤ 8)
- a = 1,.
- b = 1,
- c = 1,
- Dx is the dextran derivative described in Formula III,
- L is a PEG central linker descirbed in Formula I,
- f₁ is an ether function, or a carbamate function,
- the divalent radical -A- is a linear, -(CH₂)ₙ₁- with m an integer comprised from 1 to 7 (1 ≤ n₁ ≤ 7), branched, or cyclic alkyl derivative,
- f₂ is an amide function,
- the divalent radical -R1- is a linear, -(CH₂)ₙ₁- with m an integer comprised from 1 to 7 (1 ≤ n₁ ≤ 7), branched, or cyclic alkyl derivative,
- f₃ is an amide function,
- the divalent radical -G₁- is a 1,4-triazole derivative
- f₄ is a carbon-nitrogen covalent bond, in particular wherein the nitrogen atom is within the triazole cycle.

In a preferred embodiment, the integer i is egal to 4, i = 4.

According to the above embodiment the triazole derivatives comprising of a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.

According to the two above embodiments the triazole derivative comprises more than 10 and less than 30 carbon atoms, optionally it comprises from 4 to 6 nitrogen atoms.

According to an embodiment the 1,4-triazole derivative is obtained through a copperless reaction.

Cross-linked dextran polymer according to the invention is chosen among the dextran polymers of formula V. 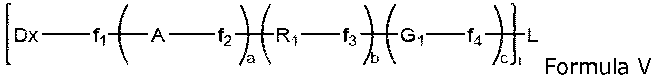 Wherein:
- I is an integer comprised from 2 to 8 (2 ≤ I ≤ 8)
- a = 1,
- b = 0,
- c = 1,
- Dx is the dextran derivative described in Formula III,
- L is a PEG central linker descirbed in Formula I,
- the divalent radical -A- is a linear, -(CH₂)ₙ₁- with m an integer comprised from 1 to 7 (1 ≤ n₁ ≤ 7), branched, or cyclic alkyl derivative,
- f₁ is an ether function, or a carbamate function,
- the divalent radical -G₁- is a 1,4-triazole derivative which is a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine,
said 1,4-triazole derivative bearing a nitrogen, said nitrogen being linked to -CORa-, Ra being an alkyl group comprising from 1 to 4 carbon atoms, via a covalent bond, thus forming an amide function, and Ra being linked to f2 which is an amide function and f₄ is a carbon-nitrogen covalent bond, wherein the nitrogen atom is within the triazole cycle.

In a preferred embodiment, the integer i is egal to 4, i = 4.

According to an embodiment the 1,4-triazole is a multicycle group comprising an acyl group linked to a nitrogen which is within one of the cycles, but not from the triazole cycle, by an amide function.

According to an embodiment the 1,4-triazole comprises a cyclooctyne bearing a nitrogen into the cycloctyne cycle.

According to an embodiment the triazole derivative comprises more than 10 and less than 30 carbon atoms, optionally it comprises from 4 to 6 nitrogen atoms.

In an embodiment the divalent radical -G₁- is a 1,4-triazole derivative as described by the following formula: 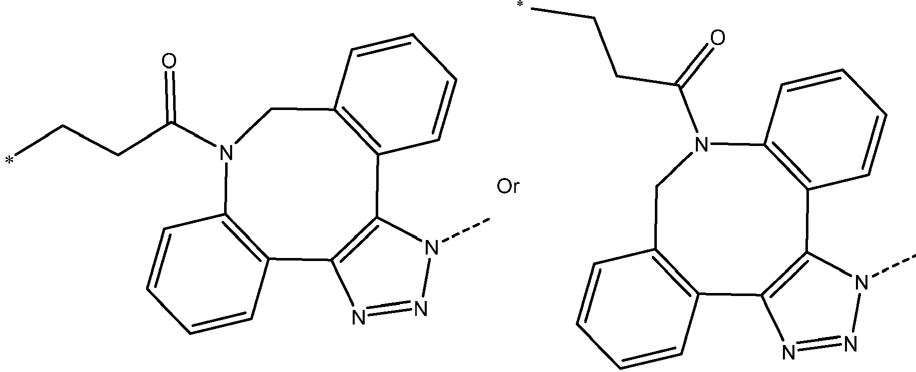 Wherein: the * represents the site of f₂, which is an amide function, and the dotted bond represents f₄, which is a carbon-nitrogen bond.
- f₄ is a carbon-nitrogen covalent bond, wherein the nitrogen atom is within the triazole cycle.

According to an embodiment the triazole 1,4 is obtained through a copperless reaction.

According to an embodiment Dx is the dextran derivative described in Formula III: 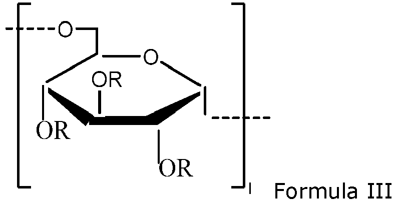 wherein R is chosen among
- -H, a anionic group of formula II, or a -W- radical bearing a L(-)ᵢ crosslinker,
- i is comprised from 20 to 5000 (20 ≤ i ≤ 5000),
- -W- and L(-)ᵢ radicals having the previously defined meanings.

According to an embodiment L is a PEG central linker described in Formula I: 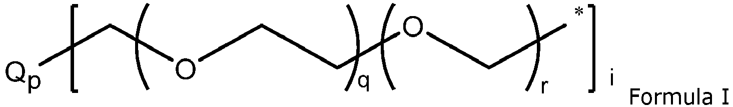 Wherein:
- i is an integer equal to 4,
- p is an integer equal to 1,
- q is an integer comprised from 8 to 1000 (8 ≤ q ≤ 1000)
- r is an integer equal to 0 or 1
- Q is either branched alkyl chain, comprising 2 to 10 carbon atoms,
- the * represents the sites of f4, which is a carbon-nitrogen covalent bond, wherein the nitrogen atom is within the triazole cycle.
the triazole 1,4 is obtained through a copperless reaction.

The invention also concerns the dextran polymers of formula VIII before the crosslinking reaction. 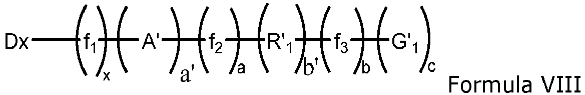 Wherein
- f₁, f₂, f₃, Dx are defined as above if none of a, a', b and b' are equal to 0,
- and
- x equal 0 or 1.
- if a, a', b and b' are equal to 0, x is equal to 0 and Dx is a dextran polymer backbone according to formula III, wherein R is chosen among -H or a anionic group of formula II,
- if one of b' and c is not equal to 0 -A'- is -A- as defined above,
- if b', b and c are equal to 0, a is equal to 0 and A' is the precursor of A before the crosslinking reaction.
- if c is not equal to 0, -R'₁- is -R₁- as defined above and -G'₁- is the precursor of -G₁-.
- if c is equal to 0, b is equal to 0 and R'₁ is the precursor of R₁ before the crosslinking , reaction.

In an embodiment, respective to formula VIII, A' is an alkyl carboxylate derivative or a poly(oxyethylene)carboxylate derivative, or an alkyl azide derivative, or a poly(oxyethylene)azide derivative, or a propargyl derivative, or a poly(oxyethylene)propargyl derivative, or a 2-hydroxyalkyl carboxylate, or a 2-hydroxyalkylamine.

The invention also concerns the dextran polymers of formula XVIII before the crosslinking reaction. 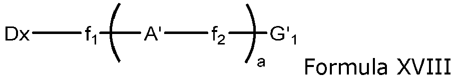 Wherein
- f₁, f₂, Dx are as defined above if a is not equal to 0,
- and
- if a is equal to 0, f₁ is an ether function and G'₁ is a propargylic derivative, if a is not equal to 0 -A' is A as defined above.

In an embodiment, respective to formula XVIII, if a=1, A' is an alkyl carboxylate derivative, or a 2-hydroxyalkyl carboxylate, or a 2-hydroxyalkylamine.

In an embodiment, respective to formula VIII, if a=1, b=0 and L is a POx derivative, A is an alkyl carboxylate derivative, or a 2-hydroxyalkyl carboxylate, or a 2-hydroxyalkylamine.

A' as carboxylate derivatives can be formalized with the following formulas:

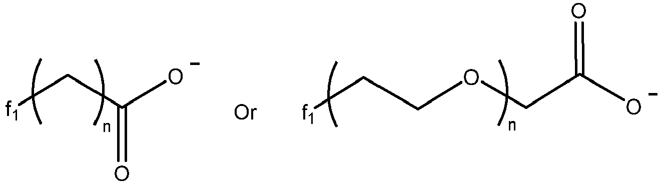

A' as azide derivatives, respective to formula VIII, can be formalized with the following formulas:

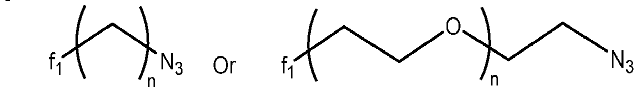

A' as propargyl derivatives, respective to formula VIII, can be formalized with the following formulas:

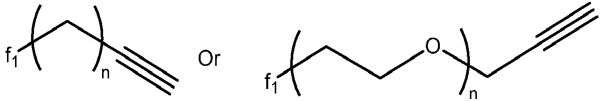

A' as a 2-hydroxyalkyl carboxylate derivative can be formalized with the following formula:

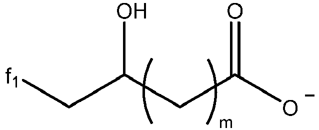

A' as a 2-hydroxyalkylamine derivatives can be formalized with the following formula: 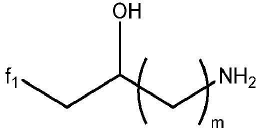 Wherein:
- n is an integer comprised from 1 to 7 (1 ≤ n ≤ 7)
- m is an integer comprised from 1 to 5 (1 ≤ m ≤ 5)
- f₁ is defined as previously.

In an embodiment, respective to formula VIII, if a=1, R'1 is an alkyl radical or a poly(oxyethylene) radical bearing a terminal amine, or a terminal hydroxyl, or a terminal thiol, or a terminal carboxylate, or a terminal azide, or a terminal alkyne.

R'1 can be formalized as follows:

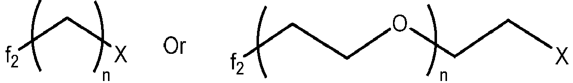

R'1 as carboxylate derivatives can be formalized with the following formulas:

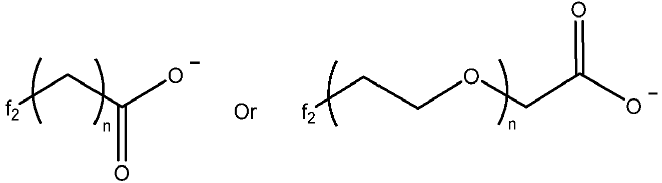

R'₁ as azide derivatives can be formalized with the following formulas: 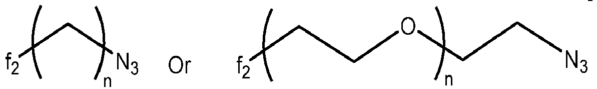 Wherein:
- n is an integer comprised from 1 to 7 (1 ≤ n ≤ 7)
- X= -NH₂, or -OH, or -SH
- f₂ is defined as previously.

Or, in another embodiment, respective to formula VIII, if a=0, then R'1 is a branched alkyl, wherein at least one hydroxyl group is attached to the alkyl chain in β position from f1, and having a terminal hydroxyl, or a terminal thiol, or a terminal azide, or a terminal alkyne.

R'1 can be formalized as follows: 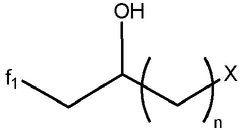 Wherein:
- n is an integer comprised from 1 to 5 (1 ≤ n ≤ 5)
- X= -NH₂, or -OH, or -SH, or -N₃, or -C=CH
- f₁ is defined as previously.

Or, in another R'1 is a propargyl derivatives, and can be formalized with the following formulas: 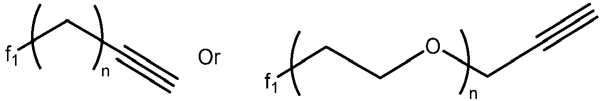 Wherein:
- n is an integer comprised from 1 to 7 (1 ≤ n ≤ 7)
- f₁ is defined as previously.

In an embodiment, respective to formula VIII, -G'1 is a maleimide derivative, or a vinylsulfone derivative, or a strained cyclooctyne derivative, or an azide derivative, or propargyl derivative, or a furane derivative, or an acrylamide derivative, or a norbornene derivative, or a trans-cyclooctene derivative, or a tetrazine derivative, or an aromatic phosphine, or a cysteine, or a thioester, or a thiol derivative, or an amine derivative, or a hydroxyl derivative.

In an embodiment, respective to formula XVIII, if a=1, G'1 is a maleimide derivative, or a vinylsulfone derivative, or a strained cyclooctyne derivative, or an azide derivative, or propargyl derivative, or a thiol derivative, or an amine derivative, or a hydroxyl derivative.

In an embodiment, respective to formula VIII, if a=1, b=0 and L is a POx derivative, G'1 is a maleimide derivative, or a vinylsulfone derivative, or a strained cyclooctyne derivative, or an azide derivative, or propargyl derivative, or a thiol derivative, or an amine derivative, or a hydroxyl derivative.

-G'1 as a thiol, an amine or a hydroxyl derivative, respective to formula VIII, can be formalized as follow:

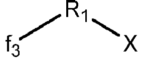

G'1 as a thiol, an amine or a hydroxyl derivative, respective to formula XVIII, can be formalized as follow:

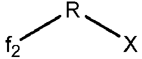

G'₁ as a maleimide can, respective to formula VIII, be formalized as follows:

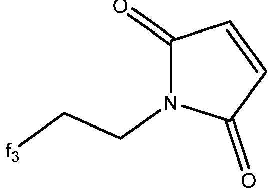

G'₁ as a maleimide, respective to formula XVIII, can be formalized as follows:

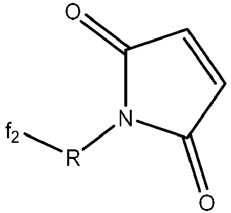

G'₁ as a vinylsulfone, respective to formula VIII, can be formalized as follows:

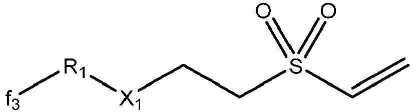

G'₁ as a vinylsulfone, respective to formula XVIII, can be formalized as follows:

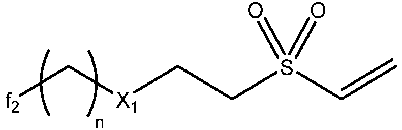

G'₁ as a strained cyclooctyne, respective to formula VIII, can be formalized as follows:

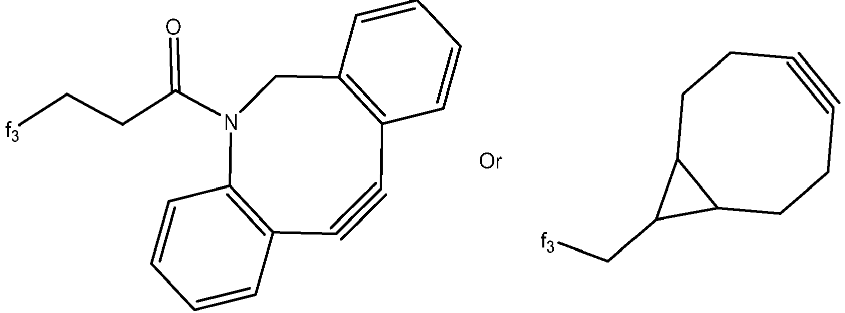

G'₁ as azide derivatives, respective to formula VIII, can be formalized with the following formula:

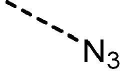

G'₁ as azide derivatives, respective to formula XVIII, can be formalized with the following formula:

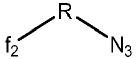

G'₁ as propargyl derivatives, respective to formula VIII, can be formalized with the following formula:

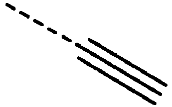

G'₁ as propargyl derivatives, respective to formula XVIII, can be formalized with the following formula: 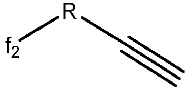 Wherein:
- n is an integer comprised from 0 to 7 (0 ≤ n₁ ≤ 7)
- R is a linear, branched, or cyclic alkyl derivative, or R is a PEG derivative
- X= -NH₂, or -OH, or -SH
- X₁ is an oxygen atom, or a sulphur atom, or a CH₂ group
- f₂ is defined as previously.

In an embodiment, respective to formula XVIII, if a=0, G'₁ is a propargyl derivative.

G'₁ as a propargyl derivative, respective to formula XVIII, can be formalized as follow: 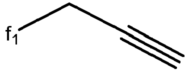 Wherein:
- f₁ is defined as previously.

G'₁ as furane derivatives, respective to formula VIII, can be formalized with the following formula:

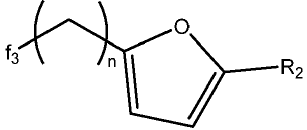

G'₁ as acrylamide derivatives, respective to formula VIII, can be formalized with the following formula:

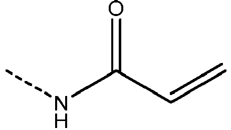

G'₁ as norbornene derivatives, respective to formula VIII, can be formalized with the following formula:

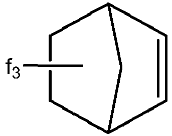

G'₁ as trans-cyclooctene, respective to formula VIII, derivatives can be formalized with the following formula:

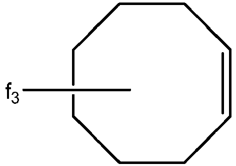

G'₁ as tetrazine derivatives, respective to formula VIII, can be formalized with the following formula:

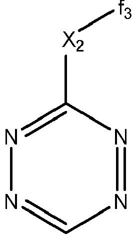

G'₁ as aromatic phosphine derivative, respective to formula VIII, can be formalized with the following formula:

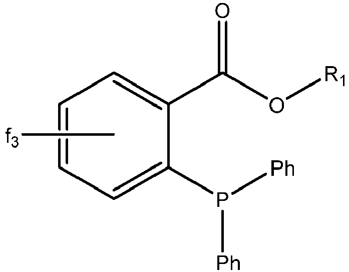

G'₁ as cysteine derivatives, respective to formula VIII, can be formalized with the following formula:

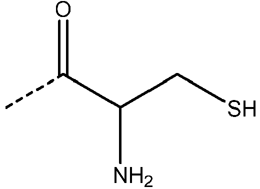

G'₁ as thioester derivatives, respective to formula VIII, can be formalized with the following formula: 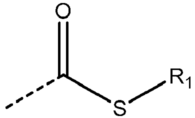 Wherein:
- X= -NH₂, or -OH, or -SH
- X₁= -O-, or -S-
- n is an integer equal to 0 or 1
- R₁= Alkyl
- X₂= -CH₂-, or aromatic
- R₂= -H, or -CH₃
- f₃ is defined as previously.
- The dotted bonds represent f₃, which is a carbon-nitrogen covalent bond, or a carbon-carbon covalent bond.

The invention also concerns a hydrogel comprising the cross-linked dextran polymer according to the invention.

In an embodiment the hydrogel is transparent.

By "transparent" is meant that in conditions disclosed in Example C21 of application PCT/EP2022/050466 for visual inspection an observer considered the sample transparent compared to the standard 2 (6 NTU) and/or the UV absorbance of the hydrogel as measured in Example C21 of application PCT/EP2022/050466 is lower than 0.06 (Absorbance Units).

In an embodiment the hydrogel is visually transparent and has a UV absorbance < 0.06 (Abs. Units).

In an embodiment the hydrogel according to the invention is characterized in that Tan δ is lower than 1.

In the present specification, Tan δ is the ratio of the loss modulus G*"* to the storage modulus (also called elastic modulus) G*'* to (Tan δ = G"/G).

In an embodiment the hydrogel according to the invention is characterized in that Tan δ is less than or equal to 0.5.

In an embodiment the hydrogel according to the invention is characterized in that Tan δ is less than or equal to 0.1.

In an embodiment the hydrogel according to the invention is characterized in that Tan δ is less than or equal to 0.05.

In an embodiment the hydrogel according to the invention is characterized in that Tan δ is less than or equal to 0.01.

In an embodiment the hydrogel according to the invention is characterized in that after swelling in water the cross-linked dextran polymer concentration is comprised from 0.01 to 0.2 g/g.

In an embodiment the hydrogel according to the invention is characterized in that after swelling in water the cross-linked dextran polymer concentration is comprised from 0.03 to 0.1 g/g.

In an embodiment the hydrogel according to the invention is characterized in that after swelling in water the cross-linked dextran polymer concentration is comprised from 0.05 to 0.1 g/g.

In an embodiment, the hydrogel is translucid.

In another embodiment the hydrogel is transparent.

In an embodiment, the hydrogel has a Young modulus comprised between 1 to 200 kPa.

In an embodiment, the hydrogel has a Young modulus comprised between 5 to 200 kPa.

In an embodiment, the hydrogel has a Young modulus comprised between 20 to 200 kPa.

In an embodiment, the hydrogel has a Young modulus comprised between 30 to 200 kPa.

In an embodiment, the hydrogel has a Young modulus comprised between 50 to 200 kPa.

In an embodiment, the hydrogel has a Young modulus comprised between 30 to 180 kPa.

In an embodiment, the hydrogel has a Young modulus comprised between 50 to 150 kPa.

In an embodiment, the hydrogel has a Young modulus comprised between 5 to 100 kPa.

In an embodiment, the hydrogel has a Young modulus comprised between 10 to 90 kPa.

In an embodiment, the hydrogel has a Young modulus comprised between 10 to 75 kPa.

In an embodiment, the hydrogel has a G*'* comprised from 0.5 to 70 kPa.

In an embodiment the hydrogel has a compression deformation at break of more than or equal to 10 %.

In an embodiment the hydrogel has a compression deformation at break of more than or equal to 15 %.

In an embodiment the hydrogel has a compression deformation at break of more than or equal to 20 %.

In an embodiment the hydrogel has a compression deformation at break of more than or equal to 25 %.

In an embodiment the hydrogel has a compression deformation at break of more than or equal to 30 %.

In an embodiment the hydrogel has a compression deformation at break of more than or equal to 35 %.

In an embodiment the hydrogel has a compression deformation at break of more than or equal to 40 %.

In an embodiment the hydrogel has a compression deformation at break of more than or equal to 45 %.

In an embodiment the hydrogel has a compression deformation at break of more than or equal to 50 %.

In an embodiment the hydrogel has a compression deformation at break of more than or equal to 55 %.

In an embodiment the hydrogel has a compression deformation at break of more than or equal to 60 %.

In an embodiment the hydrogel has a traction deformation at break of more than or equal to 10 %.

In an embodiment the hydrogel has a traction deformation at break of more than or equal to 15 %.

In an embodiment the hydrogel has a traction deformation at break of more than or equal to 20 %.

In an embodiment the hydrogel has a traction deformation at break of more than or equal to 25 %.

In an embodiment the hydrogel has a traction deformation at break of more than or equal to 30 %.

In an embodiment the hydrogel has a traction deformation at break of more than or equal to 35 %.

In an embodiment the hydrogel has a traction deformation at break of more than or equal to 40 %.

In an embodiment the hydrogel has a swelling ratio of more than 0.7.

In an embodiment the hydrogel has a swelling ratio of more than 0.8.

In an embodiment the hydrogel has a swelling ratio of more than 0.9.

In an embodiment the hydrogel has a swelling ratio of more than 1.

In an embodiment the hydrogel has a swelling ratio of more than 1.1.

In an embodiment the hydrogel has a swelling ratio of more than or equal to 1.2.

In an embodiment the hydrogel has a swelling ratio of more than or equal to 1.3.

In an embodiment the hydrogel has a swelling ratio of more than or equal to 1.4.

In an embodiment the hydrogel has a swelling ratio of more than or equal to 1.5.

In an embodiment the hydrogel has a swelling ratio of more than or equal to 1.6.

In an embodiment the hydrogel has a swelling ratio of less than or equal to 5.

In an embodiment the hydrogel has a swelling ratio of less than or equal to 4.

In an embodiment the hydrogel has a swelling ratio of less than or equal to 3.

In an embodiment the hydrogel has a swelling ratio of less than or equal to 2.8.

In an embodiment the hydrogel has a swelling ratio of less than or equal to 2.5.

In an embodiment the hydrogel has a swelling ratio of less than or equal to 2.3.

In an embodiment the hydrogel has a water content of at least 80 wt%.

In an embodiment the hydrogel has a water content of at least 85 wt%.

In an embodiment the hydrogel has a water content of at least 90 wt%.

In an embodiment the hydrogel has a water content of at least 97 wt%.

In an embodiment the hydrogel has a water content of at least 96 wt%.

In an embodiment the hydrogel has a water content of at least 95 wt%.

In an embodiment the hydrogel has a water content of at least 94 wt%.

In an embodiment the hydrogel has a water content of at least 93 wt%.

In an embodiment the hydrogel has a water content of at most 99 wt%.

In an embodiment the hydrogel has a water content of at most 98 wt%.

In an embodiment the hydrogel according to the invention is characterized in that it further comprises biological cells.

In an embodiment the cells are cells from human or animal origin.

In an embodiment the cells are cell lines.

In an embodiment the cells are stem-cells derived.

In an embodiment the stem cells are chosen from embryonic-stem cells, from induced-pluripotent-stem-cells or from mesenchymal-stem-cells.

In an embodiment the cells are primary cells.

In an embodiment the cells are proteins, hormones or peptide secreting cells.

In an embodiment the cells are chosen from:
- insulin secreting cells for diabetes treatment
- Factor VIII or Factor IX secreting cells for hemophilia treatment and
- β-glucocerebrosidase secreting cells for Gaucher disease.

In an embodiment the cells are chosen from the group consisting of cells secreting :
- growth hormone
   ∘ human growth hormone (hGH),
   ∘ recombinant human growth hormone (rhGH)
- growth hormone-releasing hormone (GHRH),
- thyroid stimulating hormone (TSH),
- thyrotropin-release hormone (TRH),
- adrenocorticotropic hormone (ACTH), and
- parathyroid hormone (PTH).

In an embodiment the cells are chosen from the group consisting of cells secreting :
- glucagon
- Insulin and
- GLP-1.

In an embodiment the cells are chosen from the group consisting of cells secreting insulin.

In an embodiment the cells are chosen from the group consisting of cells secreting insulin.

In an embodiment the cells are chosen from the group consisting of cells secreting growth factor
- vascular endothelial growth factor (VEGF),
- nerve growth factor (NGF),
- platelet-derived growth factor (PDGF),
- fibroblast growth factor (FGF),
- epidermal growth factor (EGF),
- transforming growth factor (TGF), and
- insulin-like growth factor-I and -II (IGF-I and IGF-II).

In an embodiment the cells are chosen from the group consisting of cells secreting blood clotting factor or a blood coagulation factor
- Factor I (e.g., fibrinogen),
- Factor II (e.g., prothrombin),
- Factor III (e.g., tissue factor),
- Factor V (e.g., proaccelerin, labile factor),
- Factor VI,
- Factor VII (e g., stable factor, proconvertin),
- Factor VIII (e.g., antihemophilic factor A),
- Factor VIIIC,
- Factor IX (e.g, antihemophilic factor B),
- Factor X (e g, Stuart-Prower factor),
- Factor XI (e.g, plasma thromboplastin antecedent),
- Factor XII (e.g., Hagerman factor),
- Factor XIII (e.g, fibrin-stabilizing factor),
- von Willebrand factor (vWF),
- prekallikrein,
- heparin cofactor II,
- high molecular weight kininogen (e.g., Fitzgerald factor),
- antithrombin III, and
- fibronectin

In an embodiment the cells are chosen from the group consisting of cells secreting immunoglobulin chain (heavy or light chain) or fragment thereof, comprising at least one immunoglobulin variable domain sequence, and optionally comprising an immunoglobulin Fc region.

In an embodiment the cells are chosen from the group consisting of cells secreting cytokine or a cytokine receptor, or a chimeric protein including cytokines or their receptors.

In an embodiment the cells are chosen from the group consisting of cells secreting erythropoietin

In an embodiment the cells are chosen from the group consisting of cells secreting interleukins (ILs):
- IL-1, IL-2 to IL-10

In an embodiment the cells are chosen from the group consisting of cells secreting replacement enzyme
- alpha-galactosidase A (GLA),
- alpha-L-iduronidase (IDVA),
- arylsulfatase B (ARSB),
- glucocerebrosidase, and
- N-sulfoglucosamine sulfohydrolase (SGSH).

In an embodiment the hydrogel according to the invention is characterized in that insulin secreting cells are chosen into the group of pancreatic cells.

In an embodiment the hydrogel according to the invention is characterized in that insulin secreting cells are Langherans islets.

In an embodiment the hydrogel according to the invention is characterized in that the biological cells are pseudoislets.

The invention also concerns the use of a cross-linked dextran copolymer according to the invention into the form of a hydrogel to prepare a cells composition.

In an embodiment the cells are chosen amongst one or multiple type of cells, either isolated or aggregated, which may secrete active principles.

In an embodiment, the cross-linked dextran polymer according to the invention is a dextran polymer bearing anionic groups of formula II wherein the at least divalent radical L(-)ᵢ is covalently bound to the dextran polymer backbone with i -W- ₋radicals, wherein i is 2, 4 or 8.

In an embodiment, the cross-linked dextran polymer according to the invention is a dextran polymer bearing anionic groups of formula II wherein the at least divalent radical L(-)ᵢ is covalently bound to the dextran polymer backbone with i radicals, wherein i is 2, 4 or 8.

In an embodiment, the cross-linked dextran polymer according to the invention is a dextran polymer bearing anionic groups of formula II wherein the at least divalent radical L(-)ᵢ is covalently bound to the dextran polymer backbone with i -W- ₋radicals, wherein, i is 2.

In an embodiment, the cross-linked dextran polymer according to the invention is a dextran polymer bearing anionic groups of formula II wherein the at least divalent radical L(-)ᵢ is covalently bound to the dextran polymer backbone with i radicals, wherein, i is 2.

In an embodiment, the cross-linked dextran polymer according to the invention is a dextran polymer bearing anionic groups of formula II wherein the at least divalent radical L(-)ᵢ is covalently bound to the dextran polymer backbone with i -W- ₋radicals, wherein, i is 4.

In an embodiment, the cross-linked dextran polymer according to the invention is a dextran polymer bearing anionic groups of formula II wherein the at least divalent radical L(-)ᵢ is covalently bound to the dextran polymer backbone with i radicals, wherein, i is 4.

In an embodiment, the cross-linked dextran polymer according to the invention is a dextran polymer bearing anionic groups of formula II wherein the at least divalent radical L(-)ᵢ is covalently bound to the dextran polymer backbone with i -W- ₋radicals, wherein, i is 8.

In an embodiment, the cross-linked dextran polymer according to the invention is a dextran polymer bearing anionic groups of formula II wherein the at least divalent radical L(-)ᵢ is covalently bound to the dextran polymer backbone with i radicals, wherein, i is 8.

The crosslinking step is a gelation step that leads to the formation of a hydrogel according to the invention.

The hydrogel formation kinetic is function of the temperature and could be modulated by the reactant's concentrations, pH and temperature.

In an embodiment the time to obtain a hydrogel according to the invention is comprised from 1 minute to 6 hours.

In an embodiment the crosslinking step is carried out for 1 hour.

In an embodiment the temperature of the crosslinking step is comprised from 4°C to room temperature (20-25°C) and could vary between the step of mixing and the step of gelation and moulding.

In an embodiment the mixing is performed at 4°C and the gelation is carried out at room temperature (20-25°C) for 1 hour.

In an embodiment the mixing is performed at room temperature (20-25°C).

In an embodiment the mixing is performed at 4°C or room temperature (20-25°C) and the gelation is carried out at room temperature (20-25°C) for 1 hour.

In an embodiment, the gelation is carried out at 37 °C.

In an embodiment, after crosslinking or gelation, the hydrogel is swelled in a buffer solution, the pH of the buffer solution is comprised from 5 to 8, preferably from 6 to 8 and more preferably from 6.8 to 7.5.

In an embodiment, the buffer solution is a PBS solution at pH 7.4.

In an embodiment, the buffer solution is a Tris solution at pH 7.4.

In an embodiment, the buffer solution is a Tris solution at pH 8.

In an embodiment the swelling allows the hydrogel mass being increased by 1, 2, 3 or 4 compared to its initial mass.

The invention also concerns a process to synthetize a cross-linked dextran polymer according to the invention, into the form of a hydrogel, comprising the steps of:
a) preparation of a sterile solution comprising a dextran bearing anionic groups of formula II and at least two precursors of -W-
b) preparation of a sterile solution of a precursor of L(-)ᵢ
c) addition of the sterile solution obtained from step b) to the solution obtained from step a),
d) the addition being directly done in a mould or the solutions are introduced into a mould after being mixed,
e) crosslinking and gelation, for example at room temperature (20-25°C) or at 37°C,
f) unmoulding and swelling to obtain an hydrogel.

The invention also concerns a process to synthetize a cross-linked dextran polymer according to the invention, into the form of a hydrogel, comprising the steps of:
a) preparation of a sterile solution comprising a dextran bearing anionic groups of formula II and at least two precursors of -(A-f₂)ₐ-G₁-, -(A'-f₂)ₐ-G'1-,
b) preparation of a sterile solution of a precursor of L(-)ᵢ
c) addition of the sterile solution obtained from step b) to the solution obtained from step a),
d) the addition being directly done in a mould or the solutions are introduced into a mould after being mixed,
e) crosslinking and gelation, for example at room temperature (20-25°C) or at 37°C,
f) unmoulding and swelling to obtain an hydrogel.

In an embodiment steps c) and d) are done simultaneously.

In an embodiment, the swelling is done into a PBS solution at pH 7,4.

Dextrans bearing anionic groups of formula II are prepared by grafting or substitution on the hydroxyl groups borne by the dextrans. In an embodiment the dextrans bearing anionic groups of formula II are prepared by grafting or substituting the carboxymethyl groups borne by the carboxymethyl dextrans.

In an embodiment of the process according to the invention an active pharmaceutical ingredient (API) is entrapped into the hydrogel.

The invention also concerns a therapeutic use of the hydrogel according to the invention as a therapeutic implant to administer the API to a mammal.

The invention also concerns a process to prepare a hydrogel comprising biological cells comprising the steps of:
a) preparation of a sterile solution comprising a dextran a dextran bearing anionic groups of formula II and at least two precursors of -W-,
b) preparation of a sterile solution of a precursor of L(-)ᵢ,
c) preparation of a suspension of biological cells,
d) mixing the biological cells suspension obtained from step c) and the solution obtained from the step b) or a),
e) addition of the sterile solution obtained from step a) or b) which is not used in step d) to the solution obtained from step d),
f) the addition of step e) being either done directly in a mould or the solutions are introduced into a mould after being mixed,
g) crosslinking and gelation reaction at room temperature (20-25°C),
h) unmoulding and swelling to obtain an hydrogel comprising biological cells.

The invention also concerns a process to prepare a hydrogel comprising biological cells comprising the steps of:
a) preparation of a sterile solution comprising a dextran a dextran bearing anionic groups of formula II and at least two precursors of -(A-f₂)ₐ-G₁-, - (A'-f₂)ₐ-G'₁-,
b) preparation of a sterile solution of a precursor of L(-)ᵢ,
c) preparation of a suspension of biological cells,
d) mixing the biological cells suspension obtained from step c) and the solution obtained from the step b) or a),
e) addition of the sterile solution obtained from step a) or b) which is not used in step d) to the solution obtained from step d),
f) the addition of step e) being either done directly in a mould or the solutions are introduced into a mould after being mixed,
g) crosslinking and gelation reaction at room temperature (20-25°C),
h) unmoulding and swelling to obtain an hydrogel comprising biological cells.

The invention also concerns a process to prepare a hydrogel comprising biological cells comprising the steps of:
a) preparation of a sterile solution comprising a dextran bearing anionic groups of formula II and at least two precursors of -W-
b) preparation of a sterile solution of a precursor of L(-)ᵢ chosen among a thiol polyethylene glycol, a mercaptopoly(oxyethylenes), a pentaerythritol poly(oxyethylene) azide or a pentaerythritol poly(dibenzocyclooctyne) polyoxyethylene
c) preparation of a sterile solution of hyaluronate de sodium,
d) preparation of a sterile suspension of biological cells,
e) mixing the sodium hyaluronate solution obtained from step c) with precursor solution from the step b)
f) mixing the biological cells suspension obtained from step d) and the solution obtained from the step e) or from step a)
g) mixing the solution obtained from step f) and the solutions obtained from step e),
h) addition of the sterile solution obtained from step a) or e) which is not used in step g) to the solution obtained from step f),
i) the addition of step g) being either done directly in a mould or the solutions are introduced into a mould after being mixed,
j) crosslinking and gelation reaction at room temperature (20-25°C),
k) unmoulding and swelling to obtain an hydrogel comprising biological cells.

In an embodiment, the mould is a Ring Net.

The ring is an outer ring and is composed of a superior part and an inferior part which are sandwiching the net. The two parts of the ring and the net are glued together.

In an embodiment, crosslinking and gelation reaction are performed at room temperature (20-25°C).

In an embodiment, crosslinking and gelation reaction are performed at a controlled temperature comprise between 15°C and 37°C.

In an embodiment, the swelling is done into a PBS solution at pH 7.4.

In an embodiment the hydrogel according to the invention is characterized in that it further comprises biological cells.

In an embodiment the cells are cells from human or animal origin.

In an embodiment the cells are cell lines.

In an embodiment the cells are stem-cells derived.

In an embodiment the stem cells are chosen from embryonic-stem cells, from induced-pluripotent-stem-cells or from mesenchymal-stem-cells.

In an embodiment the cells are primary cells.

In an embodiment the cells are protein(s), hormone(s) or peptide(s) secreting cells.

In an embodiment the cells are chosen from
- insulin secreting cells for diabetes treatment
- Factor VIII or Factor IX secreting cells for hemophilia treatment and
- β-glucocerebrosidase secreting cells for Gaucher disease.

In an embodiment the hydrogel according to the invention is characterized in that insulin secreting cells are chosen into the group of pancreatic cells.

In an embodiment the hydrogel according to the invention is characterized in that insulin secreting cells are Langherans islets.

In an embodiment the hydrogel according to the invention is characterized in that the biological cells are pseudoislets.

The invention also concerns a therapeutic use of the hydrogel according to the invention for treating a disorder or disease in a mammal wherein the disorder or disease is due to lack or malfunction of endocrine function of pancreas organ.

The invention also concerns a hydrogel for use as a medicament.

The invention also concerns a hydrogel for use in the treatment of a disease such as diabetes.

The invention also concerns an implantable device comprising at least a hydrogel according to the invention and obtained according to the process of the invention.

The invention also concerns an implant consisting of the hydrogel according to the invention.

The invention also concerns an implant comprising the hydrogel according to the invention.

The invention also concerns an implant comprising the hydrogel according to the invention and cells or islets.

The invention also concerns a kit comprising:
- A solution of dextran polymer of formula VIII before the crosslinking reaction: 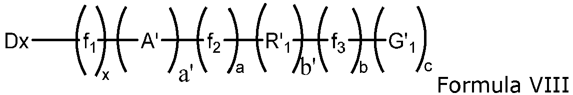 Wherein
   - f₁, f₂, f₃, f₄, Dx are defined as in formula IV if none of a, a', b and b' are not equal to 0,
   - and
   - x equal 0 or 1
   - if a, a', b and b' are equal to 0, x is equal to 0 and Dx is a dextran polymer backbone is according to formula III, wherein R is chosen among -H or a anionic group of formula II,
   - if one of b' and c is not equal to 0 -A' is A as defined above,
   - if b', b and c are equal to 0, a is equal to 0 and A' is the precursor of A before the crosslinking reaction.
   - if c is not equal to 0, R'₁ is R₁ as defined above and G'₁ is the precursor of G₁.
   - if c is equal to 0, b is equal to 0 and R'₁ is the precursor of R₁ before the crosslinking , reaction.
- a solution of a thiol polyethylene glycol, a mercaptopoly(oxyethylenes), a pentaerythritol poly(oxyethylene) azide or a pentaerythritol poly(dibenzocyclooctyne) polyoxyethylene.
- biological cells.

The invention also concerns a kit comprising:
- A solution of dextran polymer of formula VIII before the crosslinking reaction: 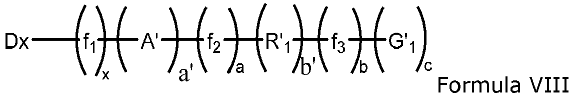 Wherein
   - f₁, f₂, f₃, f₄, Dx are defined as in formula IV if none of a, a', b and b' are not equal to 0,
   - and
   - x equal 0 or 1
   - if a, a', b and b' are equal to 0, x is equal to 0 and Dx is a dextran polymer backbone is according to formula III, wherein R is chosen among -H or a anionic group of formula II,
   - if one of b' and c is not equal to 0 -A' is A as defined above,
   - if b', b and c are equal to 0, a is equal to 0 and A' is the precursor of A before the crosslinking reaction.
   - if c is not equal to 0, R'₁ is R₁ as defined above and G'₁ is the precursor of G₁.
   - if c is equal to 0, b is equal to 0 and R'₁ is the precursor of R₁ before the crosslinking , reaction.
- a solution of a thiol polyethylene glycol, a mercaptopoly(oxyethylenes), a pentaerythritol poly(oxyethylene) azide or a pentaerythritol poly(dibenzocyclooctyne) polyoxyethylene.
- biological cells.
- a solution of non crosslinked sodium hyaluronate.

The invention also concerns a kit comprising:
- A solution of dextran polymer of formula VIII before the crosslinking reaction: 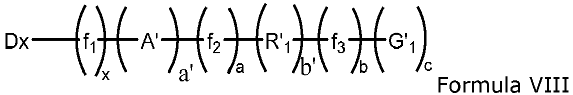 Wherein
   - f₁, f₂, f₃, f₄, Dx are defined as in formula IV if none of a, a', b and b' are not equal to 0,
   - and
   - x equal 0 or 1
   - if a, a', b and b' are equal to 0, x is equal to 0 and Dx is a dextran polymer backbone is according to formula III, wherein R is chosen among -H or a anionic group of formula II,
   - if one of b' and c is not equal to 0 -A' is A as defined above,
   - if b', b and c are equal to 0, a is equal to 0 and A' is the precursor of A before the crosslinking reaction.
   - if c is not equal to 0, R'₁ is R₁ as defined above and G'₁ is the precursor of G₁.
   - if c is equal to 0, b is equal to 0 and R'₁ is the precursor of R₁ before the crosslinking , reaction.
- a solution of a thiol polyethylene glycol, a mercaptopoly(oxyethylenes), a pentaerythritol poly(oxyethylene) azide or a pentaerythritol poly(dibenzocyclooctyne) polyoxyethylene.
- biological cells.

In an embodiment, at least 50 % of the surface the hydrogel is directly in contact with the medium in which it is implanted.

In an embodiment, at least 75 % of the surface the hydrogel is directly in contact with the medium in which it is implanted.

In an embodiment, at least 90 % of the surface the hydrogel is directly in contact with the medium in which it is implanted.

In an embodiment, at least 95 % of the surface the hydrogel is directly in contact with the medium in which it is implanted.

In an embodiment, 99 % of the surface the hydrogel is directly in contact with the medium in which it is implanted.

In an embodiment, at least 50 % of the surface the hydrogel is directly in contact with the exterior of the device or implant.

By « directly in contact with the exterior » means there is no separation between the hydrogel and the exterior, for example no wall made of a non-hydrogel material between the hydrogel and the exterior of the device or implant.

In an embodiment, at least 75 % of the surface the hydrogel is directly in contact with the exterior of the device or implant.

In an embodiment, at least 90 % of the surface the hydrogel is directly in contact with the exterior of the device or implant.

In an embodiment, at least 95 % of the surface the hydrogel is directly in contact with the exterior of the device or implant.

In an embodiment, at least 99 % of the surface the hydrogel is directly in contact with the exterior of the device or implant.

In an embodiment, 100 % of the surface the hydrogel is directly in contact with the exterior of the device or implant.

The cells or the API are entrapped into the maze of cross-linked dextran hydrogel.

In this specification the word "entrapped" is equivalent to "encapsulated" or "encapsulation".

The hydrogel matrix allows passage of small molecules e.g. nutrients and API, API being entrapped into the hydrogel or secreted by the entrapped cells.

Typically, API are hormone and peptide drugs chosen amongst PTH protein, insulin and coagulation factors.

In an embodiment, the mesh size of the matrix is immunoisolant and stops the T lymphocytes in order to preserve the cells.

In an embodiment, this mesh size is less than 1 µm.

In another embodiment it is less that 100 nanometers, preferably less than 10 nanometers, and more preferably around 5 nanometers.

In an embodiment the invention concerns an implant comprising a ring, a net, the hydrogel according to the invention and cells.

In an embodiment the implant has a thickness of less than 3 000 µm.

In an embodiment the implant has a thickness of less than 2 000 µm.

In an embodiment the implant has a thickness of less than 1 000 µm.

In an embodiment the implant has a thickness of less than 900 µm.

In an embodiment the implant has a thickness of more than 300 µm.

In an embodiment the implant has a thickness of more than 400 µm.

In an embodiment the implant has a thickness of more than 500 µm.

In an embodiment the implant has total surface of between 10 cm² to 200 cm².

In an embodiment the implant has total surface of between 15 cm² to 100 cm².

In an embodiment the implant comprises from 0.5 to 20 ml of hydrogel.

In an embodiment the implant comprises from 0.75 to 10 ml of hydrogel.

In an embodiment the implant comprises from 0.8 to 5 ml of hydrogel.

In an embodiment the ring and the impland is a parallelepiped rectangle, in particular with round corner.

The ring and net structure allow the hydrogel to be easily manipulated, a good resistance to manipulation, including for implantation. This is also true even with a hydrogel having a larger mesh size (for example with a lower DS of -W- and lower concentrations of reactive groups during crosslinking).

The ring and net structure allow the hydrogel to be easily manipulated, a good resistance to manipulation, including for implantation. This is also true even with a hydrogel having a larger mesh size (for example with a lower DS -(A-f₂)ₐ-G₁- and lower concentrations of reactive groups during crosslinking).

In an embodiment the ring has an internal diameter of 10 to 100 mm.

In an embodiment the ring has an internal diameter of 15 to 50 mm.

In an embodiment the ring has a diameter of 0.5 to 5 mm

In an embodiment the ring has a diameter of 0.5 to 10 mm.

In an embodiment the ring has a diameter of 0.5 to 5 mm.

In an embodiment the ring has a total (lower plus upper part and glue) thickness of 100 to 3000 µm.

In an embodiment the ring has a total (lower plus upper part and glue) thickness of 150 to 2000 µm.

In an embodiment the ring has a total thickness of 200 to 5000 µm.

In an embodiment the ring has a total thickness of 500 to 3000 µm.

In an embodiment the ring has a rectangular, square or round section.

In an embodiment the ring material is a bioinert material.

In an embodiment, the ring material is a biocompatible elastomer.

In an embodiment the ring material is chosen from the group consisting of silicone, in particular PDMS, polyurethanes, polyether, polyether polyester copolymers and polypropylene oxide.

In an embodiment the ring material is silicone.

In an embodiment the ring material is PDMS.

In an embodiment the net is non-biodegradable.

In an embodiment the net is biocompatible.

In an embodiment the net is non absorbable.

In an embodiment the net is a surgical mesh.

In an embodiment the filament material of the net material is chosen among the group consisting of Polypropylene, Polyethylene, polyester, in particular PET, PTFE, PVDF (polyvinylidene fluoride) and ePVDF (extended PVDF).

In a particular embodiment the filament material of the net material is chosen among the group consisting of PTFE PVDF and ePVDF.

In an embodiment the filament material of the net is chosen among the group consisting of Polypropylene, polyester, in particular PET, PTFE and PVDF (polyvinylidene fluoride).

In an embodiment the filament material of the net is chosen among the group consisting of Polypropylene and polyester, in particular PET.

In an embodiment the filament material of the net is chosen among the group consisting of Polypropylene.

In an embodiment the filament material of the net is chosen among the group consisting of is polyester, in particular PET.

In an embodiment the net has a thickness ranging from 50 to 500 µm.

In an embodiment the m net has a thickness ranging from 100 to 300 µm.

In an embodiment, the filament diameter is ranging from 0.08 to 0.2 mm.

In an embodiment the pore size of the net is ranging from 0.4 to 4 mm.

In an embodiment the pore size of the net is ranging from 0.6 to 2 mm.

In an embodiment the net is having pores with side sizes ranging from 0.4 to 4 mm.

In an embodiment the net is having pores with side sizes ranging from 0.6 to 3 mm.

In an embodiment fabric of the net is chosen from the group consisting of knitted fabric, warp knitted fabric, woven fabric, non-woven fabric.

In an embodiment fabric of the net is chosen from the group consisting of warp knitted fabric, in particular multi-filament.

In an embodiment the net is treated in order to increase the hydrophilicity.

In an embodiment the net is treated with a base, in particular on polyester, more particularly on PET.

In an embodiment this treatment is functionalisation of the surface from reactive function, such as -OH, -COOH, and reactive molecules or polymer.

The grafted polymer could thus expose reactive functions for a further reaction with the hydrogel or a precursor of hydrogel, such as a thiol function.

In an embodiment this treatment is done by adsorption of synthetic polymers, such as poloxamers or polyvinyl pyrrolidone (PVP) or of natural polymers, such as collagen, or of surfactants after a chemical or physical treatment.

In an embodiment the net remains below the exterior end of the ring.

In an embodiment the net is not in contact with the exterior of the implant comprising a ring, a net and the hydrogel.

In an embodiment the glue is biocompatible.

In an embodiment the glue is a biocompatible silicone glue, such as Silbione MED ADH 4200 supplied by Elkem.

In an embodiment the glue remains below the exterior end of the ring.

For example, a warp knit Polyester surgical net fabric type PETKM3002 (1x0.9mm pore size) supplied by SurgicalNet^{™} was treated in NaOH 1M for 5 hours at 70°C and rinsed with deionized water and ethanol 96%. The treatment led to an increased hydrophilicity of the net fabric leading to an improved wetting with aqueous solutions.

For example, a ring net construct may be obtained following the process below:
- Biocompatible PDMS sheets supplied by Grace Biolabs or Interstate Speciality Product is cut in a form of a square incorporating a circular empty disc using a stainless-steel punch,
- A part of the treated polyester surgical net described is introduced in between two square PDMS pieces: The two PDMS pieces and the surgical net are glued together with biocompatible silicone glue (Silbione MED ADH 4200 supplied by Elkem). The circular empty discs are aligned, and the surgical net is kept tense during gluing,
- then, the square construct is cut with a strainless steel punch to obtain the final object constituted by two PDMS rings sandwiching a surgical net and glued together, and
- The pieces are washed with a solution of poloxamer F127 at 1% and rinsed with water before steam sterilization.

In an embodiment, the implant can be obtained by the following process:
- Hydrogel compositions are incorporated in the Ring Mesh constructs. The concentrated polymer solutions are mixed with a pipette and a controlled volume of the mixture is introduced in a ring mesh construct adhering to a glass slide,
- Crosslinking leading to gelation is carried out. Then the Ring Mesh + Hydrogel composition is introduced in a Tris 150 mM /NaCl 30 mM / Cystein 10 mM solution at pH 8 or in PBS at pH 7.4,
- The hydrogel was rinsed with PBS solution without cysteine and further immersed in the PBS solution overnight at 37°C. The hydrogel piece was then stored in PBS solution at 4°C until being used.

Hydrogel/Ring Mesh implants can then be easily manipulated with tweezers and are foldable for the need of surgical implantation. Moreover, it is possible to fix the ring with sutures.

The hydrogel volume can be adjusted with the internal diameter and the thickness or the ring mesh construct. For the same ring mesh construct the hydrogel volume can be adjusted to control the convexity / concavity of the hydrogel above the ring level.

In an embodiment, the hydrogel comprises a first layer of the hydrogel wich does not comprise cells or islets and a second layer of the hydrogel which comprises cells or islets.

Such a structure may be obtained with a process as disclosed in this application, but with two steps of adding hydrogel precursors first step is adding hydrogel precursors without cells or islets as a first layer, and the second step is adding the hydrogel precursor with cells or islets while the gelation of the first step is not finished, in particular at a time corresponding to 5 to 25 % of the gelation time of the first hydrogel, as a second layer.

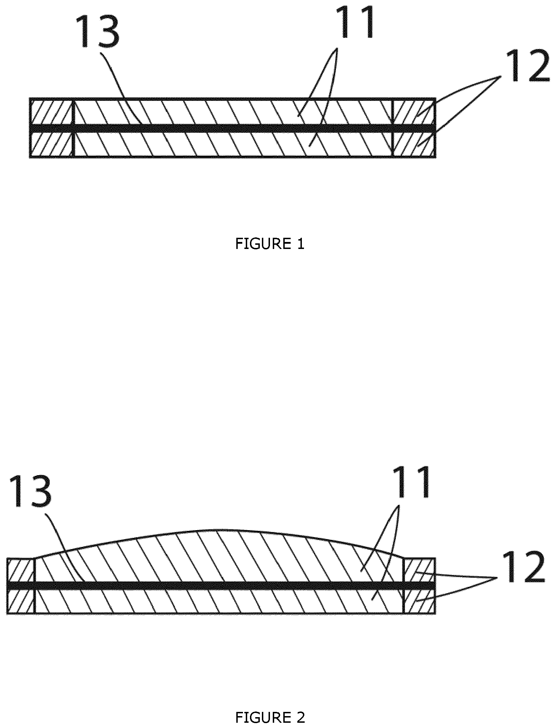

Figure 1 represents an implant (1) comprising a hydrogel (11) comprising cells or islets (not represented), a ring (12) and a net (13). Upper part of the ring, lower part of the ring and net can be glued together (not represented).
Figure 2 represents an implant (1) comprising a hydrogel (11) comprising cells or islets (not represented), a ring (12) and a net (13), where the hydrogel is concave.
Figure 3 is a top view of an implant (1) comprising a hydrogel (11) comprising cells or islets (not represented), a ring (12) and a net (13).
Figure 4 represents an implant (1) comprising a hydrogel without cells (20) sandwiching a hydrogel comprising cells (21). The upper part of 20 being optional.

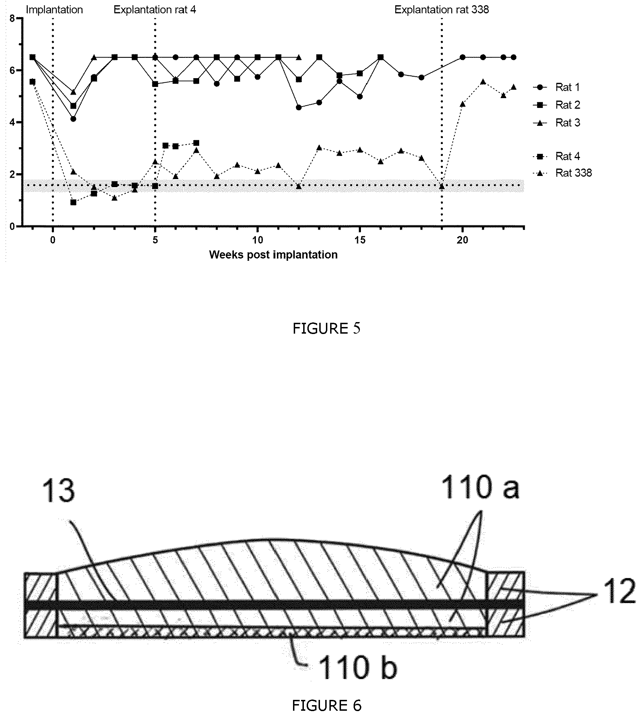

Figure 5 represents non-fasted glycemia (in g/L) measured in control rats (rats 1, 2 and 3) and rats implanted with C16B-18 (rat 4) or with C16**B**-3A / C16**B**-3B (rat 338). Rat 4 received 6 200 IEQ and rat 338 received 4000 IEQ. Vertical dotted lines represent respectively implantation, explantation of rat 4, and explantation of rat 338. Grey area with dotted line represents blood glucose values (minimum, maximum, average) measured in animals before induction of diabetes with streptozotocin. Glycemic values > 6 g/L were not measurable on the glucose meter (measured as High by the device) and were randomly set at 6.5 g/L.
Figure 6 represents an implant comprising a first layer of hydrogel (110a) comprising cells or islets (not represented), a second layer of hydrogel without cells (110b), a ring (12) and a net (13), where the hydrogel is concave. The first layer and the second layer adhering together.
Figure 7 is a picture of an example of superimposed filament layers by extrusion based 3D printing in air. The final porosity is about 2mm (scale bar represents 2mm).

The invention concerns all the embodiments hereafter listed.
cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a polyethylene glycol chain, or this at least radical being a at least divalent linear, branched or cyclic alkyl radical comprising at least a poly(oxazoline) (POx)chain.

The problem is solved by the provision of a new cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a polyethylene glycol chain.

The problem is solved by the provision of a new cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a poly(oxazoline) (POx)chain.

In an embodiment, the cross-linked dextran polymer according to the invention is not a dextran polymer bearing carboxylate groups wherein the at least divalent radical L(-)ᵢ is covalently bound to the dextran polymer backbone with i W radicals, wherein,
- L(-)ᵢ is a linear or branched polyether bearing at its ends, heteroatoms such as oxygen, nitrogen or sulfur,
- i is the valence of L and the number of -(R₁)ₘG₁- ₋ radicals and is an integer comprised from 2 to 8 (2 ≤ i ≤ 8),
- m is an integer equal to 0 or 1,
- W is a -(R₁)ₘG₁- radical, wherein
   - -R₁- is a linear or branched alkyl divalent radical comprising from 1 to 6 carbon atoms and optionally heteroatoms such as oxygen, nitrogen or sulfur,
   - -G₁- is a linear or branched or cyclic alkyl divalent radical comprising from 1 to 6 carbon atoms and may comprise heteroatoms such as oxygen, nitrogen or sulfur.

In an embodiment, the dextran polymer is not a dextran polymer wherein the at least divalent radical L(-)ᵢ covalently bound to the dextran polymer backbone with i W radicals results is a radical issued from a linear or branched mercaptopolyethyleneglycol comprising at least 2 sulfur atoms and comprising at most 8 arms which
- Mn is comprised from 1000 to 25 000 g/mol (1000 ≤ Mn ≤ 25 000 g/mol) or
- polymerisation degree (DP) is comprised from 15 to 600 (15 ≤ DP ≤ 600).

In an embodiment, the dextran polymer is not a dextran polymer wherein the at least divalent radical L(-)ᵢ covalently bound to the dextran polymer backbone with i W radicals is not a radical issued from a linear or branched mercaptopolyethyleneglycol comprising at least 2 sulfur atoms and comprising at most 8 arms, which
- number-average molecular weight (Mn) is comprised from 500 to 40 000 g/mol (500 ≤ Mn ≤ 40 000 g/mol) or
- polymerisation degree (DP) is comprised from 8 to 1000 (8 ≤ DP ≤ 1000).

The cross-linked dextran polymer according to the invention is a dextran polymer Dx bearing anionic groups wherein the at least divalent radical L(-)ᵢ is covalently bound to the dextran polymer backbone with i -W- radicals, wherein,
- L(-)ᵢ is a linear or branched polyether, or L(-)ᵢ is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether or poly(oxazoline) derivatives and that does not comprise two or more alpha aminoacid residues in particular linked by peptidic bond(s).

The cross-linked dextran polymer according to the invention is a dextran polymer Dx bearing anionic groups wherein the at least divalent radical L(-)ᵢ is covalently bound to the dextran polymer backbone with i -W- radicals, wherein,
- L(-)ᵢ is a linear or branched polyether
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

The cross-linked dextran polymer according to the invention is a dextran polymer Dx- bearing anionic groups wherein the at least divalent radical L is covalently bound to the dextran polymer backbone with i W radicals, wherein,
- L is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

The cross-linked dextran polymer according to the invention is a dextran polymer Dx bearing anionic groups wherein the at least divalent radical L(-)ᵢ is covalently bound to the dextran polymer backbone with i -W- radicals, wherein,
- L(-)ᵢ is a linear or branched polyether, or L(-)ᵢ is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether or poly(oxazoline) derivatives and that does not comprise two or more alpha aminoacid residues in particular linked by peptidic bond(s).

The cross-linked dextran polymer according to the invention is a dextran polymer Dx bearing anionic groups wherein the at least divalent radical L(-)ᵢ is covalently bound to the dextran polymer backbone with i -W- radicals, wherein,
- L(-)ᵢ is a linear or branched polyether
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

The cross-linked dextran polymer according to the invention is a dextran polymer Dx- bearing anionic groups wherein the at least divalent radical L is covalently bound to the dextran polymer backbone with i W radicals, wherein,
- L is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In one embodiment, the crosslinked dextran hydrogel according to the invention is a dextran polymer wherein the central-linker L(-)ᵢ is a linear, or a branched polyethylene glycol (PEG) radical.

In an embodiment, the central-linker L(-)ᵢ is a PEG chosen among the PEG of formula I : 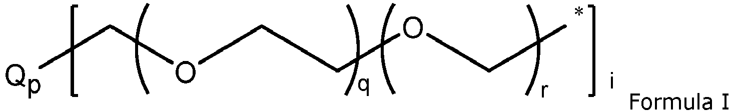 Wherein:
- i is an integer comprised from 2 to 8 (2 ≤ i ≤ 8)
- p is an integer equal to 0 or 1, and if i=2 then p=0
- q is an integer comprised from 8 to 1000 (8 ≤ q ≤ 1000)
- r is an integer equal to 0 or 1
- Q is either a carbon atom, or a linear, branched, or cyclic alkyl chain, or an aromatic, comprising 2 to 10 carbon atoms and may comprise heteroatoms such as nitrogen, oxygen, or sulphur
- the * represents the sites of f₄, which is an amine function, or an ether, or a thioether function, or an amide function, or a carbamate function or a carbon-nitrogen covalent bond, or a carbon-aromatic carbon covalent bond, or a carbon-carbon covalent bond if the crosslinking process is made by a Native Chemical Ligation (NCL).

In one embodiment, the crosslinked dextran hydrogel according to the invention is a dextran polymer wherein the central-linker L is a linear, or a branched POx radical.

In one embodiment, the POx central linker is a 2-arm POx, chosen among the linkers of formula XII. 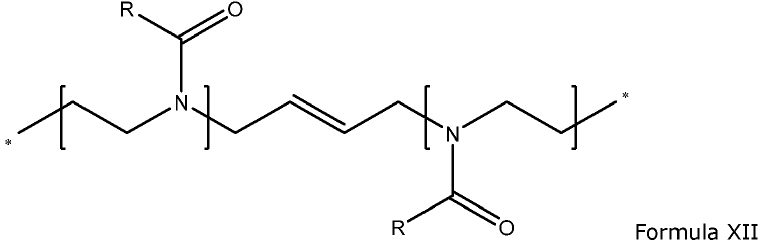 Wherein:
- The radical -R is a linear, -(CH₂)ₙ₁-CH₃ with m an integer comprised from 0 to 4 (0 ≤ n₁ ≤ 4), branched, or cyclic alkyl derivative.
- The * represents the sites of f₃, which is an amine function, or an ether function, or a thioether function, or an amide function, or a carbamate function, or a carbon-nitrogen covalent bond, or a carbon-aromatic carbon covalent bond.

In one embodiment, the POx central linker is a 2-arm POx, chosen among the linkers of formula XIIbis. 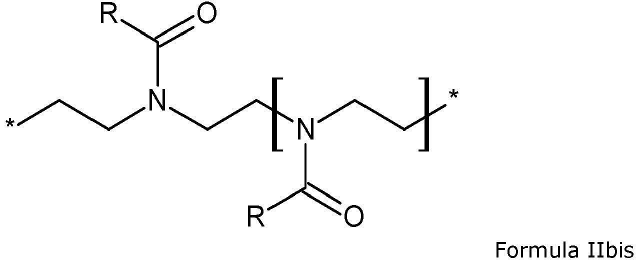 Wherein:
- The radical -R is a linear, -(CH₂)ₙ₁-CH₃ with m an integer comprised from 0 to 4 (0 ≤ n₁ ≤ 4), branched, or cyclic alkyl derivative.
- The * represents the sites of f₃, which is an amine function, or an ether function, or a thioether function, or an amide function, or a carbamate function, or a carbon-nitrogen covalent bond, or a carbon-aromatic carbon covalent bond.

In one embodiment, the POx central linker is a 4-arm POx, chosen among the linkers of formula XIII. 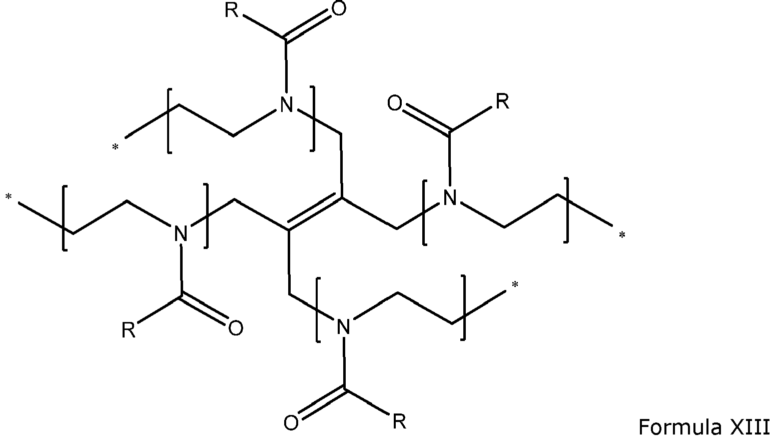 Wherein:
- The radical -R is a linear, -(CH₂)ₙ₁-CH₃ with m an integer comprised from 0 to 4 (0 ≤ n₁ ≤ 4), branched, or cyclic alkyl derivative.
- The * represents the sites of f₃, which is an amine function, or an ether function, or a thioether function, or an amide function, or a carbamate function, or a carbon-nitrogen covalent bond, or a carbon-aromatic carbon covalent bond.

In another embodiment, the POx central linker is a 4-arm POx, chosen among the linkers of formula XIV: 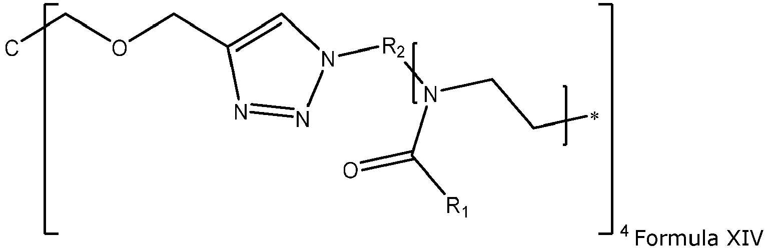 Wherein:
- The radical -R₁ is a linear, -(CH₂)ₙ₁-CH₃ with m an integer comprised from 0 to 4 (0 ≤ n₁ ≤ 4), branched, or cyclic alkyl derivative.
- The divalent radical -R₂- is a linear, -(CH₂)ₙ₂- with n₂ an integer comprised from 2 to 6 (2 ≤ n₂ ≤ 6).
- The * represents the sites of f₃, which is an amine function, or an ether function, or a thioether function, or an amide function, or a carbamate function, or a carbon-nitrogen covalent bond, or a carbon-aromatic carbon covalent bond.

In another embodiment, the POx central linker is a 4-arm POx, chosen among the linkers of formula XV: 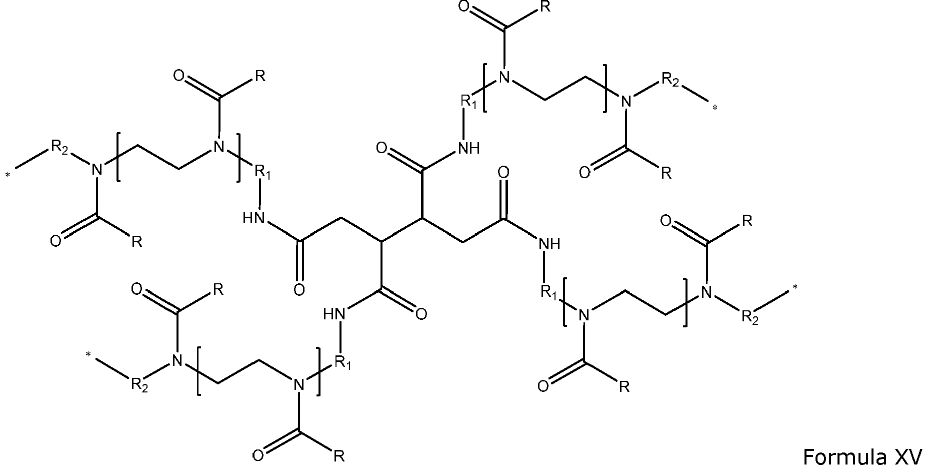 Wherein:
- The radical -R is a linear, -(CH₂)ₙ₁-CH₃ with m an integer comprised from 0 to 4 (0 ≤ n₁ ≤ 4), branched, or cyclic alkyl derivative.
- In one embodiment, R₁ = -CH₂-CH₂- and R₂ is a linear, -(CH₂)ₙ₂- with n₂ an integer comprised from 2 to 6 (2 ≤ n₂ ≤ 6)
- In another embodiment, R₂ = -CH₂-CH₂- and R₁ is a linear, *-(CH₂)ₙ₂-* with nz an integer comprised from 2 to 6 (2 ≤ n₂ ≤ 6)
- The * represents the sites of f₃, which is an amine function, or an ether function, or a thioether function, or an amide function, or a carbamate function, or a carbon-nitrogen covalent bond, or a carbon-aromatic carbon covalent bond.

In an embodiment -W- is chosen among the radicals of formula IV. 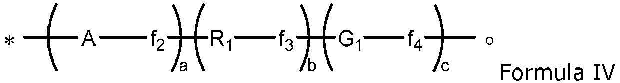 Wherein
- * represents the site of f₁ and ° represents the site of attachment with L.
- a is an integer equal to 0 or 1.
- b is an integer equal to 0 or 1.
- c is an integer equal to 0 or 1.
- In one embodiment a=0, f₁ is an ether function, or a carbamate function.
- In one embodiment a=1,
   ∘ the divalent radical -A- is a linear, -(CH₂)ₙ₁- with m an integer comprised from 1 to 7 (1 ≤ n₁ ≤ 7), branched, or cyclic alkyl derivative. It may also be branched by at least one hydroxyl group, -CH₂-CH(OH)-(CH₂)ₙ₂- with n₂ an integer comprised from 1 to 5 (1 ≤ n₂ ≤ 5); f₁ is an ether function, or a carbamate function, and f₂ is an amide function.
      Or,
   ∘ the divalent radical -A- is a linear polyether (PEG) derivative; f₁ is an ether function, or a carbamate function, and f₂ is an amide function.
      Or,
   ∘ the divalent radical -A- is a 4-Alkyl-1,4-triazole derivative or a 4-PEG-1,4-triazole derivative; f₁ is an ether function, or a carbamate function, and f₂ is a carbon-nitrogen covalent bond.
      Or,
   ∘ the divalent radical -A- is a 1-Alkyl-1,4-triazole derivative or a 1-PEG-1,4-triazole derivative; f₁ is an ether function, or a carbamate function, and f₂ is a carbon-aromatic carbon covalent bond.
- The divalent radical -R₁- is a linear, branched, or cyclic alkyl derivative, and/or an aromatic derivative, and/or a polyether (PEG) derivative, which can contain heteroatoms such as nitrogen, oxygen, or sulphur.
   ∘ If b=0, then f₁ is an ether function, or a carbamate function.
   ∘ If b=1, then f₁ is an ether function, or a carbamate function, and f₃ is an amide function, or an amine function, or an ether function, or a thioether function, or a carbamate function, or a carbon-nitrogen covalent bond, or a carbon-aromatic carbon covalent bond or a carbon-carbon covalent bond if the crosslinking process is made by a Native Chemical Ligation (NCL).
- The divalent radical -G₁- is a linear, branched, or cyclic alkyl derivative, or an aromatic derivative, which can contain heteroatoms such as: at most 5 nitrogen atoms, at most 10 oxygen atoms, at most 5 sulphur atoms, or at most one phosphorus atom. In a preferred embodiment, -G₁- is a succinimide derivative, or an alkyl sulfone derivative which can contain one heteroatom such as oxygen or sulphur, or an ethyl amide derivative, or a 1,4-triazole derivative, or a multicycle derivative from a Diels-Alder reaction, or an aromatic phosphine derivative created by a Staudinger ligation, or a cysteine derivative coming from a Native Chemical Ligation.

∘ If c=0, then f₁, is an ether function, or a carbamate function.
∘ If c=1, then f₁, is an ether function, or a carbamate function, and f₄ is an amine function, or an amide function, or a carbamate function, or a thioether function, or an ether function, or a carbon-nitrogen covalent bond, or carbon-aromatic carbon covalent bond, or a carbon-carbon covalent bond if the crosslinking process is made by a Native Chemical Ligation (NCL)

The cross-linked dextran polymer according to the invention is a dextran polymer Dx- bearing anionic groups wherein an at least divalent radical L(-)ᵢ is covalently bound to the dextran polymer backbone with i radicals, chosen among the dextrans of formula X, 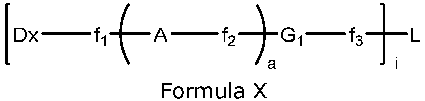 Wherein :
- a is an integer equal to 0 or 1.
- i is an integer comprised from 2 to 8, (2 ≤ i ≤ 8).
- L can be linked to the same [Dx-f₁-(A-f₂)ₐ-G₁-f₃] radicals, or to different ones.
- Dx- is a dextran moiety, which can be substituted by specific anionic groups in salified form, and optionally by alkyl carboxylate derivatives in salified form.
- f₁ is an ether function.
- The divalent radical -A- is a linear, -(CH₂)ₙ₁- with m an integer comprised from 1 to 7 (1 ≤ n₁ ≤ 7), branched, or cyclic alkyl derivative. It may also be branched by at least one hydroxyl group, -CH₂-CH(OH)-(CH₂)ₙ₂- with n₂ an integer comprised from 1 to 5 (1 ≤ n₂ ≤ 5)
- f₂ is an amide function.
- The divalent radical -G₁- is a linear, branched, or cyclic alkyl derivative, or an aromatic derivative, which can contain heteroatoms such as: at most 5 nitrogen atoms, at most 10 oxygen atoms, at most 5 sulphur atoms. In a preferred embodiment, -G₁- is a succinimide derivative, or an alkyl sulfone derivative which can contain one heteroatom such as oxygen or sulphur, or a 1,4-triazole derivative.
- The integer i is the valence of the central-linker L, and the number of, identical or different, [Dx-f₁-(A-f₂)ₐ-G₁-f₃] radicals connected to L.
- f₃ is an amine function, or a thioether function, or an ether function, or an amide function, or a carbamate function, or a carbon-nitrogen covalent bond, or carbon-aromatic carbon covalent bond.
- The central-linker L is a poly(oxazoline) (POx) derivative, which can be linear or branched.

In this embodiment, the cross-linked dextran polymer according to the invention is chosen among the dextran polymers of formula V. 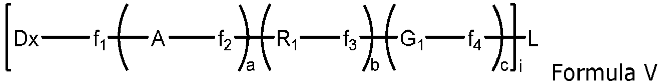 Wherein
- f₁, f₂, f₃, f₄, -A-, -R₁-, -G₁- are defined as above in Formula IV, and
- Dx- is a dextran moiety, which can be substituted by specific anionic groups in salified form, and optionally by alkyl carboxylate derivatives in salified form as previously defined.
- The integer i is the valence of the central-linker L, and the number of, identical or different, [Dx-f₁-(A-f₂)ₐ-(R₁-f₃)_{b}-(G₁-f₄)_{c}] radicals connected to L.
- The central-linker L is a polyether (PEG) derivative, which can be linear or branched.
- In one embodiment, if b=0 and c=1, then the central-linker L can be a poly(oxazoline) (POx) derivative, which can be linear or branched

In this embodiment, the cross-linked dextran polymer according to the invention is chosen among the dextran polymers of formula V. 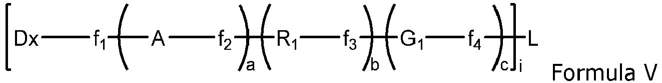 Wherein
- f₁, f₂, f₃, f₄, -A-, -R₁-, -G₁- are defined as above in Formula IV, and
- Dx- is a dextran moiety, which can be substituted by specific anionic groups in salified form, and optionally by alkyl carboxylate derivatives in salified form as previously defined.
- The integer i is the valence of the central-linker L, and the number of, identical or different, [Dx-f₁-(A-f₂)ₐ-(R₁-f₃)_{b}-(G₁-f₄)_{c}] radicals connected to L.
- The central-linker L is a polyether (PEG) derivative, which can be linear or branched.

The hydroxyl functions of the dextran polymer Dx- can be functionalised by at least one specific anionic group such as: alkyl carboxylate, sulphate anions, or sulfonate anions, or phosphate anions, or phosphonate anions.

In an embodiment, the hydroxyl functions of the dextran polymer Dx- are functionalised by one specific anionic group : alkyl carboxylates anions.

In an embodiment, the cross-linked dextran polymer bearing anionic groups according to the invention is a dextran polymer wherein the dextran polymer backbone is according to formula III, 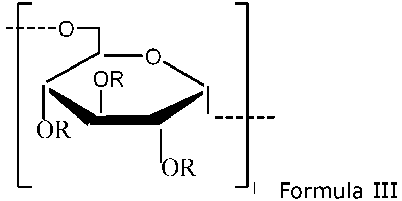 wherein R is chosen among
- -H, a anionic group of formula II, or a -W- radical bearing a L(-)ᵢ crosslinker,
- i is comprised from 20 to 5000 (20 ≤ i ≤ 5000),
- -W- and L(-)ᵢ radicals having the previously defined meanings.

In an embodiment, the cross-linked dextran polymer bearing anionic groups according to the invention is a dextran polymer wherein the dextran polymer backbone is according to formula XI, 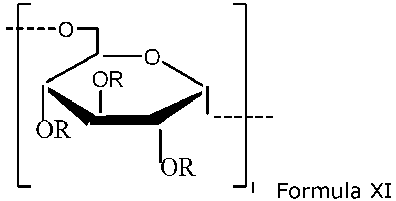 wherein R is chosen among
- -H, a anionic group of formula II, or a -(A-f₂)ₐ-G₁- radical bearing a L(-)ᵢ crosslinker,
- I is comprised from 20 to 5000 (20 ≤ I ≤ 5000),
- -(A-f₂)ₐ-G₁- and L(-)i radicals having the previously defined meanings.

In an embodiment, the cross-linked dextran polymer according to the invention is chosen among the dextran polymers of formula V. 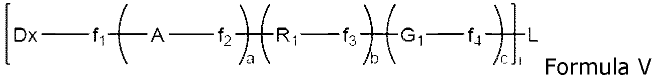 Wherein:
- i is an integer comprised from 2 to 8 (2 ≤ i ≤ 8)
- a = 1,.
- b = 1,
- c = 1,
- Dx is the dextran derivative described in Formula III,
- L is a PEG central linker descirbed in Formula I,
- f₁ is an ether function, or a carbamate function,
- the divalent radical -A- is a linear, -(CH₂)ₙ₁- with m an integer comprised from 1 to 7 (1 ≤ n₁ ≤ 7), branched, or cyclic alkyl derivative,
- f₂ is an amide function,
- the divalent radical -R1- is a linear, -(CH₂)ₙ₁- with m an integer comprised from 1 to 7 (1 ≤ n₁ ≤ 7), branched, or cyclic alkyl derivative,
- f₃ is an amide function,
- the divalent radical -G₁- is a 1,4-triazole derivative
- f₄ is a carbon-nitrogen covalent bond, in particular wherein the nitrogen atom is within the triazole cycle.
-

In a preferred embodiment, the integer i is equal to 4, i = 4.

According to the above embodiment the triazole derivatives comprising of a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.

According to the two above embodiments the triazole derivative comprises more than 10 and less than 30 carbon atoms, optionally it comprises from 4 to 6 nitrogen atoms.

According to an embodiment the 1,4-triazole derivative is obtained through a copperless reaction.

In an embodiment, the cross-linked dextran polymer according to the invention is chosen among the dextran polymers of formula V. 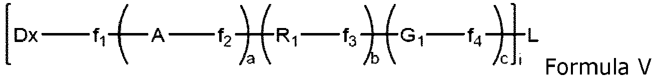 Wherein:
- i is an integer comprised from 2 to 8 (2 ≤ i ≤ 8)
- a = 1,
- b = 0,
- c = 1,
- Dx is the dextran derivative described in Formula III,
- L is a PEG central linker descirbed in Formula I,
- the divalent radical -A- is a linear, -(CH₂)ₙ₁- with n₁ an integer comprised from 1 to 7 (1 ≤ n₁ ≤ 7), branched, or cyclic alkyl derivative,
- f₁ is an ether function, or a carbamate function,
- the divalent radical -G₁- is a 1,4-triazole derivative which is a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine,
said 1,4-triazole derivative bearing a nitrogen, said nitrogen being linked to -CORa-, Ra being an alkyl group comprising from 1 to 4 carbon atoms, via a covalent bond, thus forming an amide function, and Ra being linked to f2 which is an amide function and f₄ is a carbon-nitrogen covalent bond, wherein the nitrogen atom is within the triazole cycle.

In a preferred embodiment, the integer i is equal to 4, i = 4.

According to an embodiment the 1,4-triazole is a multicycle group comprising an acyl group linked to a nitrogen which is within one of the cycles, but not from the triazole cycle, by an amide function.

According to an embodiment the 1,4-triazole comprises a cyclooctyne bearing a nitrogen into the cycloctyne cycle.

According to an embodiment the triazole derivative comprises more than 10 and less than 30 carbon atoms, optionally it comprises from 4 to 6 nitrogen atoms.

In an embodiment the divalent radical -G₁- is a 1,4-triazole derivative as described by the following formula: 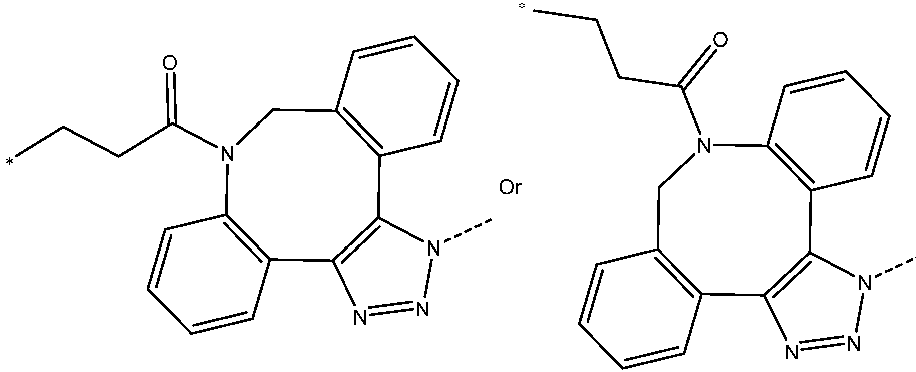 Wherein: the * represents the site of f₂, which is an amide function, and the dotted bond represents f₄, which is a carbon-nitrogen bond.
- f₄ is a carbon-nitrogen covalent bond, wherein the nitrogen atom is within the triazole cycle.

According to an embodiment the triazole 1,4 is obtained through a copperless reaction.

According to an embodiment Dx is the dextran derivative described in Formula III: 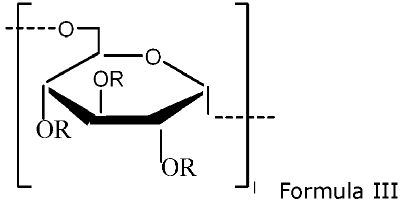 wherein R is chosen among
- -H, a anionic group of formula II, or a -W- radical bearing a L(-)ᵢ crosslinker,
- i is comprised from 20 to 5000 (20 ≤ i ≤ 5000),
- -W- and L(-)ᵢ radicals having the previously defined meanings.

According to an embodiment L is a PEG central linker descirbed in Formula I: 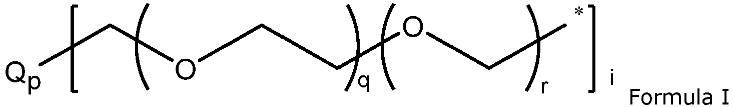 Wherein:
- i is an integer equal to 4,
- p is an integer equal to 1,
- q is an integer comprised from 8 to 1000 (8 ≤ q ≤ 1000)
- r is an integer equal to 0 or 1
- Q is either branched alkyl chain, comprising 2 to 10 carbon atoms,
- the * represents the sites of f4, which is a carbon-nitrogen covalent bond, wherein the nitrogen atom is within the triazole cycle.

In an embodiment, the cross-linked dextran polymer according to the invention is a dextran polymer bearing anionic groups of formula II wherein the at least divalent radical L(-)ᵢ is covalently bound to the dextran polymer backbone with i -W-radicals, wherein i is 2, 4 or 8.

In an embodiment, the cross-linked dextran polymer according to the invention is a dextran polymer bearing anionic groups of formula II wherein the at least divalent radical L(-)ᵢ is covalently bound to the dextran polymer backbone with i radicals, wherein i is 2, 4 or 8.

In an embodiment, the cross-linked dextran polymer according to the invention is a dextran polymer bearing anionic groups of formula II wherein the at least divalent radical L(-)ᵢ is covalently bound to the dextran polymer backbone with i -W-radicals, wherein, i is 2.

In an embodiment, the cross-linked dextran polymer according to the invention is a dextran polymer bearing anionic groups of formula II wherein the at least divalent radical L(-)ᵢ is covalently bound to the dextran polymer backbone with i radicals, wherein, i is 2.

In an embodiment, the cross-linked dextran polymer according to the invention is a dextran polymer bearing anionic groups of formula II wherein the at least divalent radical L(-)ᵢ is covalently bound to the dextran polymer backbone with i -W- radicals, wherein, i is 4.

In an embodiment, the cross-linked dextran polymer according to the invention is a dextran polymer bearing anionic groups of formula II wherein the at least divalent radical L(-)ᵢ is covalently bound to the dextran polymer backbone with i radicals, wherein, i is 4.

In an embodiment, the cross-linked dextran polymer according to the invention is a dextran polymer bearing anionic groups of formula II wherein the at least divalent radical L(-)ᵢ is covalently bound to the dextran polymer backbone with i -W- _ radicals, wherein, i is 8.

In an embodiment, the cross-linked dextran polymer according to the invention is a dextran polymer bearing anionic groups of formula II wherein the at least divalent radical L(-)ᵢ is covalently bound to the dextran polymer backbone with i radicals, wherein, i is 8.

In an embodiment, the cross-linked dextran polymer according to the invention is a dextran polymer wherein the dextran polymer backbone is a dextran having a weight average molecular weight (Mw) comprised from 5 to 1000 kDa before cross-linking and substitution.

In an embodiment, the cross-linked dextran polymer according to the invention is a cross-linked dextran polymer wherein the degree of substitution (DS₁) of the dextran backbone with the a -W- radical or a -(A-f₂)ₐ-G₁- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the degree of substitution of sulfate, sulfonate, phosphate, phosphonate (DS₃) of the dextran backbone is comprised in the range from 0.2 to 2.5 (0.2 ≤ DS₃ ≤ 2.5).

The invention also concerns the dextran polymers of formula VIII before the crosslinking reaction. 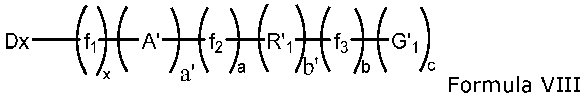 Wherein
- f₁, f₂, f₃, Dx are defined as above if none of a, a', b and b' are equal to 0,
- and
- x equal 0 or 1.
- if a, a', b and b' are equal to 0, x is equal to 0 and Dx is a dextran polymer backbone according to formula III, wherein R is chosen among -H or a anionic group of formula II,
- if one of b' and c is not equal to 0 -A'- is -A- as defined above,
- if b', b and c are equal to 0, a is equal to 0 and A' is the precursor of A before the crosslinking reaction.
- if c is not equal to 0, -R'₁- is -R₁- as defined above and -G'₁- is the precursor of -G1-.
- if c is equal to 0, b is equal to 0 and R'₁ is the precursor of R₁ before the crosslinking , reaction.

The invention concerns an hydrogel comprising:
- - a crosslinked dextran polymer Dx bearing anionic groups wherein the at least divalent radical L(-)ᵢ is covalently bound to the dextran polymer backbone with i W radicals, wherein,

- L(-)¡ is a linear or branched polyether
   - i is the valence of L and the number of W radicals bound to the dextran polymer and is an integer comprised from 2 to 8 (2 ≤ i ≤ 8),
   - -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment the hydrogel comprises a non crosslinked hyaluronate in the form of a solution.

In an embodiment the invention concerns an hydrogel comprising:
- biological cells,
- a non crosslinked hyaluronate in the form of a solution, and
- a crosslinked dextran polymer Dx bearing anionic groups wherein the at least divalent radical L(-)ᵢ is covalently bound to the dextran polymer backbone with i W radicals, wherein,

- L(-)¡ is a linear or branched polyether
   - i is the valence of L and the number of W radicals bound to the dextran polymer and is an integer comprised from 2 to 8 (2 ≤ i ≤ 8),
   - -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment, the cross-linked dextran polymer comprised in the hydrogel according to the invention is not a dextran polymer bearing carboxylate groups wherein the at least divalent radical L(-)ᵢ is covalently bound to the dextran polymer backbone with i W radicals, wherein,
- L(-)¡ is a linear or branched polyether bearing at its ends, heteroatoms such as oxygen, nitrogen or sulfur,
- i is the valence of L and the number of -(R₁)ₘG₁- - radicals and is an integer comprised from 2 to 8 (2 ≤ i ≤ 8),
- m is an integer equal to 0 or 1,
- W is a -(R₁)ₘG₁- radical, wherein
   - -R₁₋ is a linear or branched alkyl divalent radical comprising from 1 to 6 carbon atoms and optionally heteroatoms such as oxygen, nitrogen or sulfur,
   - -G₁- is a linear or branched or cyclic alkyl divalent radical comprising from 1 to 6 carbon atoms and may comprise heteroatoms such as oxygen, nitrogen or sulfur.

In an embodiment, the dextran polymer comprised in the hydrogel is not a dextran polymer wherein the at least divalent radical L(-)ᵢ covalently bound to the dextran polymer backbone with i W radicals results is a radical issued from a linear or branched mercaptopolyethyleneglycol comprising at least 2 sulfur atoms and comprising at most 8 arms which
- Mn is comprised from 1000 to 25 000 g/mol (1000 ≤ Mn ≤ 25 000 g/mol) or
- polymerisation degree (DP) is comprised from 15 to 600 (15 ≤ DP ≤ 600).

In an embodiment, the dextran polymer comprised in the hydrogel is not a dextran polymer wherein the at least divalent radical L(-)ᵢ covalently bound to the dextran polymer backbone with i W radicals is not a radical issued from a linear or branched mercaptopolyethyleneglycol comprising at least 2 sulfur atoms and comprising at most 8 arms, which
- number-average molecular weight (Mn) is comprised from 500 to 40 000 g/mol (500 ≤ Mn ≤ 40 000 g/mol) or
- polymerisation degree (DP) is comprised from 8 to 1000 (8 ≤ DP ≤ 1000).

The cross-linked dextran polymer comprised in the hydrogel according to the invention is a dextran polymer Dx bearing anionic groups wherein the at least divalent radical L(-)ᵢ is covalently bound to the dextran polymer backbone with i -W-radicals, wherein,
- L(-)¡ is a linear or branched polyether, or L(-)¡ is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether or poly(oxazoline) derivatives and that does not comprise two or more alpha aminoacid residues in particular linked by peptidic bond(s).

The cross-linked dextran polymer comprised in the hydrogel according to the invention is a dextran polymer Dx bearing anionic groups wherein the at least divalent radical L(-)ᵢ is covalently bound to the dextran polymer backbone with i -W-radicals, wherein,
- L(-)ᵢ is a linear or branched polyether
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

The cross-linked dextran polymer comprised in the hydrogel according to the invention is a dextran polymer Dx- bearing anionic groups wherein the at least divalent radical L is covalently bound to the dextran polymer backbone with i W radicals, wherein,
- L is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment the hydrogel comprises hyaluronic acid or sodium or potassium hyaluronate salts.

The cross-linked dextran polymer comprised in the hydrogel according to the invention is a dextran polymer Dx bearing anionic groups wherein the at least divalent radical L(-)ᵢ is covalently bound to the dextran polymer backbone with i -W-radicals, wherein,
- L(-)¡ is a linear or branched polyether, or L(-)¡ is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether or poly(oxazoline) derivatives and that does not comprise two or more alpha aminoacid residues in particular linked by peptidic bond(s).

The cross-linked dextran polymer comprised in the hydrogel according to the invention is a dextran polymer Dx bearing anionic groups wherein the at least divalent radical L(-)ᵢ is covalently bound to the dextran polymer backbone with i -W-radicals, wherein,
- L(-)¡ is a linear or branched polyether
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

The cross-linked dextran polymer comprised in the hydrogel according to the invention is a dextran polymer Dx- bearing anionic groups wherein the at least divalent radical L is covalently bound to the dextran polymer backbone with i W radicals, wherein,
- L is a linear or branched poly(oxazoline)
- i is the valence of L and the number of -W- radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment the hydrogel according to the invention is characterized in that Tan δ is lower than 1.

In an embodiment the hydrogel is transparent.

In an embodiment, the hydrogel is translucid.

In an embodiment the hydrogel according to the invention is characterized in that after swelling in water the cross-linked dextran polymer concentration is comprised from 0.01 to 0.2 g/g.

In an embodiment, the hydrogel has a Young modulus comprised between 1 to 200 kPa.

In an embodiment, the hydrogel has a G' comprised from 0.5 to 70 kPa.

In an embodiment the hydrogel has a compression deformation at break of more than or equal to 10 %.

In an embodiment the hydrogel has a swelling ratio of more than 0.7.

In an embodiment the hydrogel has a water content of at least 80 wt%.

In an embodiment the hydrogel according to the invention is characterized in that it further comprises biological cells.

In an embodiment the cells are proteins, hormones or peptide secreting cells.

In an embodiment the cells are chosen from:
- insulin secreting cells for diabetes treatment
- Factor VIII or Factor IX secreting cells for hemophilia treatment andβ-glucocerebrosidase secreting cells for Gaucher disease.

In an embodiment the hydrogel according to the invention is characterized in that the biological cells are pseudoislets.

The invention also concerns a process to synthetize a cross-linked dextran polymer according to the invention, into the form of a hydrogel, comprising the steps of:
g) preparation of a sterile solution comprising a dextran bearing anionic groups of formula II and at least two precursors of -W-
h) preparation of a sterile solution of a precursor of L(-)ᵢ
i) addition of the sterile solution obtained from step b) to the solution obtained from step a),
j) the addition being directly done in a mould or the solutions are introduced into a mould after being mixed,
k) crosslinking and gelation, for example at room temperature (20-25°C) or at 37°C,
l) unmoulding and swelling to obtain an hydrogel.

The invention also concerns a process to synthetize a cross-linked dextran polymer according to the invention, into the form of a hydrogel, comprising the steps of:
g) preparation of a sterile solution comprising a dextran bearing anionic groups of formula II and at least two precursors of -(A-f₂)ₐ-G₁-, -(A'-f₂)ₐ-G'1-,
h) preparation of a sterile solution of a precursor of L(-)ᵢ
i) addition of the sterile solution obtained from step b) to the solution obtained from step a),
j) the addition being directly done in a mould or the solutions are introduced into a mould after being mixed,
k) crosslinking and gelation, for example at room temperature (20-25°C) or at 37°C,
l) unmoulding and swelling to obtain an hydrogel.

In an embodiment steps c) and d) are done simultaneously.

In an embodiment, the swelling is done into a PBS solution at pH 7,4.

Dextrans bearing anionic groups of formula II are prepared by grafting or substitution on the hydroxyl groups borne by the dextrans. In an embodiment the dextrans bearing anionic groups of formula II are prepared by grafting or substituting the carboxymethyl groups borne by the carboxymethyl dextrans.

The crosslinking step is a gelation step that leads to the formation of a hydrogel according to the invention.

The invention also concerns a process to prepare a hydrogel comprising biological cells comprising the steps of:
i) preparation of a sterile solution comprising a dextran a dextran bearing anionic groups of formula II and at least two precursors of -W-,
j) preparation of a sterile solution of a precursor of L(-)ᵢ,
k) preparation of a suspension of biological cells,
l) mixing the biological cells suspension obtained from step c) and the solution obtained from the step b) or a),
m) addition of the sterile solution obtained from step a) or b) which is not used in step d) to the solution obtained from step d),
n) the addition of step e) being either done directly in a mould or the solutions are introduced into a mould after being mixed,
o) crosslinking and gelation reaction at room temperature (20-25°C),
p) unmoulding and swelling to obtain an hydrogel comprising biological cells.

The invention also concerns a process to prepare a hydrogel comprising biological cells comprising the steps of:
i) preparation of a sterile solution comprising a dextran a dextran bearing anionic groups of formula II and at least two precursors of -(A-f₂)ₐ-G₁-, - (A'-f₂)ₐ-G'₁-,
j) preparation of a sterile solution of a precursor of L(-)ᵢ,
k) preparation of a suspension of biological cells,
l) mixing the biological cells suspension obtained from step c) and the solution obtained from the step b) or a),
m) addition of the sterile solution obtained from step a) or b) which is not used in step d) to the solution obtained from step d),
n) the addition of step e) being either done directly in a mould or the solutions are introduced into a mould after being mixed,
o) crosslinking and gelation reaction at room temperature (20-25°C),
p) unmoulding and swelling to obtain an hydrogel comprising biological cells.

The invention also concerns a process to prepare a hydrogel comprising biological cells comprising the steps of:
l) preparation of a sterile solution comprising a dextran bearing anionic groups of formula II and at least two precursors of -W-
m) preparation of a sterile solution of a precursor of L(-)ᵢ chosen among a thiol polyethylene glycol, a mercaptopoly(oxyethylenes), a pentaerythritol poly(oxyethylene) azide or a pentaerythritol poly(dibenzocyclooctyne) polyoxyethylene
n) preparation of a sterile solution of hyaluronate de sodium,
o) preparation of a sterile suspension of biological cells,
p) mixing the sodium hyaluronate solution obtained from step c) with precursor solution from the step b)
q) mixing the biological cells suspension obtained from step d) and the solution obtained from the step e) or from step a)
r) mixing the solution obtained from step f) and the solutions obtained from step e),
s) addition of the sterile solution obtained from step a) or e) which is not used in step g) to the solution obtained from step f),
t) the addition of step g) being either done directly in a mould or the solutions are introduced into a mould after being mixed,
u) crosslinking and gelation reaction at room temperature (20-25°C),
v) unmoulding and swelling to obtain an hydrogel comprising biological cells.

According to an embodiment, in the step of moulding the solution comprises an osmotic agent which is a non-ionic osmotic agent, such as trehalose.

According to an embodiment, in the step of moulding the solution comprises a weight ratio nonionic osmotic agent to NaCl which is more than 2, in particular more than 5, more particularly more than 10.

According to an embodiment, in the step of moulding the solution comprises from 5 to 50 mg/ml of non-ionic osmotic agent, in particular trehalose.

In an embodiment, the mould is a Ring Net.

In an embodiment, crosslinking and gelation reaction are performed at room temperature (20-25°C),

The invention also concerns a kit comprising:
- A solution of dextran polymer of formula VIII before the crosslinking reaction: 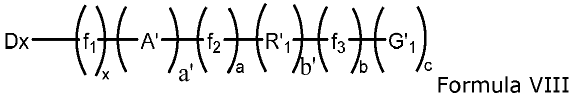 Wherein
   - f₁, f₂, f₃, f₄, Dx are defined as in formula IV if none of a, a', b and b' are not equal to 0,
   - and
   - x equal 0 or 1
   - if a, a', b and b' are equal to 0, x is equal to 0 and Dx is a dextran polymer backbone is according to formula III, wherein R is chosen among -H or a anionic group of formula II,
   - if one of b' and c is not equal to 0 -A' is A as defined above,
   - if b', b and c are equal to 0, a is equal to 0 and A' is the precursor of A before the crosslinking reaction.
   - if c is not equal to 0, R'₁ is R₁ as defined above and G'₁ is the precursor of G₁.
   - if c is equal to 0, b is equal to 0 and R'₁ is the precursor of R₁ before the crosslinking , reaction.
- a solution of a thiol polyethylene glycol, a mercaptopoly(oxyethylenes), a pentaerythritol poly(oxyethylene) azide or a pentaerythritol poly(dibenzocyclooctyne) polyoxyethylene.
- biological cells.

The invention also concerns a kit comprising:
- A solution of dextran polymer of formula VIII before the crosslinking reaction: 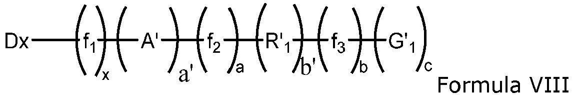 Wherein
   - f₁, f₂, f₃, f₄, Dx are defined as in formula IV if none of a, a', b and b' are not equal to 0,
   - and
   - x equal 0 or 1
   - if a, a', b and b' are equal to 0, x is equal to 0 and Dx is a dextran polymer backbone is according to formula III, wherein R is chosen among -H or a anionic group of formula II,
   - if one of b' and c is not equal to 0 -A' is A as defined above,
   - if b', b and c are equal to 0, a is equal to 0 and A' is the precursor of A before the crosslinking reaction.
   - if c is not equal to 0, R'₁ is R₁ as defined above and G'₁ is the precursor of G₁.
   - if c is equal to 0, b is equal to 0 and R'₁ is the precursor of R₁ before the crosslinking reaction.
- a solution of a thiol polyethylene glycol, a mercaptopoly(oxyethylenes), a pentaerythritol poly(oxyethylene) azide or a pentaerythritol poly(dibenzocyclooctyne) polyoxyethylene.
- biological cells.
- a solution of non crosslinked sodium hyaluronate.

The invention also concerns a kit comprising:
- A solution of dextran polymer of formula VIII before the crosslinking reaction: 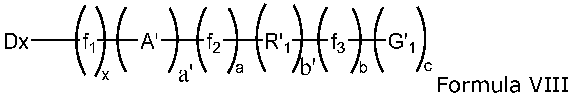 Wherein
   - f₁, f₂, f₃, f₄, Dx are defined as in formula IV if none of a, a', b and b' are not equal to 0,
   - and
   - x equal 0 or 1
   - if a, a', b and b' are equal to 0, x is equal to 0 and Dx is a dextran polymer backbone is according to formula III, wherein R is chosen among -H or a anionic group of formula II,
   - if one of b' and c is not equal to 0 -A' is A as defined above,
   - if b', b and c are equal to 0, a is equal to 0 and A' is the precursor of A before the crosslinking reaction.
   - if c is not equal to 0, R'₁ is R₁ as defined above and G'₁ is the precursor of G₁.
   - if c is equal to 0, b is equal to 0 and R'₁ is the precursor of R₁ before the crosslinking , reaction.
- a solution of a thiol polyethylene glycol, a mercaptopoly(oxyethylenes), a pentaerythritol poly(oxyethylene) azide or a pentaerythritol poly(dibenzocyclooctyne) polyoxyethylene.
- biological cells.

The invention also concerns the use of a cross-linked dextran copolymer according to the invention into the form of a hydrogel to prepare a cell composition.

The invention also concerns a therapeutic use of the hydrogel according to the invention as a therapeutic implant to administer at least an API to a mammal.

The invention also concerns a therapeutic use of the hydrogel according to the invention for treating a disorder or disease in a mammal wherein the disorder or disease is due to lack or malfunction of endocrine function of pancreas organ.

The invention also concerns a hydrogel for use as a medicament.

The invention also concerns a hydrogel for use in the treatment of a disease such as diabetes.

The invention also concerns an implant comprising the hydrogel of the invention.

In an embodiment the invention concerns an implant comprising a ring, a net, the hydrogel according to the invention and cells.

The ring and net structure allow the hydrogel to be easily manipulated, to have a good resistance to manipulation, including for implantation. This is also true even with a hydrogel having a larger mesh size (for example with a lower DS of -W- and lower concentrations of reactive groups during crosslinking).

The ring and net structure allow the hydrogel to be easily manipulated, to have a good resistance to manipulation, including for implantation. This is also true even with a hydrogel having a larger mesh size (for example with a lower DS -(A-f₂)ₐ-G₁- and lower concentrations of reactive groups during crosslinking).

The ring and net structure allow the hydrogel to be easily manipulated, a good resistance to manipulation, including for implantation in the case the hydrogel is very thin but have a rather large planar surface.

In an embodiment the implant is a parallelepiped rectangle with round corner.

In an embodiment the implant has a thickness of less than 3 000 µm.

In an embodiment the implant has total surface of between 10 cm² to 200 cm².

In an embodiment the implant comprises from 0.5 to 20 ml of hydrogel.

In an embodiment the ring has an internal diameter of 10 to 100 mm.

In an embodiment the ring is a parallelepiped rectangle with rounded corner.

In an embodiment the ring material is a bioinert material.

In an embodiment, the ring material is a biocompatible elastomer.

In an embodiment the ring material is chosen from the group consisting of silicone, in particular PDMS, polyurethanes, polyether, polyether polyester copolymers and polypropylene oxide.

In an embodiment the net is non-biodegradable.

In an embodiment the net is biocompatible.

In an embodiment the net is non absorbable.

In an embodiment the net is a surgical mesh.

In an embodiment the filament material of the net material is chosen among the group consisting of Polypropylene, Polyethylene, polyester, in particular PET, PTFE, PVDF (polyvinylidene fluoride) and ePVDF (extended PVDF).

In an embodiment the net has a thickness ranging from 50 to 500 µm.

In an embodiment the pore size of the net is ranging from 0.4 to 4 mm.

In an embodiment the net is having pores with side sizes ranging from 0.4 to 4 mm.

In an embodiment fabric of the net is chosen from the group consisting of knitted fabric, warp knitted fabric, woven fabric, non-woven fabric.

In an embodiment, the implant can be obtained by the following process:
- Hydrogel compositions are incorporated in the Ring Mesh constructs, the concentrated polymer solutions are mixed with a pipette and a controlled volume of the mixture is introduced in a ring mesh construct adhering to a glass slide,
- Crosslinking leading to gelation is carried out, then the Ring Mesh + Hydrogel composition is introduced in a Tris 150 mM /NaCl 30 mM / Cystein 10 mM solution at pH 8 or in PBS at pH 7.4,
- the hydrogel is rinsed with PBS solution without cysteine and further immersed in the PBS solution overnight at 37°C and then. the hydrogel piece is stored in PBS solution at 4°C until being used.

Another problem to be solved is to find a biological glue allowing adherence between a biological tissue and another biological tissue or between a tissue and a device, in particular a hydrogel.

The invention also relates on a bicomponent glue characterized in that it comprises precursors of a hydrogel, said hydrogel comprising:
a crosslinked dextran polymer Dx bearing anionic groups wherein the at least divalent radical L(-)_{I} is covalently bound to the dextran polymer backbone with I W radicals, wherein,
- L(-)i is a linear or branched polyether
- I is the valence of L and the number of W radicals bound to the dextran polymer and is an integer comprised from 2 to 8 (2 ≤ I ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment of the bicomponent glue according to the invention, precursors of the hydrogel comprise at least one dextran polymer of formula VIII before the crosslinking reaction: 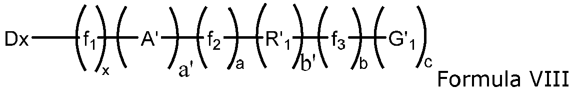 Wherein
- f₁, f₂, f₃, f₄, Dx are defined as in formula IV if none of a, a', b and b' are not equal to 0,
- and
- x equal 0 or 1
- if a, a', b and b' are equal to 0, x is equal to 0 and Dx is a dextran polymer backbone is according to formula III, wherein R is chosen among -H or a anionic group of formula II,
- if one of b' and c is not equal to 0 -A' is A as defined above,
- if b', b and c are equal to 0, a is equal to 0 and A' is the precursor of A before the crosslinking reaction.
- if c is not equal to 0, R'₁ is R₁ as defined above and G'₁ is the precursor of G₁.
- if c is equal to 0, b is equal to 0 and R'₁ is the precursor of R₁ before the crosslinking , reaction and
at least one thiol polyethylene glycol, mercaptopoly(oxyethylenes), pentaerythritol poly(oxyethylene) azide and/or pentaerythritol poly(dibenzocyclooctyne) polyoxyethylene.

In an embodiment of the bicomponent glue according to the invention, the bicomponent glue consist of at least one dextran polymer of formula VIII before the crosslinking reaction: 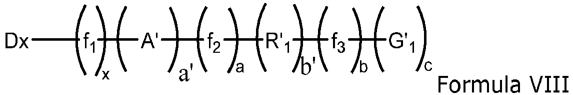 Wherein
- f₁, f₂, f₃, f₄, Dx are defined as in formula IV if none of a, a', b and b' are not equal to 0,
- and
- x equal 0 or 1
- if a, a', b and b' are equal to 0, x is equal to 0 and Dx is a dextran polymer backbone is according to formula III, wherein R is chosen among -H or a anionic group of formula II,
- if one of b' and c is not equal to 0 -A' is A as defined above,
- if b', b and c are equal to 0, a is equal to 0 and A' is the precursor of A before the crosslinking reaction.
- if c is not equal to 0, R'₁ is R₁ as defined above and G'₁ is the precursor of G₁.
- if c is equal to 0, b is equal to 0 and R'₁ is the precursor of R₁ before the crosslinking , reaction and
at least one thiol polyethylene glycol, mercaptopoly(oxyethylenes), pentaerythritol poly(oxyethylene) azide and/or pentaerythritol poly(dibenzocyclooctyne) polyoxyethylene.

In an embodiment of the bicomponent glue according to the invention, the glue is a surgical glue.

The invention also relates on the use of a composition comprising precursors of a hydrogel, characterized in that said hydrogel comprises:
a crosslinked dextran polymer Dx bearing anionic groups wherein the at least divalent radical L(-)_{I} is covalently bound to the dextran polymer backbone with I W radicals, wherein,
- L(-)i is a linear or branched polyether
- I is the valence of L and the number of W radicals bound to the dextran polymer and is an integer comprised from 2 to 8 (2 ≤ I ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment of the use of a composition comprising precursors of a hydrogel according to invention, precursors of the hydrogel comprise at least one dextran polymer of formula VIII before the crosslinking reaction: 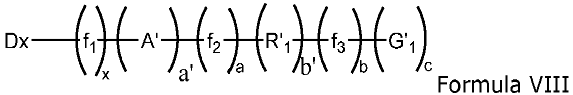 Wherein
- f₁, f₂, f₃, f₄, Dx are defined as in formula IV if none of a, a', b and b' are not equal to 0,
- and
- x equal 0 or 1
- if a, a', b and b' are equal to 0, x is equal to 0 and Dx is a dextran polymer backbone is according to formula III, wherein R is chosen among -H or a anionic group of formula II,
- if one of b' and c is not equal to 0 -A' is A as defined above,
- if b', b and c are equal to 0, a is equal to 0 and A' is the precursor of A before the crosslinking reaction.
- if c is not equal to 0, R'₁ is R₁ as defined above and G'₁ is the precursor of G₁.
- if c is equal to 0, b is equal to 0 and R'₁ is the precursor of R₁ before the crosslinking , reaction and
at least one thiol polyethylene glycol, mercaptopoly(oxyethylenes), pentaerythritol poly(oxyethylene) azide and/or pentaerythritol poly(dibenzocyclooctyne) polyoxyethylene.

In an embodiment of the use of a composition comprising precursors of a hydrogel according to invention the composition consists of at least one dextran polymer of formula VIII before the crosslinking reaction: 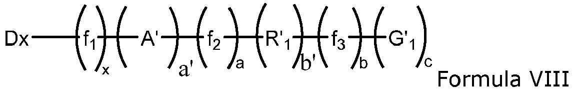 Wherein
- f₁, f₂, f₃, f₄, Dx are defined as in formula IV if none of a, a', b and b' are not equal to 0,
- and
- x equal 0 or 1
- if a, a', b and b' are equal to 0, x is equal to 0 and Dx is a dextran polymer backbone is according to formula III, wherein R is chosen among -H or a anionic group of formula II,
- if one of b' and c is not equal to 0 -A' is A as defined above,
- if b', b and c are equal to 0, a is equal to 0 and A' is the precursor of A before the crosslinking reaction.
- if c is not equal to 0, R'₁ is R₁ as defined above and G'₁ is the precursor of G₁.
- if c is equal to 0, b is equal to 0 and R'₁ is the precursor of R₁ before the crosslinking , reaction and
at least one thiol polyethylene glycol, mercaptopoly(oxyethylenes), pentaerythritol poly(oxyethylene) azide and/or pentaerythritol poly(dibenzocyclooctyne) polyoxyethylene.

The invention also concerns bicomponent glue according to the invention for therapeutical and/or surgical use.

The invention also concerns a bicomponent glue according to the invention for therapeutical.

The invention also concerns a bicomponent glue according to the invention for surgical use.

The invention also concerns the bicomponent glue for therapeutical and/or surgical use according to the invention for improving adhesion and/or immobilizing *in vivo* an implant onto biological tissue of the recipient patient.

The invention also concerns the bicomponent glue for therapeutical and/or surgical use according to the invention, for improving adhesion and/or immobilizing *in vivo* an implant onto biological tissue of the recipient patient.

The invention also concerns the bicomponent glue for therapeutical and/or surgical use according to the invention, for improving adhesion *in vivo* of an implant onto biological tissue of the recipient patient.

The invention also concerns the bicomponent glue for therapeutical and/or surgical use according to the invention, for immobilizing *in vivo* an implant onto biological tissue of the recipient patient.

The invention also concerns the bicomponent glue for therapeutical and/or surgical use according to the invention, for improving adhesion and/or immobilizing *in vivo* a hydrogel onto biological tissue of the recipient patient and/or a biological tissue graft onto biological tissue of the recipient patient.

The invention also concerns the bicomponent glue for therapeutical and/or surgical use according to the invention, for improving adhesion *in vivo* of a hydrogel onto biological tissue of the recipient patient and/or a biological tissue graft onto biological tissue of the recipient patient.

The invention also concerns the bicomponent glue for therapeutical and/or surgical use according to the invention, for immobilizing *in vivo* a hydrogel onto biological tissue of the recipient patient and/or a biological tissue graft onto biological tissue of the recipient patient.

In an embodiment of the bicomponent glue for therapeutical and/or surgical use according to the invention, the implant is a biological tissue.

In an embodiment of the bicomponent glue for therapeutical and/or surgical use according to the invention, the implant comprises active ingredients.

In an embodiment of the bicomponent glue for therapeutical and/or surgical use according to the invention, the active ingredient is secreting cells.

In an embodiment of the bicomponent glue for therapeutical and/or surgical use according to the invention, the active ingredient is insulin secreting cells.

In an embodiment of the bicomponent glue for therapeutical and/or surgical use according to the invention, the active ingredient is Factor VIII or Factor IX secreting cells.

In an embodiment of the bicomponent glue for therapeutical and/or surgical use according to the invention, the active ingredient is β-glucocerebrosidase secreting cells.

In an embodiment of the bicomponent glue for therapeutical and/or surgical use according to the invention, the implant is an organ.

In an embodiment of the bicomponent glue for therapeutical and/or surgical use according to the invention, the implant is an organ in the context of an organ transplant.

In an embodiment of the bicomponent glue for therapeutical and/or surgical use according to the invention, the implant is an organ in the context of an organ transplant selected from the group consisting of a liver transplant, a lung transplant, a pancreas transplant, a cornea transplant, an organ system transplant, a bone marrow transplant, a pancreatic islet cell transplant, a stem cell transplant, a skin tissue transplant, a skin cell transplant, and a xenotransplant.

In an embodiment of the bicomponent glue for therapeutical and/or surgical use according to the invention, the implant is an artificial organ.

In an embodiment of the bicomponent glue for therapeutical and/or surgical use according to the invention, the implant is an artificial pancreas.

In an embodiment of the bicomponent glue for therapeutical and/or surgical use according to the invention, the artificial pancreas is a hydrogel comprising active ingredients.

In an embodiment of the bicomponent glue for therapeutical and/or surgical use according to the invention, the artificial pancreas is a hydrogel comprising secreting cells.

In an embodiment of the bicomponent glue for therapeutical and/or surgical use according to the invention, the artificial pancreas is a hydrogel comprising secreting cells as defined above.

In an embodiment of the bicomponent glue for therapeutical and/or surgical use according to the invention, the artificial pancreas is a hydrogel comprising insulin secreting cells.

In an embodiment of the bicomponent glue for therapeutical and/or surgical use according to the invention, the artificial pancreas is a hydrogel comprising Factor VIII or Factor IX secreting cells.

In an embodiment of the bicomponent glue for therapeutical and/or surgical use according to the invention, the artificial pancreas is a hydrogel comprising Factor VIII or Factor IX secreting cells β-glucocerebrosidase secreting cells.

In an embodiment of the bicomponent glue for therapeutical and/or surgical use according to the invention, the implant is a hydrogel.

In an embodiment of the bicomponent glue for therapeutical and/or surgical use according to the invention, the implant is a hydrogel comprising active ingredients.

In an embodiment of the bicomponent glue for therapeutical and/or surgical use according to the invention, the implant is a hydrogel comprising active ingredients as defined above.

In an embodiment of the bicomponent glue for therapeutical and/or surgical use according to the invention, the hydrogel comprising secreting cells comprise a crosslinked dextran polymer Dx bearing anionic groups wherein the at least divalent radical L(-)i is covalently bound to the dextran polymer backbone with I W radicals, wherein,
- L(-)i is a linear or branched polyether
- I is the valence of L and the number of W radicals bound to the dextran polymer and is an integer comprised from 2 to 8 (2 ≤ I ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment of the bicomponent glue for therapeutical and/or surgical use according to the invention, the hydrogel comprising secreting cells further comprises a non crosslinked hyaluronate in the form of a solution.

In an embodiment of the bicomponent glue for therapeutical and/or surgical use according to the invention, the hydrogel comprising insulin secreting cells is chosen amongst the dextran polymer Dx bearing anionic groups wherein the at least divalent radical L(-)_{I} is covalently bound to the dextran polymer backbone with I -W-radicals, wherein,
- L(-)_{I} is a linear or branched polyether, or L(-)_{I} is a linear or branched poly(oxazoline)
- I is the valence of L and the number of -W- radicals bound to the dextran polymer and is an integer comprised from 2 to 8 (2 ≤ I ≤ 8),
- -W- is a radical comprising at least a radical alkyl linear or branched and optionally comprising heteroatoms such as oxygen, nitrogen or sulfur, aromatic cycles, polyether or poly(oxazoline) derivatives and that does not comprise two or more alpha aminoacid residues in particular linked by peptidic bond(s).

In an embodiment of the bicomponent glue for therapeutical and/or surgical use according to the invention, the hydrogel comprising secreting cells is characterized in that Tan δ is lower than 1.

In an embodiment of the bicomponent glue for therapeutical and/or surgical use according to the invention, the hydrogel comprising secreting cells is a transparent hydrogel.

In an embodiment of the bicomponent glue for therapeutical and/or surgical use according to the invention, the hydrogel comprising secreting cells is a translucid hydrogel.

Bicomponent glue for therapeutical and/or surgical use according to the invention, wherein after swelling in water the cross-linked dextran polymer concentration is comprised from 0.01 to 0.2 g/g.

In an embodiment of the bicomponent glue for therapeutical and/or surgical use according to the invention, the hydrogel comprising secreting cells is characterized in that its Young modulus is comprised between 1 to 200 kPa.

In an embodiment of the bicomponent glue for therapeutical and/or surgical use according to the invention, the hydrogel comprising secreting cells is characterized in that its G' is comprised from 0.5 to 70 kPa.

In an embodiment of the bicomponent glue for therapeutical and/or surgical use according to the invention, the hydrogel comprising secreting cells is characterized in that its compression deformation at break is of more than or equal to 10 %.

In an embodiment of the bicomponent glue for therapeutical and/or surgical use according to the invention, the hydrogel comprising secreting cells is characterized in that it has a swelling ratio of more than 0.7.

In an embodiment of the bicomponent glue for therapeutical and/or surgical use according to the invention, the hydrogel comprising secreting cells is characterized in that it has a water content of at least 80 wt%.

In an embodiment of the bicomponent glue for therapeutical and/or surgical use according to the invention, the hydrogel comprising secreting cells is characterized in that the secreting cells are proteins, hormones or peptide secreting cells.

In an embodiment of the bicomponent glue for therapeutical and/or surgical use according to the invention, the hydrogel comprising secreting cells is characterized in that the secreting cells are chosen from:
- insulin secreting cells for diabetes treatment
- Factor VIII or Factor IX secreting cells for hemophilia treatment andβ-glucocerebrosidase secreting cells for Gaucher disease.

In an embodiment of the bicomponent glue for therapeutical and/or surgical use according to the invention, the the hydrogel comprising secreting cells is characterized in that the secreting cells are pseudoislets.

In an embodiment of the bicomponent glue for therapeutical and/or surgical use according to the invention, the artificial pancreas further comprises a ring and/or a net.

In an embodiment of the bicomponent glue for therapeutical and/or surgical use according to the invention, the artificial pancreas is characterized in that it is a parallelepiped rectangle with round corner.

In an embodiment of the bicomponent glue for therapeutical and/or surgical use according to the invention, the artificial pancreas is characterized in that its thickness is of less than 3 000 µm.

In an embodiment of the bicomponent glue for therapeutical and/or surgical use according to the invention, the artificial pancreas is characterized in that it has total surface of between 10 cm² to 200 cm².

In an embodiment of the bicomponent glue for therapeutical and/or surgical use according to the invention, the artificial pancreas is characterized in that it comprises from 0.5 to 20 ml of hydrogel comprising insulin secreting cells.

In an embodiment of the bicomponent glue for therapeutical and/or surgical use according to the invention, the artificial pancreas is characterized in that the ring has an internal diameter of 10 to 100 mm.

In an embodiment of the bicomponent glue for therapeutical and/or surgical use according to the invention, the artificial pancreas is characterized in that the ring is a parallelepiped rectangle with rounded corner.

In an embodiment of the bicomponent glue for therapeutical and/or surgical use according to the invention, the artificial pancreas is characterized in that the ring material is a bioinert material.

In an embodiment of the bicomponent glue for therapeutical and/or surgical use according to the invention, the artificial pancreas is characterized in that the ring material is a biocompatible elastomer.

In an embodiment of the bicomponent glue for therapeutical and/or surgical use according to the invention, the artificial pancreas is characterized in that the ring material is chosen from the group consisting of silicone, in particular PDMS, polyurethanes, polyether, polyether polyester copolymers and polypropylene oxide.

In an embodiment of the bicomponent glue for therapeutical and/or surgical use according to the invention, the artificial pancreas is characterized in that the net is non-biodegradable.

In an embodiment of the bicomponent glue for therapeutical and/or surgical use according to the invention, the artificial pancreas is characterized in that the net is biocompatible.

In an embodiment of the bicomponent glue for therapeutical and/or surgical use according to the invention, the artificial pancreas is characterized in that the net is non absorbable.

In an embodiment of the bicomponent glue for therapeutical and/or surgical use according to the invention, the artificial pancreas is characterized in that the net is a surgical mesh.

In an embodiment of the bicomponent glue for therapeutical and/or surgical use according to the invention, the artificial pancreas is characterized in that the filament material of the net material is chosen among the group consisting of Polypropylene, Polyethylene, polyester, in particular PET, PTFE, PVDF (polyvinylidene fluoride) and ePVDF (extended PVDF).

In an embodiment of the bicomponent glue for therapeutical and/or surgical use according to the invention, the artificial pancreas is characterized in that the net has a thickness ranging from 50 to 500 µm.

In an embodiment of the bicomponent glue for therapeutical and/or surgical use according to the invention, the artificial pancreas is characterized in that the pore size of the net is ranging from 0.4 to 4 mm.

In an embodiment of the bicomponent glue for therapeutical and/or surgical use according to the invention, the artificial pancreas is characterized in that fabric of the net is chosen from the group consisting of knitted fabric, warp knitted fabric, woven fabric, non-woven fabric.

In an embodiment of the bicomponent glue for therapeutical and/or surgical use according to the invention, the artificial pancreas is characterized in that it is obtained by the following process:
- Hydrogel compositions are incorporated in the Ring Mesh constructs, the concentrated polymer solutions are mixed with a pipette and a controlled volume of the mixture is introduced in a ring mesh construct adhering to a glass slide,
- Crosslinking leading to gelation is carried out, then the Ring Mesh + Hydrogel composition is introduced in a Tris 150 mM /NaCl 30 mM / Cystein 10 mM solution at pH 8 or in PBS at pH 7.4,
- the hydrogel is rinsed with PBS solution without cysteine and further immersed in the PBS solution overnight at 37°C and then. The hydrogel piece is stored in PBS solution at 4°C until being used.

In an embodiment of the bicomponent glue for therapeutical and/or surgical use according to the invention, the implant is sutured to the biological tissue of the recipient patient.

In an embodiment of the bicomponent glue for therapeutical and/or surgical use according to the invention, the biological tissue of the recipient patient is a connective tissue.

In an embodiment of the bicomponent glue for therapeutical and/or surgical use according to the invention, the biological tissue of the recipient patient is a mucous membrane.

In an embodiment of the bicomponent glue for therapeutical and/or surgical use according to the invention, the recipient patient is a mammal.

In an embodiment of the bicomponent glue for therapeutical and/or surgical use according to the invention, the recipient patient is a human.

In an embodiment of the bicomponent glue for therapeutical and/or surgical use according to the invention, the recipient patient has a disorder or disease due to lack or malfunction of endocrine function of pancreas organ.

In an embodiment of the bicomponent glue for therapeutical and/or surgical use according to the invention, the recipient patient has diabetes.

In an embodiment of the bicomponent glue for therapeutical and/or surgical use according to the invention, the bicomponent glue is applied on the surface of the implant, on the surface of the biological tissue of the recipient patient and/or on the contact area.

In an embodiment of the bicomponent glue for therapeutical and/or surgical use according to the invention, the bicomponent glue is applied on the surface of the implant.

In an embodiment of the bicomponent glue for therapeutical and/or surgical use according to the invention, the bicomponent glue is applied on the surface of the biological tissue of the recipient patient.

In an embodiment of the bicomponent glue for therapeutical and/or surgical use according to the invention, the bicomponent glue is applied on the contact area.

According to the invention, "contact area" is understood to refer to the specific area where the implant comes into direct contact with the biological tissue of the recipient patient.

In an embodiment of the bicomponent glue for therapeutical and/or surgical use according to the invention, the quantity of bicomponent glue applied per square centimeters of contact area is from 0,01 µL/cm² to 500 µL/cm².

In an embodiment of the bicomponent glue for therapeutical and/or surgical use according to the invention, the quantity of bicomponent glue applied per square centimeters of contact area is from 0,1 µL/cm² to 250 µL/cm².

In an embodiment of the bicomponent glue for therapeutical and/or surgical use according to the invention, the quantity of bicomponent glue applied per square centimeters of contact area is from 1 µL/cm² to 100 µL/cm².

In an embodiment of the bicomponent glue for therapeutical and/or surgical use according to the invention, the quantity of bicomponent glue applied per square centimeters of contact area is from 10 µL/cm² to 50 µL/cm².

In an embodiment of the bicomponent glue for therapeutical and/or surgical use according to the invention, the quantity of bicomponent glue applied per square centimeters of contact area is from 20 µL/cm² to 30 µL/cm².

In an embodiment of the bicomponent glue for therapeutical and/or surgical use according to the invention, the bicomponent glue is applied before, during and/or after the implantation and/or the implant positioning.

In an embodiment of the bicomponent glue for therapeutical and/or surgical use according to the invention, the bicomponent glue is applied before the implantation and/or the implant positioning.

In an embodiment of the bicomponent glue for therapeutical and/or surgical use according to the invention, the bicomponent glue is applied before the implantation and/or the implant positioning within a sufficiently short timeframe for the bicomponent glue not to crosslink before the implantation and/or the implant positioning.

In an embodiment of the bicomponent glue for therapeutical and/or surgical use according to the invention, the bicomponent glue is applied before the implantation and/or the implant positioning within a sufficiently short timeframe for the bicomponent glue to still be reactive.

In an embodiment of the bicomponent glue for therapeutical and/or surgical use according to the invention, the bicomponent glue is applied up to 15 minutes before the implantation and/or the implant positioning.

In an embodiment of the bicomponent glue for therapeutical and/or surgical use according to the invention, the bicomponent glue is applied up to 10 minutes before the implantation and/or the implant positioning.

In an embodiment of the bicomponent glue for therapeutical and/or surgical use according to the invention, the bicomponent glue is applied up to 5 minutes before the implantation and/or the implant positioning.

In an embodiment of the bicomponent glue for therapeutical and/or surgical use according to the invention, the bicomponent glue is applied up to 1 minutes before the implantation and/or the implant positioning.

In an embodiment of the bicomponent glue for therapeutical and/or surgical use according to the invention, the bicomponent glue is applied during the implantation and/or the implant positioning.

In an embodiment of the bicomponent glue for therapeutical and/or surgical use according to the invention, the bicomponent glue is applied after the implantation and/or the implant positioning.

In an embodiment of the bicomponent glue for therapeutical and/or surgical use according to the invention, the bicomponent glue is applied up to 30 minutes after the implantation and/or the implant positioning.

In an embodiment of the bicomponent glue for therapeutical and/or surgical use according to the invention, the bicomponent glue is applied up to 15 minutes after the implantation and/or the implant positioning.

In an embodiment of the bicomponent glue for therapeutical and/or surgical use according to the invention, the bicomponent glue is applied up to 10 minutes after the implantation and/or the implant positioning.

In an embodiment of the bicomponent glue for therapeutical and/or surgical use according to the invention, the bicomponent glue is applied up to 5 minutes after the implantation and/or the implant positioning.

In an embodiment of the bicomponent glue for therapeutical and/or surgical use according to the invention, the bicomponent glue is applied up to 3 minutes after the implantation and/or the implant positioning.

In an embodiment of the bicomponent glue for therapeutical and/or surgical use according to the invention, the bicomponent glue is applied before, during and/or after the implantation.

In an embodiment of the bicomponent glue for therapeutical and/or surgical use according to the invention, the bicomponent glue is applied before the implantation.

In an embodiment of the bicomponent glue for therapeutical and/or surgical use according to the invention, the bicomponent glue is applied before the implantation within a sufficiently short timeframe for the bicomponent glue not to crosslink before the implantation.

In an embodiment of the bicomponent glue for therapeutical and/or surgical use according to the invention, the bicomponent glue is applied before the implantation within a sufficiently short timeframe for the bicomponent glue to still be reactive.

In an embodiment of the bicomponent glue for therapeutical and/or surgical use according to the invention, the bicomponent glue is applied up to 15 minutes before the implantation.

In an embodiment of the bicomponent glue for therapeutical and/or surgical use according to the invention, the bicomponent glue is applied up to 10 minutes before the implantation.

In an embodiment of the bicomponent glue for therapeutical and/or surgical use according to the invention, the bicomponent glue is applied up to 5 minutes before the implantation.

In an embodiment of the bicomponent glue for therapeutical and/or surgical use according to the invention, the bicomponent glue is applied up to 1 minutes before the implantation.

In an embodiment of the bicomponent glue for therapeutical and/or surgical use according to the invention, the bicomponent glue is applied during the implantation.

In an embodiment of the bicomponent glue for therapeutical and/or surgical use according to the invention, the bicomponent glue is applied after the implantation.

In an embodiment of the bicomponent glue for therapeutical and/or surgical use according to the invention, the bicomponent glue is applied up to 30 minutes after the implantation.

In an embodiment of the bicomponent glue for therapeutical and/or surgical use according to the invention, the bicomponent glue is applied up to 15 minutes after the implantation.

In an embodiment of the bicomponent glue for therapeutical and/or surgical use according to the invention, the bicomponent glue is applied up to 10 minutes after the implantation.

In an embodiment of the bicomponent glue for therapeutical and/or surgical use according to the invention, the bicomponent glue is applied up to 5 minutes after the implantation.

In an embodiment of the bicomponent glue for therapeutical and/or surgical use according to the invention, the bicomponent glue is applied up to 3 minutes after the implantation.

In an embodiment of the bicomponent glue for therapeutical and/or surgical use according to the invention, the bicomponent glue is applied before, during and/or after the implant positioning.

In an embodiment of the bicomponent glue for therapeutical and/or surgical use according to the invention, the bicomponent glue is applied before the implant positioning.

In an embodiment of the bicomponent glue for therapeutical and/or surgical use according to the invention, the bicomponent glue is applied before the implant positioning within a sufficiently short timeframe for the bicomponent glue not to crosslink before the implant positioning.

In an embodiment of the bicomponent glue for therapeutical and/or surgical use according to the invention, the bicomponent glue is applied before the implant positioning within a sufficiently short timeframe for the bicomponent glue to still be reactive.

In an embodiment of the bicomponent glue for therapeutical and/or surgical use according to the invention, the bicomponent glue is applied up to 15 minutes before the implant positioning.

In an embodiment of the bicomponent glue for therapeutical and/or surgical use according to the invention, the bicomponent glue is applied up to 10 minutes before the implant positioning.

In an embodiment of the bicomponent glue for therapeutical and/or surgical use according to the invention, the bicomponent glue is applied up to 5 minutes before the implant positioning.

In an embodiment of the bicomponent glue for therapeutical and/or surgical use according to the invention, the bicomponent glue is applied up to 1 minutes before the implant positioning.

In an embodiment of the bicomponent glue for therapeutical and/or surgical use according to the invention, the bicomponent glue is applied during the implant positioning.

In an embodiment of the bicomponent glue for therapeutical and/or surgical use according to the invention, the bicomponent glue is applied after the implant positioning.

In an embodiment of the bicomponent glue for therapeutical and/or surgical use according to the invention, the bicomponent glue is applied up to 30 minutes after the implant positioning.

In an embodiment of the bicomponent glue for therapeutical and/or surgical use according to the invention, the bicomponent glue is applied up to 15 minutes after the implant positioning.

In an embodiment of the bicomponent glue for therapeutical and/or surgical use according to the invention, the bicomponent glue is applied up to 10 minutes after the implant positioning.

In an embodiment of the bicomponent glue for therapeutical and/or surgical use according to the invention, the bicomponent glue is applied up to 5 minutes after the implant positioning.

In an embodiment of the bicomponent glue for therapeutical and/or surgical use according to the invention, the bicomponent glue is applied up to 3 minutes after the implant positioning.

The invention also relates on a process for improving adhesion and/or immobilizing in vivo an implant onto biological tissue of the recipient patient, characterized in that it comprises the step of applying the bicomponent glue according to the invention on the surface of the implant, on the surface of the biological tissue of the recipient patient and/or on the contact area.

In an embodiment of the process for improving adhesion and/or immobilizing in vivo an implant onto biological tissue of the recipient patient according to the invention, said process comprises the step of applying the bicomponent glue according to the invention on the surface of the implant, on the surface of the biological tissue of the recipient patient and/or on the contact area.

In an embodiment of the process for improving adhesion and/or immobilizing in vivo an implant onto biological tissue of the recipient patient according to the invention, said process comprises the step of applying the bicomponent glue according to the invention on the surface of the implant, on the surface of the biological tissue of the recipient patient and/or on the contact area.

In an embodiment of the process for improving adhesion and/or immobilizing in vivo an implant onto biological tissue of the recipient patient according to the invention, said process comprises a step of suturing the implant to the biological tissue.

In an embodiment of the process for improving adhesion and/or immobilizing in vivo an implant onto biological tissue of the recipient patient according to the invention, said process comprises a step of suturing 2 to 4 points on the implant to the biological tissue.

In an embodiment of the process for improving adhesion and/or immobilizing in vivo an implant onto biological tissue of the recipient patient according to the invention, said process comprises a step of suturing 2 to 4 points on the edge of the implant to the biological tissue.

In an embodiment of the process for improving adhesion and/or immobilizing in vivo an implant onto biological tissue of the recipient patient according to the invention, the step of suturing the implant to the biological tissue occurs before or after the step of applying the bicomponent glue.
The invention also concerns the process for improving adhesion and/or immobilizing in vivo an implant onto biological tissue of the recipient patient according to the invention,

The invention also concerns the process for improving adhesion and/or immobilizing in vivo an implant onto biological tissue of the recipient patient according to the invention, for improving adhesion and/or immobilizing *in vivo* a hydrogel onto biological tissue of the recipient patient and/or a biological tissue graft onto biological tissue of the recipient patient.

The invention also concerns the process for improving adhesion and/or immobilizing in vivo an implant onto biological tissue of the recipient patient according to the invention, for improving adhesion *in vivo* of a hydrogel onto biological tissue of the recipient patient and/or a biological tissue graft onto biological tissue of the recipient patient.

The invention also concerns the process for improving adhesion and/or immobilizing in vivo an implant onto biological tissue of the recipient patient according to the invention, for immobilizing *in vivo* a hydrogel onto biological tissue of the recipient patient and/or a biological tissue graft onto biological tissue of the recipient patient.

In an embodiment of the process for improving adhesion and/or immobilizing in vivo an implant onto biological tissue of the recipient patient according to the invention, the implant is a biological tissue.

In an embodiment of the process for improving adhesion and/or immobilizing in vivo an implant onto biological tissue of the recipient patient according to the invention, the implant comprises active ingredients.

In an embodiment of the process for improving adhesion and/or immobilizing in vivo an implant onto biological tissue of the recipient patient according to the invention, the active ingredient is secreting cells.

In an embodiment of the process for improving adhesion and/or immobilizing in vivo an implant onto biological tissue of the recipient patient according to the invention, the active ingredient is insulin secreting cells.

In an embodiment of the process for improving adhesion and/or immobilizing in vivo an implant onto biological tissue of the recipient patient according to the invention, the active ingredient is Factor VIII or Factor IX secreting cells.

In an embodiment of the process for improving adhesion and/or immobilizing in vivo an implant onto biological tissue of the recipient patient according to the invention, the active ingredient is β-glucocerebrosidase secreting cells.

In an embodiment of the process for improving adhesion and/or immobilizing in vivo an implant onto biological tissue of the recipient patient according to the invention, the implant is an organ.

In an embodiment of the process for improving adhesion and/or immobilizing in vivo an implant onto biological tissue of the recipient patient according to the invention, the implant is an organ in the context of an organ transplant.

In an embodiment of the process for improving adhesion and/or immobilizing in vivo an implant onto biological tissue of the recipient patient according to the invention, the implant is an organ in the context of an organ transplant selected from the group consisting of a liver transplant, a lung transplant, a pancreas transplant, a cornea transplant, an organ system transplant, a bone marrow transplant, a pancreatic islet cell transplant, a stem cell transplant, a skin tissue transplant, a skin cell transplant, and a xenotransplant.

In an embodiment of the process for improving adhesion and/or immobilizing in vivo an implant onto biological tissue of the recipient patient according to the invention, the implant is an artificial organ.

In an embodiment of the process for improving adhesion and/or immobilizing in vivo an implant onto biological tissue of the recipient patient according to the invention, the implant is an artificial pancreas.

In an embodiment of the process for improving adhesion and/or immobilizing in vivo an implant onto biological tissue of the recipient patient according to the invention, the artificial pancreas is a hydrogel.

In an embodiment of the process for improving adhesion and/or immobilizing in vivo an implant onto biological tissue of the recipient patient according to the invention, the artificial pancreas is a hydrogel as defined above.

In an embodiment of the process for improving adhesion and/or immobilizing in vivo an implant onto biological tissue of the recipient patient according to the invention, the artificial pancreas is a hydrogel comprising active ingredients.

In an embodiment of the process for improving adhesion and/or immobilizing in vivo an implant onto biological tissue of the recipient patient according to the invention, the artificial pancreas is a hydrogel comprising secreting cells.

In an embodiment of the process for improving adhesion and/or immobilizing in vivo an implant onto biological tissue of the recipient patient according to the invention, the artificial pancreas is a hydrogel comprising secreting cells as defined above.

In an embodiment of the process for improving adhesion and/or immobilizing in vivo an implant onto biological tissue of the recipient patient according to the invention, the artificial pancreas is a hydrogel comprising insulin secreting cells.

In an embodiment of the process for improving adhesion and/or immobilizing in vivo an implant onto biological tissue of the recipient patient according to the invention, the artificial pancreas is a hydrogel comprising Factor VIII or Factor IX secreting cells.

In an embodiment of the process for improving adhesion and/or immobilizing in vivo an implant onto biological tissue of the recipient patient according to the invention, the artificial pancreas is a hydrogel comprising β-glucocerebrosidase secreting cells.

In an embodiment of the process for improving adhesion and/or immobilizing in vivo an implant onto biological tissue of the recipient patient according to the invention, the implant is a hydrogel.

In an embodiment of the process for improving adhesion and/or immobilizing in vivo an implant onto biological tissue of the recipient patient according to the invention, the implant is a hydrogel as defined above.

In an embodiment of the process for improving adhesion and/or immobilizing in vivo an implant onto biological tissue of the recipient patient according to the invention, the implant is a hydrogel comprising active ingredients.

In an embodiment of the process for improving adhesion and/or immobilizing in vivo an implant onto biological tissue of the recipient patient according to the invention, the implant is a hydrogel comprising active ingredients.

In an embodiment of the process for improving adhesion and/or immobilizing in vivo an implant onto biological tissue of the recipient patient according to the invention, the implant is a hydrogel comprising secreting cells.

In an embodiment of the process for improving adhesion and/or immobilizing in vivo an implant onto biological tissue of the recipient patient according to the invention, the implant is a hydrogel comprising secreting cells as defined above.

In an embodiment of the process for improving adhesion and/or immobilizing in vivo an implant onto biological tissue of the recipient patient according to the invention, the hydrogel comprising secreting cells comprise a crosslinked dextran polymer Dx bearing anionic groups wherein the at least divalent radical L(-)_{I} is covalently bound to the dextran polymer backbone with I W radicals, wherein,
- L(-)i is a linear or branched polyether
- I is the valence of L and the number of W radicals bound to the dextran polymer and is an integer comprised from 2 to 8 (2 ≤ I ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment of the process for improving adhesion and/or immobilizing in vivo an implant onto biological tissue of the recipient patient according to the invention, the hydrogel comprising secreting cells further comprises a non crosslinked hyaluronate in the form of a solution.

In an embodiment of the process for improving adhesion and/or immobilizing in vivo an implant onto biological tissue of the recipient patient according to the invention, the hydrogel comprising insulin secreting cells is chosen amongst the dextran polymer Dx bearing anionic groups wherein the at least divalent radical L(-)i is covalently bound to the dextran polymer backbone with I -W- radicals, wherein,
- L(-)_{I} is a linear or branched polyether, or L(-)_{I} is a linear or branched poly(oxazoline)
- I is the valence of L and the number of -W- radicals bound to the dextran polymer and is an integer comprised from 2 to 8 (2 ≤ I ≤ 8),
- -W- is a radical comprising at least a radical alkyl linear or branched and optionally comprising heteroatoms such as oxygen, nitrogen or sulfur, aromatic cycles, polyether or poly(oxazoline) derivatives and that does not comprise two or more alpha aminoacid residues in particular linked by peptidic bond(s).

In an embodiment of the process for improving adhesion and/or immobilizing in vivo an implant onto biological tissue of the recipient patient according to the invention, the hydrogel comprising secreting cells is characterized in that Tan δ is lower than 1.

In an embodiment of the process for improving adhesion and/or immobilizing in vivo an implant onto biological tissue of the recipient patient according to the invention, the hydrogel comprising secreting cells is a transparent hydrogel.

In an embodiment of the process for improving adhesion and/or immobilizing in vivo an implant onto biological tissue of the recipient patient according to the invention, the hydrogel comprising secreting cells is a translucid hydrogel.

In an embodiment of the process for improving adhesion and/or immobilizing in vivo an implant onto biological tissue of the recipient patient according to the invention, after swelling in water the cross-linked dextran polymer concentration is comprised from 0.01 to 0.2 g/g.

In an embodiment of the process for improving adhesion and/or immobilizing in vivo an implant onto biological tissue of the recipient patient according to the invention, the hydrogel comprising secreting cells is characterized in that its Young modulus is comprised between 1 to 200 kPa.

In an embodiment of the process for improving adhesion and/or immobilizing in vivo an implant onto biological tissue of the recipient patient according to the invention, the hydrogel comprising secreting cells is characterized in that its G' is comprised from 0.5 to 70 kPa.

In an embodiment of the process for improving adhesion and/or immobilizing in vivo an implant onto biological tissue of the recipient patient according to the invention, the hydrogel comprising secreting cells is characterized in that its compression deformation at break is of more than or equal to 10 %.

In an embodiment of the process for improving adhesion and/or immobilizing in vivo an implant onto biological tissue of the recipient patient according to the invention, the hydrogel comprising secreting cells is characterized in that it has a swelling ratio of more than 0.7.

In an embodiment of the process for improving adhesion and/or immobilizing in vivo an implant onto biological tissue of the recipient patient according to the invention, the hydrogel comprising secreting cells is characterized in that it has a water content of at least 80 wt%.

In an embodiment of the process for improving adhesion and/or immobilizing in vivo an implant onto biological tissue of the recipient patient according to the invention, the hydrogel comprising secreting cells is characterized in that the secreting cells are proteins, hormones or peptide secreting cells.

In an embodiment of the process for improving adhesion and/or immobilizing in vivo an implant onto biological tissue of the recipient patient according to the invention, the hydrogel comprising secreting cells is characterized in that the secreting cells are chosen from:
- insulin secreting cells for diabetes treatment
- Factor VIII or Factor IX secreting cells for hemophilia treatment andβ-glucocerebrosidase secreting cells for Gaucher disease.

In an embodiment of the process for improving adhesion and/or immobilizing in vivo an implant onto biological tissue of the recipient patient according to the invention, the hydrogel comprising secreting cells is characterized in that the secreting cells are pseudoislets.

In an embodiment of the process for improving adhesion and/or immobilizing in vivo an implant onto biological tissue of the recipient patient according to the invention, the artificial pancreas further comprises a ring and/or a net.

In an embodiment of the process for improving adhesion and/or immobilizing in vivo an implant onto biological tissue of the recipient patient according to the invention, the artificial pancreas is characterized in that it is a parallelepiped rectangle with round corner.

In an embodiment of the process for improving adhesion and/or immobilizing in vivo an implant onto biological tissue of the recipient patient according to the invention, the artificial pancreas is characterized in that its thickness is of less than 3 000 µm.

In an embodiment of the process for improving adhesion and/or immobilizing in vivo an implant onto biological tissue of the recipient patient according to the invention, the artificial pancreas is characterized in that it has total surface of between 10 cm² to 200 cm².

In an embodiment of the process for improving adhesion and/or immobilizing in vivo an implant onto biological tissue of the recipient patient according to the invention, the artificial pancreas is characterized in that it comprises from 0.5 to 20 ml of hydrogel comprising insulin secreting cells.

In an embodiment of the process for improving adhesion and/or immobilizing in vivo an implant onto biological tissue of the recipient patient according to the invention, the artificial pancreas is characterized in that the ring has an internal diameter of 10 to 100 mm.

In an embodiment of the process for improving adhesion and/or immobilizing in vivo an implant onto biological tissue of the recipient patient according to the invention, the artificial pancreas is characterized in that the ring is a parallelepiped rectangle with rounded corner.

In an embodiment of the process for improving adhesion and/or immobilizing in vivo an implant onto biological tissue of the recipient patient according to the invention, the artificial pancreas is characterized in that the ring material is a bioinert material.

In an embodiment of the process for improving adhesion and/or immobilizing in vivo an implant onto biological tissue of the recipient patient according to the invention, the artificial pancreas is characterized in that the ring material is a biocompatible elastomer.

In an embodiment of the process for improving adhesion and/or immobilizing in vivo an implant onto biological tissue of the recipient patient according to the invention, the artificial pancreas is characterized in that the ring material is chosen from the group consisting of silicone, in particular PDMS, polyurethanes, polyether, polyether polyester copolymers and polypropylene oxide.

In an embodiment of the process for improving adhesion and/or immobilizing in vivo an implant onto biological tissue of the recipient patient according to the invention, the artificial pancreas is characterized in that the net is non-biodegradable.

In an embodiment of the process for improving adhesion and/or immobilizing in vivo an implant onto biological tissue of the recipient patient according to the invention, the artificial pancreas is characterized in that the net is biocompatible.

In an embodiment of the process for improving adhesion and/or immobilizing in vivo an implant onto biological tissue of the recipient patient according to the invention, the artificial pancreas is characterized in that the net is non absorbable.

In an embodiment of the process for improving adhesion and/or immobilizing in vivo an implant onto biological tissue of the recipient patient according to the invention, the artificial pancreas is characterized in that the net is a surgical mesh.

In an embodiment of the process for improving adhesion and/or immobilizing in vivo an implant onto biological tissue of the recipient patient according to the invention, the artificial pancreas is characterized in that the filament material of the net material is chosen among the group consisting of Polypropylene, Polyethylene, polyester, in particular PET, PTFE, PVDF (polyvinylidene fluoride) and ePVDF (extended PVDF).

In an embodiment of the process for improving adhesion and/or immobilizing in vivo an implant onto biological tissue of the recipient patient according to the invention, the artificial pancreas is characterized in that the net has a thickness ranging from 50 to 500 µm.

In an embodiment of the process for improving adhesion and/or immobilizing in vivo an implant onto biological tissue of the recipient patient according to the invention, the artificial pancreas is characterized in that the pore size of the net is ranging from 0.4 to 4 mm.

In an embodiment of the process for improving adhesion and/or immobilizing in vivo an implant onto biological tissue of the recipient patient according to the invention, the artificial pancreas is characterized in that fabric of the net is chosen from the group consisting of knitted fabric, warp knitted fabric, woven fabric, non-woven fabric.

In an embodiment of the process for improving adhesion and/or immobilizing in vivo an implant onto biological tissue of the recipient patient according to the invention, the artificial pancreas is characterized in that it is obtained by the following process:
- Hydrogel compositions are incorporated in the Ring Mesh constructs, the concentrated polymer solutions are mixed with a pipette and a controlled volume of the mixture is introduced in a ring mesh construct adhering to a glass slide,
- Crosslinking leading to gelation is carried out, then the Ring Mesh + Hydrogel composition is introduced in a Tris 150 mM /NaCl 30 mM / Cystein 10 mM solution at pH 8 or in PBS at pH 7.4,
- the hydrogel is rinsed with PBS solution without cysteine and further immersed in the PBS solution overnight at 37°C and then. The hydrogel piece is stored in PBS solution at 4°C until being used.

In an embodiment of the process for improving adhesion and/or immobilizing in vivo an implant onto biological tissue of the recipient patient according to the invention, the implant is sutured to the biological tissue of the recipient patient.

In an embodiment of the process for improving adhesion and/or immobilizing in vivo an implant onto biological tissue of the recipient patient according to the invention, the biological tissue of the recipient patient is a connective tissue.

In an embodiment of the process for improving adhesion and/or immobilizing in vivo an implant onto biological tissue of the recipient patient according to the invention, the biological tissue of the recipient patient is a mucous membrane.

In an embodiment of the process for improving adhesion and/or immobilizing in vivo an implant onto biological tissue of the recipient patient according to the invention, the recipient patient is a mammal.

In an embodiment of the process for improving adhesion and/or immobilizing in vivo an implant onto biological tissue of the recipient patient according to the invention, the recipient patient is a human.

In an embodiment of the process for improving adhesion and/or immobilizing in vivo an implant onto biological tissue of the recipient patient according to the invention, the recipient patient has a disorder or disease due to lack or malfunction of endocrine function of pancreas organ.

In an embodiment of the process for improving adhesion and/or immobilizing in vivo an implant onto biological tissue of the recipient patient according to the invention, the recipient patient has diabetes.

In an embodiment of the process for improving adhesion and/or immobilizing in vivo an implant onto biological tissue of the recipient patient according to the invention, the bicomponent glue is applied on the surface of the implant, on the surface of the biological tissue of the recipient patient and/or on the contact area.

In an embodiment of the process for improving adhesion and/or immobilizing in vivo an implant onto biological tissue of the recipient patient according to the invention, the bicomponent glue is applied on the surface of the implant.

In an embodiment of the process for improving adhesion and/or immobilizing in vivo an implant onto biological tissue of the recipient patient according to the invention, the bicomponent glue is applied on the surface of the biological tissue of the recipient patient.

In an embodiment of the process for improving adhesion and/or immobilizing in vivo an implant onto biological tissue of the recipient patient according to the invention, the bicomponent glue is applied on the contact area.

In an embodiment of the process for improving adhesion and/or immobilizing in vivo an implant onto biological tissue of the recipient patient according to the invention, the quantity of bicomponent glue applied per square centimeters of contact area is from 0,01 µL/cm² to 500 µL/cm².

In an embodiment of the process for improving adhesion and/or immobilizing in vivo an implant onto biological tissue of the recipient patient according to the invention, the quantity of bicomponent glue applied per square centimeters of contact area is from 0,1 µL/cm² to 250 µL/cm².

In an embodiment of the process for improving adhesion and/or immobilizing in vivo an implant onto biological tissue of the recipient patient according to the invention, the quantity of bicomponent glue applied per square centimeters of contact area is from 1 µL/cm² to 100 µL/cm².

In an embodiment of the process for improving adhesion and/or immobilizing in vivo an implant onto biological tissue of the recipient patient according to the invention, the quantity of bicomponent glue applied per square centimeters of contact area is from 10 µL/cm² to 50 µL/cm².

In an embodiment of the process for improving adhesion and/or immobilizing in vivo an implant onto biological tissue of the recipient patient according to the invention, the quantity of bicomponent glue applied per square centimeters of contact area is from 20 µL/cm² to 30 µL/cm².

In an embodiment of the process for improving adhesion and/or immobilizing in vivo an implant onto biological tissue of the recipient patient according to the invention, the bicomponent glue is applied before, during and/or after the implantation and/or the implant positioning.

In an embodiment of the process for improving adhesion and/or immobilizing in vivo an implant onto biological tissue of the recipient patient according to the invention, the bicomponent glue is applied before the implantation and/or the implant positioning.

In an embodiment of the process for improving adhesion and/or immobilizing in vivo an implant onto biological tissue of the recipient patient according to the invention, the bicomponent glue is applied before the implantation within a sufficiently short timeframe for the bicomponent glue not to crosslink before the implantation and/or the implant positioning.

In an embodiment of the process for improving adhesion and/or immobilizing in vivo an implant onto biological tissue of the recipient patient according to the invention, the bicomponent glue is applied before the implantation and/or the implant positioning within a sufficiently short timeframe for the bicomponent glue to still be reactive.

In an embodiment of the process for improving adhesion and/or immobilizing in vivo an implant onto biological tissue of the recipient patient according to the invention, the bicomponent glue is applied up to 15 minutes before the implantation and/or the implant positioning.

In an embodiment of the process for improving adhesion and/or immobilizing in vivo an implant onto biological tissue of the recipient patient according to the invention, the bicomponent glue is applied up to 10 minutes before the implantation and/or the implant positioning.

In an embodiment of the process for improving adhesion and/or immobilizing in vivo an implant onto biological tissue of the recipient patient according to the invention, the bicomponent glue is applied up to 5 minutes before the implantation and/or the implant positioning.

In an embodiment of the process for improving adhesion and/or immobilizing in vivo an implant onto biological tissue of the recipient patient according to the invention, the bicomponent glue is applied up to 1 minutes before the implantation and/or the implant positioning.

In an embodiment of the process for improving adhesion and/or immobilizing in vivo an implant onto biological tissue of the recipient patient according to the invention, the bicomponent glue is applied during the implantation and/or the implant positioning.

In an embodiment of the process for improving adhesion and/or immobilizing in vivo an implant onto biological tissue of the recipient patient according to the invention, the bicomponent glue is applied after the implantation and/or the implant positioning.

In an embodiment of the process for improving adhesion and/or immobilizing in vivo an implant onto biological tissue of the recipient patient according to the invention, the bicomponent glue is applied up to 30 minutes after the implantation and/or the implant positioning.

In an embodiment of the process for improving adhesion and/or immobilizing in vivo an implant onto biological tissue of the recipient patient according to the invention, the bicomponent glue is applied up to 15 minutes after the implantation and/or the implant positioning.

In an embodiment of the process for improving adhesion and/or immobilizing in vivo an implant onto biological tissue of the recipient patient according to the invention, the bicomponent glue is applied up to 10 minutes after the implantation and/or the implant positioning.

In an embodiment of the process for improving adhesion and/or immobilizing in vivo an implant onto biological tissue of the recipient patient according to the invention, the bicomponent glue is applied up to 5 minutes after the implantation and/or the implant positioning.

In an embodiment of the process for improving adhesion and/or immobilizing in vivo an implant onto biological tissue of the recipient patient according to the invention, the bicomponent glue is applied up to 3 minutes after the implantation and/or the implant positioning.

In an embodiment of the process for improving adhesion and/or immobilizing in vivo an implant onto biological tissue of the recipient patient according to the invention, the bicomponent glue is applied before, during and/or after the implantation.

In an embodiment of the process for improving adhesion and/or immobilizing in vivo an implant onto biological tissue of the recipient patient according to the invention, the bicomponent glue is applied before the implantation.

In an embodiment of the process for improving adhesion and/or immobilizing in vivo an implant onto biological tissue of the recipient patient according to the invention, the bicomponent glue is applied before the implantation within a sufficiently short timeframe for the bicomponent glue not to crosslink before the implantation.

In an embodiment of the process for improving adhesion and/or immobilizing in vivo an implant onto biological tissue of the recipient patient according to the invention, the bicomponent glue is applied before the implantation within a sufficiently short timeframe for the bicomponent glue to still be reactive.

In an embodiment of the process for improving adhesion and/or immobilizing in vivo an implant onto biological tissue of the recipient patient according to the invention, the bicomponent glue is applied up to 15 minutes before the implantation.

In an embodiment of the process for improving adhesion and/or immobilizing in vivo an implant onto biological tissue of the recipient patient according to the invention, the bicomponent glue is applied up to 10 minutes before the implantation.

In an embodiment of the process for improving adhesion and/or immobilizing in vivo an implant onto biological tissue of the recipient patient according to the invention, the bicomponent glue is applied up to 5 minutes before the implantation.

In an embodiment of the process for improving adhesion and/or immobilizing in vivo an implant onto biological tissue of the recipient patient according to the invention, the bicomponent glue is applied up to 1 minutes before the implantation.

In an embodiment of the process for improving adhesion and/or immobilizing in vivo an implant onto biological tissue of the recipient patient according to the invention, the bicomponent glue is applied during the implantation.

In an embodiment of the process for improving adhesion and/or immobilizing in vivo an implant onto biological tissue of the recipient patient according to the invention, the bicomponent glue is applied after the implantation.

In an embodiment of the process for improving adhesion and/or immobilizing in vivo an implant onto biological tissue of the recipient patient according to the invention, the bicomponent glue is applied up to 30 minutes after the implantation.

In an embodiment of the process for improving adhesion and/or immobilizing in vivo an implant onto biological tissue of the recipient patient according to the invention, the bicomponent glue is applied up to 15 minutes after the implantation.

In an embodiment of the process for improving adhesion and/or immobilizing in vivo an implant onto biological tissue of the recipient patient according to the invention, the bicomponent glue is applied up to 10 minutes after the implantation.

In an embodiment of the process for improving adhesion and/or immobilizing in vivo an implant onto biological tissue of the recipient patient according to the invention, the bicomponent glue is applied up to 5 minutes after the implantation.

In an embodiment of the process for improving adhesion and/or immobilizing in vivo an implant onto biological tissue of the recipient patient according to the invention, the bicomponent glue is applied up to 3 minutes after the implantation.

In an embodiment of the process for improving adhesion and/or immobilizing in vivo an implant onto biological tissue of the recipient patient according to the invention, the bicomponent glue is applied before, during and/or after the implant positioning.

In an embodiment of the process for improving adhesion and/or immobilizing in vivo an implant onto biological tissue of the recipient patient according to the invention, the bicomponent glue is applied before the implant positioning.

In an embodiment of the process for improving adhesion and/or immobilizing in vivo an implant onto biological tissue of the recipient patient according to the invention, the bicomponent glue is applied before the implant positioning within a sufficiently short timeframe for the bicomponent glue not to crosslink before the implant positioning.

In an embodiment of the process for improving adhesion and/or immobilizing in vivo an implant onto biological tissue of the recipient patient according to the invention, the bicomponent glue is applied before the implant positioning within a sufficiently short timeframe for the bicomponent glue to still be reactive.

In an embodiment of the process for improving adhesion and/or immobilizing in vivo an implant onto biological tissue of the recipient patient according to the invention, the bicomponent glue is applied up to 15 minutes before the implant positioning.

In an embodiment of the process for improving adhesion and/or immobilizing in vivo an implant onto biological tissue of the recipient patient according to the invention, the bicomponent glue is applied up to 10 minutes before the implant positioning.

In an embodiment of the process for improving adhesion and/or immobilizing in vivo an implant onto biological tissue of the recipient patient according to the invention, the bicomponent glue is applied up to 5 minutes before the implant positioning.

In an embodiment of the process for improving adhesion and/or immobilizing in vivo an implant onto biological tissue of the recipient patient according to the invention, the bicomponent glue is applied up to 1 minutes before the implant positioning.

In an embodiment of the process for improving adhesion and/or immobilizing in vivo an implant onto biological tissue of the recipient patient according to the invention, the bicomponent glue is applied during the implant positioning.

In an embodiment of the process for improving adhesion and/or immobilizing in vivo an implant onto biological tissue of the recipient patient according to the invention, the bicomponent glue is applied after the implant positioning.

In an embodiment of the process for improving adhesion and/or immobilizing in vivo an implant onto biological tissue of the recipient patient according to the invention, the bicomponent glue is applied up to 30 minutes after the implant positioning.

In an embodiment of the process for improving adhesion and/or immobilizing in vivo an implant onto biological tissue of the recipient patient according to the invention, the bicomponent glue is applied up to 15 minutes after the implant positioning.

In an embodiment of the process for improving adhesion and/or immobilizing in vivo an implant onto biological tissue of the recipient patient according to the invention, the bicomponent glue is applied up to 10 minutes after the implant positioning.

In an embodiment of the process for improving adhesion and/or immobilizing in vivo an implant onto biological tissue of the recipient patient according to the invention, the bicomponent glue is applied up to 5 minutes after the implant positioning.

In an embodiment of the process for improving adhesion and/or immobilizing in vivo an implant onto biological tissue of the recipient patient according to the invention, the bicomponent glue is applied up to 3 minutes after the implant positioning.

The invention also concerns an implant comprising at least two layers of hydrogels, a first layer of hydrogel comprising biological cells and a second layer of hydrogel, also called sub-layer, comprising a hydrogel as disclosed in the instant specification and which does not comprise biological cells. Said first and second layers adhering together.

In an embodiment the hydrogel comprises a cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a polyethylene glycol chain, or this at least radical being a at least divalent linear, branched or cyclic alkyl radical comprising at least a poly(oxazoline) (POx) chain.

In an embodiment the first layer of hydrogel is also a hydrogel as disclosed in the instant specification.

In an embodiment the first layer of hydrogel and the second layer of hydrogel are different.

In an embodiment the first layer of hydrogel and the second layer of hydrogel are different.

In an embodiment the first layer of hydrogel and the second layer of hydrogel are the same.

In an embodiment the second layer of hydrogel protects the first layer of hydrogel.

In an embodiment, the second layer of hydrogel is coating the first layer of hydrogel, totally or in part.

In an embodiment, the implant is a wire or a tube where the first layer is the core of the wire or of the tube and the second layer is coating completely the first layer. The extremities of the layer may be uncoated.

In an embodiment, the second layer is defining a surface protecting the first layer from being in contact with the outside.

In an embodiment, the second layer is surrounding more than 90 %, more than 95 % of the surface of the first layer.

In an embodiment, the implant is having:
- an upper face of more than 1 cm²
- a bottom face parallel to the upper face, the distance between the upper and bottom faces defining a thickness of the device said thickness being smaller than 5 mm,
- at least one lateral face between the upper and bottom faces,
- the first layer occupying the volume between the upper face and the second layer and the second layer occupying the volume between the first layer and the upper face.

In an embodiment, the precursors of the first layer have been casted on the surface of the second layer.

In an embodiment, the thickness of the second layer is between 50 µm to 500 µm.

In an embodiment the volume of the second layer is between 5 to 25% of the total volume of the implant.

Process for obtaining an implant comprising at least two layers of hydrogels as defined above:
- casting a mixture of precursors of hydrogel to form the second layer,
- when the second layer began gelifying, casting a mixture of precursors of the first layer and biological,
- wait for the gelification to be finished, and
- unmold the bilayer hydrogel.

Device or implant comprising hydrogel as defined in the instant specification obtainable or obtained by 3D printing. The mixture of precursors being mixed before 3D printing.

In an embodiment, the hydrogel comprises the cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a polyethylene glycol chain, or this at least radical being a at least divalent linear, branched or cyclic alkyl radical comprising at least a poly(oxazoline) (POx) chain.

In an embodiment the device or implant has the shape of a net.

Process of treatment of a hydrogel comprising biological cells said process comprising the step of treatment of said hydrogel comprising cells by anti-inflammatory compound(s).

Process of treatment a hydrogel comprising biological cells, as defined in the specification, said process comprising the step of treatment of said hydrogel comprising cells by anti-inflammatory compound(s).

In an embodiment, the hydrogel comprises a cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a polyethylene glycol chain, or this at least radical being a at least divalent linear, branched or cyclic alkyl radical comprising at least a poly(oxazoline) (POx) chain.

Said process may improve resistance to hypoxia, in particular to hypoxia after said hydrogel comprising cells is implanted in vivo.

Said process may improve resistance to cytokines, in particular after implantation in vivo.

In an embodiment, the anti-inflammatory compound is neutralizing the biological activity of IL1-alpha and/or IL1-beta.

In a preferred embodiment, the anti-inflammatory compound is anakinra.

In an embodiment, the cells encapsulated in the hydrogel are treated by adding anakinra to the culture medium at a final concentration of between 1 to 50 µg/ml, in particular 5 to 15 µg/ml, and more particularly at 10 µg/ml.

In an embodiment, the treatment lasts 24 to 72h.

Hydrogel as defined in the instant specification, wherein the islets have a mean size, as defined in the examples, of less than 200 µm, in particular less than 180 µm, the islets of less than 50 µm being excluded for size distribution analysis.

Hydrogel as defined in the instant specification, wherein the islets are obtained by filtration with a filter of 200 µm pore size,

In an embodiment, the islets are obtained by filtration with a filter of 150 µm pore size.

In an embodiment, the islets are coming from stem cells.

### EXAMPLES

### Part A - CHEMISTRY

### Example A1: Synthesis of substituted dextrans

**Table 1: List of synthesized polysaccharides**

| **Polysaccharides** | **Structure** | |
|---|---|---|
| Polysaccharide 1 | 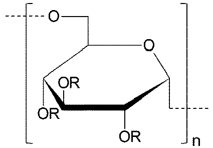 | |
| | Mw (Dextran) = 40 kg/mol | |
| | n = 205 | |
| | R = H or | 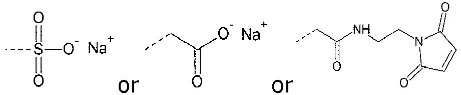 |
| | DS₃ = 1.5 | |
| | DS₂ = 0.8 | |
| | DS₁ = 0.25 | |
| Polysaccharide 2 | 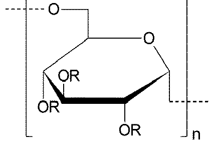 | |
| | Mw (Dextran) = 40 kg/mol | |
| | n = 205 | |
| | R = H or | 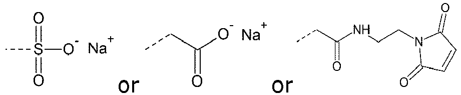 |
| | DS₃ = 1.9 | |
| | DS₂ = 0.5 | |
| | DS₁ = 0.23 | |
| Polysaccharide 3 | 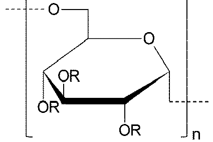 | |
| | Mw (Dextran) = 40 kg/mol | |
| | n = 205 | |
| | R = H or | 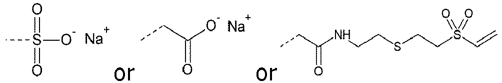 |
| | DS₃ = 1.5 | |
| | DS₂ = 0.8 | |
| | DS₁ = 0.28 | |
| Polysaccharide 4 | 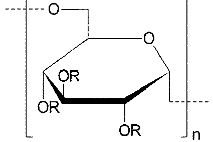 | |
| | Mw (Dextran) = 40 kg/mol | |
| | n = 205 | |
| | R = H or | 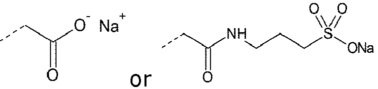 |
| | or | 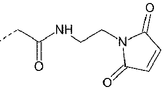 |
| | DS₃ = 1.15 | |
| | DS₂ = 2.1 | |
| | DS₁ = 0.25 | |
| Polysaccharide 5 | 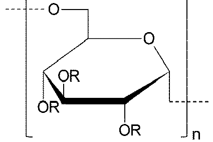 | |
| | Mw (Dextran) = 40 kg/mol | |
| | n = 205 | |
| | R = H or | 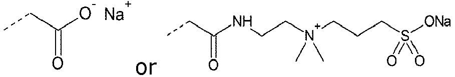 |
| | or 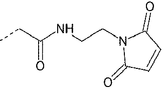 | |
| | DS₃ = 0.7 | |
| | DS₂ = 2.1 | |
| | DS₁ = 0.22 | |
| Polysaccharide 6 | 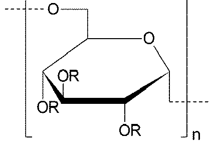 | |
| | Mw (Dextran) = 40 kg/mol | |
| | n = 205 | |
| | R = H or | 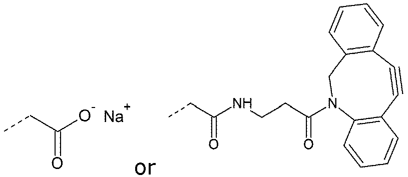 |
| | DS₂ = 2.1 | |
| | DS₁ = 0.28 | |
| Polysaccharide 7 | 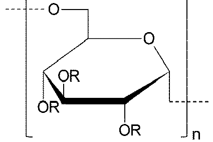 | |
| | Mw (Dextran) = 40 kg/mol | |
| | n = 205 | |
| | | 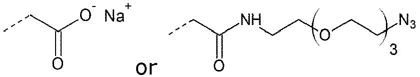 |
| | R = H or | |
| | DS₂ = 2.1 | |
| | DS₁ = 0.28 | |
| Polysaccharide 8 | 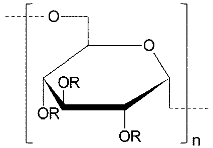 | |
| | Mw (Dextran) = 40 kg/mol | |
| | n = 205 | |
| | | 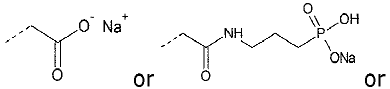 |
| | R = H or | |
| | 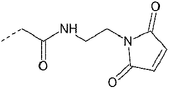 | |
| | DS₃ = 0.05 | |
| | DS₂ = 2.1 | |
| | DS₁ = 0.25 | |
| Polysaccharide 9 | 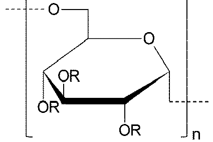 | |
| | Mw (Dextran) = 40 kg/mol | |
| | n = 205 | |
| | R = H or | 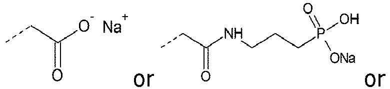 |
| | 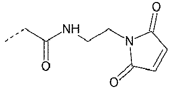 | |
| | DS₃ = 0.75 | |
| | DS₂ = 2.1 | |
| | DS₁ = 0.21 | |
| Polysaccharide 10 | 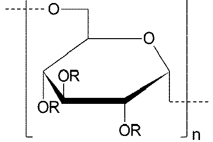 | |
| | Mw (Dextran) = 40 kg/mol | |
| | n = 205 | |
| | R = H or | 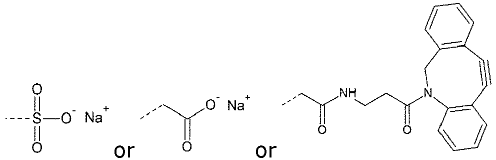 |
| | DS₃ = 1.5 | |
| | DS₂ = 0.8 | |
| | DS₁ = 0.23 | |
| Polysaccharide 11 | 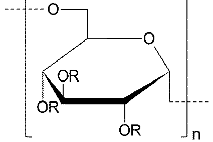 | |
| | Mw (Dextran) = 250 kg/mol | |
| | n = 1100 | |
| | | 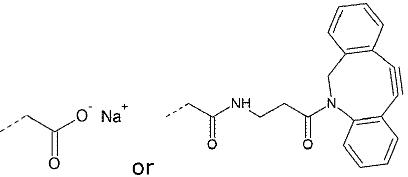 |
| | R = H or | |
| | DS₂ = 2.0 | |
| | DS₁ = 0.06 | |
| Polysaccharide 12 | 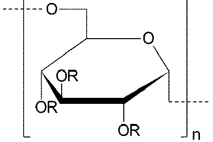 | |
| | Mw (Dextran) = 500 kg/mol | |
| | n = 2200 | |
| | R = H or | 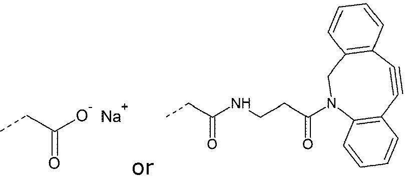 |
| | DS₂ = 2.0 | |
| | DS₁ = 0.06 | |
| Polysaccharide 14 | 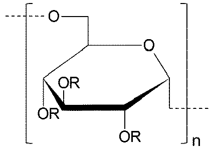 | |
| | Mw (Dextran) = 40 kg/mol | |
| | n = 205 | |
| | R = H or | 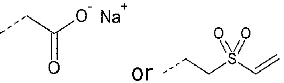 |
| | DS₂ = 1.2 | |
| | DS₁ = 0.17 | |
| Polysaccharide 15 | 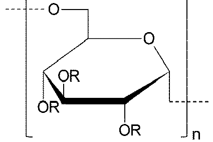 | |
| | Mw (Dextran) = 40 kg/mol | |
| | n = 205 | |
| | R = H or | 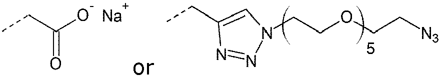 |
| | DS₂ = 1.8 | |
| | DS₁ = 0.24 | |
| Polysaccharide 17 | 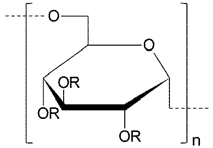 | |
| | Mw (Dextran) = 40 kg/mol | |
| | n = 205 | |
| | R = H or | 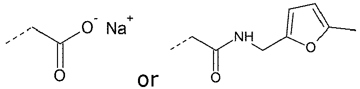 |
| | DS₂ = 2.1 | |
| | DS₁ = 0.30 | |
| Polysaccharide 18 | 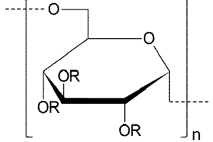 | |
| | Mw (Dextran) = 40 kg/mol | |
| | n = 205 | |
| | R = H or | 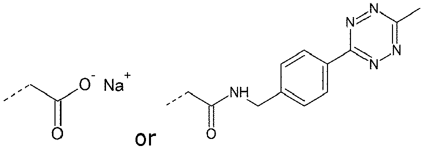 |
| | DS₂ = 2.1 | |
| | DS₁ = 0.25 | |
| Polysaccharide 24 | 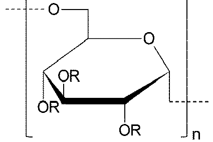 | |
| | Mw (Dextran) = 40 kg/mol | |
| | n = 205 | |
| | R = H or | 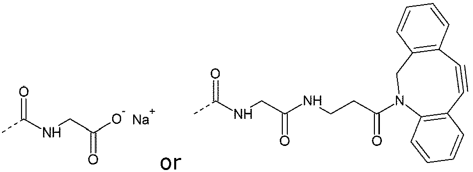 |
| | DS₂ = 2.4 | |
| | DS₁ = 0.28 | |
| Polysaccharide 25 | 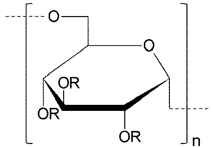 | |
| | Mw (Dextran) = 40 kg/mol | |
| | n = 205 | |
| | R = H or | 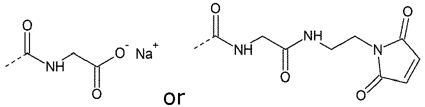 |
| | DS₂ = 2.4 | |
| | DS₁ = 0.26 | |
| Polysaccharide 26 | 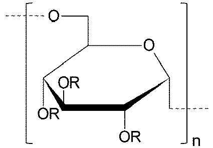 | |
| | Mw (Dextran) = 40 kg/mol | |
| | n = 205 | |
| | R = H or | 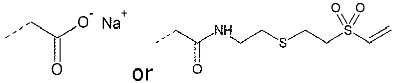 |
| | DS₂ = 2.1 | |
| | DS₁ = 0.25 | |

Polysaccharide 1 to 25 are synthesized as disclosed in PCT/EP2023/069586

### Polysaccharide 26 - Dextran Methyl Carboxylate and Vinyl Sulfone

To 207 g of the solution of polysaccharide 4.1 (48.4 mg/g, DS₂ = 2.1, 10.0 g, 30.28 mmol of glucoside units), 2-hydroxypyridine 1-oxide (HOPO) (1.68 g, 15.14 mmol) is added and the mixture is cooled to 4°C. To this solution are added 2-[[2-(ethenylsulfonyl)ethyl]thio]ethanamine hydrochloride (VS) (2.10 g, 9.08 mmol) (synthesized according: S. A. Stewart et al., Soft Matter, 2018, 14, 8317), Et₃N (1.27 mL, 9.08 mmol) and *N*-ethyl-*N*'-(3-dimethylaminopropyl)carbodiimide hydrochloride (EDC) (2.90 g, 15.14 mmol) and the reaction mixture is stirred between 4°C and 25°C for 2 h. Two additional additions of EDC (2.90 g, 15.14 mmol) are performed every 2 h. The mixture is diluted with NaCl (9 g/L in water), then purified by ultrafiltration on PES membrane (MWCO 5 kDa) against NaCl (9 g/L in water), carbonate buffer pH 9-10, NaCl (9 g/L in water), phosphate buffer pH 7, NaCl (9 g/L in water), and then water. The polysaccharide 26 concentration of the final solution is determined by dry extract, and the degree of substitution with vinyl sulfone is determined by ¹H NMR in D₂O. The final solution is stored at -20°C.

According to the dry extract: [polysaccharide 26] = 21.3 mg/g.

According to ¹H NMR (D₂O), degree of substitution with vinyl sulfone (DS₁) = 0.25.

### Example A2 : Polyethylene glycol derivatives comprising at least two reactive functions

Commercial Polyethylene glycol (PEG) derivatives functionalized with reactive functions were purchased. The reactive functions include thiols ("PEG-SH"), Azides (PEG-N3) and Alcynes (PEG-DBCO). Linear homo bifunctional and multi-arm homofunctional having different molecular weights and bearing different functions were used and are shown in the following Table 2.

**Table 2: List of commercial PEG derivatives used**

| **PEG** | **Chemical Name *(Supplier)*** | **Structure** |
|---|---|---|
| PEG-SH-1 | 2-arm PEG-thiol *(NOF; Ref. SUNBRIGHT DE-034SH)* | 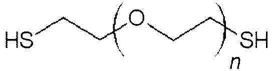 |
| | | Mn (PEG) = 3.4 kg/mol |
| | | n = 77 |
| PEG-SH-2 | 2-arm PEG-thiol *(Sinopeg; Ref. 06020201301)* | 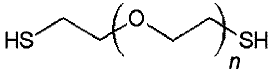 |
| | | Mn (PEG) = 1 kg/mol |
| | | n = 23 |
| PEG-SH-3 | 4-arm PEG-thiol, pentaerythritol core *(NOF; Ref. SUNBRIGHT PTE-050SH)* | 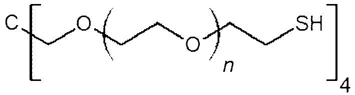 |
| | | Mn (PEG) = 5.2 kg/mol |
| | | n = 30 |
| PEG-SH-4 | 4-arm PEG-thiol, pentaerythritol core *(Jenkem; Ref. 4ARM-SH-20K)* | 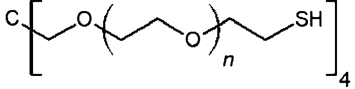 |
| | | Mn (PEG) = 20 kg/mol |
| | | n = 114 |
| PEG-SH-5 | 4-arm PEG-thiol, pentaerythritol core *(Sinopeg; Ref. 06020701315)* | 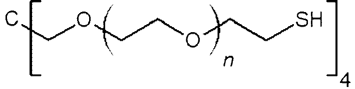 |
| | | Mn (PEG) = 40 kg/mol |
| | | n = 227 |
| PEG-N3-1 | 4-arm PEG-azide, pentaerythritol core *(Jenkem; Ref. 4ARM-AZIDE-20K)* | 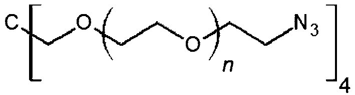 |
| | | Mn (PEG) = 20 kg/mol |
| | | n = 114 |
| PEG-DBCO-1 | 4-arm PEG-DBCO (Dibenzocyclooctyne), pentaerythritol core *(Jenkem; Ref. 4ARM-DBCO-20K)* | 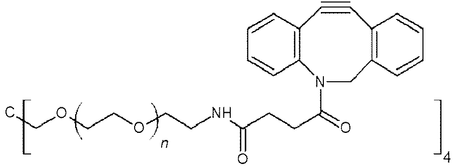 |
| | | Mn (PEG) = 20 kg/mol |
| | | n = 114 |
| PEG-Mal-1 | 4-arm PEG-Maleimide, pentaerythritol core *(Jenkem; Ref. 4ARM-MAL-20K)* | 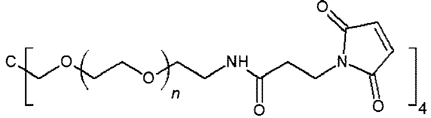 |
| | | Mn (PEG) = 20 kg/mol |
| | | n = 114 |
| PEG-Nb-1 | 4-arm PEG-norbornene(amide), pentaerythritol core *(Sinopeg; Ref. 06020717412)* | 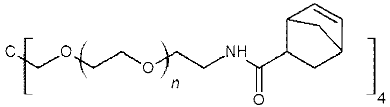 |
| | | Mn (PEG) = 20 kg/mol |
| | | n = 114 |

These PEG derivatives correspond to the precursors of the -L of Formula I.

### Part B - BIOLOGY

### Example B1A: Preparation of pseudoislets

Min-6 cell line (Caltag Medsystems) were cultivated in culture medium indicated in Table 3, in an incubator at 37°C and 5% CO2. Cells were subcultured 3 times a week using 0.05% trypsin / EDTA to detach the cells and diluted 5 times in culture medium.

**Table 3: Culture media compositions**

| **Culture media for Min-6 pseudoislets** |
|---|
| Dulbecco's Modified Eagle Medium (DMEM) |
| 10% FBS |
| 1% penicillin / streptomycin |
| 1mM pyruvate |
| 0.05 mM beta-mercaptoethanol |

Pseudoislets with a mean diameter of 150 µm were formed using Min-6 cell line using 400 µm microwell Eplasia plates (Corning) by seeding 500 cells per microwell and incubating them at 37°C and 5% CO₂ for 3 days. Pseudoislets were then collected, concentrated by centrifugation, and finally suspended in 0.9% NaCl.

### Example B2A: Isolation of primary human islets

Pancreata were obtained from human brain-dead donors. Pancreatic islets were produced following the method described in Technique of pancreatic procurement for pancreatic islet isolation, Pattou et al., Anchir 2005. Briefly, the pancreas was isolated from the tissue, and perfused in the Wirsung canal for digestion with a mix of collagenases I and II (Liberase^{®}, Roche, France) in order to ensure the releasing of islets. Islets were then purified using a density gradient centrifugation (EuroFicoll, SigmaAldrich). Purified islets were finally cultured in culture flasks at 37°C under 5% CO2 in CMRL medium supplemented with 0.625% BSA and 1% penicillin / streptomycin. Culture medium was replaced every 2-3 days.

### Example B2B: Primary rat islet isolation

Pancreatic islets were isolated from male Wistar or Lewis rats (approximative weight: 300g) following a similar method as described in A Pratical Guide to Rodent Islet Isolation and Assessment, Carter et al. Biological Procedures Online 2009.

Briefly, pancreases were perfused with Collagenase injected via the common bile duct. After perfusion, pancreata were excised and the digestion was performed at 37°C for 10 min. The islets were then purified through density gradient centrifugation. Purified islets were cultured in non-adherent culture flasks, in DMEM medium (Gibco) supplemented with 10% Foetal Bovine Serum, 2g/L glucose and 1% Penicillin/Streptomycin or CMRL medium at 37°C and 5% CO2. Culture medium was replaced every 2-3 days.

### Example 3A: Islet equivalent counting

To normalize the quantity of islets or pseudoislets used in each experiment, islets or pseudoislets were counted to determine the islet equivalent count (IEQs). One IEQ corresponds to the volume of a perfectly spherical islet/pseudoislet with a diameter of 150 µm. During counting, a multiplicative factor was applied to each islet depending on its size. This mathematical compensation for islet varying diameters allows for normalization between preparations (See NIH CIT Consortium Chemistry Manufacturing Controls Monitoring Committee; Purified Human Pancreatic Islet: Qualitative and Quantitative Assessment of Islets Using Dithizone (DTZ): Standard Operating Procedure of the NIH Clinical Islet Transplantation Consortium. CellR4 Repair Replace Regen Reprogram).

Two 50 µL samples from each islet or pseudoislet batch were counted on a glass slide with a 50 µm grid. Islets or pseudoislets were categorized by size according to Table 4A.

**Table 4A: Multiplication factor of islet size for islet equivalent determination**

| **Islet size** | **Multiplicative factor** |
|---|---|
| 50-100 µm | 1/6 |
| 100-150 µm | 1/1.5 |
| 150-200 µm | 1.7 |
| 200-250 µm | 3.5 |
| 250-300 µm | 6.3 |

The islet equivalent count was determined by averaging the count results from two independent samples.

### Example B3B: Islet equivalent counting

To normalize the quantity of islets used in each experiment, islets were counted to determine the islet equivalent count (IEQs). One IEQ corresponds to the volume of a perfectly spherical islet with a diameter of 150 µm. During counting, a multiplicative factor was applied to each islet depending on its size. This mathematical compensation for islet varying diameters allows for normalization between islet preparations (See NIH CIT Consortium Chemistry Manufacturing Controls Monitoring Committee; Purified Human Pancreatic Islet: Qualitative and Quantitative Assessment of Islets Using Dithizone (DTZ): Standard Operating Procedure of the NIH Clinical Islet Transplantation Consortium. CellR4 Repair Replace Regen Reprogram).

Two 50 µL samples from each islet batch were counted on a glass slide with a 50 µm grid. Islets were categorized by size according to Table 4B.

**Table 4B: Multiplication factor of islet size for islet equivalent determination**

| **Islet size** | **Multiplicative factor** |
|---|---|
| 50-100 µm | 1/6 |
| 100-150 µm | 1/1.5 |
| 150-200 µm | 1.7 |
| 200-250 µm | 3.5 |
| 250-300 µm | 6.3 |

The islet equivalent count was determined by averaging the count results from two independent samples.

### Part C - PHYSICO-CHEMISTRY

### Example C1A: Preparation of solutions of concentrated polysaccharide functionalized with maleimide (Mal) groups

A concentrated polysaccharide solution was prepared by weighing the appropriate weight of a sterile freeze-dried polysaccharide obtained according to part A1 and adding the appropriate weight of sterile deionised water. The solution was placed on an orbital shaker overnight at 70 rpm for complete solubilization. The pH of the solution was adjusted to pH 4 by addition of concentrated HCl before sterile filtration (0.22 µm). The mass concentration of the solution of polysaccharide (mg/g) was determined by dry exact. The volume concentration of the solution of polysaccharide (mg/mL) was determined by density measurements, weighing three times 100 µL of solution. The solution was frozen at -20°C until being used.

### Example C1B: Preparation of solutions of concentrated polysaccharide functionalized with vinyl sulfone (VS), DBCO or azide groups

A concentrated polysaccharide solution was prepared by weighing the appropriate weight of a sterile freeze-dried polysaccharide obtained according to part A1 and adding the appropriate weight of sterile deionized water. The solution was placed on an orbital shaker overnight at 70 rpm for complete solubilization. The pH of the solution was adjusted to pH 7.4 by addition of NaOH before sterile filtration (0.22 µm). The mass concentration of the solution of polysaccharide (mg/g) was determined by dry extract. The volume concentration of the solution of polysaccharide (mg/mL) was determined by density measurements, weighing three times 100 µL of solution. The sterile solution was frozen at -20°C until being used.

### Example C2: Preparation of solutions of concentrated PEG derivative

A concentrated solution of PEG (from the list according to table 2) was prepared by weighing the appropriate weight of a PEG powder and adding the appropriate weight of sterile deionized water. The solution was placed on roller shaker at 15 rpm for 2 h for complete solubilization before sterile filtration (0.22 µm). The mass concentration of the PEG solution (mg/g) was determined by dry extract. The volume concentration of the PEG solution (mg/mL) was determined by density measurements, weighing three times 100 µL of solution. The sterile solution was frozen at -20°C until being used.

### Example C3: Preparation of solution of concentrated sodium hyaluronate

A concentrated solution of sodium hyaluronate (Pharma Grade 150 supplied by Novamatrix) was prepared by weighing the appropriate weight of sodium hyaluronate powder and adding the appropriate weight of sterile deionized water. Alternatively, another hyaluronate (Pharma Grade 300 supplied by echelon biosens, HTL Biotechnologies) was used in particular for C4B-16 gel. The solution was placed on roller shaker at 10 rpm overnight to complete solubilization before sterile filtration (0.22 µm).

### Example C3bis: Preparation of solution of concentrated trehalose dihydrate

A concentrated solution of trehalose dihydrate D(+) (Sigma-Aldrich) was prepared by weighing the appropriate mass of trehalose dihydrate D(+) powder and adding the appropriate weight of sterile deionized water. The solution was heated at 45°C under mixing to complete solubilization and cooled down to ambient temperature before sterile filtration (0.22 µm).

### Example C3A: Hydrogels preparation

The preparation of the hydrogels was made in an aseptic environment.

Polysaccharide and PEG derivatives concentrated sterile solutions prepared according to example C1A or C1B and example C2, respectively, were adjusted with a concentrated NaCl solution to obtain isotonic stock solutions (300 mOsm/kg) and equilibrated either at room temperature (20-25°C) or at 4°C. Optionally concentrated solution of polysaccharide bearing VS or DBCO groups was supplemented by a tris buffer at pH 7.4 or pH 8.

A concentrated solution of PEG was added to a concentrated solution of polysaccharide in a 2 mL Eppendorf. The volume ratio of the PEG solution to the polysaccharide solution was 70:30 (%:%) or 80: 20 (%:%). The solutions were mixed with a pipette and a controlled volume of the mixture was introduced in a circular silicone isolator adhering to a glass slide. Different molded hydrogel geometries were prepared.

**Table 5A: Molded hydrogel geometries conditions.**

| **Geometry** | **Silicone Isolator Diameter (mm)** | **Silicone Isolator Thickness (mm)** | **Hydrogel Volume (µL)** |
|---|---|---|---|
| C3A-1 | 4.5 | 0.5 | 10 |
| C3A-2 | 9 | 0.5 | 32 |
| C3A-3 | 9 | 1.6 | 100 |
| C3A-4 | 10 | 0.5 | 50 |
| C3A-5 | 13 | 0.5 | 66 |
| C3A-6 | 20 | 0.5 | 160 |

Cross-linking process leading to gelation was carried out for 1 h at room temperature (20-25°C) or at 37°C. The hydrogel was unmolded and introduced in a Tris 150 mM /NaCl 30 mM / Cystein 10 mM solution (2 mL) or in PBS at pH 7.4 for 1h at 37°C.

The hydrogel was rinsed with 20 mL of PBS solution without cysteine and further immersed in 10 mL of the PBS solution overnight at 37°C. The hydrogel piece was then stored in 10 mL of PBS solution at 4°C until being used.

### Example C3B: Hydrogels preparation

The preparation of the hydrogels was made in an aseptic environment.

Polysaccharide and PEG derivatives concentrated sterile solutions prepared according to example C1A or C1B and example C2, respectively, were adjusted with a concentrated NaCl solution to obtain isotonic stock solutions (300 mOsm/kg) and equilibrated either at room temperature (20-25°C) or at 4°C. Alternatively, osmolarity can be adjusted with a combination of NaCl and non-ionic agent such as trehalose in particular in example C4B24. Optionally concentrated solution of polysaccharide bearing VS or DBCO groups was supplemented by a tris buffer at pH 7.4 or pH 8.

A concentrated solution of PEG/Hyaluronate was prepared by mixing a concentrated solution of PEG prepared according to example C2 and a concentrated solution of sodium hyaluronate prepared according to example C3.

A concentrated solution of polysaccharide supplemented by pluronic F127 (Sigma-Aldrich) was prepared by mixing a concentrated solution of polysaccharide prepared according to example C1B.

A concentrated solution of PEG or PEG/Hyaluronate was added to a concentrated solution of polysaccharide or polysaccharide/pluronic, in particular Pluronic F127, in a 2 mL Eppendorf. The volume ratio of the PEG solution to the polysaccharide solution was 70:30 (%:%) or 80:20 (%:%) when hyaluronate is added to the PEG solution. The solutions were mixed with a pipette and a controlled volume of the mixture was introduced in a circular silicone isolator adhering to a glass slide. Different molded hydrogel geometries were prepared.

Addition of a non-ionic surfactant of the pluronic type may help to improve the wettability of the composition.

**Table 5B: Molded hydrogel geometries conditions.**

| **Geometry** | **Silicone Isolator Diameter (mm)** | **Silicone Isolator Thickness (mm)** | **Hydrogel Volume (µL)** |
|---|---|---|---|
| C3B-1 | 4.5 | 0.5 | 10 |
| C3B-2 | 4.5 | 1.6 | 25 |
| C3B-3 | 9 | 0.5 | 32 |
| C3B-4 | 9 | 1.6 | 100 |
| C3B-5 | 10 | 0.5 | 50 |
| C3B-6 | 13 | 0.5 | 66 |
| C3B-7 | 20 | 0.5 | 160 |

Crosslinking leading to gelation was carried out for 1 h at room temperature (20-25°C) or at 37°C. The hydrogel was unmolded and introduced in a Tris 150 mM /NaCl 30 mM / Cystein 10 mM solution at pH 8 or in PBS at pH 7.4 for 1h at 37°C.

The hydrogel was rinsed with PBS solution without cysteine and further immersed in the PBS solution overnight at 37°C. The hydrogel piece was then stored in PBS solution at 4°C until being used.

### Example C4A: Hydrogel compositions

Different hydrogel compositions were prepared according to the protocol described in Example C3A are shown in Table 6A. Concentrations of the two reactive groups (maleimide (Mal) or vinylsulfone (VS) reacting with thiol (SH) or alcyne (DBCO) reacting with azide (N3)) and polymers (polysaccharide and PEG derivatives) correspond to the final concentration upon mixing of the polymer solutions.

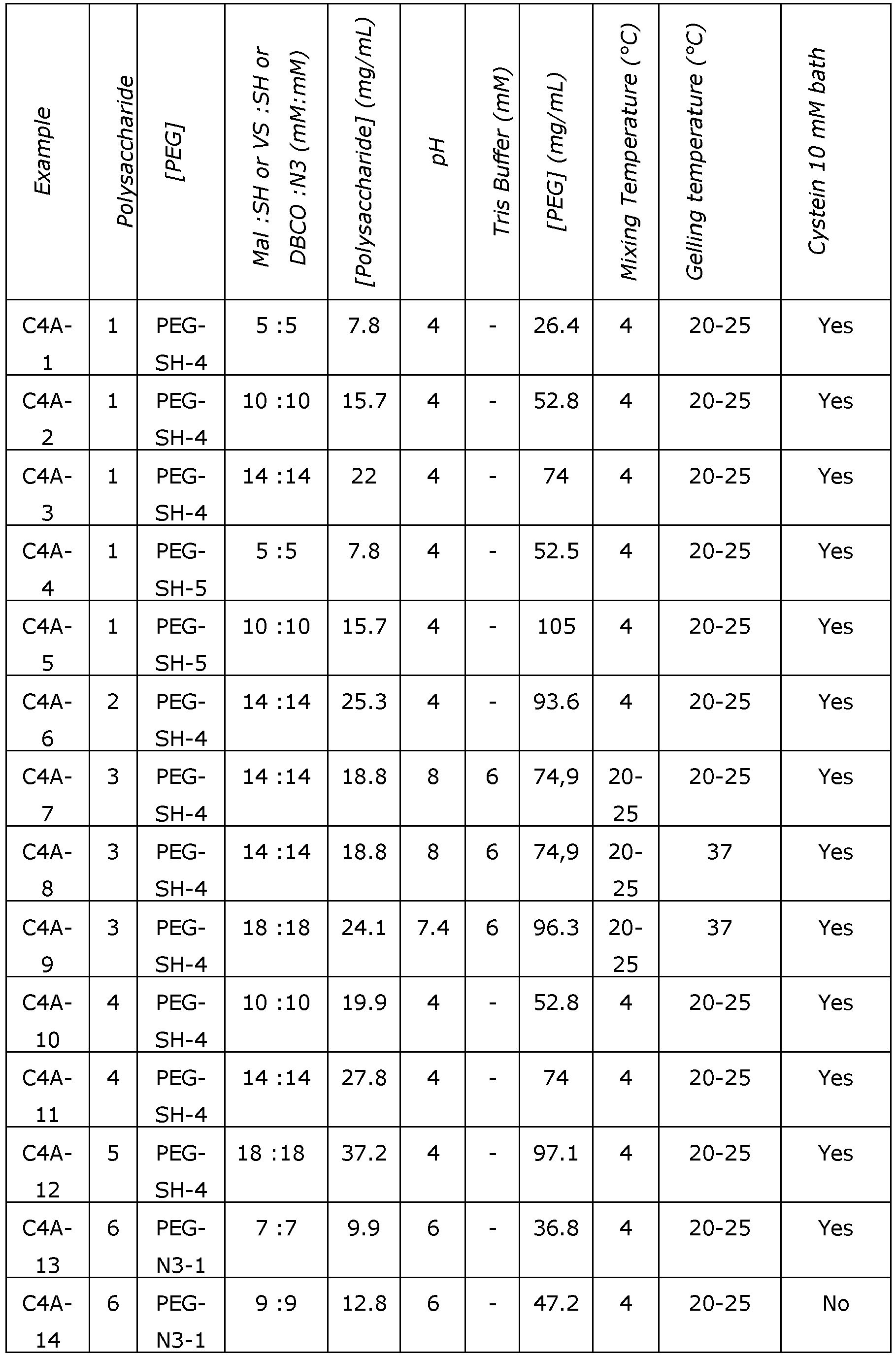

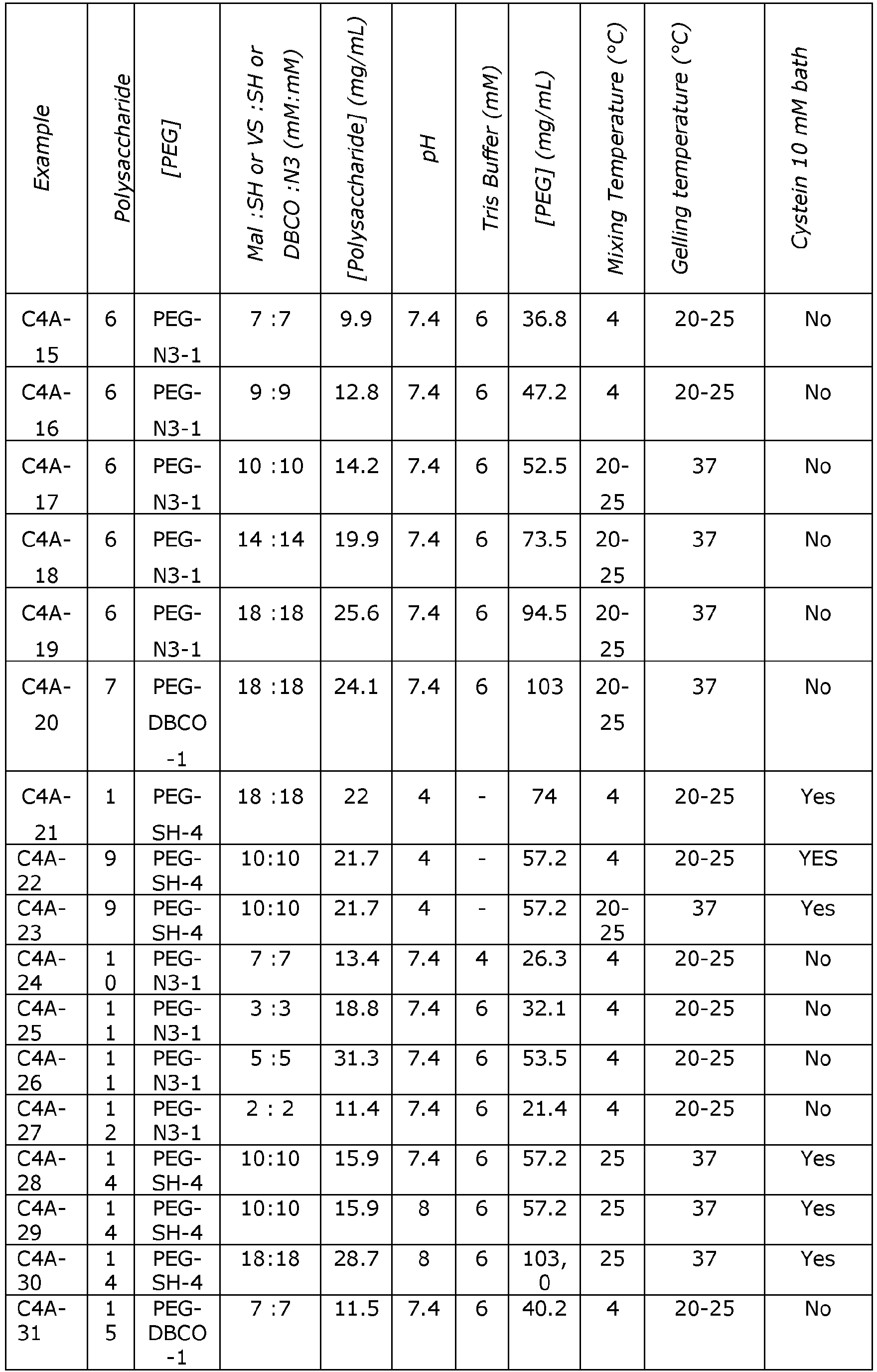

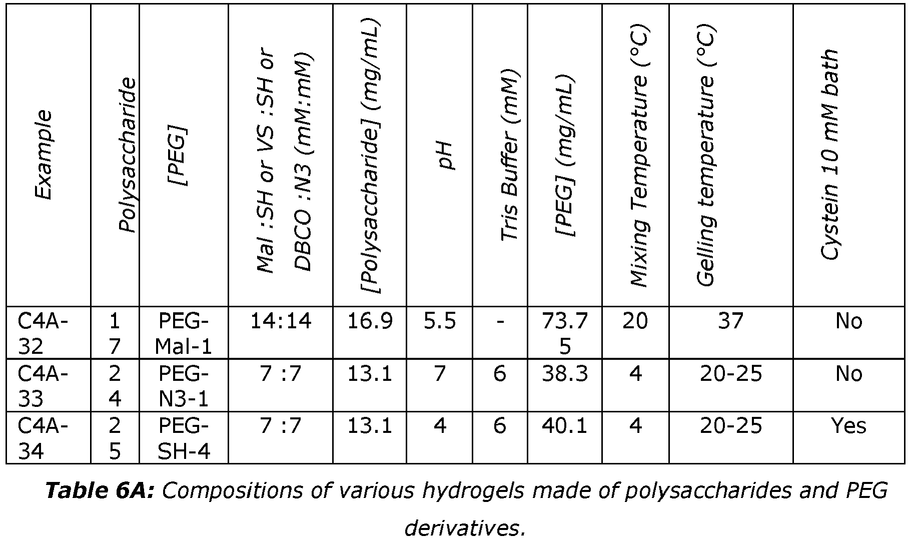

Solid disc-shaped hydrogel pieces were obtained. The hydrogels pieces were easily unmoulded and handled with tweezers for characterization.

**Table 7A: Structures of the hydrogels**

| **Hydrogels (concentration in reactive species (mM))** | **Structure** | | |
|---|---|---|---|
| C4A-1 (5) | 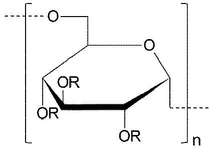 | | |
| C4A-2 (10) | | | |
| C4A-3 (14) | | | |
| C4A-21 (18) | | | |
| | Mw (Dextran) = 40 kg/mol | | |
| | n = 205 | | |
| | R = H or | 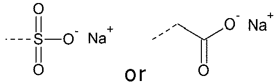 | or |
| | 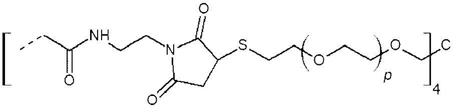 | | |
| | p = 114 | | |
| | DS₃ (sulfate) = 1.5 | | |
| | DS₂ (methylcarboxylate) = 0.8 | | |
| | DS₁ (succinimide derivative) = 0.25 | | |
| C4A-4 (5) | 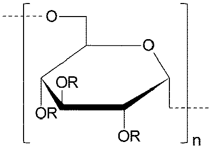 | | |
| C4A-5 (10) | | | |
| | Mw (Dextran) = 40 kg/mol | | |
| | n = 205 | | |
| | R = H or | 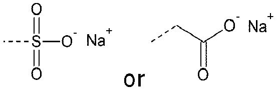 | or |
| | 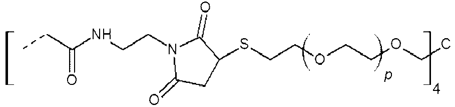 | | |
| | p = 227 | | |
| | DS₃ (sulfate) = 1.5 | | |
| | DS₂ (methylcarboxylate) = 0.8 | | |
| | DS₁ (succinimide derivative) = 0.25 | | |
| C4A-6 (14) | 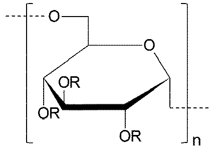 | | |
| | Mw (Dextran) = 40 kg/mol | | |
| | n = 205 | | |
| | R = H or | 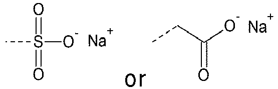 | or |
| | 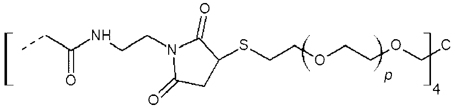 | | |
| | p = 114 | | |
| | DS₃ (sulfate) = 1.9 | | |
| | DS₂ (methylcarboxylate) = 0.5 | | |
| | DS₁ (succinimide derivative) = 0.23 | | |
| C4A-7 (14) | 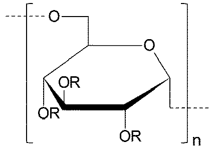 | | |
| C4A-8 (14) | | | |
| C4A-9 (18) | | | |
| | Mw (Dextran) = 40 kg/mol | | |
| | n = 205 | | |
| | R = H or | 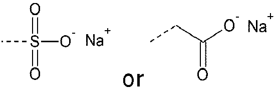 | or |
| | 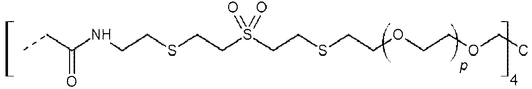 | | |
| | p = 114 | | |
| | DS₃ (sulfate) = 1.5 | | |
| | DS₂ (methylcarboxylate) = 0.8 | | |
| | DS₁ (sulfone derivative) = 0.28 | | |
| C4A-10 (10) | 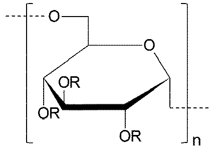 | | |
| C4A-11 (14) | | | |
| | Mw (Dextran) = 40 kg/mol | | |
| | n = 205 | | |
| | R = H or | 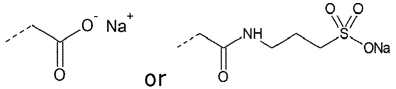 | or |
| | 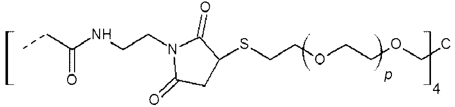 | | |
| | p = 114 | | |
| | DS₃ (homotaurine) = 1.15 | | |
| | DS₂ (methylcarboxylate) = 2.1 | | |
| | DS₁ (succinimide derivative) = 0.25 | | |
| C4A-12 (18) | 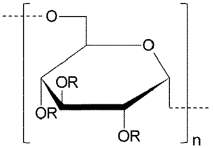 | | |
| | Mw (Dextran) = 40 kg/mol | | |
| | n = 205 | | |
| | R = H or | 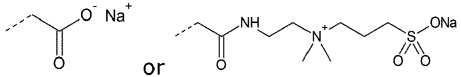 | or |
| | 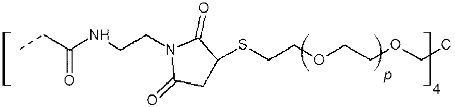 | | |
| | p = 114 | | |
| | DS₃ (SB) = 0.7 | | |
| | DS₂ (methylcarboxylate) = 2.1 | | |
| | DS₁ (succinimide derivative) = 0.22 | | |
| C4A-13 (7) | 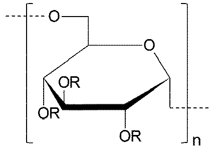 | | |
| C4A-14 (9) | | | |
| C4A-15 (7) | | | |
| C4A-16 (9) | | | |
| C4A-17 (10) | Mw (Dextran) = 40 kg/mol | | |
| C4A-18 (14) | n = 205 | | |
| C4A-19 (18) | R = H or | 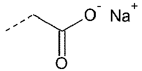 | or |
| | 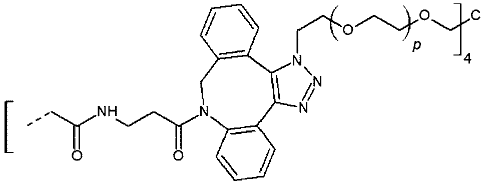 | | |
| | or | | |
| | 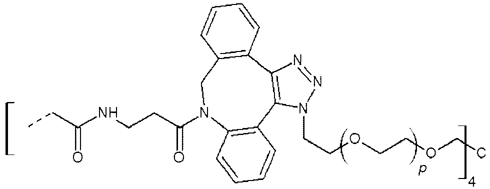 | | |
| | p = 114 | | |
| | DS₂ (methylcarboxylate) = 2.1 | | |
| | DS₁ (triazole derivatives (mixture of 2 isomers)) = 0.28 | | |
| C4A-20 (18) | 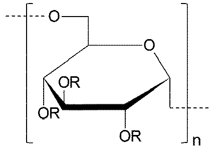 | | |
| | Mw (Dextran) = 40 kg/mol | | |
| | n = 205 | | |
| | R = H or | 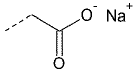 | or |
| | 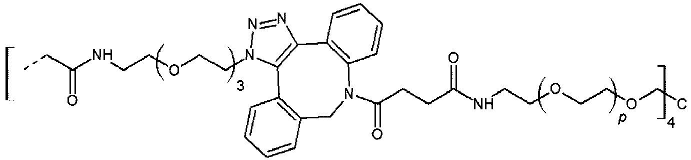 | | |
| | or | | |
| | 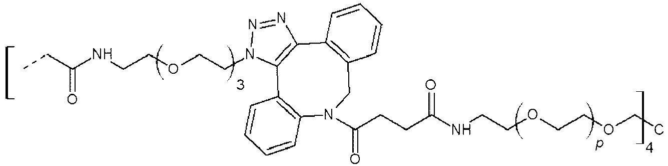 | | |
| | p = 114 | | |
| | DS₂ (methylcarboxylate) = 2.1 | | |
| | DS₁ (triazole derivatives (mixture of 2 isomers)) = 0.28 | | |
| C4A-22 (10) | 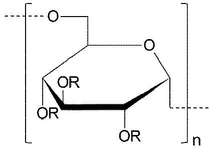 | | |
| C4A-23 (10) | | | |
| | Mw (Dextran) = 40 kg/mol | | |
| | n = 205 | | |
| | R = H or | 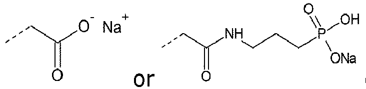 | or |
| | 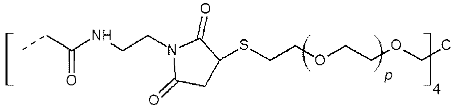 | | |
| | p = 114 | | |
| | DS₃ (phosphonate) = 0.75 | | |
| | DS₂ (methylcarboxylate) = 2.1 | | |
| | DS₁ (succinimide derivative) = 0.21 | | |
| C4A-24 (7) | 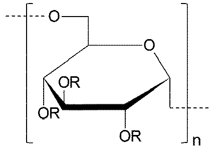 | | |
| | Mw (Dextran) = 40 kg/mol | | |
| | n = 205 | | |
| | R = H or | 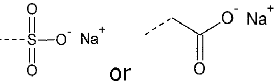 | or |
| | 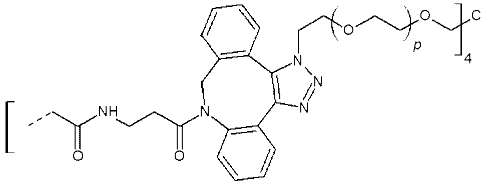 | | |
| | or | | |
| | 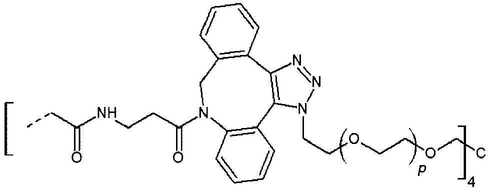 | | |
| | p = 114 | | |
| | DS₃ (sulfate) = 1.5 | | |
| | DS₂ (methylcarboxylate) = 0.8 | | |
| | DS₁ (triazole derivatives (mixture of 2 isomers)) = 0.23 | | |
| C4A-25 (3) | 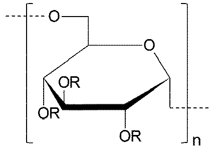 | | |
| C4A-26 (5) | | | |
| | Mw (Dextran) = 250 kg/mol | | |
| | n = 1100 | | |
| | R = H or | 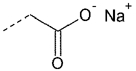 | or |
| | 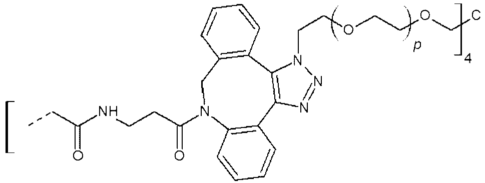 | | |
| | or | | |
| | 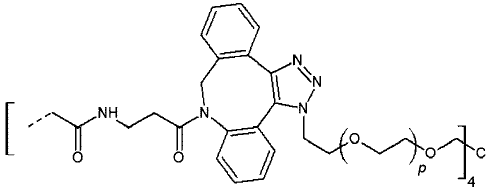 | | |
| | p = 114 | | |
| | DS₂ (methylcarboxylate) = 2.0 | | |
| | DS₁ (triazole derivatives (mixture of 2 isomers)) = 0.06 | | |
| C4A-27 (2) | 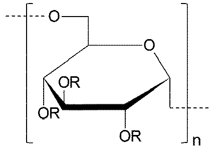 | | |
| | Mw (Dextran) = 500 kg/mol | | |
| | n = 2200 | | |
| | R = H or | 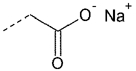 | or |
| | 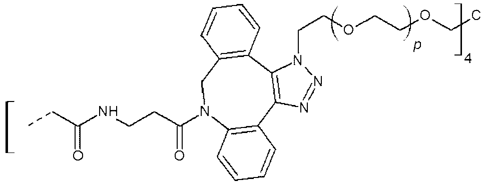 | | |
| | or | | |
| | 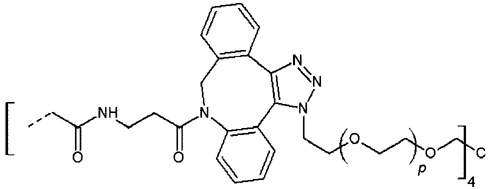 | | |
| | p = 114 | | |
| | DS₂ (methylcarboxylate) = 2.0 | | |
| | DS₁ (triazole derivatives (mixture of 2 isomers)) = 0.06 | | |
| C4A-28 (10) | 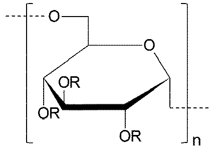 | | |
| C4A-29 (10) | | | |
| C4A-30 (18) | | | |
| | Mw (Dextran) = 40 kg/mol | | |
| | n = 205 | | |
| | R = H or | 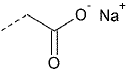 | or |
| | 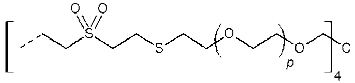 | | |
| | p = 114 | | |
| | DS₂ (methylcarboxylate) = 1.2 | | |
| | DS₁ (sulfone derivative) = 0.17 | | |
| C4A-31 (7) | 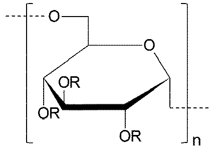 | | |
| | Mw (Dextran) = 40 kg/mol | | |
| | n = 205 | | |
| | R = H or | 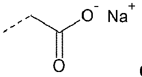 | or |
| | 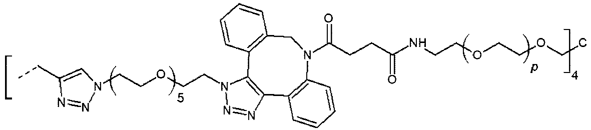 | | |
| | or | | |
| | 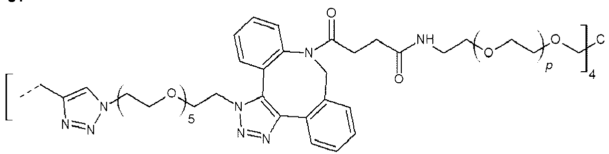 | | |
| | p = 114 | | |
| | DS₂ (methylcarboxylate) = 1.8 | | |
| | DS₁ (triazole derivatives (mixture of 2 isomers)) = 0.24 | | |
| C4A-32 (14) | 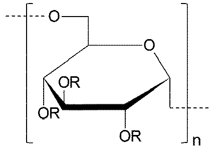 | | |
| | Mw (Dextran) = 40 kg/mol | | |
| | n = 205 | | |
| | R = H or | 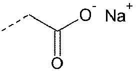 | or |
| | 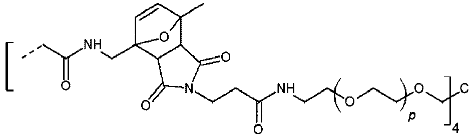 | | |
| | p = 114 | | |
| | DS₂ (methylcarboxylate) = 2.1 | | |
| | DS₁ (succinimide derivative) = 0.30 | | |
| C4A-33 (7) | 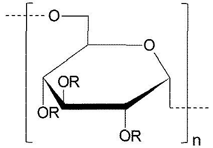 | | |
| | Mw (Dextran) = 40 kg/mol | | |
| | n = 205 | | |
| | R = H or | 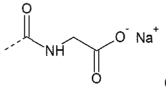 | or |
| | 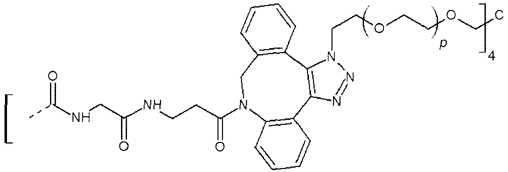 | | |
| | or | | |
| | 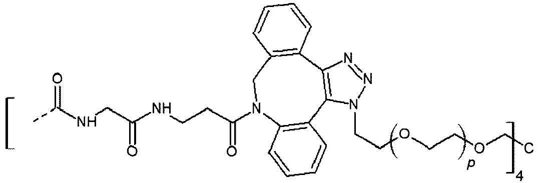 | | |
| | p = 114 | | |
| | DS₂ (glycine carbamate) = 2.4 | | |
| | DS₁ (triazole derivative) = 0.28 | | |
| C4A-34 (7) | 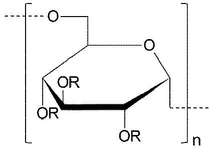 | | |
| | Mw (Dextran) = 40 kg/mol | | |
| | n = 205 | | |
| | R = H or | 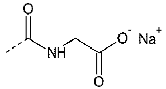 | or |
| | 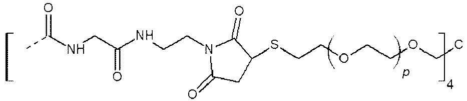 | | |
| | p = 114 | | |
| | DS₂ (glycine carbamate) = 2.4 | | |
| | DS₁ (succinimide derivative) = 0.26 | | |

### Example C4B: Hydrogel compositions

Different hydrogel compositions were prepared according to the protocol described in Example C3B (Table 6B). Concentrations of the two reactive groups (maleimide (Mal) or vinylsulfone (VS) reacting with thiol (SH) or alcyne (DBCO) reacting with azide (N3)) and polymers (polysaccharide and PEG derivatives) correspond to the final concentration upon mixing of the polymer solutions.

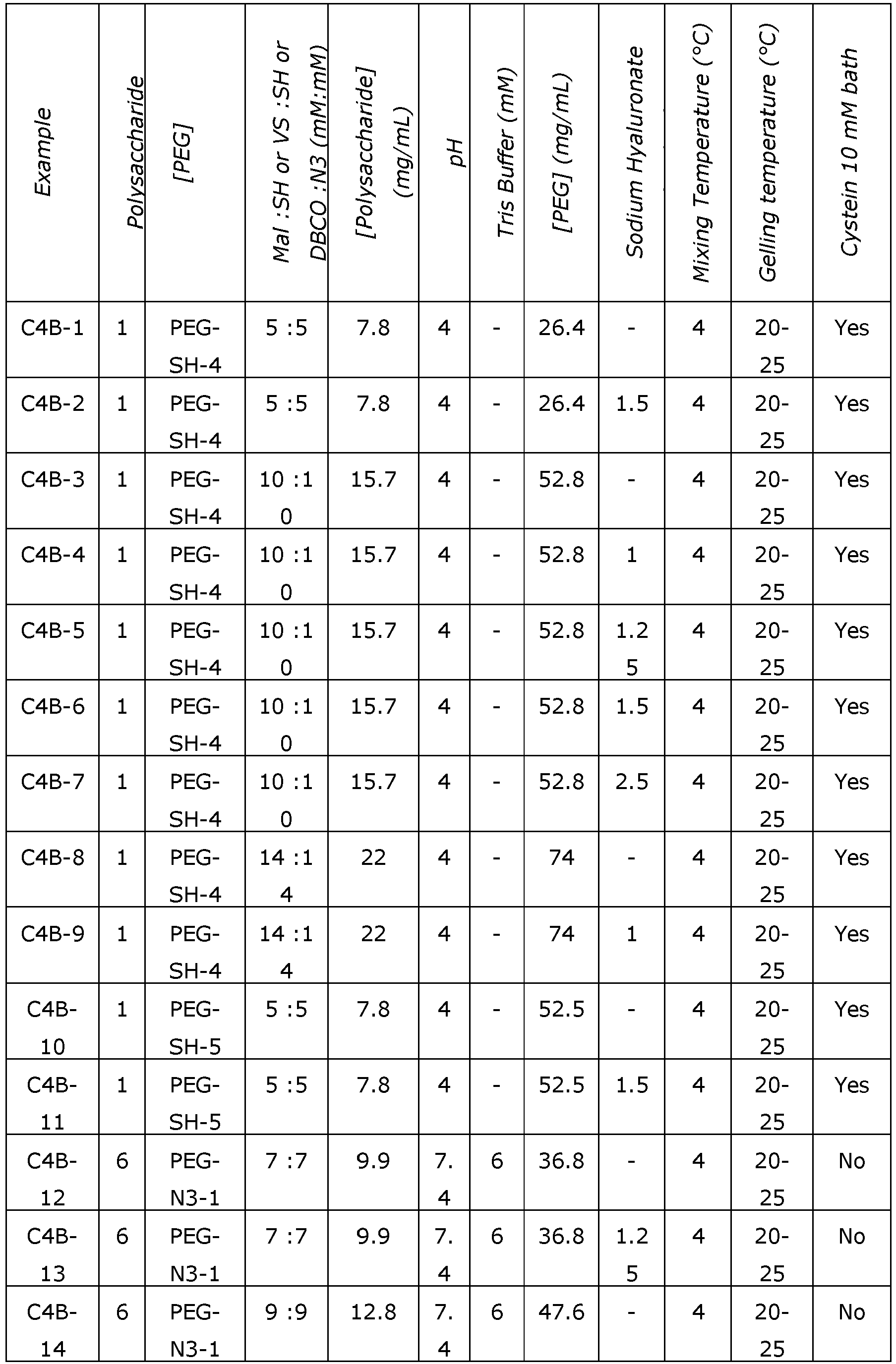

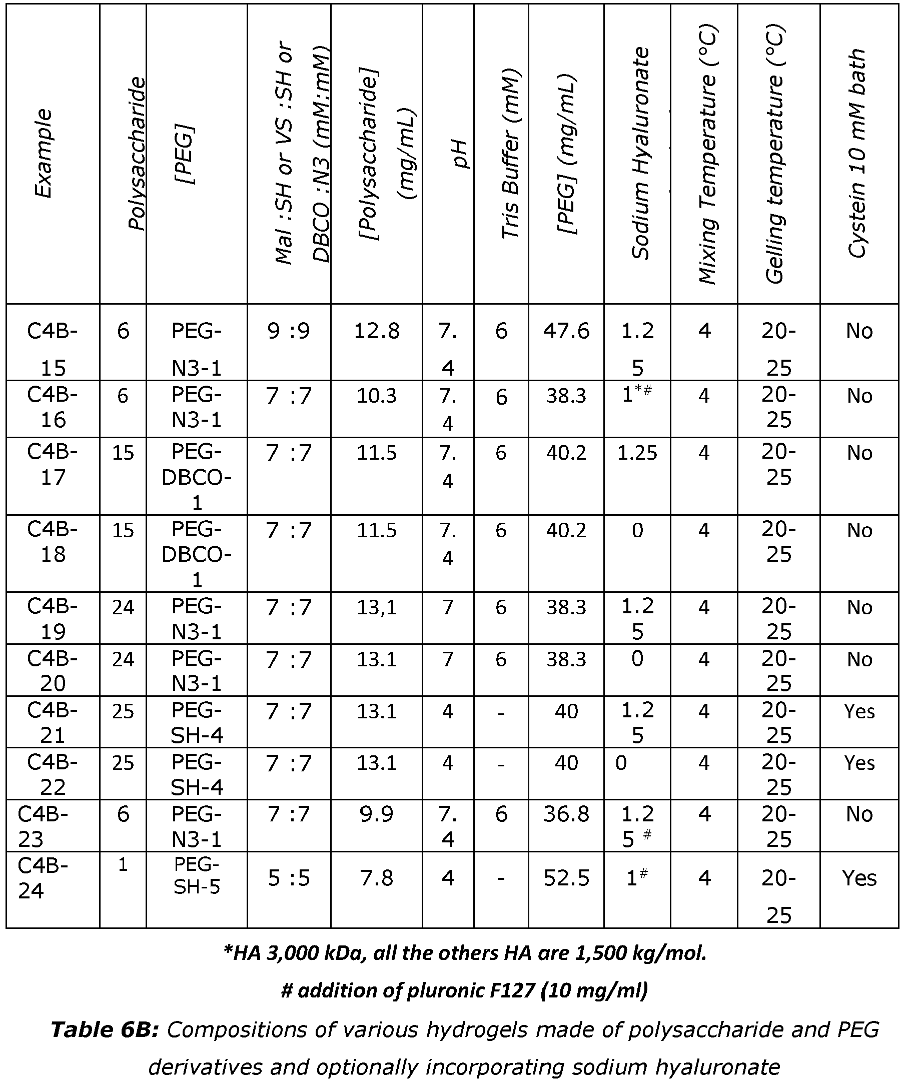

Solid disc-shaped hydrogel pieces were obtained. The hydrogels pieces were easily unmolded and handled with tweezers for characterization. /

**Table 7B: Structures of the hydrogels**

| **Hydrogels (concentration in reactive species (mM))** | **Structure** | | |
|---|---|---|---|
| C4B-1 (5) | 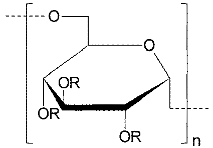 | | |
| C4B-2 (5) | | | |
| C4B-3 (10) | | | |
| C4B-4 (10) | | | |
| C4B-5 (10) | Mw (Dextran) = 40 kg/mol | | |
| C4B-6 (10) | n = 205 | | |
| C4B-7 (10) | | 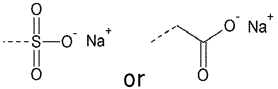 | |
| C4B-8 (14) | R = H or | | or |
| C4B-9 (14) | 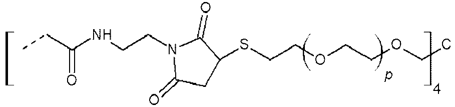 | | |
| | p = 114 | | |
| | DS₃ (sulfate) = 1.5 | | |
| | DS₂ (methylcarboxylate) = 0.8 | | |
| | DS₁ (succinimide derivative) = 0.25 | | |
| C4B-10 (5) | 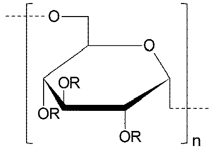 | | |
| C4B-11 (5) | | | |
| | Mw (Dextran) = 40 kg/mol | | |
| | n = 205 | | |
| | R = H or | 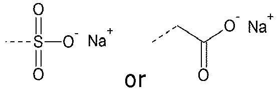 | or |
| | 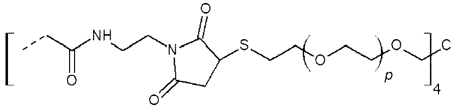 | | |
| | p = 227 | | |
| | DS₃ (sulfate) = 1.5 | | |
| | DS₂ (methylcarboxylate) = 0.8 | | |
| | DS₁ (succinimide derivative) = 0.25 | | |
| C4B-12 (7) | 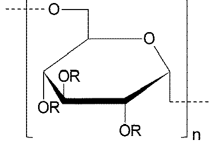 | | |
| C4B-13 (7) | | | |
| C4B-14 (9) | | | |
| C4B-15 (9) | | | |
| C4B-16 (7) | Mw (Dextran) = 40 kg/mol | | |
| | n = 205 | | |
| | R = H or | 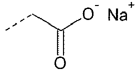 | or |
| | 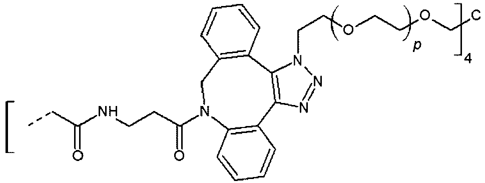 | | |
| | or | | |
| | 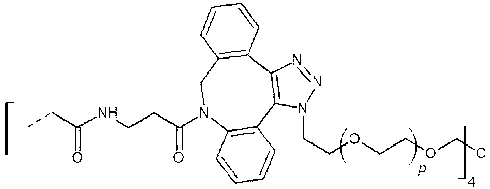 | | |
| | p = 114 | | |
| | DS₂ (methylcarboxylate) = 2.1 | | |
| | DS₁ (triazole derivatives (mixture of 2 isomers)) = 0.28 | | |
| C4B-17 (7) | 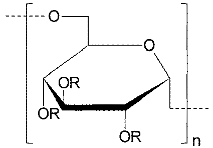 | | |
| C4B-18 (7) | | | |
| | | | |
| | Mw (Dextran) = 40 kg/mol | | |
| | n = 205 | | |
| | R = H or | 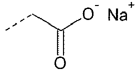 | or |
| | 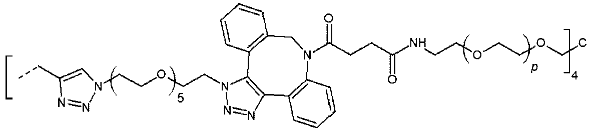 | | |
| | or | | |
| | 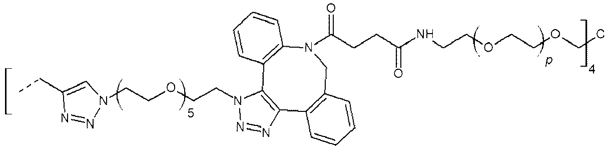 | | |
| | p = 114 | | |
| | DS₂ (methylcarboxylate) = 1.8 | | |
| | DS₁ (triazole derivatives (mixture of 2 isomers)) = 0.24 | | |
| C4B-19 (7) | 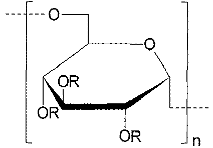 | | |
| C4B-20 (7) | | | |
| | Mw (Dextran) = 40 kg/mol | | |
| | n = 205 | | |
| | R = H or | 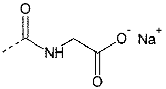 | or |
| | 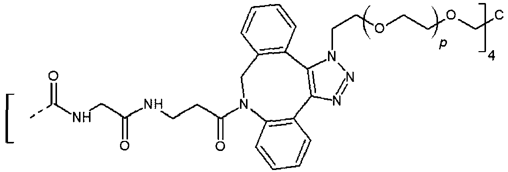 | | |
| | or | | |
| | 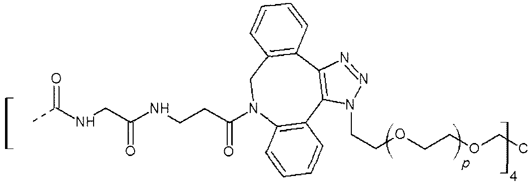 | | |
| | p = 114 | | |
| | DS₂ (glycine carbamate) = 2.4 | | |
| | DS₁ (triazole derivative) = 0.28 | | |
| C4B-21 (7) | 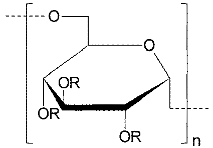 | | |
| C4B-22 (7) | | | |
| | Mw (Dextran) = 40 kg/mol | | |
| | n = 205 | | |
| | R = H or | 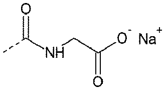 | or |
| | 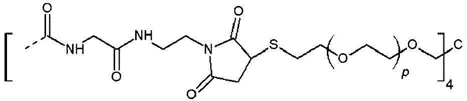 | | |
| | p = 114 | | |
| | DS₂ (glycine carbamate) = 2.4 | | |
| | DS₁ (succinimide derivative) = 0.26 | | |

### Example C5A : Hydrogel rheological characterization

Oscillatory shear test was carried out with a rotational rheometer (AR2000, TA instrument) equipped with a cone plate geometry. The cross-linking process leading to gelation was done *"in situ",* meaning that drops of polysaccharide and PEG concentrated solutions were introduced between cone one and plate and mixed by rotation of the geometry before starting oscillations measurements. Oscillation time sweep tests were carried out at 25°C or 37°C, with a constant strain of 0.1% and constant oscillation frequency of 1 Hz. The storage modulus G' (i.e. elasticity) and Tan δ (ratioG"/G') values were reported at 1600 s in the plateau region of the measure of (G',G") as a function of time.

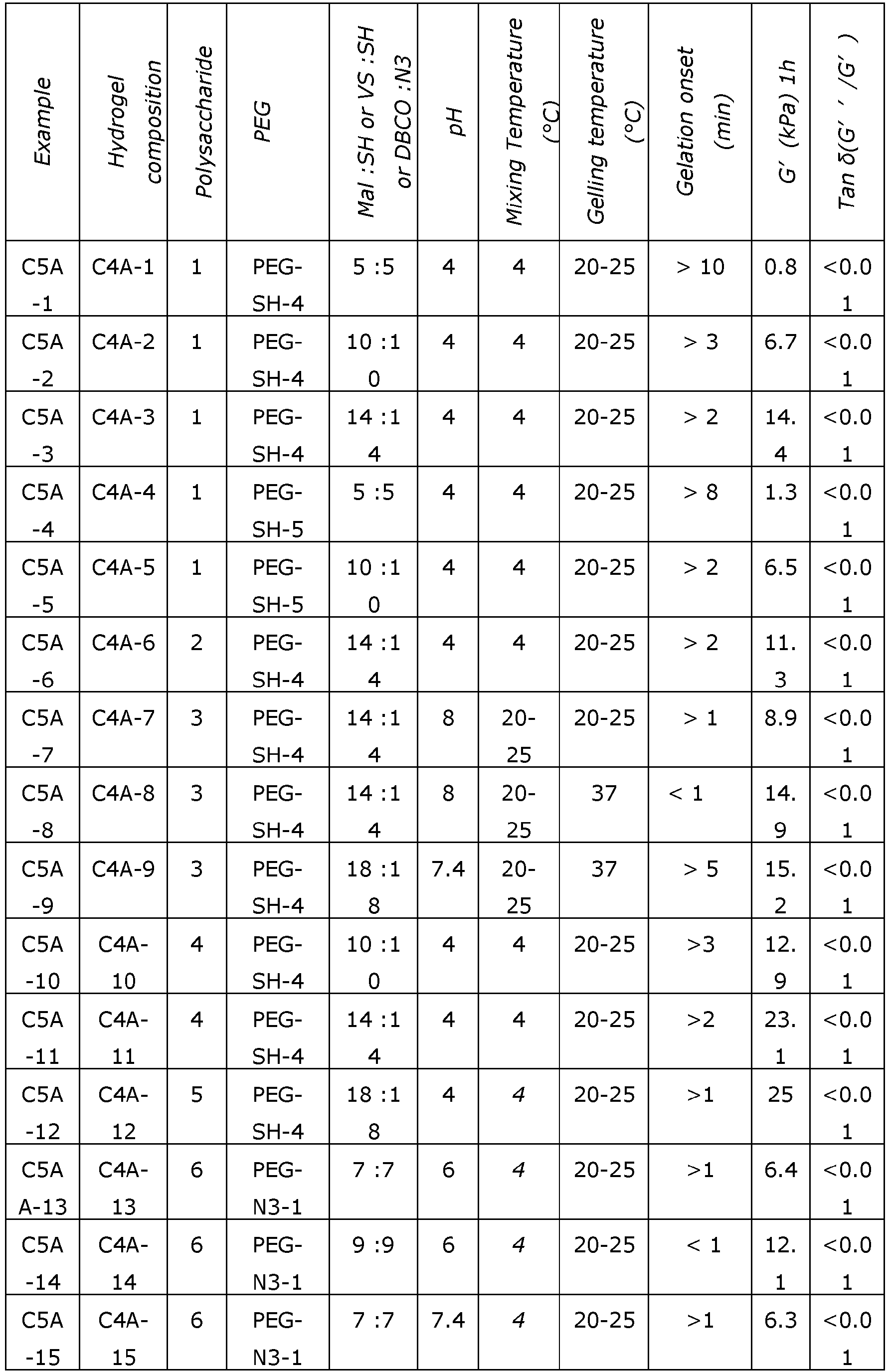

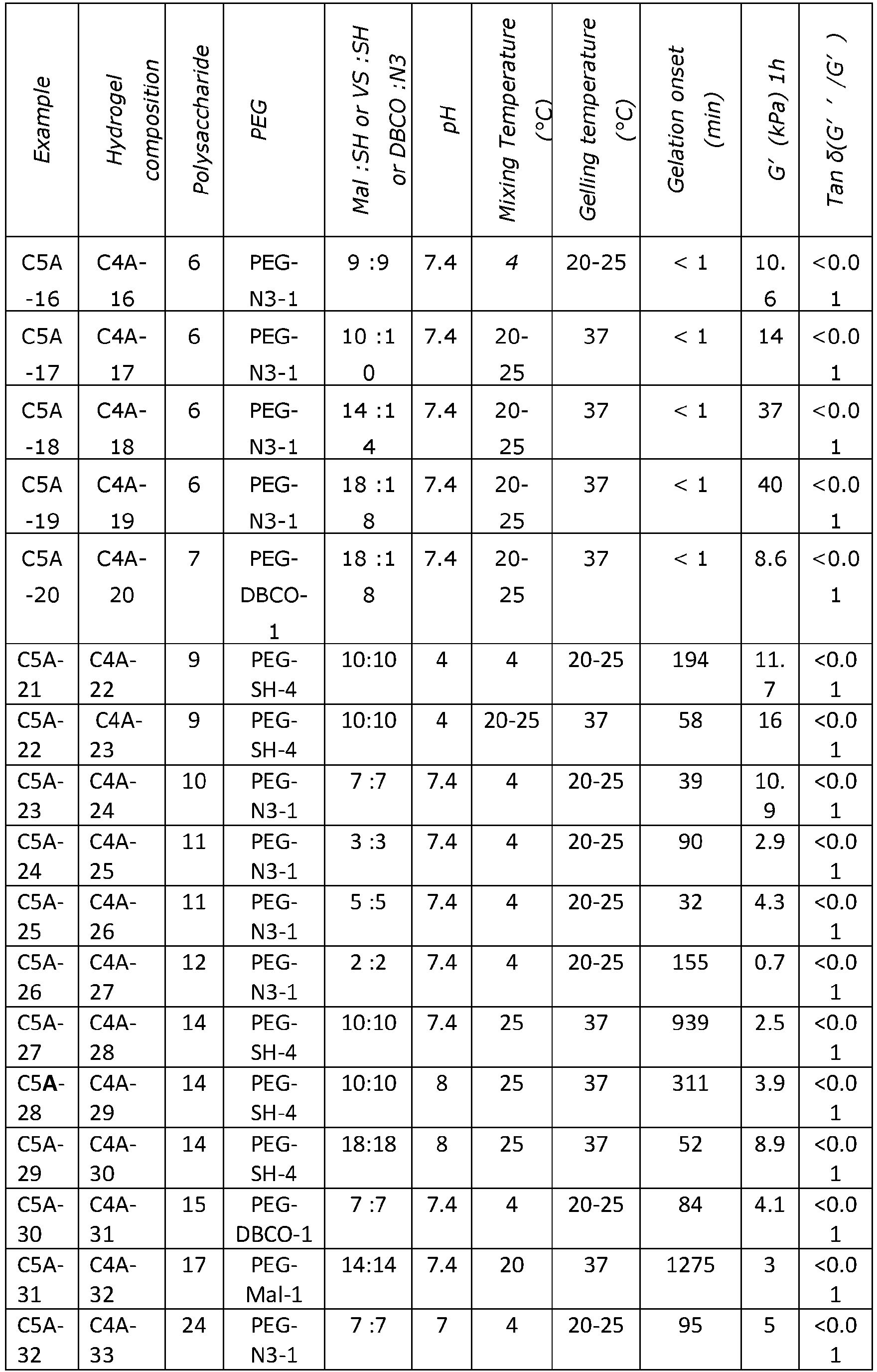

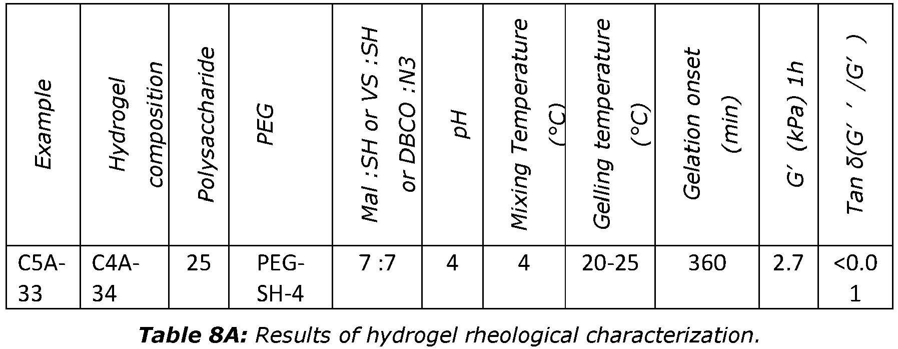

Hydrogels present low values of Tan δ, meaning that G' was much higher than G" which is a typical property of chemically cross-linked hydrogels behaving as solid elastic materials (see Polysaccharide Hydrogels: Characterization and Biomedical Applications, 2016 Pan Stanford Publishing Pte. Ltd.; Chapter 3, page 97). The increase of the concentration in Mal:SH leads to an increase of the value of elastic modulus G'.

Increasing the temperature and the pH were two ways to accelerate the cross-linking process leading to the gelation of hydrogels prepared from polysaccharide bearing VS groups and PEG-SH. For example, going from neutral pH to pH 8 and/or increasing the temperature from 25°C to 37°C leads to a faster gelation.

This can allow a fine tuning of the gelation speed which could be convenient as fast gelation could be beneficial to avoid cells sedimentation whereas slow gelation could be beneficial for polymer mix casting before gelation.

### Example C5B: Hydrogel rheological characterization

Oscillatory shear test was carried out with a rotational rheometer (AR2000, TA instrument) equipped with a cone plate geometry. The crosslinking leading to gelation was done *"in situ",* meaning that drops of polysaccharide and PEG concentrated solutions were introduced between cone one and plate and mixed by rotation of the geometry before starting oscillations measurements. Oscillation time sweep tests were carried out at 25°C or 37°C, with a constant strain of 0.1% and constant oscillation frequency of 1 Hz. The storage modulus G' (i.e. elastic modulus) and Tan δ (ratio G"/G') values were reported at 1600 s in the plateau region of the measure of (G",G') as a function of time.

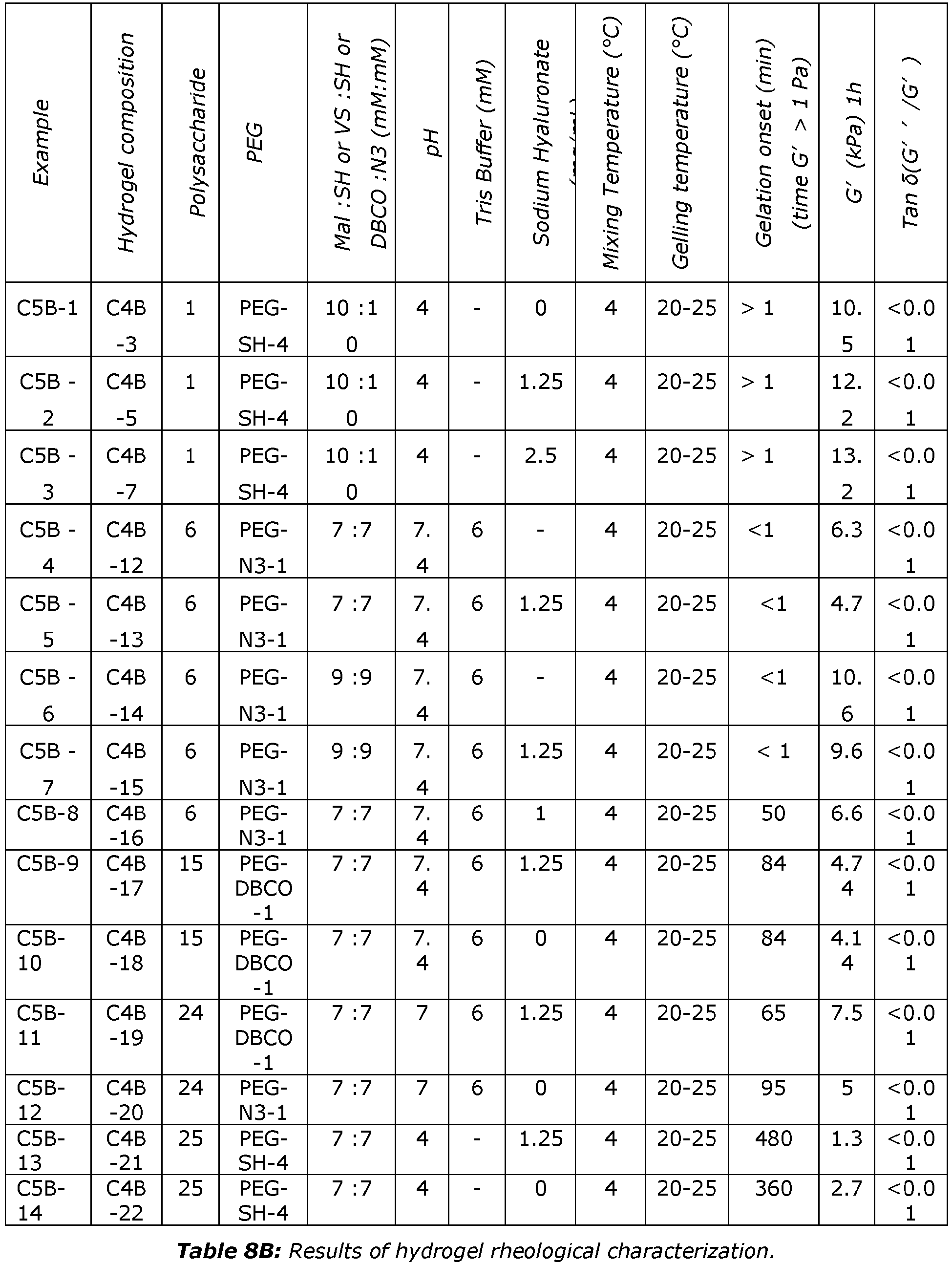

Hydrogels present low values of Tan δ, meaning that G' was much higher than G" which is a typical property of chemically cross-linked hydrogels behaving as solid elastic materials (see Polysaccharide Hydrogels: Characterization and Biomedical Applications, 2016 Pan Stanford Publishing Pte. Ltd.; Chapter 3, page 97).

The increase of the concentration in reactive groups leads to an increase of the value of elastic modulus G'.

This can allow a fine tuning of the crosslinking leading to gelation speed which could be convenient as fast gelation could be beneficial to avoid cells sedimentation whereas slow gelation could be beneficial for polymer mix casting before gelation.

Gelation properties are maintained in presence of non-reactive sodium hyaluronate.

This can allow a further tuning of hydrogel composition to avoid cells sedimentation, even for slow gelling compositions.

### Example C6A: Hydrogel swelling and water content

The hydrogel piece was weighed right after unmoulding (w0) and after overnight swelling (wovernight) in the PBS solution. The swelling ratio was defined as the mass ratio wovernight/w0. The water content of the hydrogel was deducted from the measurement of hydrogel mass in the swollen state and the control of polymer precursors concentrations implemented to synthetize the hydrogel.

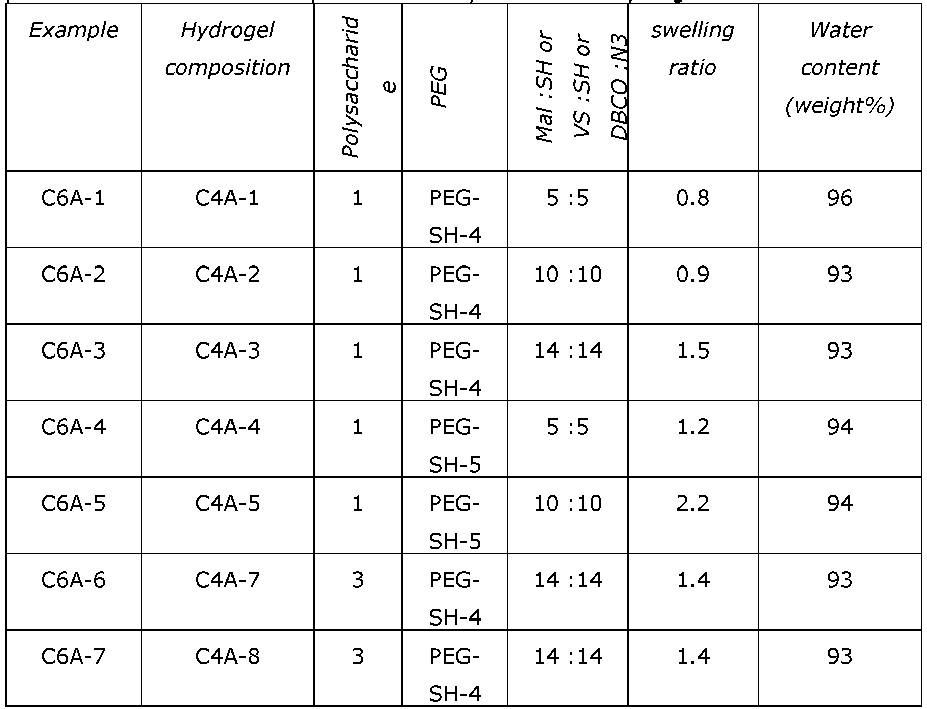

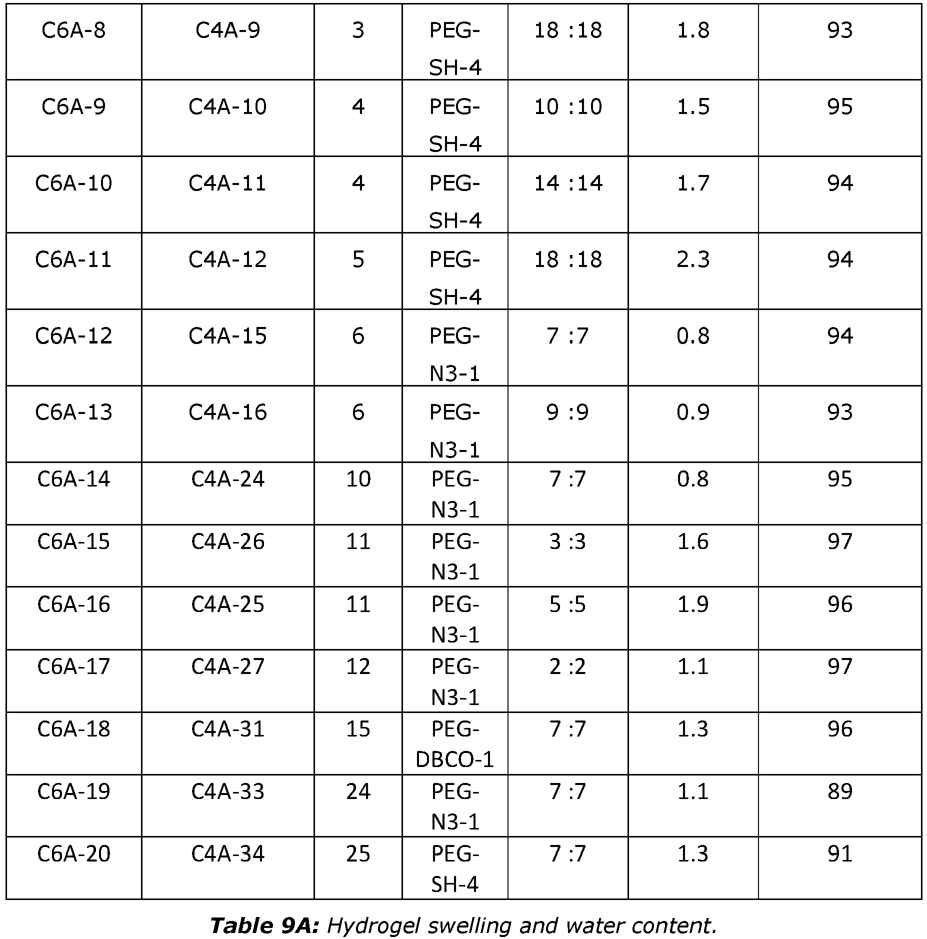

Hydrogels contain high water content. Water content varies depending on polymer precursors structures and concentrations.

### Example C6B: Hydrogel swelling and water content

The hydrogel piece was weighed right after unmoulding (w0) and after overnight swelling (overnight) in the PBS solution. The swelling ratio was defined as the mass ratio overnight/w0. The water content of the hydrogel was deducted from the measurement of hydrogel mass in the swollen state and the control of polymer precursors concentrations implemented to synthetize the hydrogel.

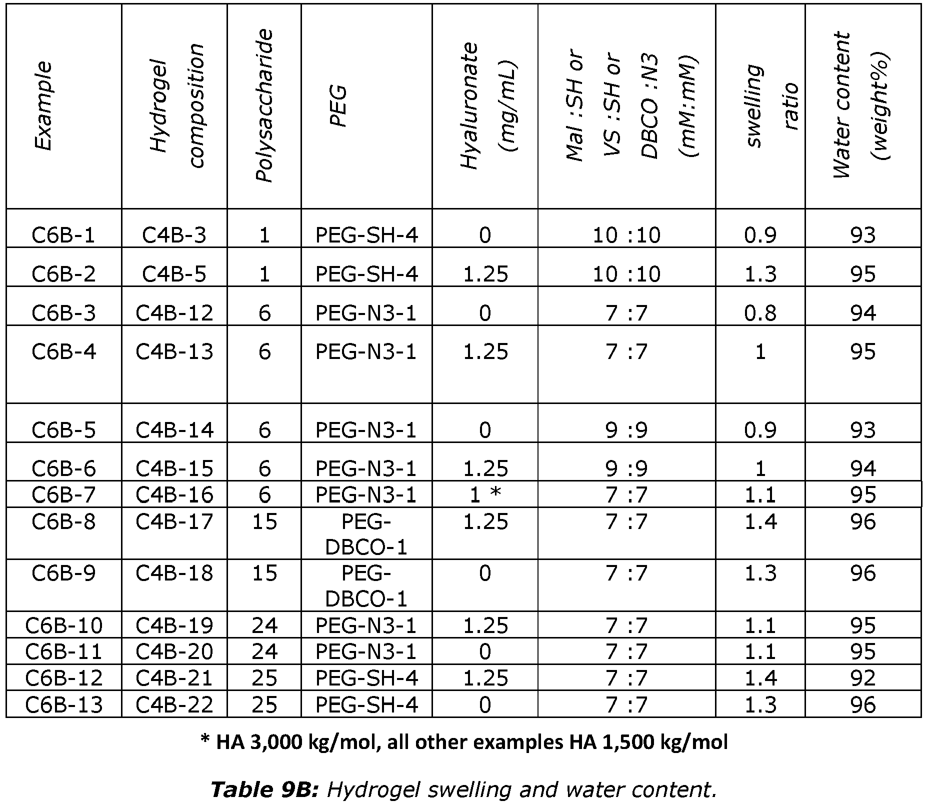

Hydrogels contain high water content.

### Example C7A: Hydrogel stability at 37°C in physiological medium

Hydrogel pieces were stored in PBS at pH 7.4 or in Serum (FBS Foetal Bovine Serum) at 37°C and weighed at different periods of time. Disc-shaped hydrogels with a diameter of 9 mm and a thickness of 1.6 mm were tested.

**Table 10A: Hydrogel stability in physiological medium**

| **Example** | **Hydrogel composition** | **medium** | **number of hydrogels weighed** | **Time at 37°C (days)** | **Gel Mass (mg)** |
|---|---|---|---|---|---|
| C7A-1 | C6A-2 | PBS | 2 | 0 | 134 ± 2 |
| | | | | 34 | 129 ± 5 |
| | | Serum | 2 | 0 | 133 ± 2 |
| | | | | 16 | 139 ± 1 |
| | | | | 34 | 137 ± 1 |

Hydrogels were retrieved intact and their mass does not evolve significantly upon storage at 37°C in physiological medium. Hydrogel swelling (mass increase) or dissolution (mass decrease) would be expected in case of network structure modification in case of hydrolysis side reaction for example. This shows that the hydrogel was stable under physiological conditions.

### Example C7B: Hydrogel stability at 37°C in physiological medium

Hydrogel pieces were stored in PBS at pH 7.4 or in Serum (FBS Foetal Bovine Serum) or acetate buffer 100mM pH=4 at 37°C. Pieces were weighed at different periods of time. Disc-shaped hydrogels with a diameter of 9 mm and a thickness of 1.6 mm were tested.

**Table 1OB: Hydrogel stability in physiological media**

| **Example** | **Hydrogel composition** | **media** | **number of hydrogels weighed** | **Gel Mass t0** | **Time t at 37°C (days)** | **Gel Mass At time t (mg)** |
|---|---|---|---|---|---|---|
| C7B-1 | C4B-3 | PBS | 2 | 134 ± 2 | 34 | 134 ± 2 |
| | | | | 133 ± 5 | 365 | 141 ± 4 |
| | | Serum | 2 | 140 ± 2 | 16 | 133 ± 2 |
| | | | | 137 ± 1 | 34 | 137 ± 1 |
| C7B-2 | C4B-5 | PBS | 2 | 138 ± 1 | 34 | 138 ± 1 |
| | | | | 139 ± 3 | 365 | 153± 2 |
| | | Serum | 2 | 143 ± 3 | 16 | 139 ± 3 |
| | | | | 134 ± 7 | 34 | 132± 7 |
| C7B-3 | C4B-13 | PBS | 3 | 114 ± 3 | 28 | 117 ± 1 |
| | | | | | 84 | 115± 3 |
| C7B-4 | C4B-17 | PBS | 3 | 124 ± 5 | 28 | 136 ± 6 |
| | | | | | 84 | 201 ± 18 |
| C7B-5 | C4B-19 | PBS | 3 | 119 ± 2 | 34 | 123 ± 3 |
| | | acetate | 3 | 129 ± 3 | 34 | 113 ± 3 |
| C7B-6 | C4B-21 | PBS | 3 | 160± 4 | 34 | 138 ± 5 |
| | | acetate | 3 | 154 ± 10 | 34 | 130 ±14 |

Hydrogels were retrieved intact and their mass does not evolve significantly upon storage at 37°C in physiological medium. Hydrogel swelling (mass increase) or dissolution (mass decrease) would be expected in case of network structure modification in case of hydrolysis side reaction for example. This shows that the hydrogel was stable under physiological conditions.

At acidic pH, a rather good stability of the gels can be obtained as no significant mass decrease is reported.

### Example C8A: Encapsulation of macromolecular probes within hydrogel

Commercial Fluorescent Dextran-FITC 3 kDa and Dextran-FITC 70 kDa were each solubilized in water to obtain concentrated stock solutions.

Polysaccharide DMCMal and PEG-SH concentrated sterile solutions prepared according to example C1A and C2, respectively, were equilibrated at 4°C. For DMCVS based hydrogel, polysaccharide DMCMal and PEG-SH concentrated solutions prepared according to example C1B and C2, respectively, were equilibrated at 20-25°C.

The solution of polysaccharide DMCMal or DMCVS was mixed with a fluorescent dextran. 100 µL of a concentrated solution of PEG-SH was added to 100 µL of a concentrated solution of polysaccharide DMCMal or DMCVS and fluorescent dextran. The solutions were mixed with a pipette and 200 µL of the latter mixture was introduced in a rectangular silicone mould (IBIDI 12x7.75 mm). Cross-linking process leading to gelation was carried out for 1 h at room temperature (20-25°C) for DMCMal based hydrogel or 1h at 37°C for DMCVS based hydrogel.

The hydrogel piece was unmoulded, introduced in a well (12 wells multi-plate) and immersed with 1.3 mL of a buffer solution of Tris (200 mM)/NaCl (50 mM) at pH 8 containing the same concentration of encapsulated fluorescent dextran. Hydrogel swelling was performed overnight at 37°C and the supernatant was weighed to estimate the degree of swelling and the quantity (mg) in the hydrogel volume.

Hydrogels were quickly rinsed twice with 1 mL of a buffer solution of Tris (200 mM)/NaCl (50 mM) at pH 8 before release experiment.

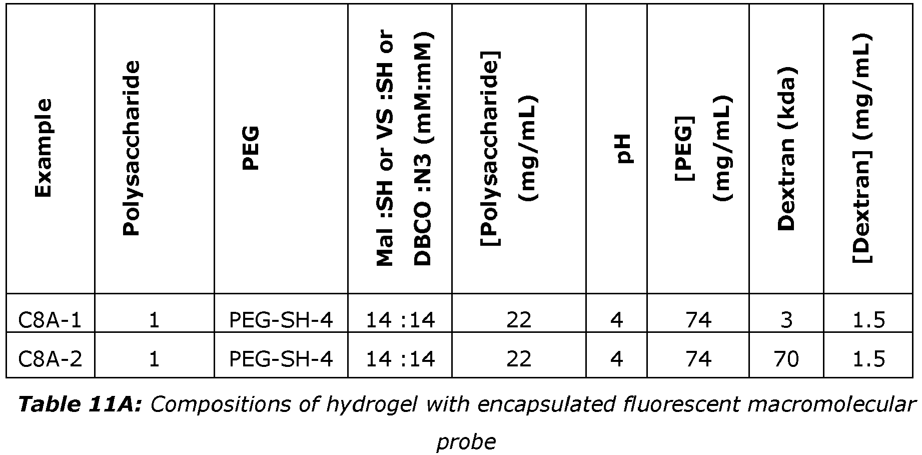

### Example C8B: Encapsulation of macromolecular probes within hydrogel

Commercial Fluorescent Dextran-FITC 3 kDa and Dextran-FITC 70 kDa were each solubilized in water to obtain concentrated stock solutions.

Polysaccharide and PEG or Polysaccharide DMCMal and PEG-SH concentrated sterile solutions prepared according to example C1A, C1B and C2, respectively, were equilibrated at 4°C. For DMCVS based hydrogel, polysaccharide DMCMal and PEG-SH concentrated solutions prepared according to example C1B and C2, respectively, were equilibrated at 20-25°C.

The solution of polysaccharide DMCMal or DMCVS or DMCDBCO was mixed with a fluorescent dextran. 100 µL of a concentrated solution of PEG-SH or PEG-N3 was added to 100 µL of a concentrated solution of polysaccharide, polysaccharide DMCMal or DMCVS and fluorescent dextran. The solutions were mixed with a pipette and 200 µL of the latter mixture was introduced in a rectangular silicone mould (IBIDI 12x7.75 mm). Crosslinking leading to gelation was carried out for 1 h at room temperature (20-25°C) for DMCMal and DMC-DBCO based hydrogel or 1h at 37°C for DMCVS based hydrogel.

The hydrogel piece was unmoulded, introduced in a well (12 wells multi-plate) and immersed with 1.3 mL of a buffer solution of Tris (200 mM)/NaCl (50 mM) at pH 8 containing the same concentration of encapsulated fluorescent dextran. Hydrogel swelling was performed overnight at 37°C and the supernatant was weighed to estimate the degree of swelling and the quantity (mg) in the hydrogel volume.

Hydrogels were quickly rinsed twice with 1 mL of a buffer solution of Tris (200 mM)/NaCl 50 mM at pH 8 before release experiment shown in example C7B.

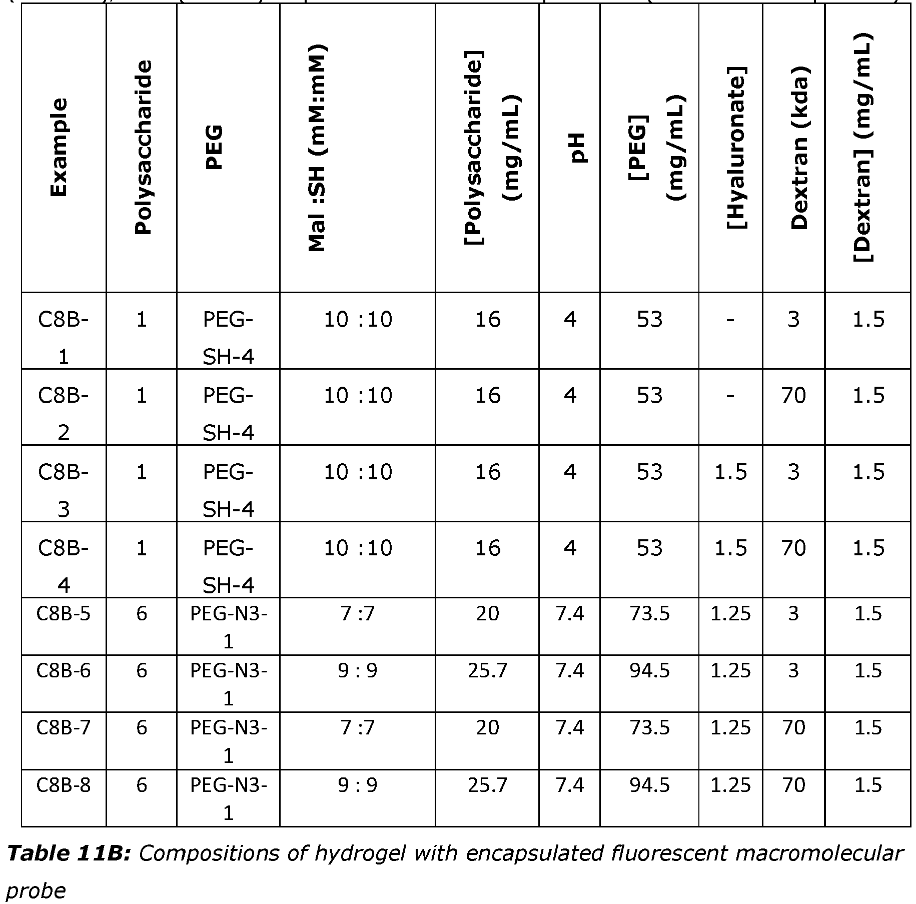

### Example C9A: Release of macromolecular probes within hydrogel

Hydrogels with encapsulated fluorescent probe as produced in Example C8A were each introduced in a well (12 wells multi-plate) and immersed with 2 mL of a buffer solution of Tris (200 mM)/NaCl (50 mM) at pH 8. The plate was covered with a film and introduced in an oven at 37°C. 200 µL of buffer were sampled at different time point and replaced by fresh buffer. Fluorescent probe concentration in the samples was determined by fluorescence (fluorescent plate reader SAFAS) using a calibration curve. The cumulative fraction of fluorescent probe released at each time point corresponds to the ratio of the cumulative quantity of fluorescent probe released to the initial quantity of fluorescent probe in the swollen hydrogel.

**Table 12A: Cumulative fraction of macromolecular probes released from the hydrogel at different time points.**

| **Example** | **Composition** | **Time (h)** | **0.25** | **0.5** | **1** | **24** |
|---|---|---|---|---|---|---|
| C9A-1 | C8A-1 | % Dextran 3 kDa released | >10 | >20 | >30 | >40 |
| C9A-2 | C8A-2 | % Dextran 70 kDa released | <5 | <5 | <5 | <30 |

Increasing the size of the macromolecular probe leads to a slower kinetic of release. This shows the permselective property of the hydrogel's network structure.

### Example C9B: Release of macromolecular probes within hydrogel

Hydrogels with encapsulated fluorescent probe as produced in Example C8B were each introduced in a well (12 wells multi-plate) and immersed with 2 mL of a buffer solution of Tris (200 mM)/NaCl (50 mM) at pH 8. The plate was covered with a film and introduced in an oven at 37°C. 200 µL of buffer were sampled at different time point and replaced by fresh buffer. Fluorescent probe concentration in the samples was determined by fluorescence (fluorescent plate reader SAFAS) using a calibration curve. The cumulative fraction of fluorescent probe released at each time point corresponds to the ratio of the cumulative quantity of fluorescent probe released to the initial quantity of fluorescent probe in the swollen hydrogel.

**Table 12B: Cumulative fraction of macromolecular probes released from the hydrogel at different time points.**

| **Example** | **Composition** | **Time (h)** | **0.25** | **0.5** | **1** | **24** |
|---|---|---|---|---|---|---|
| C9B-1 | C8B-1 | % Dextran 3 kDa released | >10 | >20 | >30 | >40 |
| C9B-2 | C8B-2 | % Dextran 70 kDa released | <5 | <5 | <5 | <20 |
| C9B-3 | C8B-3 | % Dextran 3 kDa released | >10 | >20 | >30 | >40 |
| C9B-4 | C8B-4 | % Dextran 70 kDa released | <5 | <5 | <5 | <20 |
| C9B-5 | C8B-5 | % Dextran 3 kDa released | >20 | >30 | >40 | >50 |
| C9B-6 | C8B-6 | % Dextran 3 kDa released | >20 | >20 | >40 | >50 |
| C9B-7 | C8B-7 | % Dextran 70 kDa released | <5 | <5 | <10 | <20 |
| C9B-8 | C8B-8 | % Dextran 70 kDa released | <5 | <5 | <10 | <20 |

Increasing the size of the macromolecular probe leads to a slower kinetic of release. This shows the permselective property of the hydrogel's network structure.

### Example C10: Moulding of thin hydrogel discs

According to the protocol described for example C3A, concentrated polymer solutions were equilibrated at 4°C. The solutions were mixed with a pipette and a controlled volume of the mixture was introduced in a circular silicone isolator (10 mm diameter and 0.5 mm, 432 or 356 µm thick) adhering to a glass slide. A second glass side is placed on the drop to promotes its spreading in the entire diameter if the mold.

Upon 1 hour of cross-linking process leading to gelation at 20-25°C hydrogel discs were unmoulded. The thickness was deduced from the diameter and the volume of the gel.

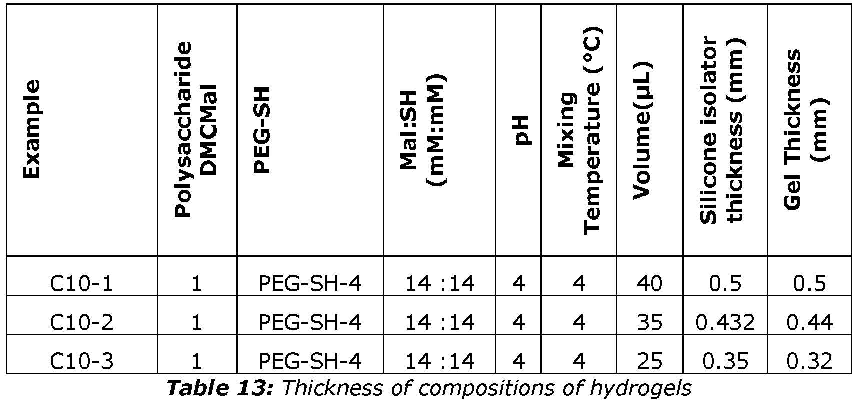

The method allows to obtain hydrogels discs of controlled diameters and thicknesses by adjusting the diameter of the mould and the volume of hydrogel.

### Example C11A: Determination of mechanical resistance of the hydrogels.

For compression, a swollen rectangular hydrogel piece as described in example C3A was introduced in a flat glass crystallizer and immersed in PBS. The uniaxial compression was done in NaCl 0.9% at 20-25°C by using a universal mechanical tester apparatus (Zwickroell) equipped with flat compression plates, at a speed of 0.2 mm/min. Initial thickness of the sample was determined from the contact of the plate with the hydrogel, when the force starts to increase. The deformation is defined by the ratio of the compression displacement (mm) and the initial thickness (mm). The deformation at break was determined from the force/displacement curve. The break is defined when a decrease of the force *versus* displacement was observed.

For traction, dog bone shaped hydrogel pieces were prepared by moulding hydrogel in a dog bone shaped silicone mould. The uniaxial traction was done in NaCl 0.9% at 20-25°C with a universal mechanical tester apparatus (Zwickroell) equipped with screw grips, at a speed of 3 mm/min. Initial length of the sample was measured between the grips with a ruler. The deformation is defined by the ratio of the traction displacement (mm) and the initial length (mm). The deformation at break was determined from the force/displacement curve. The break is defined when a decrease of the force *versus* displacement was observed. The Young modulus was determined from the slope of the true strain / deformation curve. The true strain (kPa) corresponds to the ratio of the force (N) to the instantaneous surface area (mm2) of the sample under compression.

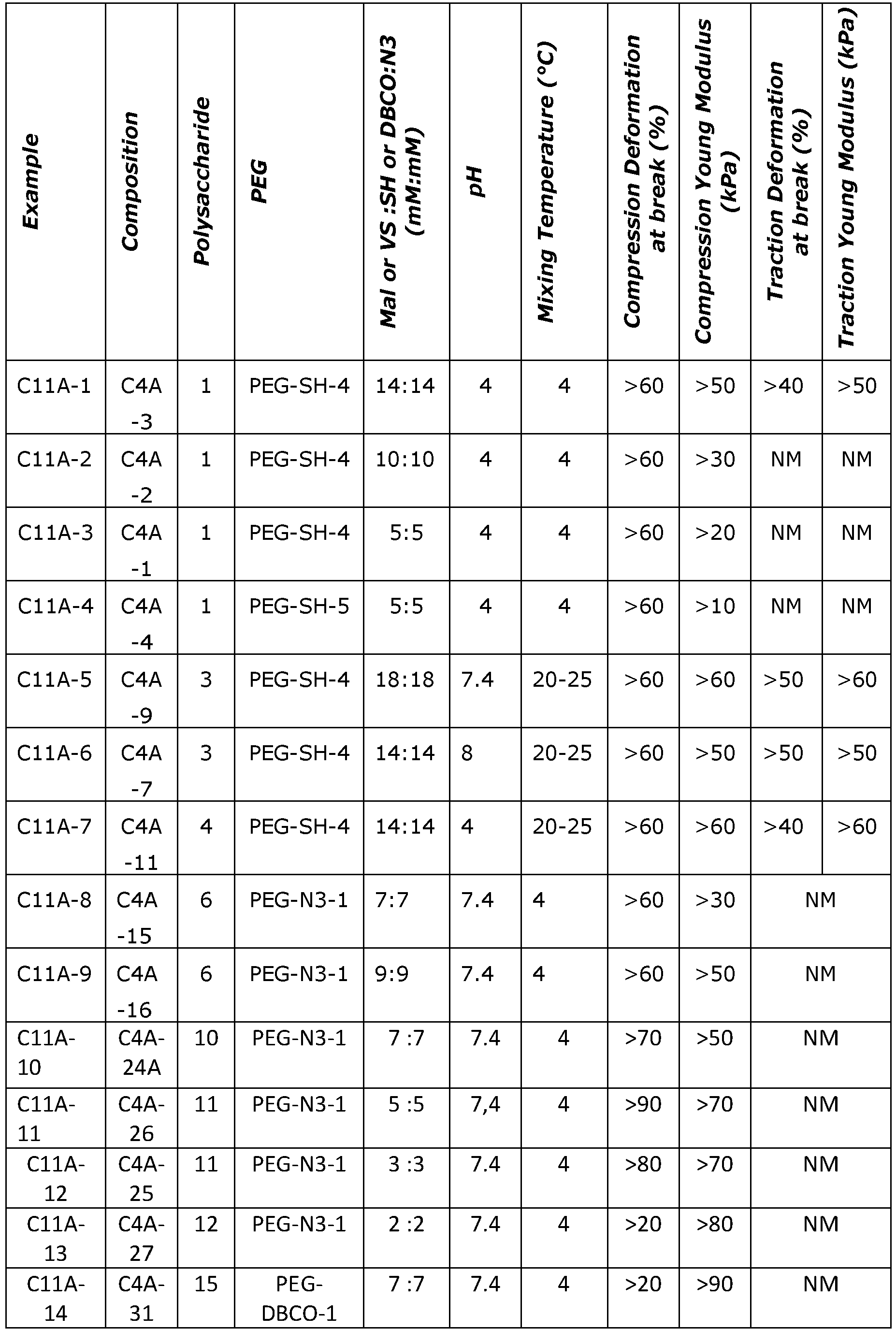

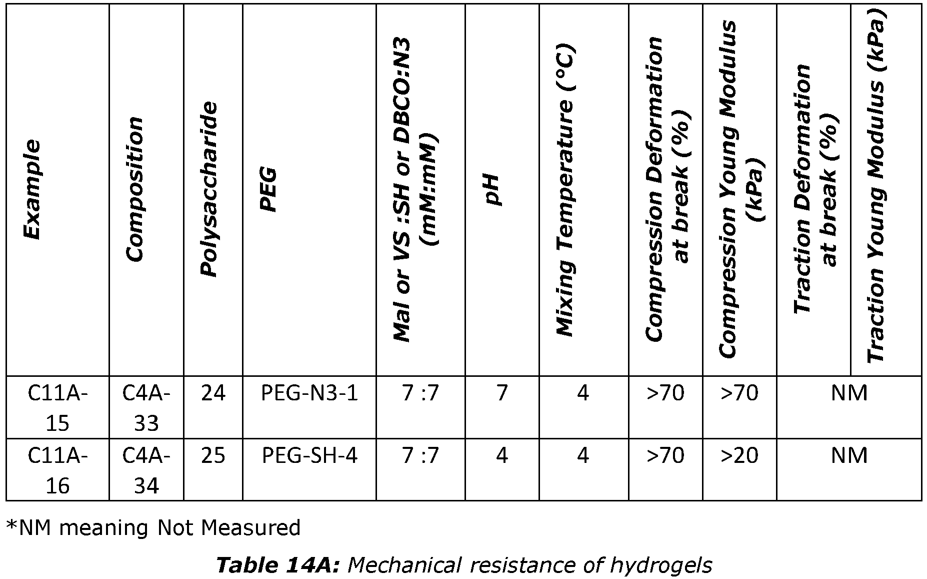

The hydrogels according to the invention exhibit very good mechanical resistances features combining deformability and stiffness suitable for surgical implantation.

### Example C11B: Determination of mechanical resistance of the hydrogels.

For compression, a swollen rectangular hydrogel piece as described in example C3B was introduced in a flat glass crystallizer and immersed in PBS. The uniaxial compression was done in NaCl 0.9% at 20-25°C by using a universal mechanical tester apparatus (Zwickroell) equipped with flat compression plates, at a speed of 0.2 mm/min. Initial thickness of the sample was determined from the contact of the plate with the hydrogel, when the force starts to increase. The deformation is defined by the ratio of the compression displacement (mm) and the initial thickness (mm). The deformation at break was determined from the force/displacement curve. The break is defined when a decrease of the force *versus* displacement was observed.

The Young modulus was determined from the slope of the true strain / deformation curve. The true strain (kPa) corresponds to the ratio of the force (N) to the instantaneous surface area (mm2) of the sample under compression.

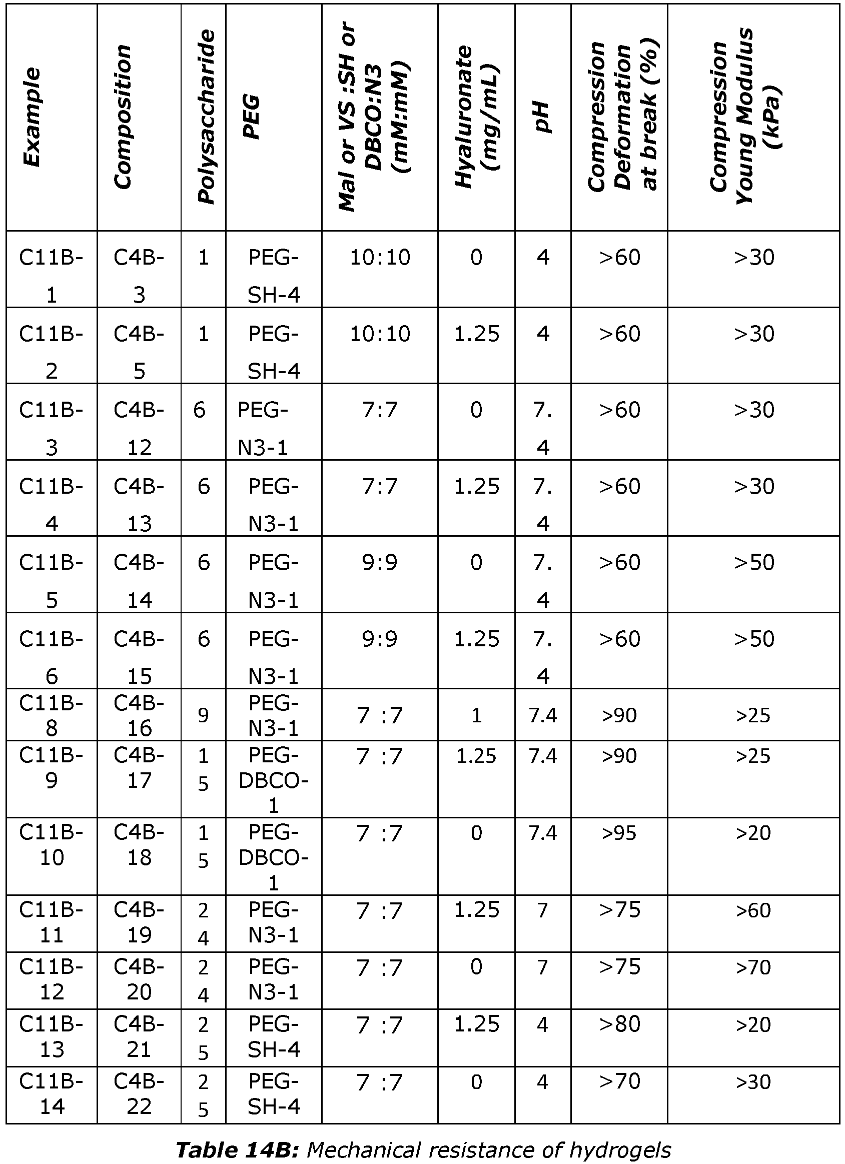

The hydrogels according to the invention exhibit very good mechanical resistances features combining deformability and stiffness suitable for surgical implantation.

### Example C12: surgical net

### Example C12A: Alkaline treatment of polyester surgical net

Warp knit Polyester multifilament surgical net fabric type PETKM3002 (1x0.9mm pore size) supplied by SurgicalMeshTM were treated in NaOH 1M for 5 hours at 70°C and rinsed with deionized water and ethanol 96%. The treatment led to an increased hydrophilicity of the net fabric leading to an improved wetting with aqueous solutions of polymers constituting the hydrogel.

### Example C12B: PVDF surgical net fabric

Warp knit PVDF surgical net fabric (1x1 mm pore size) supplied by Dynamesh formed by knitted monofilament of 100 µm diameter.

### Example C12C: PTFE perforated surgical film

PTFE surgical film of 150 µm thickness perforated by star patterns (1x2mm pore size) supplied by Aran Biomedical.

### Example C13: Fabrication of Constructs

### Example C13A: Fabrication of Ring Net constructs

Biocompatible PDMS sheets supplied by Grace Biolabs or Interstate Speciality Product or Limitless Shielding were cut in a form of a square incorporating a circular empty disc using a stainless steel punch.

A part of the treated polyester surgical net or part of the surgical net described in example C12 was introduced in between two square PDMS pieces: The two PDMS pieces and the surgical net were glued together with biocompatible silicone glue (Silbione MED ADH 4200 supplied by Elkem). The circular empty discs were aligned, and the surgical net was kept tense during gluing.

Finally, the square construct was cut with a stainless steel punch to obtain the final object constituted by two PDMS rings sandwiching a surgical net and glued together.

The pieces were washed with a solution of poloxamer F127 at 1% and rinsed with water before steam or ETO (Ethylene oxide) sterilization.

### Example C13B: Fabrication of Scaled rectangular constructs

For human surgery, to allow an easier surgical implantation via a trocar of interior diameter of 15mm, the implant could have an elongated form such as a rectangle with width of less than 100 mm.

Biocompatible PDMS sheets supplied by Grace Biolabs or Interstate Specialty Product or Limitless Shielding were cut in a empty frame of different form such as elongated rectangle.

A part of the surgical net described in example C12 was introduced in between frame PDMS pieces: The two PDMS pieces and the surgical net were glued together with biocompatible silicone glue (Silbione MED ADH 4200 supplied by Elkem). The empty frames were aligned, and the surgical net was kept tense during gluing.

Finally, the surplus of net surrounded the frame was cut to obtain the final object constituted by two PDMS frames sandwiching a surgical net and glued together.

A surplus of net surrounded the frame can be deliberated uncut to make to make easier the fixation to tissue via resorbable or non-resorbable tacks.

The pieces were washed with a solution of poloxamer F127 at 1% and rinsed with water before steam or ETO sterilization.

### Example C14: Constructs of different dimensions

### Example C14A: Ring net constructs of different dimensions

Different Ring Net constructs were produced according to example C13A by varying parameters such as internal/external diameter and thickness of the PDMS rings and the thickness of the glue.

**Table 15A: Dimensions of Ring Net constructs.**

| **Example** | **PDMS Ring thickness (each part) (µm)** | **Interior Ring diameter (mm)** | **Exterior Ring Diameter (mm)** | **Net material** | **Net type** | **Net thickness (µm)** | **Glue thickness (µm)** | **Total thickness (µm)** |
|---|---|---|---|---|---|---|---|---|
| C14A-1 | 430 | 12 | 16 | PET | 3002 | 230 | ~200 | 1250 |
| C14A-2 | 250 | 12 | 16 | PET | 3002 | 230 | ~200 | 900 |
| C14A-3 | 250 | 16 | 20 | PET | 3002 | 230 | ~200 | 900 |
| C14A-4 | 250 | 36 | 44 | PET | 3002 | 230 | ~200 | 900 |
| C14A-5 | 150 | 12 | 16 | PET | 3002 | 230 | ~200 | 700 |
| C14A-6 | 150 | 12 | 16 | PET | 3002 | 230 | ~100 | 600 |
| C14A-7 | 100 | 12 | 16 | PET | 3002 | 230 | ~100 | 500 |
| C14A-8 | 100 | 12 | 16 | PVDF | - | 230 | ~100 | 500 |
| C14A-9 | 100 | 12 | 16 | PTFE | - | 150 | ~100 | 450 |

### Example C14B: Scaled constructs of different dimensions

Different constructs were produced according to example C13B by varying parameters such as internal/external diameter and thickness of the PDMS rings and the thickness of the glue.

**Table 15B: Dimensions of scaled constructs.**

| **Exampl e** | **PDMS Ring thicknes s (each part) (µm)** | **PDM S widt h (mm** ) | **Interio r width (mm)** | **Interio r length (mm)** | **Net** | **Net surplu s width (mm)** | **Glue thicknes s (µm)** | **Total thicknes s (µm)** |
|---|---|---|---|---|---|---|---|---|
| C14B-1 | 300 | 10 | 50 | 60 | C12 A | 0 | ∼200 | 1000 |
| C14B-2 | 100 | 10 | 50 | 60 | C12 A | 0 | ∼200 | 650 |
| C14B-3 | 300 | 5 | 30 | 220 | C12 B | 0 | ∼200 | 1000 |
| C14B-4 | 300 | 5 | 30 | 120 | C12 B | 0 | ∼200 | 1000 |
| C14B-5 | 300 | 5 | 50 | 60 | C12 B | 10 | ∼200 | 1000 |
| C14B-6 | 100 | 5 | 40 | 60 | C12 C | 10 | ∼200 | 550 |

### Exemple C15: Hydrogel / construct composites compositions

### Exemple C15A: Hydrogel / Ring Net composites compositions

Hydrogel compositions prepared according to example C3B and described in example C4B were incorporated in the Ring Net constructs described in example C14. The concentrated polymer solutions were mixed with a pipette and a controlled volume of the mixture was introduced in a ring net construct adhering to a glass slide.

Crosslinking leading to gelation was carried out for 1h at room temperature (20-25°C) or at 37°C. The Ring Net / Hydrogel composition was then introduced in a Tris 150 mM /NaCl 30 mM / Cystein 10 mM solution at pH 8 or in PBS at pH 7.4 for 1h at 37°C.

The hydrogel was rinsed with PBS solution without cysteine and further immersed in the PBS solution overnight at 37°C. The hydrogel piece was then stored in PBS solution at 4°C until being used.

Different Hydrogel / Ring Net composites compositions were prepared (Table 16A):

**Table 16A: Hydrogel / Ring Net or scaled composites compositions.**

| **Example** | **Hydrogel Composition** | **Ring Net Construct** | **Hydrogel Volume (µL)** | **Cysteine 10 mM bath** |
|---|---|---|---|---|
| C15A-1 | C4B-5 | C14A-1 | 220 | Yes |
| C15A-2 | C4B-3 | C14A-2 | 120 | Yes |
| C15A-3 | C4B-5 | C14A-2 | 120 | Yes |
| C15A-4 | C4B-5 | C14A-2 | 140 | Yes |
| C15A-5 | C4B-5 | C14A-3 | 230 | Yes |
| C15A-6 | C4B-5 | C14A-4 | 500 | Yes |
| C15A-7 | C4B-5 | C14A-5 | 110 | Yes |
| C15A-8 | C4B-13 | C14A-6 | 90 | Yes |
| C15A-9 | C4B-13 | C14A-6 | 80 | No |
| C15A-10 | C4B-13 | C14A-7 | 90 | No |
| C15A-11 | C4B-13 | C14A-7 | 80 | No |
| C15A-12 | C4B-13 | C14A-8 | 90 | No |
| C15A-13 | C4B-13 | C14A-9 | 90 | No |

Hydrogel/Ring Net composites are thus easily manipulated with tweezers and are foldable for the need of surgical implantation, in particular for mini-invasive surgery. Moreover, it is possible to fix the ring to tissue with sutures.

The hydrogel volume can be adjusted with the internal diameter and the thickness or the ring net construct. For the same ring net construct the hydrogel volume can be adjusted to control the convexity of the hydrogel above the ring level.

### Exemple C15B: Hydrogel / Scaled composites compositions

To facilitate the implantation, to ensure the absence of filling defects and to limit the sedimentation of the islets, the scaled implant for human assay were manufactured via a device allowing its filling by hydrogel horizontally or vertically:

The device consists of glass plates on which are affixed on one side a sheet of silicone of thickness of 50 to 2000 µm. One of the glass plates and the corresponding sheet are perforated with two holes of diameter 1 to 6 mm. A female luer lock is glued in the middle of one of the holes. The empty implant is placed between the two sheets of laminated glass. The assembly is tightened on both sides against the silicone frame of the construct in order to guarantee the desired thickness and the sealing during the casting of the hydrogel.

The adapted volumes of the hydrogel composition were injected via two methods into the scaled construct respectively:
a. Reconstitution and injection: After mixing the gel precursor solutions, the gel solution was placed in a syringe with a male Luer lock. The syringe was screwed onto the Luer lock on the glass plate and then the gel was injected into the implant.
b. Dual syringe injection with mixing chamber (Twin-Syringe Biomaterial Delivery System (M-System) from MedMix^{®}): Hydrogel precursor solutions were placed separately in two syringes. A coupling to connect the flux of the two syringes was extended by a mixing chamber with a male Luer lock that was screwed onto the filling device. By pushing on both syringes at the same time with a medmix^{®} clamp, the hydrogel was reconstituted and immediately injected into the device.

**Table 16B: Hydrogel / Ring Net or scaled composites compositions.**

| **Example** | **Hydrogel Composition** | **Ring Net Construct** | **Hydrogel Volume (µL)** | **Cysteine 10 mM bath** |
|---|---|---|---|---|
| C15B-1 | C4B-13 | C14B-1 | 3000 | NO |
| C15B-2 | C4B-13 | C14B-2 | 2000 | NO |
| C15B-3 | C4B-13 | C14B-3 | 6600 | NO |
| C15B-4 | C4B-13 | C14B-4 | 3600 | NO |
| C15B-5 | C4B-13 | C14B-5 | 3000 | NO |
| C15B-6 | C4B-13 | C14B-6 | 1300 | NO |

### Example C16A: Encapsulation of insulin producing Islets in Hydrogels

Islets encapsulation was made in an aseptic environment.

Polysaccharide and PEG concentrated sterile solutions prepared according to examples C1A or C1B and C2, respectively, were adjusted with a concentrated NaCl solution in order to obtain isotonic stock solutions (300 mOsm/kg). Solutions were equilibrated either at room temperature (20-25°C or at 4°C).

A concentrated mixture of PEG and islets was prepared by mixing equal volumes of isotonic PEG solution (prepared according to example C2) and islets or pseudoislet suspension (prepared according to example B1A or B2A). Then the mixture containing PEG and islets was gently mixed with an isotonic concentrated solution of polysaccharide by a 70:30 Volume:Volume proportion of islets suspension/PEG: polysaccharide.

The solutions were gently mixed with a pipette and a controlled volume of the mixture was introduced in a circular silicone isolator from Grace Biolab adhering to a glass slide. Different hydrogel sizes were made depending on the volume and silicone mold diameter.

**Table 17A: Moulded hydrogels volume and size**

| Volume (µL) | Mould Diameter (mm) |
|---|---|
| 10 | 4.5 |
| 32 | 9 |
| 50 | 10 |
| 66 | 13 |
| 160 | 20 |

Cross-linking process leading to gelation was carried out for 1 h at room temperature (RT, 20-25°C) or in an oven at a controlled temperature such as 20, 25 or 37°C. The hydrogel incorporating islets was unmolded and introduced in a Tris 150 mM / NaCl 30 mM / Cystein 10 mM solution at pH 8 or in culture medium for 15 min at room temperature. Hydrogels were then immersed in culture medium containing 10 mM cysteine for 1 h at 37°C. After 1 h the culture medium containing cysteine was removed and replaced by culture medium. Sterile hydrogels containing cells were stored at 37°C and 5% CO2 before further in vitro testing or *in vivo* implantation.

Different hydrogel compositions were prepared according to this process, either with pseudo islets or primary islets.

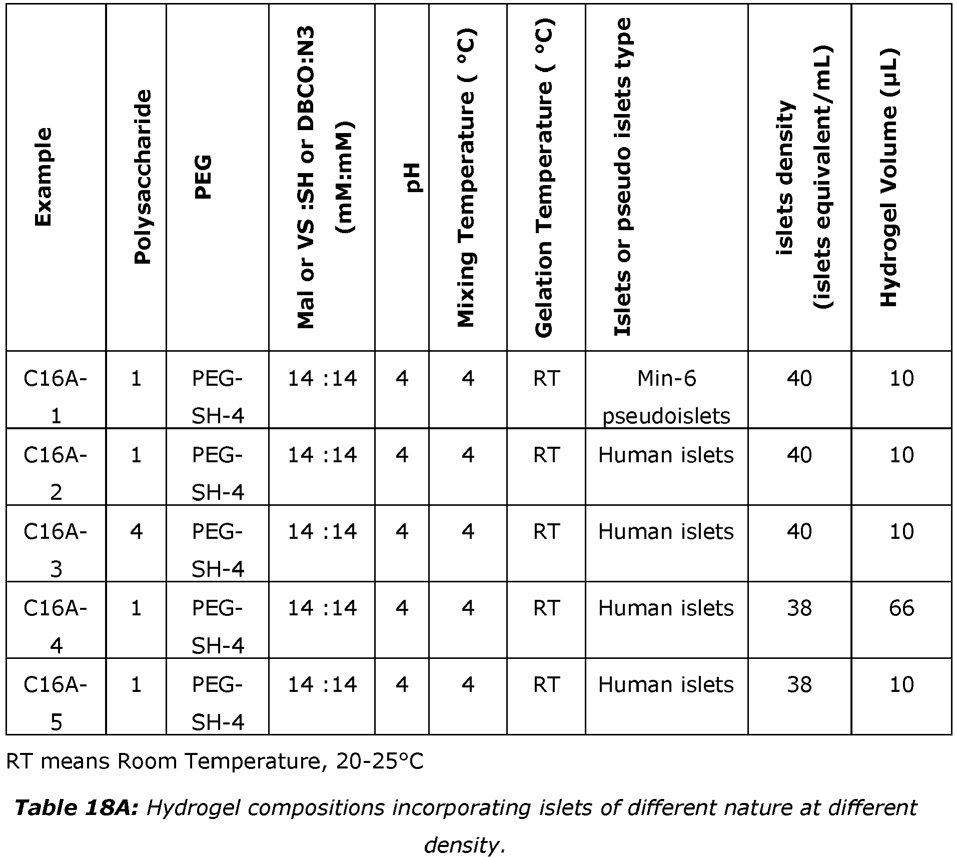

### Example C16B: Encapsulation of insulin producing cell islets in Hydrogels

Cell islets encapsulation was made in an aseptic environment.

Polysaccharide and PEG concentrated sterile solutions prepared according to examples C1A or C1B and C2, respectively, were adjusted with a concentrated NaCl solution in order to obtain isotonic stock solutions (300 mOsm/kg). Solutions were equilibrated either at room temperature (20-25°C or at 4°C).

A concentrated mixture of PEG and islets was prepared by mixing equal volumes of isotonic PEG solution or PEG / Sodium hyaluronate solution (prepared according to example C2, C3 and C3B) and islets suspension (prepared according to example B1B). Then the mixture containing PEG and islets was gently mixed with an isotonic concentrated solution of polysaccharide by a 70:30 Volume:Volume proportion of islets suspension/PEG : polysaccharide or 80:20 Volume:Volume proportion of islets suspension/ PEG/Hyaluronate : polysaccharide.

The solutions were gently mixed with a pipette and a controlled volume of the mixture was introduced in a circular silicone isolator (from Grace Biolab) adhering to a glass slide or in a Ring Net construct. Different hydrogel sizes were made by using different silicone molds (Table 5B) or ring net constructs (Table 16)

Crosslinking leading to gelation was carried out for 1 h at room temperature (RT, 20-25°C) or in an oven at a controlled temperature such as 20, 25 or 37°C. The hydrogel disc or hydrogel / ring net incorporating islets was introduced in a Tris 150 mM / NaCl 30 mM / Cystein 10 mM solution at pH 8 (Mal or VS : SH cross-linking) or in culture medium for 15 min at room temperature (DBCO:N3 cross-linking). Hydrogels were then immersed in culture medium containing 10 mM cysteine for 1 h at 37°C or in culture medium without cysteine. After 1 h the culture medium containing cysteine was removed and replaced by culture medium. Sterile hydrogels containing cells were stored at 37°C and 5% CO2 before further in vitro testing or *in vivo* implantation.

Different hydrogel / Islets compositions were prepared according to this process.

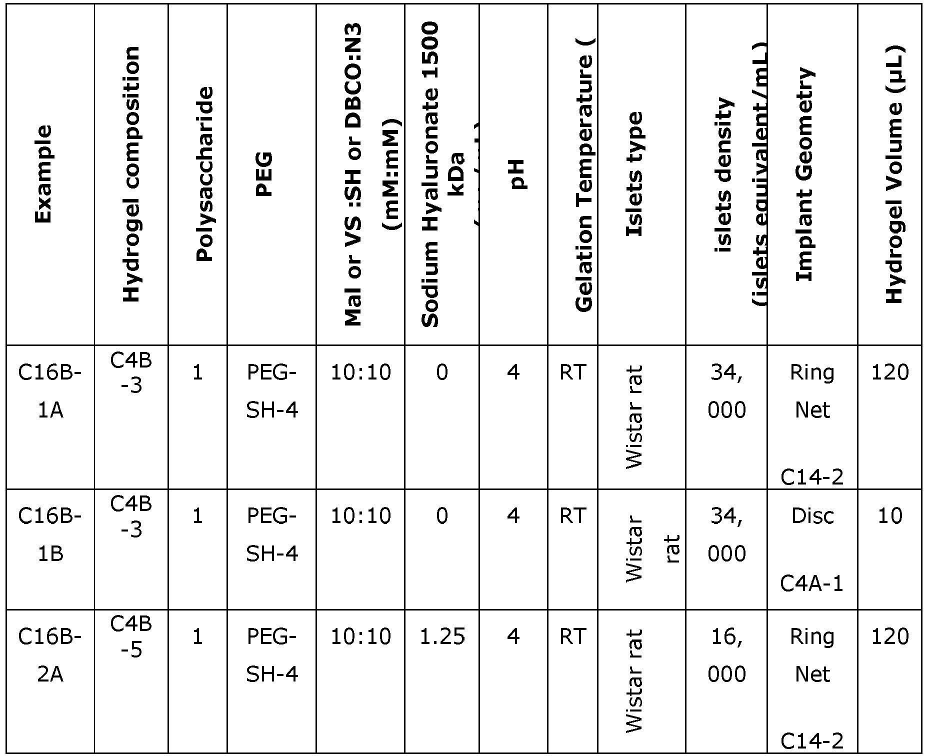

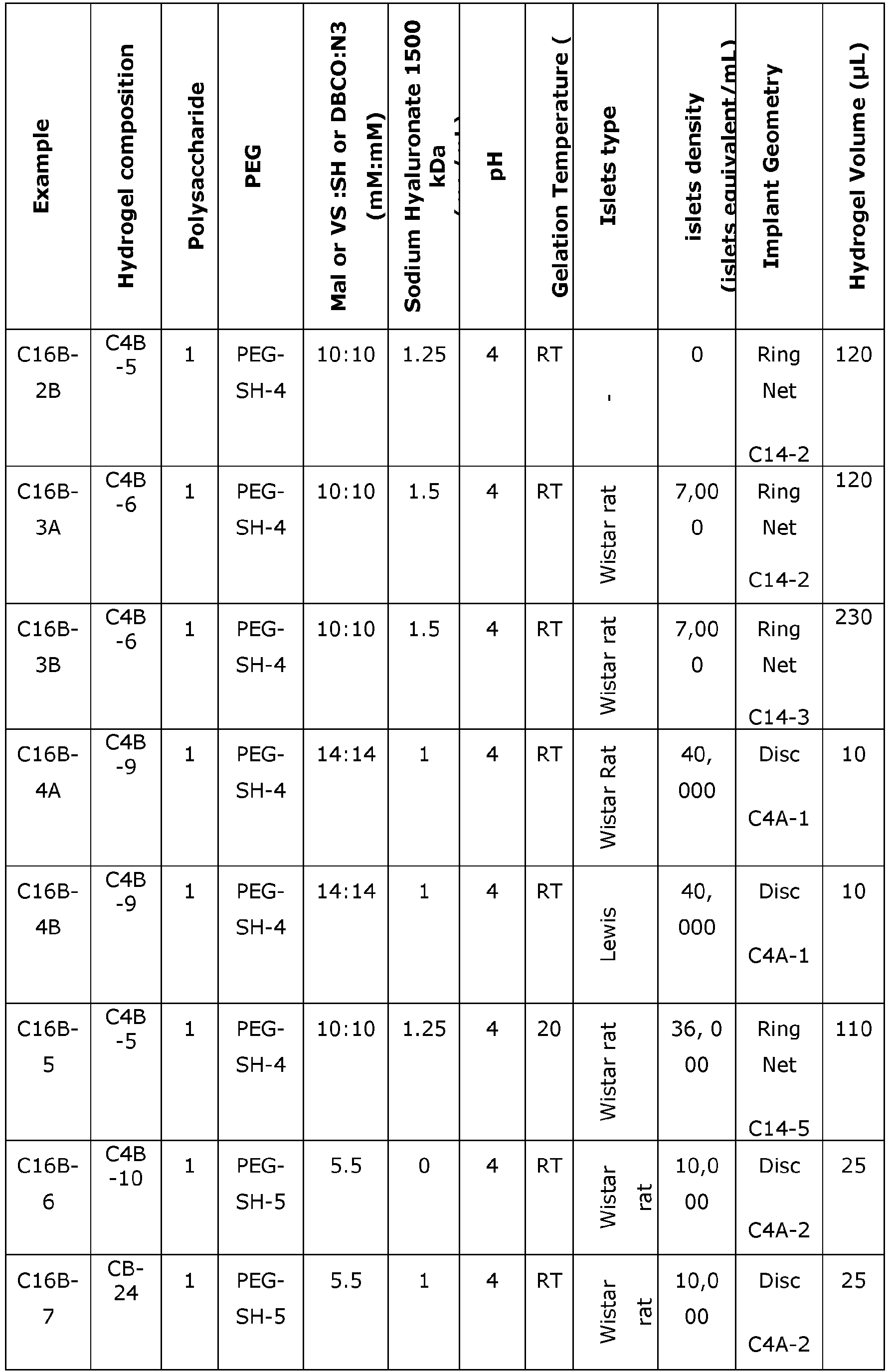

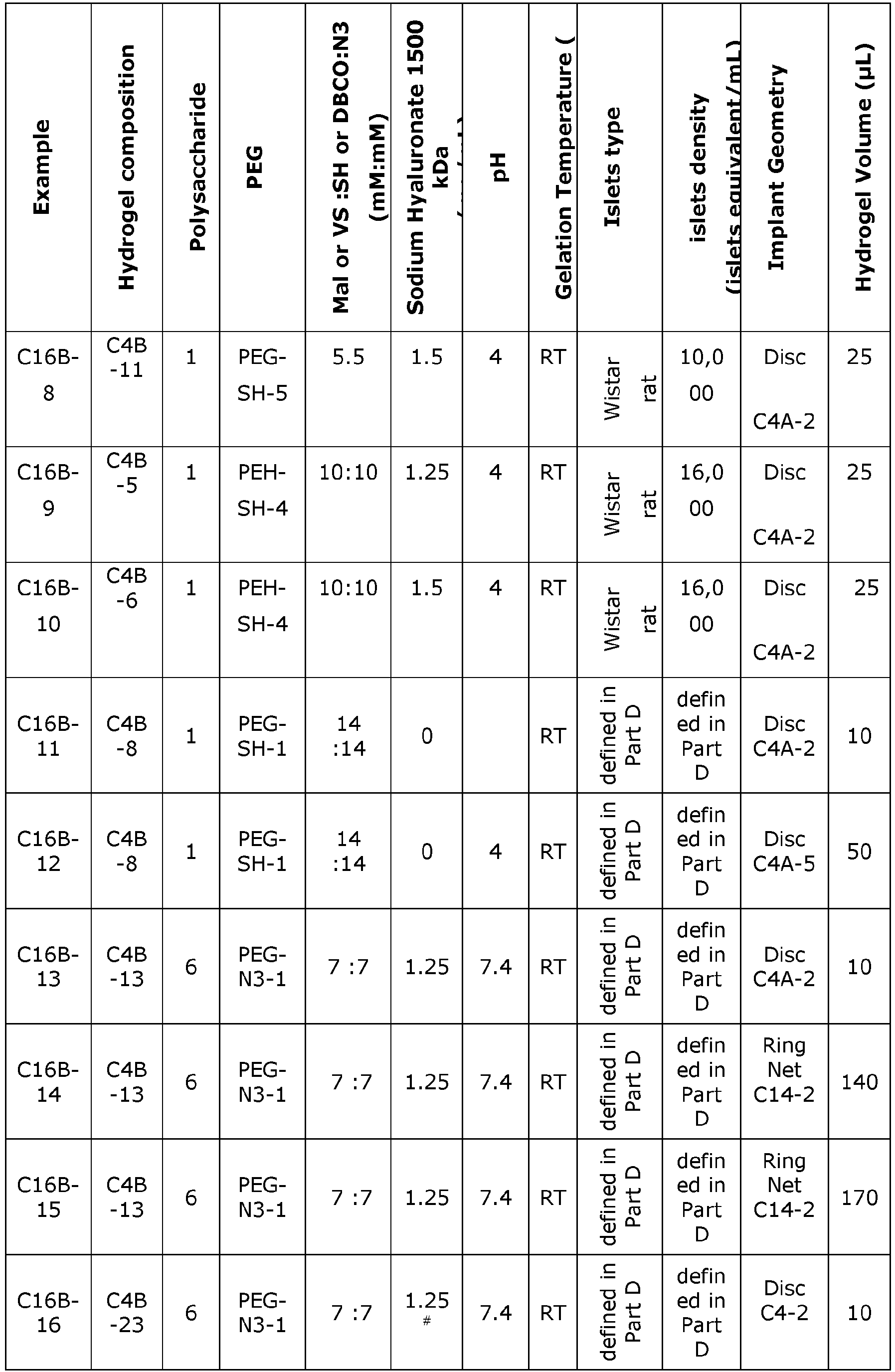

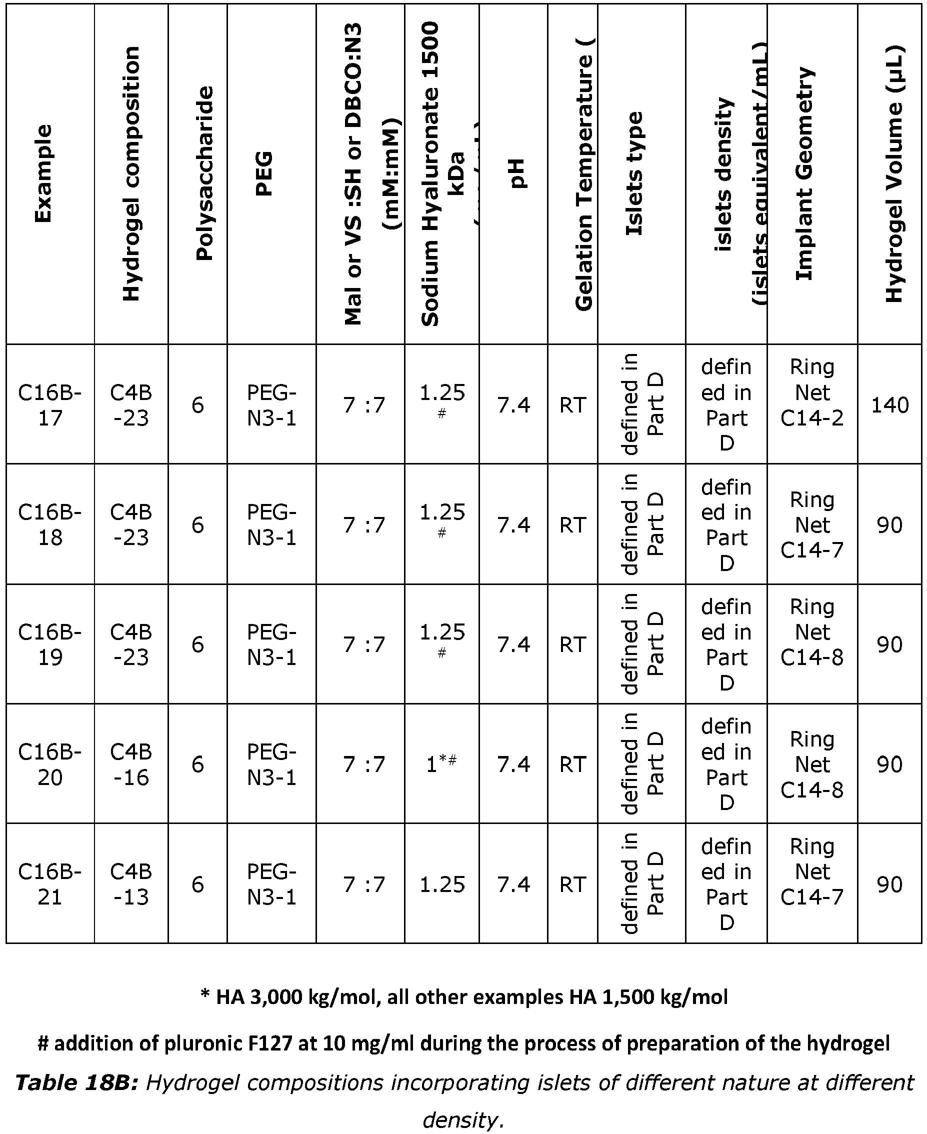

### Example C17: Spatial homogeneity of encapsulated cells in hydrogel thickness

Hydrogels discs incorporating islets were prepared as described in example C16B. Hydrogel pictures were taken from side to evaluate islets homogeneity through the thickness of the hydrogel. Homogeneity is achieved when the islets are located within the whole thickness of the hydrogel and not essentially localized on one side of the hydrogel.

**Table 178 : Islets homogeneity in hydrogels**

| **Example** | **Composition** | **Islet Homogeneity** |
|---|---|---|
| C17-1 | C16B-6 | No |
| C17-2 | C16B-7 | No |
| C17-3 | C16B-8 | Yes |
| C17-4 | C16B-9 | Yes |
| C17-5 | C16B-10 | Yes |
| C17-6 | C4B-13 | Yes |
| C17-7 | C4B-23 | Yes |
| C17-8 | C4B-26 | Yes |

The addition of sodium hyaluronate increases the mixture viscosity and permit to obtain homogeneity of the spatial localization of the islets in the thickness of the hydrogel.

The use of longer hyaluronate chain allows to reach higher level of homogeneity.

### Example C18: Evaluation and control of hydrogel transparence

Hydrogel compositions described in examples C4A and C4B and other hydrogels disclosed in Examples C are visually transparent.

To quantify the transparency property of the hydrogels, UV absorbance measurements (Spectrofluorimetrer XENIUS^{®} SAFAS) were done by casting 80µL precursor solution in an 96 well plate. 100µL of PBS were added on the top on the gel to avoid drying. Absorbance measurement at 400 nm is used to quantify the turbidity level of the samples. Five measurements performed at different positions were averaged. Measurements were conducted 3hours after casting at fully swollen equilibrate state. The samples in the table below were also visually inspected in standardized conditions in front of black panel with a light level between 2,000 and 3,750 Lux (Adelphi^{®} Apollo II Liquid Inspection Unit).

Hydrogel of composition C4B-13 was prepared similarly to example C3B. Polysaccharide/pluronic DMCDBCO and PEG-N3 concentrated sterile solutions prepared according to example C1 and example C2, respectively, were adjusted with a concentrated NaCl solution in order to obtain isotonic stock solutions (300 mOsm/kg) and equilibrated at 4°C. PEG-N3 concentrated sterile solutions prepared according to

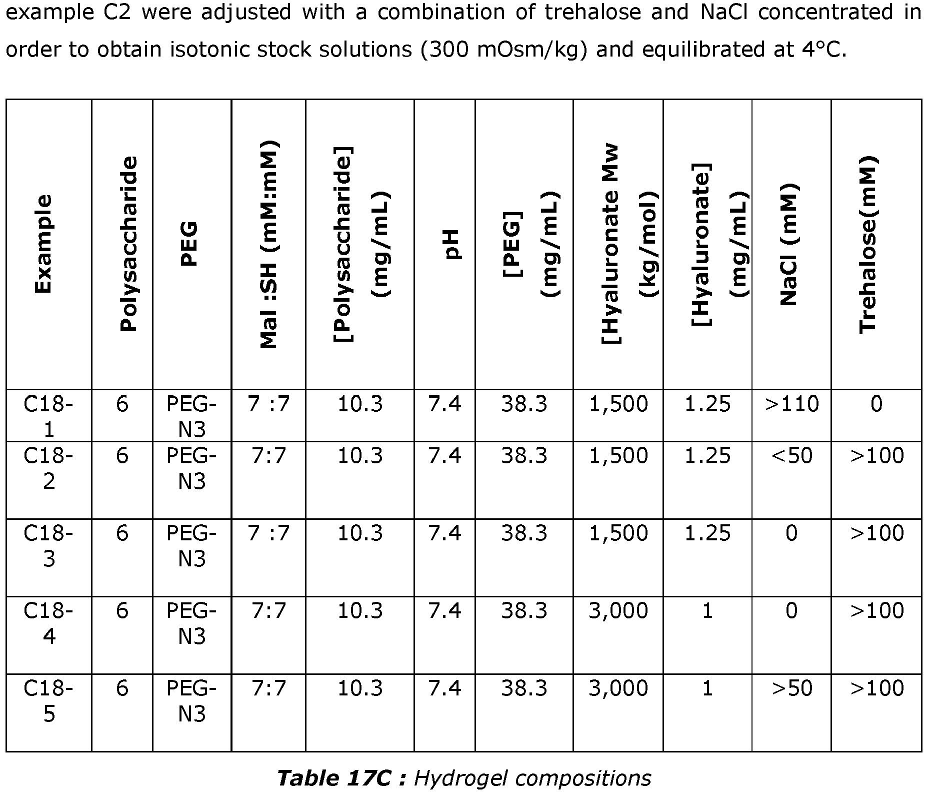

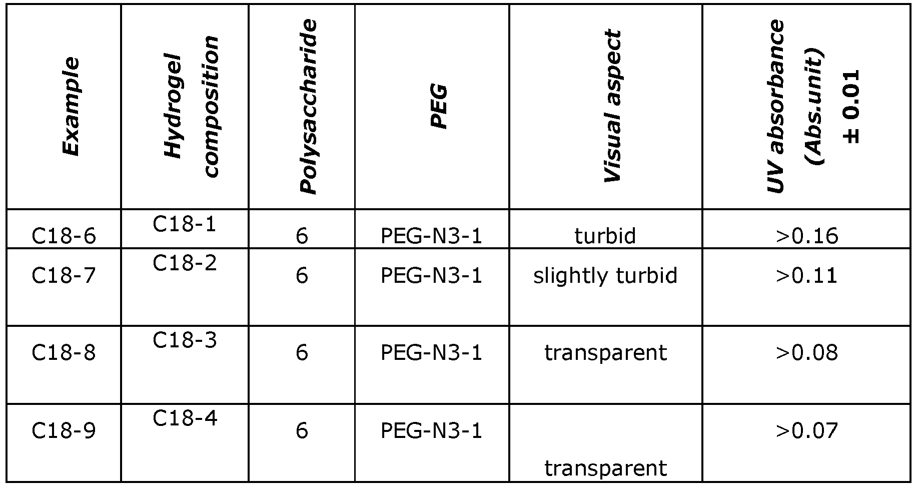

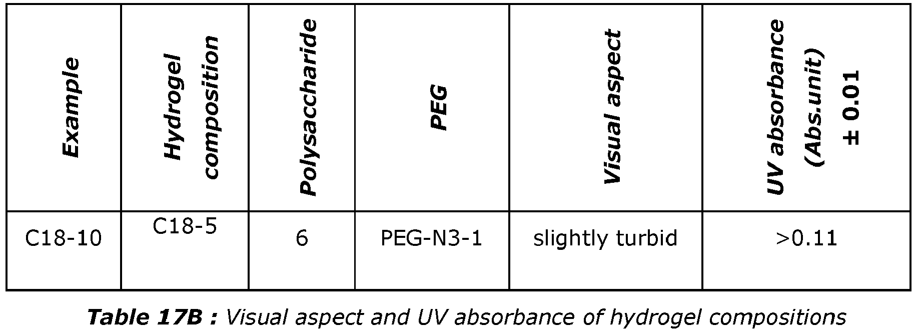

All the gels are transparent under "normal" conditions (real life).

However, as the changes of absorbance assess variable homogeneity state of the macroscopic network, for grading the impact of the change of osmotic agent on transparency, we had to the use harsh conditions as disclosed above. These results show that, in presence of HA, the turbidity of the hydrogel decreased when changing the osmotic agent NaCl to trehalose.

Others non ionic osmotic agent such as mannitol or glycerin could also be used to enhance transparency in the presence of HA.

Such characteristics do not imply a gel properties modification and no permearbility variations. Hence, no matter the degree of the turbidity here, the diffusion of small molecule such as insulin is maintained.

### Example C19: Laparoscopic implantation of Hydrogel / Scaled composites

The implantability of Hydrogel / Scaled composites was evaluated on domestic pig by laparoscopic surgery.

Two implants (C15B-1 and C15B-4) were each first inserted into a surgical pouch and then rolled up to be inserted into the peritoneal cavity via a trocar 15 mm. The implant is then extracted from the pouch and then fixed on the peritoneal (C15B-1) or at the level of a peritoneal breach (C15B-4) in contact with the muscle using stiches or non-resorbable surgery tacks fixed through the silicone frame.

The implants were intact / undamaged during the various operation and at the end of the implantation.

### Part D - BIOLOGICAL EVALUATIONS OF HYDROGELS

### Example D1A: In vitro evaluation of hydrogel cytotoxicity profile by extract test

The cytotoxicity profile of the hydrogel was evaluated using the extraction method following ISO 10993-5: Biological evaluation of medical devices recommendations.

The hydrogels were placed in culture medium (DMEM supplemented with 10% Fetal Bovine Serum and 1% Penicilin/Streptomycin) at 3 cm²/ml in a 24-well plate. They were incubated for 24 h at 37°C and 5% CO₂ under orbital agitation (70 rpm) to obtain the hydrogel extraction medium. In parallel, 3T3 cells were plated at 5000 cells/well in culture medium in 96-well plates and incubated overnight at 37°C and 5% CO₂. The next day, the medium of the cells was removed and replaced with the hydrogel incubation medium. After 24 h of incubation at 37°C and 5% CO₂ of the 3T3 cells with the extraction medium, the viability was measured by quantification of the intra-cellular ATP concentration with the ATPLite kit (Perkin Elmer), following the instruction of the manufacturer.

The viability percentage was calculated using the following formula: $Viability \left(%\right) = \frac{{Signal}_{test item}}{{Signal}_{control}} \times 100$

The cytotoxicity of hydrogel compositions C4A-21 was evaluated by this extract test. The results are shown in the Table 19 below.The viability percentage was compared to untreated control. The standard deviations (S.D.) of the mean viability of the triplicate wells was calculated (n=2 Hydrogels, each extraction was deposited in triplicate cell wells).

**Table 19A: Viability of hydrogel by extract test (ISO 10993-5). N=2 independent experiments.**

| | **Example C4A-21** | |
|---|---|---|
| | Mean (%) | S.D. (%) |
| Viability | 93.9 | 7.0 |

The selected composition did not show any significant cytotoxicity compared to untreated cells (t-test, p=0.48) (see Table 19).

### Example D1B: In vitro evaluation of C16B-18, C16B-19, C16B-B20 hydrogel cytotoxicity profile by extract test in presence or absence of Human islets

The cytotoxicity profiles of C16B-18, C16B-19 and C16B-20 were evaluated using the extraction method following ISO 10993-5: Biological evaluation of medical devices recommendations. The tested items were C16B-18, C16B-19 and C16B-20, in presence or absence of primay Human islets. Primary human islets were isolated following Examples B3A and encapsulated as described in examples C16B-18, C16B-19 and C16B-20.

C16B-18, C16B-19 and C16B-20 in presence or absence of islets were placed in culture medium (DMEM supplemented with 10% Fetal Bovine Serum and 1% Penicillin/Streptomycin) at 6cm²/mL in 6 well plates. They were incubated for 24 h at 37°C and 5% CO₂ under orbital agitation (70 rpm) to obtain the hydrogel extraction medium. In parallel, 3T3 cells were plated at 5000 cells/well in culture medium in 96-well plates and incubated overnight at 37°C and 5% CO₂. The next day, the medium of the cells was removed and replaced with the hydrogel incubation medium. After 24 h of incubation at 37°C and 5% CO₂ of the 3T3 cells with the extraction medium, the viability was measured by quantification of the intra-cellular ATP concentration with the ATPLite kit (Perkin Elmer), following the instruction of the manufacturer.

The viability percentage was calculated using the following formula: $Viability \left(%\right) = \frac{{Signal}_{test item}}{{Signal}_{control}} \times 100$ where control is untreated 3T3 cells.

The cytotoxicity of C16B-18, C16B-19 and C16B-20 were evaluated by extract test. Results are shown in table 19B. Values were normalized by untreated 3T3.

**Table 19B: Viability of 3T3 cells after treatment with C16B-18, C16B-19 and C16B-20 in the presence or absence of islets cells measured by extract test (ISO 10993-5). Negative control showed a viability of 75.2 ± 24.8 % (Mean ± SD). SD means Standard Deviation. N=2 independent experiments, n=3 independent hydrogels per experiment.**

| | Viability as compared to untreated 3T3 | | | | | |
|---|---|---|---|---|---|---|
| | C16B-18 | | C16B-19 | | C16B-20 | |
| | Mean (%) | SD (%) | Mean (%) | SD (%) | Mean (%) | SD (%) |
| No islets | 91.2 | 6.9 | 96.9 | 0.6 | 88.4 | 9.0 |
| With islets | 82.8 | 6.3 | 79.9 | 1.1 | 86.7 | 4.4 |

The selected compositions did not show any impact on 3T3 cells viability in the presence or absence of encapsulated Human islets (One-way ANOVA, p=0.7868; p= 0.7293; p= 0.5853; p= 0.9105; p= 0.8582; p= 0.6743, respectively for C16B-18, C16B-18 with islets, C16B-19, C16B-19 with islets, C16B -20, and C16B-20 with islets

In conclusion C16B-18, C16B-19 and C16B-20 in the presence or absence of encapsulated human islets is not associated with any cytotoxicity.

### Example D1C: Short-term in vitro functionality of rat primary islets encapsulated in Example C16B-11

2 days after isolation, rat primary islets were encapsulated as described in Example C16B-11 at 40 kIEQ/mL, and maintained in culture medium in a 37°C / 5% CO2 incubator (see Example B2B).

The functionality of rat primary islets was evaluated, 2 and 8 days after encapsulation. For the first time point, naked islets from the same islet preparation were evaluated in parallel. See Table 19C.

**Table 19C: Functionality of rat primary islets naked or encapsulated in Example C16B-11. Errors are standard deviations. N.A. Not Applicable. N=1 islet preparation, n = number of replicate. Ex = x days after encapsulation**

| Example C16B-11 | Time point | n= | Insulin secretion (nIU/IEQ/min) | Secretion index |
|---|---|---|---|---|
| Naked islets | E2 | 1 | 55.3 | 2.5 +/- N.A. |
| Encapsulated islets | | 2 | 27.1 +/- 1.4 | 2.4 +/- 1.9 |
| | E8 | | 73.7 +/- 53.2 | 4.6 +/- 1.3 |

The secretion indexes were maintained above 2 up to 8 days after encapsulation and were not different from control naked islets (One way ANOVA, p value = 0.4699). In addition, insulin secreted levels were not decreased up to 8 days after encapsulation as compared to control naked islets (One way ANOVA, p value = 0.5632). Altogether, these results demonstrate that the functionality of rat primary islet is maintained at least 8 days after encapsulation in C16B-11.

### Example D1D: Short term in vitro functionality of rat primary islets encapsulated in Example C16B-13

To evaluate the *in vitro* functionality of naked or encapsulated rat primary islets, GSIS (Glucose Stimulated Insulin Secretion) experiments were performed as described in Example D2C.

2 days after isolation, rat primary islets were encapsulated as described in Example C16B-13, at 20kIEQ/mL, and maintained in culture medium in a 37°C / 5% CO2 incubator (see Example B2B. Their functionality was evaluated after 8 days of culture *in vitro,* in parallel with naked islets from the same islet preparation. See Table 19D.

**Table 19D: Functionality of naked rat primary islets or islets encapsulated in Example C16B-13. N=1 islet preparation, n=2 hydrogel / naked islets samples. Errors are standard deviations. The evaluation is performed 8 days after encapsulation.**

| Example **C16B-13** | Time point | Secretion index |
|---|---|---|
| Naked islets | E8 | 3.2 +/- 0.4 |
| Encapsulated islets | | 2.2 +/- 0.1 |

*The secretion index was maintained above 2 after up to 8 days after encapsulation and was not different from control naked islets (t-test, p value = 0.07), demonstrating the maintenance of rat primary islets functionality up to 8 days after encapsulation in C16B-*13.

### Example D1D: Short term in vitro functionality of rat primary islets encapsulated in Example C16B-16

### a/ Total insulin content quantification method

After functionality evaluation, naked or encapsulated islets were lysed in RIPA buffer for 10 min on ice. Samples were then sonicated for 1 min and stored at - 80°C. Insulin levels in these samples were quantified using a qualified ELISA assay specific for insulin. Insulin levels were normalized by the IEQ content of the samples (see Example B3A).

### b/ The encapsulation process in Example C16B-16, does not impact neither insulin synthesis nor islet functionality of rat primary islets.

3 days after isolation, rat primary islets were encapsulated as described in Example C16B-16 at 15kIEQ/mL,and maintained in culture medium in a 37°C / 5% CO2 incubator as described in Example B2B. Their functionality was evaluated after 1, 2 and 8 days of culture *in vitro* (see Example D2C. In parallel, the total insulin content of samples was analysed. See Table 19E.

**Table 19E: Functionality of rat primary islets encapsulated in Example C16B-16. Errors are standard deviations. N=1 islet preparation, n = number of replicates. Ex = x days after encapsulation**

| **Example C16B-16** | **Time point** | **n=** | **Secretion index** | **Total insulin content (µIU/IEQ)** |
|---|---|---|---|---|
| Encapsulated islets | E8 | 4 | 10.4 +/-13.6 | 310.9 +/- 76.3 |

Rat islets encapsulated in C16B-16 still contained insulin 8 days after encapsulation. Moreover, they were able to secrete insulin in response to glucose as illustrated by mean secretion indexes above 3.

Altogether, these results show that both insulin synthesis and primary islet functionality are maintained for at least 8 days after encapsulation in C16B-16.

### Example D2A: In vitro viability and functionality of encapsulated pseudo islets and primary human islets

### a) Viability evaluation

Live/Dead staining (ThermoFisher) was used on encapsulated or naked primary or pseudo islets to determine their viability. Phosphate Buffer Saline (PBS) was used to wash the samples. They were then incubated in PBS supplemented with 2µM calcein-AM and 8µM ethidium bromide and incubated for 60min. They were finally washed in PBS and imaged using epifluorescence microscopy.

Quantification of the viability was performed by segmenting the images. The integrated intensity (sum of area pixel intensity) in the green channel, V (live cells), and the integrated intensity in the red channel, D (dead cells), were calculated.

A viability ratio was obtained by calculating $\frac{V}{\left(V+D\right)}$.

### b) Functionality evaluation methods

To evaluate the functionality of primary or pseudo islets (naked or encapsulated), a perifusion experiment was performed. At a selected date after encapsulation, 400 IEQs (see Example B3A) from the batch used for encapsulation (primary islets or pseudo islets) were introduced in a perifusion chamber, as well as encapsulated samples. Chambers were then simultaneously perfused following the protocols described in Table 20A.

**Table 20A: Perifusion conditions**

| ***Step*** | ***Solution*** | ***Duration (min)*** | ***Flow Collection Frequency*** |
|---|---|---|---|
| *Equilibration* - *G3* | *Krebs buffer* + *3mM Glucose (G3)* | 50 | *100µL*/*min No collection* |
| *1^{st} Basal secretion* - *G3* | | 14 | *100µL*/*min* |
| *Stimulation* - *G17* | *Krebs buffer* + *17mM Glucose (G17)* | 38 | |
| *2^{nd} Basal secretion* - *G3* | *Krebs buffer* + *3mM Glucose (G3)* | 18 | |
| *Stimulation* - *KCl* | *Krebs buffer* + *25mM KCI* | 6 | *1 well*/*2min* |
| *3^{rd} Basal secretion* - *G3* | *Krebs buffer* + *3mM Glucose (G3)* | 8 | |

Flow-through buffer was collected, and insulin was quantified using a qualified sandwich ELISA assay specific to insulin.

The Insulin Output for each sample was calculated as the sum of insulin concentrations measured over the 1st basal secretion, the stimulation, and the 2nd basal secretion steps.

The Secretion Index for each sample was calculated as the ratio of the average insulin concentration measured during the stimulation step over the average insulin concentration measured during the 1st basal secretion step.

### c) Encapsulation maintains the viability and the functionality of encapsulated Min-6 pseudoislets

Min-6 pseudo islets were encapsulated as described in Example C16A-1 and maintained in culture medium (see Example B1A) in 37°C / 5% CO2 incubator. Their viability and as described in example D2A were evaluated 4 days after encapsulation and compared to naked pseudo islets from the same batch. See Table 21A.

**Table 21A: Viability and functionality of encapsulated pseudoislets 4 days after encapsulation. Errors are standard deviation. N=1, n=2 hydrogels.**

| **Example** | **Viability (%)** | **Insulin output (mUI/L)** | **Secretion index** |
|---|---|---|---|
| Naked pseudoislets | 93 ±1 | 8890 ±327 | 2.7 ±0.3 |
| C16A-1 | 92 ±1 | 5311 ±833 | 4.2 ±0.4 |

The encapsulation process shows no negative impact on viability nor on secretion index 4 days after encapsulation. The insulin output shows a slight decrease that is non-significant (Welch's test, p=0.19). In conclusion, the viability and the functionality of encapsulated pseudoislets is maintained after encapsulation.

### d) Encapsulation maintains the viability and the functionality of encapsulated primary human islets at least 22 days after encapsulation

Primary human islets were encapsulated as described in Examples C16B-2 and C16B-3, and maintained in culture medium (See Example B2A) in a 37°C / 5% CO2 incubator. Their viability was evaluated 14 and 22 days after encapsulation (E14 and E22 respectively) and their functionality was evaluated 11 and 22 days after encapsulation (E11 and E22 respectively). See Table 22A.

**Table 22A: Viability (E14 and E22) and functionality (E11 and E22) of encapsulated primary human islets. Errors are standard deviation. N=1, n=2 hydrogels/naked islet samples, n=1 for naked islets functionality at E22. Naked islets viability was not measured. N.A. : Not applicable. N.M. : Not Measured**

| **Example** | **Viability (%)** | | **Insulin output (mUI/L)** | | **Functionality Secretion index** | |
|---|---|---|---|---|---|---|
| | E14 | E22 | E11 | E22 | E11 | E22 |
| Naked Islets | N.M. | N.M. | 1912 ±1108 | 1146 ±N.A. | 11.5 ±4.8 | 3.1 ±N.A. |
| C16A-2 | 94 ±0 | 91 ±8 | 2290 ±328 | 2576 ±768 | 2.8 ±0.1 | 5.2 ±0.6 |
| C16A-3 | 94 ±1 | 90 ±3 | 2262 ±1351 | 1732 ±731 | 5.6 ±1.2 | 4.7 ±2.6 |

The encapsulation process shows viability above 90% after encapsulation. Encapsulation shows no significant negative impact on insulin output and secretion index 11 and 22 days after encapsulation compared to naked islets (two way ANOVA, p=0.46 for insulin output ; p=0.39 for secretion index). In conclusion, the encapsulation maintains the viability and the functionality of encapsulated primary human islets at least 22 days after encapsulation.

### c) Encapsulated primary human islets maintains a stable functionality for at least 3 months

Primary human islets were encapsulated as described in Examples C16A-13 and C16A-16 at 20kIEQ/ml, and maintained in culture medium (See Example B2A) in a 37°C / 5% CO2 incubator. Their functionality was evaluated 7 days, 1 month, and from 3 to 4 months after encapsulation and compared to naked islets, as described in Example D2A. See Table **22B:** 22B.

**Table 22B: Functionality of encapsulated primary human islets. S.D. : Standard Deviation. N.A. = Not Applicable N=4 independent islet preparations, n=2 hydrogels/naked islet samples per preparation.**

| **Timing** | **Secretion** | | **C16B13/C16B-16** | **Naked Islets** |
|---|---|---|---|---|
| 1 week after encapsulation | Basal ± S.D. | (nUI/min/IEQ) | 25.8 ± 19.6 | 11.1 ± 3.3 |
| | Stimulated ± S.D. | (nUI/min/IEQ) | 102.4 ± 99.7 | 64.9 ± 65.8 |
| | Secretion index ± S.D. | | 4.5 ± 1.8 | 5.4 ± 5.0 |
| 1 month after encapsulation | Basal ± S.D. | (nUI/min/IEQ) | 25.0 ± 12.2 | 10.5 ± 3.9 |
| | Stimulated ± S.D. | (nUI/min/IEQ) | 125.2 ± 118.2 | 83.9 ± 111.4 |
| | Secretion index ± S.D. | | 4.6 ± 3.1 | 6.7 ± 6.7 |
| 3-4 months after encapsulation | Basal ± S.D. | (nUI/min/IEQ) | 18.1 ± 14.2 | N.A. |
| | Stimulated ± S.D. | (nUI/min/IEQ) | 144.3 ± 142.5 | N.A. |
| | Secretion index ± S.D. | | 7.1 ± 4.4 | N.A. |

Encapsulated islets showed no significative difference of basal and stimulated insulin secretion, nor of the secretion index (ratio of stimulated over basal secretion) one week or one month after encapsulation compared to naked islets (Two-way ANOVA, p=0.1071, p=0.5161, and p=0.7642, respectively).

As expected, naked islets could not be maintained in culture longer than 1 month (Marchini A, Ciulla MG, Antonioli B, Agnoli A, Bovio U, Visnoviz V, Bertuzzi F, Gelain F. Long-term cultures of human pancreatic islets in self-assembling peptides hydrogels. Front Bioeng Biotechnol. 2023 Feb 23;11: 1105157. doi: 10.3389/fbioe.2023.1105157. PMID: 36911193; PMCID: PMC9995881). Encapsulated islets showed no decrease of basal and stimulated insulin secretion, nor of the secretion index over time, at least 3 to 4 months after encapsulation using a two-way ANOVA (p=0.3394, p=0.6737, and p=0.4482, respectively).

Overall, these data demonstrate that the encapsulation maintains the viability and the functionality of encapsulated primary human islets at least 3 months after encapsulation.

### Example D2B: In vitro viability and functionality of encapsulated rat islets in C16B-4A and C16B-4B

### a) Rat primary islet encapsulation

Rat primary islets (from Wistar or Lewis rat donors) were isolated as described in Example B2B and encapsulated 1 or 3 days after isolation, as described in example C16B-4A, C16B-4B and C16B-14 (17.3 kIEQ/ml), and maintained in culture in vitro.

### b) In vitro viability evaluation

Live/Dead staining (ThermoFisher) was used on encapsulated or naked primary or pseudo islets to determine their viability. Phosphate Buffer Saline (PBS) was used to wash the samples. They were then incubated in PBS supplemented with 2µM calcein-AM and 8µM ethidium bromide and incubated for 60min. They were finally washed in PBS and imaged using epifluorescence microscopy.

Quantification of the viability was performed by segmenting the images. The integrated intensity (sum of area pixel intensity) in the green channel, V (live cells), and the integrated intensity in the red channel, D (dead cells), were calculated.

A viability ratio was obtained by calculating $\frac{V}{\left(V+D\right)}$.

Briefly, samples were first washed in Phosphate Buffer Saline (PBS) and then incubated for 60min in PBS supplemented with 2µM calcein-AM and 8µM ethidium bromide. They were finally washed in PBS and imaged using epifluorescence microscopy. Quantification of the viability was performed by segmenting the images. The integrated intensity (sum of area pixel intensity) in the green channel, V (live cells), and the integrated intensity in the red channel, D (dead cells), were calculated. A viability ratio was obtained by calculating $\frac{V}{\left(V+D\right)}$.

### c) In vitro functionality evaluation method

To evaluate the functionality of encapsulated rat primary islets, a perifusion experiment was performed. At the selected date after encapsulation, samples were introduced in a perifusion chamber. Chambers were then simultaneously perfused following the protocols described in Table 1720B.

Flow-through buffer was collected, and insulin was quantified with a qualified ELISA specific for insulin.

The Secretion Index for each sample was calculated as the ratio of the average insulin concentration measured during the stimulation step over the average insulin concentration measured during the 1st basal secretion step.

**Table 208: Perifusion conditions**

| **Step** | **Solution** | **Duration (min)** | **Flow Collection Frequency** |
|---|---|---|---|
| Equilibration - G3 | Krebs buffer + 3mM Glucose (G3) | 50 | 100µL/min No collection |
| 1^{st} Basal secretion - G3 | | 12 | 100µL/min |
| Stimulation - G17 | Krebs buffer + 17mM Glucose (G17) | 36 | |
| 2^{nd} Basal secretion - G3 | Krebs buffer + 3mM Glucose (G3) | 12 | |
| Stimulation - KCl | Krebs buffer + 25mM KCl | 6 | 1 well/2min |
| 3^{rd} Basal secretion - G3 | Krebs buffer + 3mM Glucose (G3) | 6 | |

To evaluate the functionality of primary or pseudo islets (naked or encapsulated), a perifusion experiment was performed. At a selected date after encapsulation, 400 IEQs (see Example B3A) from the batch used for encapsulation (primary islets or pseudo islets) were introduced in a perifusion chamber, as well as encapsulated samples. Chambers were then simultaneously perfused following the protocols described in Table 20C.

**Table 20C: Perifusion conditions**

| **Step** | **Solution** | **Duration (min)** | **Flow Collection Frequency** |
|---|---|---|---|
| Equilibration - G3 | Krebs buffer + 3mM Glucose (G3) | 50 | 100µL/min No collection |
| 1^{st} Basal secretion - G3 | | 14 | 100µL/min |
| Stimulation - G17 | Krebs buffer + 17mM Glucose (G17) | 38 | |
| 2^{nd} Basal secretion - G3 | Krebs buffer + 3mM Glucose (G3) | 18 | |
| Stimulation - KCl | Krebs buffer + 25mM KCl | 6 | 1 well/2min |
| 3^{rd} Basal secretion - G3 | Krebs buffer + 3mM Glucose (G3) | 8 | |

Flow-through buffer was collected, and insulin was quantified using a qualified sandwich ELISA assay specific to insulin.

Basal secretion and stimulated secretion were calculated as the average of the insulin levels of the 1st basal secretion - G3 period and the stimulation - G17 period respectively.

The Insulin Output for each sample was calculated as the sum of insulin concentrations measured over the 1st basal secretion, the stimulation, and the 2nd basal secretion steps.

The Secretion Index for each sample was calculated as the ratio of the average insulin concentration measured during the stimulation step over the average insulin concentration measured during the 1st basal secretion step.

### d) Islet intracellular content of insulin

After functionality evaluation, naked or encapsulated islets were lysed in RIPA buffer for 10 min on ice. Samples were then sonicated for 1 min and stored at - 80°C +/- 20°C. Insulin levels in these samples were quantified using a qualified ELISA assay specific for insulin. Insulin levels were normalized by the IEQ content of the samples (see Example B3B).

### e) Rat primary islet viability and functionality is maintained in C16B-4A and C16B-4B

Functionality and viability of rat primary islets encapsulated in C16B-4A and C16B-4B were evaluated 8 and 9 days after encapsulation, respectively (Table 21A). The results were pooled.

**Table 21B: Viability and functionality of encapsulated rat primary islets. Errors are standard deviation**

| **Example** | **Viability Day 9** | **Functionality Day 8 Secretion index** |
|---|---|---|
| C16B-4A and C16B-4B | 87.2% +/- 6.9 | 3.45 +/- 1.68 |

| | | |
|---|---|---|
| *N* = *2 independent preparations (C16**B**-4A n*=*2 replicates and C16**B**-4B n=2 replicates)* | | |

The viability score of rat primary islets encapsulated in C16B-4A and C16B-4B was high at day 9: 87.2% +/- 6.9. In addition, the rat primary islets encapsulated in C16B-4A and C16B-4B maintain their ability to secrete insulin in response to glucose stimulation: secretion index = 3.45 +/- 1.68.

### f) Rat primary islet functionality is maintained in C16B-14

Functionality of rat primary islets encapsulated in C16B-14 was evaluated as described in Example D2C, 2 days after encapsulation, see Table 21C.

In parallel, the total insulin content of samples was analysed, see Table 21C.

**Table 21C: Functionality of rat islets encapsulated in Example C16B-14. Data are presented as Mean ± Standard Deviations. N=1 islet preparation, n = number of replicate. Ex = x days after encapsulation**

| **Example C16B-14** | **Time point** | **n=** | **Insulin secretion (µIU/IEQ)** | **Secretion index** | **Total insulin content (µIU/IEQ)** |
|---|---|---|---|---|---|
| Encapsulated islets | E2 | 2 | 2.1 ± 0.6 | 6.5 ± 1.1 | 188.8 ± 8.3 |

Rat islets encapsulated in C16B-14 still contained insulin 2 days after encapsulation. Moreover, they were able to secrete insulin in response to glucose as illustrated by mean secretion indexs of 6.5.

Altogether, these results show that both insulin synthesis and primary islet functionality are maintained for at least 2 days after encapsulation in C16B-14.

In conclusion, rat primary islet viability and functionality are maintained in C16B-4A, C16B-4B and C16B-14.

### Example D2C: Long-term in vitro functionality of islets encapsulated in C16B-1A

### Rat primary islet encapsulation

Rat primary islets were isolated as described in Example B1 and encapsulated 1 day after isolation, as described in example C16B and maintained in *in vitro* culture for 28 days.

### a) In vitro functionality evaluation - method 1

To evaluate the in vitro functionality of encapsulated rat primary islets, GSIS (Glucose Stimulated Insulin Secretion) experiments were performed.

Briefly, encapsulated islets were first washed 3 times in Krebs buffer containing 0.1% BSA en 3mM of glucose (Solution G3) (Table 1923). Insulin secretion was then equilibrated by incubating the encapsulated islets in Solution G3 for 2 x 30 min at 37°C. Basal insulin secretion was obtained by incubating encapsulated islets in Solution G3 for another 60 min at 37°C. At the end of this incubation, media were collected (Basal insulin secretion samples). Encapsulated islets were then stimulated in Krebs buffer containing 0.1% BSA and 17mM of glucose (Solution G17) for 60 min at 37°C. At the end of the incubation, media were collected (Stimulated insulin secretion samples).

**Table 23: GSIS buffers and conditions.**

| **Step** | **Solution** | **Duration (min)** | **Insulin quantification** |
|---|---|---|---|
| Wash | Krebs buffer + 3mM Glucose (Solution G3) | 2x 10min | Not performed |
| Equilibration - G3 | | 2x 30 min | |
| Basal secretion - G3 | | 60 min | Basale insulin secretion |
| Stimulation - G17 | Krebs buffer + 17mM Glucose (Solution G17) | 60min | Stimulated insulin secretion |

Insulin levels in these samples were the quantified using a qualified ELISA assay specific for insulin. Secretion index was calculated as the ratio of insulin concentrations measured between Stimulation and Basal secretion steps.

### b) The long term functionality of rat primary islets is maintained when encapsulated in C16B-1A

Functionality rat primary islets encapsulated in C16B-1A was evaluated 28 days after encapsulation. The secretion index was 2.9 showing that the functionality of rat primary islets is maintained in C16B-1A.

In conclusion, the long-term functionality of rat primary islets is maintained when encapsulated in C16B-1A.

### Example D2D: In vitro functionality of islets encapsulated in C16B-5

### a) Rat primary islet encapsulation

Rat primary islets were isolated as described in Example BA and encapsulated 1 day after isolation, as described in example C16B-5.

### b) The functionality of rat primary islets is maintained in vitro when encapsulated in C16B-5

In vitro functionality of rat primary islets encapsulated in C16B-5 was evaluated as described in Example D2B, 1 day after encapsulation (Table 24A).

**Table 24A: Functionality of encapsulated rat primary islets. N=1 hydrogel per condition**

| **Example** | **Functionality D1 Secretion index** |
|---|---|
| C16B-5 | 3.1 |

The secretion indexes were 3.1 and 3.0 respectively, showing that the functionality of encapsulated rat primary islets was maintained in C16B-5.

In conclusion, the functionality of rat primary islets is maintained in vitro when encapsulated in C16B-5.

### Example D2E: Long term in vitro functionality of Human islets encapsulated in C16B-18 and C16B-19

### a) Human primary islet encapsulation

Human primary islets from 3 independent preparations were isolated as described in Example B3, encapsulated in C16B-18, (10 kIEQ/mL) and C16B-19 (10 kIEQ/mL) 6, 7 and 5 days after isolation, as described in example C16, and maintained in *in vitro* culture for 33 days.

### b) In vitro functionality evaluation

The *in vitro* functionality of naked and encapsulated Human primary islets was evaluated as described in Example D2C.

### c) Long term functionality of human primary islets is maintained when encapsulated in C16B-18 and C16B-19

Naked and encapsulated Human islets were maintained in culture medium in a 37°C / 5% CO2 incubator up to 1 month. Their functionality was evaluated 1 week and 1 month after the day of encapsulation and compared, see Table 24B.

**Table 24B: Functionality of naked and encapsulated primary Human islets in C16B-18 and C16B-19. S.D.: Standard Deviation. N=3 independent islet preparations analyzed 1 week after encapsulation, N=2 islet independent preparations analyzed 1 month after encapsulation, n=2 hydrogels/naked islet samples evaluated for each preparation and at each timepoint.**

| **Timing** | **1 week after encapsulation** | | **1 month after encapsulation** | |
|---|---|---|---|---|
| Secretion | Insulin secretion(nIU/min/IEQ) (Mean ± S.D.) | Secretion index (Mean ± S.D.) | Insulin secretion (nIU/min/IEQ) (Mean ± S.D.) | Secretion index (Mean ± S.D.) |
| Naked Islets | 37.8 ± 7.7 | 2.9 ± 2.5 | 57.1 ± 5.1 | 4.8 ± 3.9 |
| *C16B-18* | 44.6 ± 29.7 | 3.5 ± 0.6 | 70.3 ± 55.1 | 3.5 ± 0.8 |
| *C16B-19* | 45.8 ± 38.2 | 7.8 ± 5.3 | 65.9 ± 61.2 | 6.4 ± 5.5 |

Naked Human islets and Human islets encapsulated in *C16B-18* and *C16B-*19 showed no significative difference of stimulated insulin secretion, nor of the secretion index one week (one-way ANOVA, p=0.7568, and p=0.6142, respectively) or one month after encapsulation (one-way ANOVA, p=0.8093, and p=0.5412, respectively).

Naked Human islets and Human islets encapsulated in *C16B-18* and *C16B-*19 showed no significant decrease of stimulated insulin secretion (paired t-test, p=0.1048, p=0.7533, and p=0.7734, respectively), nor of the secretion index (paired t-test, p=0.4108, p=0.5411, and p=0.0615, respectively) over time, at least 1 month after encapsulation.

In conclusion, the long-term functionality of Human primary islets is maintained at least 1 month when encapsulated inC16B-18 and *C16B-19.*

### Example D3: In vivo long-term local tolerance of hydrogels

a) Local tolerance of hydrogels was evaluated based on the guideline ISO10993 Part 6 (2016): Tests for local effects after implantation.
b) C4A-3 hydrogel and a disc-shaped HDPE, as the negative control (NC), of the same size as the disc-shaped hydrogel were implanted in the dorsal subcutaneous tissue of rats for 13 weeks. The local tissue effects as well as the aspects of the hydrogel were evaluated macroscopically and by histopathologic analysis (n=5 sites per article and per time-period) as recommended by ISO 10993 Part 6 as follows: after sacrifice of the animals and explantation of the hydrogels, tissue reactivity produced by the implants was evaluated by scoring its various components on a 0 to 4 scale. The components evaluated were polymorphonuclear cells, lymphocytes, plasma cells, macrophages, giant cells, necrosis on one part, and neovascularization, fibrosis and fatty infiltrate on a second part. The various inflammatory cell types or morphologic features were only reported if present. The average score obtained for each test article, minus the average score obtained by the negative control article HDPE (high density polyethylene; as recommended by ISO 10993 Part 6), gives a tissue reactivity score.
c) Macroscopically, C4A-3 hydrogel was well preserved in shape, size, color and consistency.
d) After histopathology examination, and according to the scoring scale for tissue reactivity from guideline ISO10993 Part 6, C4A-3 was found to induce "minimal to no reaction", and with an average score reaction score close to the negative control HDPE.

**Table 25: Scoring and tissue reactivity for C4A-3 hydrogel versus the negative control (NC) HDPE after 13 weeks of subcutaneous implantation in the rat. n/a: not applicable. Because the number of samples were different between C4A-3 (n = 5) and HDPE (n = 4), F1 and F2 scores are reported as averages.**

| **Average score** | **C4A-3** | **HDPE (NC)** |
|---|---|---|
| F1. Inflammation (x2) | 7.2 | 5.5 |
| F2. Neovascularization | 2.0 | 1.3 |
| Fibrosis | 2.0 | 3.0 |
| Fatty infiltrate | 0.0 | 0.0 |
| Ave | 11.2 | 9.75 |
| Reaction score vs HDPE | 1.45 | n/a |
| Tissue reactivity | No | n/a |

Therefore, the hydrogel shows an excellent local tolerance after 13 weeks of implantation in the rat subcutis. In particular fibrosis was lower, and neovascularization higher, than for the negative control, which are both critical parameters for a successful tissue integration.

### Example D4A: In vivo survival and functionality of human islets encapsulated in C16A-4

### a) Human platelet rich plasma preparation

50mL of fresh human blood was introduced in a centrifuge tube. The blood was centrifugated for 20min at 300g at 20°C. The supernatant (plasma) was centrifugated for 20min at 2000g at 20°C. The top 2/3 (v/v) of the centrifugated plasma was discarded to collect the bottom 1/3 (v/v) fraction, which corresponds to the platelet rich plasma (PRP).

### b) Primary human islet encapsulation

Primary human islets were encapsulated as described in example C16A-4. They were incubated for 3h in human PRP in a 37°C / 5% CO2 incubator, washed in PBS, and implanted in the omentum of healthy rats for up to 30 days. In vitro samples were prepared extemporaneously from the same islet batch following example C16A-5.

### c) In vivo implantation

Wistar rats weighing between approx. 350 g and 400 g at surgery were used.

Anesthesia was performed with isoflurane.

Each animal was placed in the supine position on a warmed pad. The fur was shaved from surgical area with a surgical blade. The surgical site was disinfected with povidone iodine solution.

A 2-3 cm long midline incision was made in the abdomen. The intestines were moved to the left side and covered with saline-soaked gauze to prevent dehydration. The stomach was fully exposed, and the omentum was spread on a humid gauze. Two implants were placed onto the omentum and covered with it (wrapping technique). Non-resorbable suture stitches (Prolene 6/0) were made on the omentum to maintain the implants in place and avoid their slippage and/or overlapping. Prior to closing the abdomen, 2 mL of physiological saline solution were dispensed into the abdominal cavity to prevent dehydration. The peritoneum with muscular layer was closed with continuous non-resorbable suture (Prolene 4/0), then the skin was sutured (Prolene 4/0) by interrupted single stitches and the incision cleaned with povidone iodine solution.

After surgery, each animal was moved to a recovery area and monitored for recovery from anesthesia until sternal recumbency was achieved. After recovery, the animals were group-housed and observed for general health.

After 30 days, the animals were sacrificed. The implantation sites were collected and one explant from each animal was dissected free from tissues for post-implantation perifusion and viability assays. Blood was collected by exsanguination at the aortic bifurcation level for dosage of human C-peptide.

### d) In vivo proof of functionality of encapsulated islets

*In vivo* functionality of encapsulated human islets was evaluated in rats implanted in the omentum with two disc-shaped C16A-4 implants containing human islets. Each animal was implanted with 5 000 IEq at the density of 38 000 IEq/mL.

Omental implantation was performed as described in Example D4Ac.

Islets' functionality was assessed by measurement of human C-peptide in the plasma at days 3-, 6-, 9- and 12- (5 animals) and at days 3-, 6-, 9-, 12-, 17-, 20-, 23-, 27- and 30- (3 animals) post implantation using a qualified specific ELISA immunoassay (Mercodia, Ref. 10.1141.01).

C-peptide plasma levels were quantifiable for all animals at 3- and 6-days post implantation, indicating that encapsulated islets were functional, and that the hydrogel allowed diffusion of insulin into the bloodstream.

40% (2/5) and 33% (1/3) of the animals display quantifiable levels of circulating C-peptide at days 12- and 30- post implantation, respectively. These results demonstrate that after 30 days *in vivo,* some C16A-4 implants are still viable and functional and that the diffusion properties of the hydrogel are conserved.

### e) Explant evaluation

Thirty days after implantation, explant viability and functionality were evaluated as described in examples D2Aa and D2Ab and compared to in vitro samples prepared from the same batch of human islets (C16A-5 composition) and kept in a 37°C / 5% CO2 incubator with medium renewal every other day. See Table 26A.

**Table 26A: Functionality of encapsulated primary human islet explants after a 30-days in vivo implantation in rat omentum. N=1 independent experiment, n=1 in vivo sample, n=4 in vitro samples. Errors are standard deviation. N.A.: Not applicable.**

| **Rat ID** | **Implant or explant** | **Viability (%)** | **Insulin output / IEQ (mUI/L/IEQ)** | **Functionality Secretion Index** |
|---|---|---|---|---|
| 006 | C16A-4 | 78.8 ±N.A. | 0.3 ±N.A. | 1.8 ±N.A. |
| 007 | | 86.6 ±N.A. | 3.6 ±N.A. | 2.5 ±N.A. |
| 847 | | 90.4 ±N.A. | 0.1 ±N.A. | 1.2 ±N.A. |
| *In vitro* sample | C16A-5 | 90. ±6.7 | 3.9 ±2.0 | 2.9 ±1.2 |

Islets encapsulated in C16A-4 display viability above 75% and in 1/3 of the rats (007), the functionality was comparable to in vitro samples suggesting that encapsulated islets are able to survive and maintain their functionality in a xenograft, and that the diffusion properties of the hydrogel are conserved.

### Example D4B: In vivo tolerance, survival and functionality of islets encapsulated in C16B-1A and C16B-12

### Induction of diabetes

Diabetes was induced by a single intraperitoneal injection of either 45 or 75 mg/kg Streptozotocin. Animals administered 75 mg/kg were supplemented with basal insulin via pellets until 5 days before implantation, followed by daily administration of a bolus subcutaneous injection of Lantus (up to 6 U/animal) up to the day of implantation to maintain satisfactory health status throughout the experiment. No insulin supplementation was necessary for the one administered 45 mg/kg Streptozotocin.

### a) Rat primary islets encapsulation

Wistar rat primary islets were encapsulated 1 day after isolation, as described in example C16B-1A and C16B12 (40 kIEQ/ml).

### b) In vivo implantation

Wistar rats weighing between 260 g and 310 g at surgery were used. Each animal was implanted with 4000 IEq.

Anesthesia was performed with isoflurane.

Each animal was placed in the supine position on a warmed pad. The fur was shaved from surgical area with a surgical blade. Alternatively, animals were shaved 2 days prior to surgery. The surgical site was disinfected with povidone iodine solution.

A 2-3 cm long midline incision was made in the abdomen. The intestines were moved to the left side and covered with saline-soaked gauze to prevent dehydration. The stomach was fully exposed, and the omentum was spread on a humid gauze. One C16B-1A implant was placed onto the omentum and covered with it (wrapping technique). Non-resorbable suture stitches (Prolene 6/0) were made on the omentum to maintain the implant in place and avoid their slippage and/or overlapping. Prior to closing the abdomen, 2 mL of physiological saline solution were dispensed into the abdominal cavity to prevent dehydration. The peritoneum with muscular layer was closed with continuous non-resorbable suture (Prolene 4/0), then the skin was sutured (Prolene 4/0) by interrupted single stitches and the incision cleaned with povidone iodine solution.

After surgery, each animal was moved to a recovery area and monitored for recovery from anesthesia until sternal recumbency was achieved. After recovery, the animals were group-housed and observed for general health.

After 4 weeks, the animals were sacrificed. The implantation site was collected; half of the explant from each animal was dissected free from tissues for post-implantation functionality and viability assays, and the other half processed for histology.

### c) Evolution of insulinemia

Islets' functionality was assessed by specific measurement of rat plasma insulin up to 21 days after implantation. Blood was collected at days 2-, 7-, 14- and 21-post implantation. Plasma was prepared and rat insulin quantified using a qualified specific ELISA immunoassay (Mercodia, Ultrasensitive Rat Insulin ELISA, Ref. 10-1251-01). See Table 26B.

**Table 26B: Insulin concentrations in plasma of diabetic rats implanted with C16B-1A for 21 days. Indicated days are days post implantation. The lower limit of quantification of the ELISA method was defined at 32.0 pM.**

| | **Rat ID** | **Plasmatic insulin concentration (pM)** | | | |
|---|---|---|---|---|---|
| | | **Day 2** | **Day 7** | **Day 14** | **Day 21** |
| C16B-1A | 129 | 162.2 | 148.9 | 248.3 | 283.8 |
| | 130 | 104.1 | 122.4 | 59.2 | 132.1 |
| | 133 | 220.7 | 309.2 | 219.0 | 184.5 |
| C16B-12 | 109 | 105.0 | 70.0 | 53.1 | 60.2 |
| | 110 | 81.3 | 76.9 | 101.3 | 56.1 |
| | 111 | 89.6 | 53.2 | 65.4 | 66.3 |
| | 112 | 59.8 | 63.6 | < LLOQ | < LLOQ |

Stable plasmatic insulin levels were measured for all animals implanted with C16B-1A and for 75% of animals implanted with C16B-12 from 2- to 21- days post implantation. These data demonstrate that islets encapsulated in C16B-1A and C16B-12 were functional *in vivo,* and that the hydrogel allowed diffusion of insulin into the bloodstream. Moreover, as the Wistar strain is not syngenic, these data also demonstrate the immune-protection property of the hydrogel matrix.

Insulin secretion was more than 3 times higher in rats implanted with C16B-1A comprising a ring net as compared to rat implanted with C16B-12 (both rats comprising the same amount of islets).

### d) Explants functional evaluation

At the end of the study (28 days post implantation), the functionality of the islets within the explants C16B-1A from the three rats was evaluated in vitro as described in example D2b. See Table 27.

**Table 27: Functionality of encapsulated rat primary islet explants, 28 days after in vivo implantation in rat omentum. N=1 independent experiment, n=3 samples.**

| **Rat ID** | **In vitro evaluation of islets functionality within the explants C16B-1A at D28** |
|---|---|
| | **GSIS Secretion Index** |
| 129 | 2.1 |
| 130 | 2.2 |
| 133 | 2.1 |

Encapsulated islets displayed insulin increase in response to glucose, 28 days after implantation: indeed, GSIS secretion index superior to 2 were measured in vitro. Therefore, the functionality of rat primary islets encapsulated in C16B-1A is maintained for at least 28 days after implantation in rat omentum.

### e) Implants tolerance evaluation

Local tolerance of implants was evaluated by histology after processing and Hermatoxilyn-Eosin staining. The implant consisting of the ring net / hydrogel / allogenic islets was well tolerated, and embedded within a fibrous capsule, with no adherence to the surrounding tissues except at the level of the suture stitches. The thickness of the surrounding capsule was related to the exposure of some fibers of the net which were incompletely embedded within the gel. The gel exerted thus a protective effect against allogeneic islets rejection but also against the proinflammatory properties of the net material and structure.

### Example D4C: In vivo survival and functionality of islets encapsulated in C16B-2A

### a) Induction of diabetes

Diabetes was induced as described in Example D4B. Animals were supplemented with a bolus administration of Lantus given in the morning, as necessary.

### b) Rat primary islet encapsulation

Wistar rat primary islets were encapsulated 1 day after isolation, as described in example C16B-2A.

### c) In vivo implantation

*In vivo* implantation was conducted as described in Example D4B. Lewis rats weighing approx. 250 g at surgery were used. Each animal was implanted with 4 000 IEq (at a density of 16 000 IEq/mL). An islets-free group implanted C16B-2B served as a control.

Two C16B-2A implants were fixed with 2 sutures onto the peritoneum, one on each side of the abdominal cavity.

After 5 weeks, the animals were sacrificed. The implantation sites were collected; one explant from each animal was dissected free from tissues for post-implantation perifusion and viability assays, and the other one processed for histology.

### d) Evolution of insulinemia

Islet functionality was assessed by specific measurement of rat plasma insulin. Blood was collected 2 days before implantation, the day of implantation (prior to surgery) and then 28 and 35 days post implantation. Plasma was prepared and rat insulin measured using a qualified specific ELISA immunoassay (Mercodia, Ultrasensitive Rat Insulin ELISA, Ref. 10-1251-01). See Table 26C.

**Table 26C: Insulin concentrations in plasma of diabetic rats implanted with C16B-2A for 35 days. Indicated days are day post implantation. The lower limit of quantification (LLOQ) of the ELISA method was defined at 32.0 pM. <LLOQ means that insulin levels were below the LLOQ of the method.**

| **Group** | **Rat ID** | **Plasmatic insulin concentration (pM)** | | |
|---|---|---|---|---|
| | | **Before implantation** | **Day 28** | **Day 35** |
| Control (C16B-2B) | 998 | < LLOQ | < LLOQ | < LLOQ |
| | 999 | < LLOQ | < LLOQ | < LLOQ |
| | 000 | < LLOQ | < LLOQ | < LLOQ |
| Implanted rats (C16B-2A) | 975 | < LLOQ | 65.7 | 43.5 |
| | 976 | < LLOQ | 69.0 | 46.6 |
| | 977 | < LLOQ | 94.9 | 56.2 |
| | 978 | < LLOQ | 36.3 | < LLOQ |

28 and 35-days post implantation, 100% and 75% of the rats implanted with C16B-2A presented detectable levels of circulating insulin, respectively, while animals treated with control hydrogels (with no encapsulated islets, C16B-2B) display no detectable insulin.

These data demonstrate that encapsulated islets were functional, and that the hydrogel allowed diffusion of insulin into the bloodstream until at least 35 days post implantation. Moreover, as the Lewis islets were implanted into Wistar rats, these data also demonstrate the immune-protection property of the hydrogel matrix for at least 35 days.

### e) Explants histological evaluation

The implants were free of any adherence within the peritoneal cavity. They were never surrounded by fibrous tissue, and no capsule was observed at histology. Islet survival observed without immune cell infiltration and no evidence of rejection.

### Example D4D: In vivo survival and functionality of islets encapsulated in C16B-3A, C16B-3B and C16B-18

### a) Rat primary islet encapsulation

Wistar rat primary islets were isolated as described in Example B2B, encapsulated 1 day after isolation, as described in example C16B-3A, C16B-3B and C16B-18, and implanted in diabetic Lewis rats. Diabetes was induced as described in Example D4B with either 45 or 75 mg/kg STZ.

### b) In vivo implantation

Recipient animals weighed between approx. 260 and 400 g at the time of surgery. Animals were implanted either in the omentum (1 implant C16B-3A) and the internal face of the peritoneum (2 implants C16B-3B) or, exclusively, on the internal face of the peritoneum (4 implants C16B-18). The animals were implanted with either 4 000 IEQ at the density of 7 000 IEQ/mL or 6 200 IEQ at a density of 16 000 IEQ/ml.

Anesthesia was performed with isoflurane.

Each animal was placed in the supine position on a warmed pad. The fur was shaved from surgical area with a surgical blade. Alternatively, animals were shaved 2 days prior to surgery. The surgical site was disinfected with povidone iodine solution.

A 3-5 cm long midline incision was made in the abdomen.

For omental implantation, the intestines were moved to the left side and covered with saline-soaked gauze to prevent dehydration. The stomach was fully exposed, and the omentum was spread on a humid gauze. One C16B-3A implant was placed onto the omentum and covered with it (wrapping technique). Non-resorbable suture stitches (Prolene 6/0) were made on the omentum to maintain the implant in place and avoid slippage.

For intraperitoneal implantation, one C16B-3B or four C16B-18 were placed onto the parietal peritoneum, on both sides and maintained in place by non-resorbable suture stitches (Prolene 6/0).

Prior to closing the abdomen, 2 mL of physiological saline solution were dispensed into the abdominal cavity to prevent dehydration. The peritoneum with muscular layer was closed with continuous non-resorbable suture (Prolene 4/0), then the skin was sutured (Prolene 4/0) by interrupted single stitches and the incision cleaned with povidone iodine solution.

Animals were explanted under anesthesia 5 (2 rats) or 19 (1 rat) weeks after implantation of the encapsulated islets and kept for 2 or 3.5 weeks. Following a midline incision of the abdomen, omental and intraperitoneal implantation sites were exposed, and the implants removed. The incisions were sutured, cleaned and disinfected with povidone iodine solution. Animals were then kept in individual cages under until recovery from surgery.

### c) Evolution of glycemia

Non fasted glycemia was measured regularly in the diabetic (streptozotocin treated) rats using a glucose meter (Roche Guide), starting the day of implantation.

A pre-implantation blood glucose measurement was performed. Then, glycemia was taken in the morning prior to Lantus administration (administered only in control animals).

A drop of blood glucose was obtained from the vein tail

The animal implanted with C16B-3A and C16B-3B showed a notable drop in glycemia from Day 1 post-implantation, that lasted until Day 132 (i.e., 19 weeks) post-implantation, at which time implants were explanted; values dropped from around 6 g/L pre-implantation to around 2.25 g/L (1.10 g/L at the lowest).

Similarly, the animal implanted with C16B-18 showed normoglycemia from Day 1 post-implantation that lasted for 5 weeks post-implantation, at which time implants were retrieved; values dropped from 5.56 g/L pre-implantation to an average of 1.38 g/L (0.92 g/L at the lowest).

Glycemia of the control animals were not regulated and all of them were hyperglycemic (average blood glucose over of 5.3 g/L).

After implants retrieval, glycemia of animals was followed for 2 or 3.5 weeks. At least three measures were performed post explantation. Glycemia sharply increased above pre explantation values (ranging from 3.08 to 5.57 g/L), and above pre diabetes induction values. See Figure 5.

These data demonstrate that the encapsulated islets in the hydrogel are able to regulate non-fasted glycemia, and therefore to reduce glycemic excursions after a meal.

### a) Evolution of insulinemia

*In vivo* islets functionality was assessed by specific measurement of rat insulin in the plasma Plasma was prepared and rat insulin measured using a qualified specific ELISA immunoassay (Mercodia, Ultrasensitive Rat Insulin ELISA, Ref. 10-1251-01).

Rat 4 was followed 3 and 1 days before implantation (D-3 and D-1, respectively), for 26 days during implantation and for 14 additional days after explantation (corresponding to D39, D43 and D49). Rat 338 was followed every week after implantation (except during the first week where 2 samplings were performed) until 132 post-implantation. See Table 24C.

**Table 24C: Insulin concentrations in plasma of a diabetic rat implanted with C16B-18 for 35 days and followed for 14 days post explantation (left panel) and a diabetic rat implanted with C16B-3A / C16B-3B for 132 days and followed for 30 days post explantation (right panel). Indicated days are days post implantation. The lower limit of quantification (LLOQ) of the ELISA method was defined at 32.0 pM. NS means No Sampling performed**

| | Rat insulin (pM) | | | | Rat insulin (pM) | |
|---|---|---|---|---|---|---|
| | C16B-18 | | | | C16B-3A, C16B-3B | |
| Rat ID | | 4 | | Rat ID | | 338 |
| Pre implantation phase | D-3 | 153.0 | | Pre implantation phase | D-3 | NS |
| | D-1 | 118.6 | | | D-1 | NS |
| Implantation phase | D2 | NS | | Implantation phase | D2 | 193.8 |
| | D3 | 671.2 | | | D3 | NS |
| | D7 | 405.3 | | | D7 | 203.1 |
| | D14 | 219.5 | | | D14 | 190.8 |
| | D19 | 237.2 | | | D19 | NS |
| | D21 | NS | | | D21 | 115 |
| | D26 | 318.0 | | | D26 | NS |
| | D28 | NS | | | D28 | 110.4 |
| | D35 | NS | | | D35 | 79.7 |
| Post explantation phase | D39 | 289.9 | | | D39 | NS |
| | D42 | NS | | | D42 | 258.6 |
| | D43 | 394.6 | | | D43 | NS |
| | D49 | 53.8 | | | D49 | 187.7 |
| | | | | | D56 | 115.8 |
| | | | | | D64 | 192.7 |
| | | | | | D70 | 160.4 |
| | | | | | D78 | 267.4 |
| | | | | | D82 | 186.8 |
| | | | | | D89 | 317.5 |
| | | | | | D96 | 274.9 |
| | | | | | D103 | 437.2 |
| | | | | | 0110 | 220.8 |
| | | | | | D117 | 367.8 |
| | | | | | D124 | 417.9 |
| | | | | | D131 | 271.6 |
| | | | | Post explantation phase | D146 | 79.5 |
| | | | | | D153 | 66.9 |
| | | | | | D161 | 45.6 |

Stable plasmatic insulin levels were detectable from 2- to 26- days post implantation (100% of timepoints) of C16B-18 in rat 4 and from 2- to 131-days post implantation (100% time points) of C16B-3A / C16B-3B in rat 338.

Insulin levels strongly decreased after explantation for rats implanted with C16B-18 or C16B-3A/C16B-3B (from D39 to D49 for rat 4 and from D146 to D161 for rat 338), demonstrating that most of circulating insulin was produced by implants.

These data demonstrate that encapsulated islets were functional, and that the hydrogel allowed diffusion of insulin into the bloodstream until at least 89, even 131-days post implantation (at which time the implant was removed).

Moreover, as Wistar rat islets were implanted into a Lewis rat, these data also demonstrate the immune-protection property of the hydrogel matrixfor at least 131 days.

### b) Explant evaluation

After explantation (D132), the explants of Rat 338 were stained for insulin using dithizone (DTZ). A DTZ signal was observed in islets, showing that after 132 days of implantation, islets encapsulated in C16B-3A / C16B-3B still contained insulin.

The tissue reaction around the implants is limited to a vascularized non fibrotic and non-inflammatory layer of connective tissue.

### Example D5: in vivo tolerance and immuno-isolation properties of the hydrogels C16A-4 implanted in D4A

a) Tolerance and immuno-isolation properties of hydrogels were assessed during the course of studies involving implantation in the omentum or in the dorsal subcutaneous tissue of either xenogenic (human in rat) or allogenic (rat in rat) encapsulated islets of Langerhans, where rats were immunocompetent.
b) The implants, which had been explanted as described in D4A c), were free of any adherence within the peritoneal cavity. They were never surrounded by fibrous tissue, and no capsule was observed at histology.

Tolerance and immuno-isolation were also assessed histologically. Explants dedicated to histology were processed into paraffin blocks, cut, and stained with Hematoxylin-Eosin. The host reaction to implanted materials was evaluated.

This histologic analysis of xenogenic transplants of human islets in rats shows that islets can survive in the hydrogel without any immune cell infiltration of hot cells, neither rejection reaction. This analysis also confirms the splendid tolerance of the hydrogel implants in the abdomen cavity and its immunoisolant role with xenogenic islets.

### Example D6: In vivo survival, functionality and efficacy of rat islets encapsulated in C16B-14

### a. Rat primary islets encapsulation

Wistar rat primary islets were isolated as described in Example B2B encapsulated 1 day after isolation, as described in examples C16B-14 at 21kIEQ/mL, and implanted in diabetic Wistar rats. Diabetes was induced by a single intraperitoneal injection of 75 mg/kg Streptozotocin. Animals were supplemented with basal insulin via pellets as described in Example D4B.

### b. In vivo implantation

Recipient animals weighed between approx. 350 (group test; 5 rats) and 370 g (control group, no islet; 4 rats) at the time of surgery. Animals were implanted either exclusively in the internal face of the peritoneum (1 implant C16B-14 no islet for the control group) or in the internal face of the peritoneum and intramuscularly into the rectus abdominal muscle (6 implants C16B-14 with islets, 3 for each site, for the test group). Animals in the test group were implanted with 21 000 IEq at the density of 3 000 IEq/mL.

The anaesthetic and surgical procedure was performed as described in example D4B.

Animals were explanted under anesthesia 10 weeks after implantation of the encapsulated islets and kept for 4 weeks. Following a midline incision of the abdomen, intraperitoneal implantation sites were exposed, and the implants removed. The incisions were sutured, cleaned and disinfected with povidone iodine solution. Animals were then kept in individual cages under until recovery from surgery.

### c. Evolution of glycemia

Fasted glycemia was measured regularly in the diabetic (streptozotocin treated) rats using a glucose meter (Roche Guide), starting 2 weeks post-implantation.

Glycemia was taken in the early afternoon each week, after a 5-hour fasting period. Lantus was not administered on the days when fasting glycemia was taken (administered only in control animals).

A drop of blood glucose was obtained from the vein tail.

The animals in the test group implanted with C16B-14 showed fasting normoglycemia of around 1.5 g/L. Fasted glycemia of the control animals was not regulate, and all of them were all hyperglycemic (average blood glucose level of 4.2 g/L).

After implants retrieval, fasted glycemia of animals was followed for 4 weeks. At least four measures were performed post explantation. Fasted glycemia significantly increased compared to post implantation values (average fasting blood glucose level of 3,1 g/L), demonstrating the efficacy of implants in regulating glycemia. The control animals were still hyperglycemic (average fasting blood glucose level of 4.2 g/L).

### d. Evolution of insulinemia and C-peptidemia

Islets functionality in rats implanted with C16B-14 (control group, no islet) and C16B-14 (with islets) assessed by specific measurement of rat insulin and C-peptide in the plasma up to 70 days after implantation and up to 30 days after explantation (corresponding to D83, D90, D97 and D104). Blood was collected approximately once weekly (except during the first week, where plasma was collected on Days 4 and 7 post-implantation). Plasma was prepared. Rat insulin was measured using a qualified specific ELISA immunoassay (Mercodia, Ultrasensitive Rat Insulin ELISA, Ref. 10-1251-01). Rat C-peptide was measured using a qualified specific ELISA immunoassay (Mercodia, Rat C-peptide ELISA, Ref. 10-1172-01). See Tables 28 and 29.

**Table 28: Insulin concentrations in plasma of diabetic rats implanted with C16B-14 (no islets) and C16B-14 (with islets) for 70 days and up to 30 days after explantation. Indicated days are day post implantation. The lower limit of quantification (LLOQ) of the ELISA method was defined at 32.0 pM. NS means No Sampling. ND means Not Done.**

| | | **Rat Insulin (pM)** | | | | | | | | |
|---|---|---|---|---|---|---|---|---|---|---|
| | | **C16B-14 No islets** | | | | **C16B-14 with islets** | | | | |
| Rat ID | | 694 | 699 | 705 | 713 | 714 | 716 | 708 | 715 | 717 |
| Implantation phase | D4 | < LLOQ | < LLOQ | < LLOQ | 183.3 | 284.1 | 291.7 | 495.8 | 347.6 | 236.9 |
| | D7 | 34.7 | 76.2 | < LLOQ | 173.6 | 315.3 | 421.7 | 280.5 | 236.6 | 215.4 |
| | D12 | < LLOQ | < LLOQ | < LLOQ | 154.1 | 214.7 | 475.3 | 291.7 | 131.5 | 116.9 |
| | D14 | < LLOQ | < LLOQ | < LLOQ | 142.6 | < LLOQ | 490.0 | 457.4 | 270.6 | 91.6 |
| | D22 | 35.5 | NS | NS | 153.6 | 178.5 | 477.2 | 322.3 | 289.7 | 208.9 |
| | D28 | < LLOQ | < LLOQ | NS | 200.4 | 157.1 | 332.3 | 424.3 | 222.3 | 235.6 |
| | D41 | < LLOQ | 56.9 | NS | 112.1 | 133.5 | 243.6 | 366.2 | 216.7 | 315.7 |
| | D48 | < LLOQ | 43.7 | NS | 100.8 | 351.0 | 259.3 | 297.2 | 275.1 | 325.9 |
| | D55 | 41.0 | 34.5 | NS | 155.9 | 169.9 | 276.8 | 134.5 | 153.3 | NS |
| | D62 | 53.5 | < LLOQ | NS | 122.0 | 122.3 | 267.5 | 166.6 | 51.2 | NS |
| | D70 | 35.5 | < LLOQ | NS | 98.1 | 112.1 | 269.9 | 308.4 | 187.0 | NS |
| Explantation phase | D83 | 55.7 | < LLOQ | < LLOQ | 132.2 | 65.3 | 49.1 | 100.3 | 69.9 | ND |
| | D90 | 152.8 | 61.7 | < LLOQ | 79.5 | 153.9 | 55.1 | 123.5 | 91.9 | 81.2 |
| | D97 | 41.6 | < LLOQ | < LLOQ | 115.5 | 104.8 | 38.0 | 105.4 | 128.3 | 114.6 |
| | D104 | < LLOQ | 62.7 | < LLOQ | 139.7 | 74.8 | 38.0 | < LLOQ | 194.4 | 100.1 |

**Table 29: C-peptide concentrations in plasma of diabetic rats implanted with C16B-14 (no islets) and C16B-14 (with islet) for 70 days and up to 30 days after explantation. Indicated days are day post implantation. The lower limit of quantification (LLOQ) of the ELISA method was defined at 50.0 pM. NS means No Sampling.**

| | | **Rat C-peptide (pM)** | | | | | | | | |
|---|---|---|---|---|---|---|---|---|---|---|
| | | **C16B-14 (no islet)** | | | | **C16B-14 (with islet)** | | | | |
| Rat ID | | 694 | 699 | 705 | 713 | 714 | 716 | 708 | 715 | 717 |
| Preimplanta tion phase | D-1 | <LLO Q | <LLO Q | <LLO Q | 252.9 | < LLOQ | < LLOQ | < LLOQ | 293.7 | 441.7 |
| | D0 | 155.6 | 83.1 | <LLO Q | 252.9 | < LLOQ | < LLOQ | < LLOQ | 321.1 | 303.4 |
| Implantatio n phase | D4 | 88.4 | 251.1 | 53.9 | 423.9 | 557.0 | 1084.4 | 1299.5 | 965.4 | 1050.7 |
| | D7 | 78.7 | 256.5 | 68.9 | 327.3 | 803.7 | 856.1 | 529.5 | 818.8 | 407.1 |
| | D12 | 136.3 | 164.7 | 54.9 | 314.0 | 575.8 | 1327.9 | 533.1 | 1010.7 | 709.2 |
| | D14 | 506.5 | 85.3 | 69.5 | 284.0 | 174.5 | 1400.3 | 932.9 | 1021.1 | 752.1 |
| | D22 | 130.1 | NS | NS | 410.9 | 480.8 | 1073.0 | 752.8 | 641.9 | 820.9 |
| | D28 | 71.0 | 114.6 | NS | 318.6 | 403.5 | 693.3 | 951.2 | 756.5 | 981.3 |
| | D41 | 91.5 | 172.4 | NS | 273.0 | 320.0 | 786.6 | 1103.9 | 872.8 | 954.6 |
| | D48 | 93.4 | 152.4 | NS | 260.1 | 343.6 | 730.1 | 836.4 | 756.0 | 995.6 |
| | D55 | 95.5 | 162.6 | NS | 338.9 | 404.4 | 805.4 | 861.7 | 844.5 | 838.8 |
| | D62 | 138.8 | 177.9 | NS | 338.9 | 433.9 | 842.5 | 916.5 | 766.4 | NS |
| | D70 | 115.4 | 146.8 | NS | 236.4 | 338.5 | 816.1 | 906.9 | 789.2 | NS |
| Explantation phase | D83 | 99.9 | 105.8 | 83.8 | 336.9 | 119.2 | 171.8 | 406.4 | 320.3 | 536.0 |
| | D90 | 113.9 | 155.9 | 104.3 | 321.1 | 184.8 | 172.6 | 430.5 | 406.4 | 353.6 |
| | D97 | 123.8 | 176.5 | 96.0 | 473.0 | 242.0 | 187.7 | 431.2 | 475.7 | 611.0 |
| | D10 4 | 122.8 | 193.2 | 89.2 | 359.7 | 235.6 | 147.5 | 379.4 | 424.9 | 619.8 |

C-peptide concentrations increased after implantation with C16B-14 (with islet) for 100% of rats, whereas no increased was observed for control rats implanted with *C16B-14 (no islets).* During the implantation phase, C-peptide and insulin secretions were stable for all rats, with significantly higher levels in rats implanted with C16B-14 (with islet) as compared to control rats implanted with *C16B-14 (no islets)* Insulin and C-peptide levels strongly decreased after explantation (from D83 to D104) for rats implanted with C16B-14 with islets, demonstrating that most of circulating insulin and C-peptide were produced by implants. No impact of explantation was observed for control rats implanted with *C16B-14* (no islets).

These data demonstrate that encapsulated islets were functional, and that the hydrogel allowed diffusion of insulin into the bloodstream until at least 70 days post implantation.

### e. Insulin immunostaining method on explant sections

After explantation, samples were fixed, embedded in paraffin and 7µm slices were performed. Paraffin sections were then treated to remove paraffin and an antigen retrieval step was performed. Sections were then stained with an anti-insulin antibody and nuclei were counterstained with Hoechst. Sections were imaged on an epifluorescent microscope using a 40x objective lens.

### f. Maintenance of beta cells in the explants of example C16B-14 with islets

After explantation (at day 74 post implantation), the presence of beta cells within islets encapsulated in C16B-14 with islets was evaluated in vitro by insulin immunostaining on paraffin embedded sections of the explants. 74 days after implantation in diabetic rats, beta cells with strong insulin staining were observed in islets. This shows that beta cells encapsulated in C16B-14 survive in vivo for least 74 days upon implantation in diabetic rats.

The tissue reaction around the intra peritoneal or intra muscular implants is limited to a vascularized non fibrotic and non-inflammatory layer of connective tissue.

### Example D6bis: In vivo survival, functionality and efficacy of rat islets encapsulated in C16B-14

### a. Induction of diabetes

Wistar rat primary islets were isolated as described in Example B2B, encapsulated 2 days after isolation, as described in examples C16B-14, at 9.5kIEQ/mL, and implanted in diabetic Wistar rats. Diabetes was induced by a single intraperitoneal injection of 75 mg/kg Streptozotocin.

Until the day of implantation, the animals were supplemented with basal insulin via pellets, followed by daily administration of a bolus subcutaneous injection of Lantus if required, from the day of implantation to maintain satisfactory health status throughout the experiment. Depending on morning and afternoon glycemia, animals were treated with a dose of 4 to 2 IU/animal.

### b. In vivo implantation

Recipient animals weighed between approx. 270g at the time of surgery. Animals were implanted exclusively in the internal face of the peritoneum (3 implants C16B-14 with islets. The animals were implanted with 4 000 IEq at the density of 9 500 IEq/mL.

The anaesthetic and surgical procedure was performed as described in example D4B.

### c. Evolution of glycemia

Fasted glycemia was measured regularly in the diabetic (streptozotocin treated) rats using a glucose meter (Roche Guide), starting 2 weeks post-implantation.

Glycemia was taken in the early afternoon each week, after a 5-hour fasting period. Lantus was not administered the day before and, on the days, when fasting glycemia was taken (administered only in control animals).

A drop of blood glucose was obtained from the vein tail.

The animals in the test group implanted with C16B-14 with islets showed fasting normoglycemia of around 2 g/L (between 1.3 g/L and 3 g/L).

### d. Tolerance Local

Tolerance was assessed by the functionality of the grafts but also by macroscopic and histopathological features. At day 180, intraperitoneal implants were still in place, non-adherent, covered by a thin transparent membrane, without capsule, or epiploic adherences. Histopathologic examinations of the explants confirmed the absence of foreign body reaction (FBR) around the explants, which were covered by a thin membrane, without fibrosis but containing vascular sections. No macrophages nor giant cells were observed.

### e. Insulin immunostaining method on explant sections

After explantation, samples are fixed, embedded in paraffin and 7µm slices are performed. Paraffin sections are first treated to remove paraffin and an antigen retrieval step is performed. Sections are then stained with an anti insulin antibody and nuclei are counterstained with Hoescht. Sections are imaged on an epifluorescent microscope using a 40x objective lens.

### f. Functional evaluation of the explants of example C16B-14

After explantation of the 4 rats (6 to 7 months post implantation), the functionality of islets within the explants C16B-14 was evaluated in vitro as described in and intramuscularly into the rectus abdominal muscle. See Table 30.

**Table 30: Functionality of rat primary islets encapsulated in C16B-14, 6 to 7 months after in vivo implantation in diabetic rats. Mean secretions index are reported. Errors are standard deviations.**

| **Rat number** | **Explantation timing** | **Secretion index (Mean ± SD)** |
|---|---|---|
| 526 | 6 months | 1.73 ± 0.23 |
| 519 | 7 months | 1.28 ± 0.17 |
| 521 | | 1.46 ± 0.75 |
| 525 | | 1.83 ± 0.98 |

For the 4 rats of the study, encapsulated islets displayed insulin secretion in response to glucose (secretion index >1). Therefore, the functionality of rat primary islets encapsulated in C16B-14 can be maintained for at least 6 to 7 months after implantation in rat.

### GSIS (Glucose Stimulated Insulin Secretion) experiment - Method 2

To evaluate the *in vitro* functionality of primary islets, GSIS (Glucose Stimulated Insulin Secretion) experiments were performed.

Briefly, explants were first washed 3 times in Krebs buffer containing 0.1% BSA and 3mM of glucose (Solution G3, 4.5mL) (Table 31). Insulin secretion was then equilibrated by incubating the islets in Solution G3 for 4 x 30 min at 37°C. After a washing step, basal insulin secretion was obtained by incubating islets in Solution G3 for another 60 min at 37°C. At the end of this incubation, media were collected (Basal insulin secretion samples). Islets were then stimulated in Krebs buffer containing 0.1% BSA and 17mM of glucose (Solution G17) for 60 min at 37°C. At the end of the incubation, 0.5mL of media were collected (1 hour Stimulated insulin secretion samples), and 0.5 mL of Solution G17 were added. After 60 min of incubation at 37°C, 0.5mL of media were collected (2 hours Stimulated insulin secretion samples), and 0.5mL of Solution G17 were added. After 60 min of incubation at 37°C, media were collected (3 hours Stimulated insulin secretion samples).

**Table 31: Buffers and conditions for GSIS Method 2.**

| **Step** | **Solution** | **Duration (min)** | **Insulin quantification** |
|---|---|---|---|
| Wash | Krebs buffer + 3mM Glucose (Solution G3) | 2x 10 min | Not performed |
| Equilibration - G3 | | 4x 30 min | |
| Basal secretion - G3 | | 60 min | Basale insulin secretion |
| Stimulation - G17 1h | Krebs buffer + 17mM Glucose (Solution G17) | 60min | 1 hour stimulated insulin secretion |
| Stimulation - G17 2h | Krebs buffer + 17mM Glucose (Solution G17) | 60min | 2 hours stimulated insulin secretion |
| Stimulation - G17 3h | Krebs buffer + 17mM Glucose (Solution G17) | 60min | 3 hours stimulated insulin secretion |

Insulin levels in these samples were the quantified using a qualified ELISA assay specific for insulin. Insulin levels were normalized by the IEQ content of the samples (see Example B3A). Insulin secretion levels correspond to the quantity of insulin secreted during the G17 stimulation step normalized by IEQ content. Secretion indexes were calculated as the ratio of insulin concentrations measured between Stimulation and Basal secretion steps.

### Example D7B: In vivo survival and functionality of Human islets encapsulated in immunocompromised SD-RG rat with C16B-18

### a. Human primary islet encapsulation

Human primary islets were isolated as described in Example B2A and encapsulated 7 days after isolation, as described in C16B-18 example.

### a. In vivo implantation

C16B-18 was implanted in immunocompromised SD-RG rats. These animals are double knockout for the Rag1 and IL-2Ry genes (Rag1-/- Il2rγ-/-), resulting in a deficiency of B, T and NK cells (adaptative immunity).

Recipient animals weighed between approx. 300 and 320 g at the time of surgery. Animals were implanted exclusively on the internal face of the peritoneum (4 implants C16B-18). Animals were implanted with 5 300 IEQ at the density of 15 000 IEq/mL.

Anesthesia was performed with isoflurane.

Each animal was placed in the supine position on a warmed pad. The fur was shaved from surgical area with clippers. The surgical site was disinfected with povidone iodine solution.

A 3-5 cm long midline incision was made on the abdomen to open and expose the peritoneal cavity.

4 implants were placed into the parietal peritoneum, on both sides and maintained in place by non-resorbable suture stitches (Prolene 6/0).

Prior to closing the abdomen, 2 mL of physiological saline solution were dispensed into the abdominal cavity to prevent dehydration. The peritoneum with muscular layer was closed with continuous non-resorbable suture (Prolene 4/0), then the skin was sutured (Prolene 4/0) by interrupted single stitches and the incision cleaned with povidone iodine solution.

Prior to closing the abdomen, 2 mL of physiological saline solution were dispensed into the abdominal cavity to prevent dehydration. The peritoneum with muscular layer was closed with continuous non-resorbable suture (Prolene 4/0), then the skin was sutured (Prolene 4/0) by interrupted single stitches and the incision cleaned with povidone iodine solution.

Animals were explanted 6 weeks after implantation of the encapsulated islets. Following a midline incision of the abdomen, intraperitoneal implantation sites were exposed, and the implants removed. The incisions were sutured, cleaned and disinfected with povidone iodine solution. Animals were then kept in individual cages under until recovery from surgery.

### b. In vivo Human islets functionality

Human islets functionality in rats implanted with C16B-18 was assessed by specific measurement of Human C-peptide in the plasma before and up to 21 days after implantation. Blood was collected one day before implantation, and then, on Days 1, 2, 5, 7, 14, 16, 21 and 28 days post-implantation. Plasma was prepared and Human C-peptide was measured using a qualified specific ELISA immunoassay (Mercodia, Ultrasensitive C-peptide ELISA, Ref. 10-1141-01). See Table 32.

**Table 32: Human C-peptide concentrations in plasma of diabetic rats implanted with C16B-18. Indicated days are days post implantation. The lower limit of quantification (LLOQ) of the ELISA method was defined at 20.0 pM.**

| | C16B-18 | | |
|---|---|---|---|
| Rat ID | A | E | F |
| D-1 | <LLOQ | <LLOQ | <LLOQ |
| D1 | 97.4 | 110.9 | 79.8 |
| D2 | 83.1 | 47.3 | 140.7 |
| D5 | 93.2 | 125.3 | 181.7 |
| D7 | 175.7 | 179.2 | 308.1 |
| D14 | 114.3 | 221.8 | 215.6 |
| D16 | 135.8 | 193.4 | 369.3 |
| D21 | 137.2 | 248.1 | 278.7 |
| D28 | 102.3 | 175.8 | 165.8 |

Before implantation, no Human C-peptide is detected in samples, demonstrating the specificity of the bioanalytical method for Human and not Rat C-peptide. After implantation, Human C-peptide secretion increased in all three rats to reach concentrations between 102 and 176 pM at D28. These data demonstrate that encapsulated human islets in C16B-18 were functional, and that the hydrogel allowed diffusion of insulin into the bloodstream until at least 28 days post implantation.

### c. Explant analysis

At the end of the study (41 days post implantation), the functionality of islets within the explants from rats A and E was evaluated in vitro as described in example D7A. Then, encapsulated islets were lysed in RIPA buffer for 10 min on ice. Samples were mechanically crushed, and then sonicated. Insulin levels in these samples were quantified using a qualified ELISA assay specific for insulin. Insulin levels were normalized by the IEQ content of the samples (see Example B2A). See Table 33.

**Table 33: Insulin content and functionality of encapsulated human primary islets explants, 41 days after in vivo implantation in rat peritoneal cavity. Mean values are reported. Errors are standard deviations.**

| **Rat ID** | **Explant functional evaluation** | |
|---|---|---|
| | **Intracellular insulin content (µIU/IEQ) (Mean ± SD)** | **GSIS Secretion Index (Mean ± SD)** |
| A | 8.1 ± 7.9 | 5.0 ± 2.4 |
| E | 12.8 ± 7.8 | 13.3 ± 2.9 |

Encapsulated islets still contained insulin 41 days after explantation. Moreover, they maintained their ability to secrete insulin in response to glucose, illustrated by GSIS secretion index superior to 5 for explants of both rats.

Therefore, encapsulated human islets in C16B-18 with human islet 15 kIEQ/mL are still functional after a 41-day implantation period in the rat peritoneal cavity.

### Example D8: In vivo functionality of islets encapsulated

For this study 2 groups of rats were used. One "Group Test" comprising implants with islets (as disclosed above) and one group for negative control, "Group Control".

The Group Control was implanted with C16B28, C16B-15, C16B-5 and C16B-14 without islets.

### a. Rat primary islets encapsulation

Rat primary islets were isolated following Example B2B and encapsulated 1 or 2 days after isolation, as described in C16B-14 at a density of 9 500 IEQ/ml, C16B-21 at a density of 30 000 IEQ/ml, C16B-15 at a density of 10 000 IEQ/ml and C16B-17 and C16B-18 at respective densities of 12 000 and 18 000 IEQ/ml.

### b. In vivo implantation

Streptozotocin-induced diabetic rats were implanted in the intraperitoneal space as described in Example D4D. Each animal received 3 or 4 implants. The Group Test comprised animals implanted with either 4 000, 5 000 or 8 000 IEQ respectively of C16B-14, C16B-15and C16B-21 or 6 500 IEQ of C16B-17 and C16B-18.

### c. Evolution of insulinemia and C-peptidemia

Islets functionality in rats from Group Test was assessed by specific measurement of rat insulin in the plasma up to 42 days after implantation, in 5 independent studies. Functionality in control rats from Group Control as assessed by specific measurement of rat insulin in the plasma up to 41 days after implantation, in 4 independent studies.

Blood was collected approximately once weekly. Plasma was prepared and rat insulin measured using a qualified specific ELISA immunoassay (Mercodia, Ultrasensitive Rat Insulin ELISA, Ref. 10-1251-01). Area under the curve for each rat was calculated using GraphPad Prism, normalized by the time, and designated as the mean insulin secretion. See Table 34.

**Table 34: Mean insulin secretion of rats implanted with C16B-18 or C16B-17 for 35 to 41 days or 39 to 42 days, respectively.**

| | **Mean Insulin secretion (pM)** | **Standard Deviation (pM)** | **Number of rats** |
|---|---|---|---|
| Group Control | 43.00 | 40.87 | 13 |
| Group Test | 129.6 | 65.37 | 25 |

Rats from Group Test secreted significantly higher levels of insulin for up to 42 days after implantation as compared to negative control, rats from Group Test(Mann-Whitney test, p-value < 0.0001). These data demonstrate that encapsulated rat islets in Group Test were functional, and that the hydrogel allowed diffusion of insulin into the bloodstream until at least 42 days post implantation. As these data were obtained in 5 different studies using 25 rats (for Group Test ) and 4 different studies using 13 rats (for Group Control), it highlighted the robustness and the reproducibility of encapsulated islets functionality and survival *in vivo.*

### Part E - ADHESION

### Example E1: Hydrogel preparation

The described hydrogels are used for preparation of preformed implants, *in situ* formed implants on tissue and as bio-adhesives implant/tissues adhesion or tissue/tissue adhesion.

### Example E2A: Preformed and in situ formed implant preparation

The preparation of the hydrogels was made in an aseptic environment.

Polysaccharide and PEG derivatives concentrated sterile solutions prepared according to example C1A or C1B and example C2, respectively, were adjusted with a concentrated NaCl solution to obtain isotonic stock solutions (300 mOsm/kg) and equilibrated either at room temperature (20-25°C) or at 4°C. Optionally concentrated solution of polysaccharide bearing VS or DBCO groups was supplemented by a tris buffer at pH 7,4 or pH 8.

A concentrated solution of PEG was added to a concentrated solution of polysaccharide. The volume ratio (70:30 (%:%), 80:20 (%:%) or 50:50 (%:%)) of the PEG solution to the polysaccharide solution was casted:
- In a rectangular silicone isolator deposited on petri dish (preformed implant) or omentum (in situ formed implant), a surgical mesh was pre-deposited to give further mechanical reinforcement to the rectangular implant specimen for peeling test. Two types of mesh were used Mesh PET (Surgical mesh) and PVDF 230µm thickness (Dynamesh).
- In a ring net construct deposited on petri dish, for *in vivo* assays.
- In a silicon ring spacer deposited on glass slide for *in vitro* assays.
- On biological tissue (for *in situ* formed implant) with a silicone ring spacer for in vivo assays on omentum.

The ring net construct is made of two PDMS ring surrounding surgical mesh as followed:
- Biocompatible 100µm silicone sheets supplied by Grace Biolabs or Interstate Speciality Product were cut in a form of a square incorporating and then a circular empty disc was punched using a stainless-steel punch.
- A piece of the net (wider than the square silicone pieces) was introduced in between the two square silicone pieces.
- The circular empty discs were aligned, and the surgical net was kept tense during gluing with biocompatible silicone glue (Silbione MED ADH 4200 supplied by Elkem).
- Finally, the square construct was cut with a stainless-steel punch to obtain the final object constituted by two silicone rings sandwiching a surgical net glued together.
- The pieces were washed with a solution of poloxamer F127 at 1% and rinsed with water.
- Resulting ring net constructs underwent steam sterilization (PET), or ETO (PVDF) or alternatively were decontaminated with a Surfa'Safe Premium (Anios) bath.

Several specimens for peeling test were used as described in table 35.

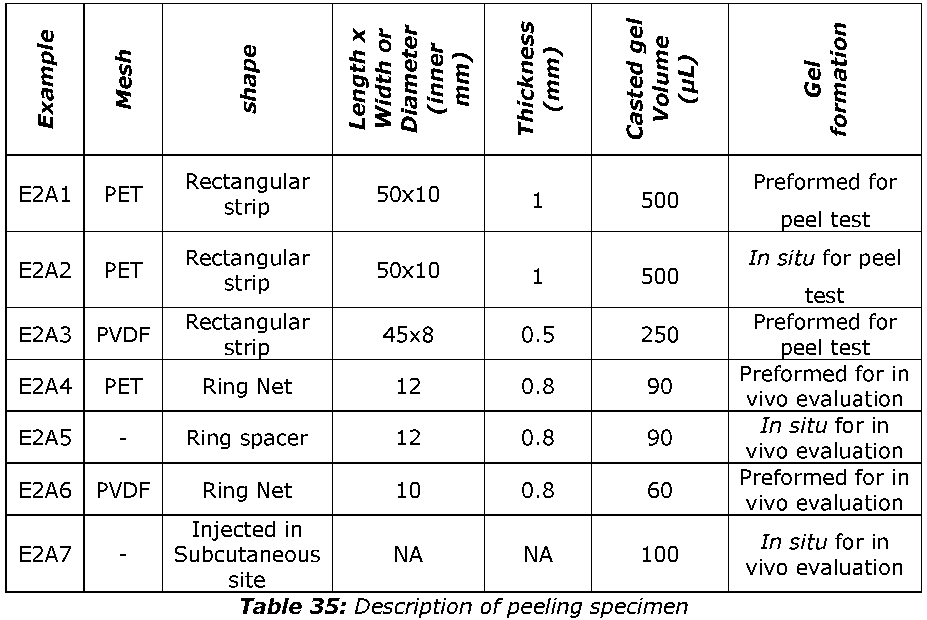

*Cross-linking process leading to gelation was carried out for 1 h at room temperature (20-25°C) for preformed implant or at 37°C for in situ formed implant.*

### Example E2B: Adhesive preparation

The preparation of the hydrogels was made in an aseptic environment.

Polysaccharide and PEG derivatives concentrated sterile solutions prepared according to example C1A or C1B and example C2, respectively, were adjusted with a concentrated NaCl or trehalose solution to obtain isotonic stock solutions (300 mOsm/kg) and equilibrated either at room temperature (20-25°C) or at 4°C. Optionally concentrated solution of polysaccharide bearing Mal, VS or DBCO groups was supplemented by a tris buffer at pH 7,4 or pH 8.

A concentrated solution of PEG was added to a concentrated solution of polysaccharide in a 2 mL Eppendorf. The volume ratio of the PEG solution to the polysaccharide solution was 50:50 (%:%) or 70:30 (%:%) or 80:20 (%:%). The adhesive solution was mixed with a pipette and deposited on the top surface of swollen implant specimen.

The freshly glued surface was gently put into contact with the desired substrate. Remaining 15 min before mechanical testing.

### Example E3: Hydrogel (implant and adhesive) compositions

Different hydrogel (as implant or adhesive) compositions were prepared according to the protocol described in Example E4A and E4B and are shown in Table 36. Concentrations of the two reactive groups (maleimide (Mal) or vinylsulfone (VS) reacting with thiol (SH) or alcyne (DBCO) reacting with azide (N3)) and polymers (polysaccharide and PEG derivatives) correspond to the final concentration upon mixing of the polymer solutions.

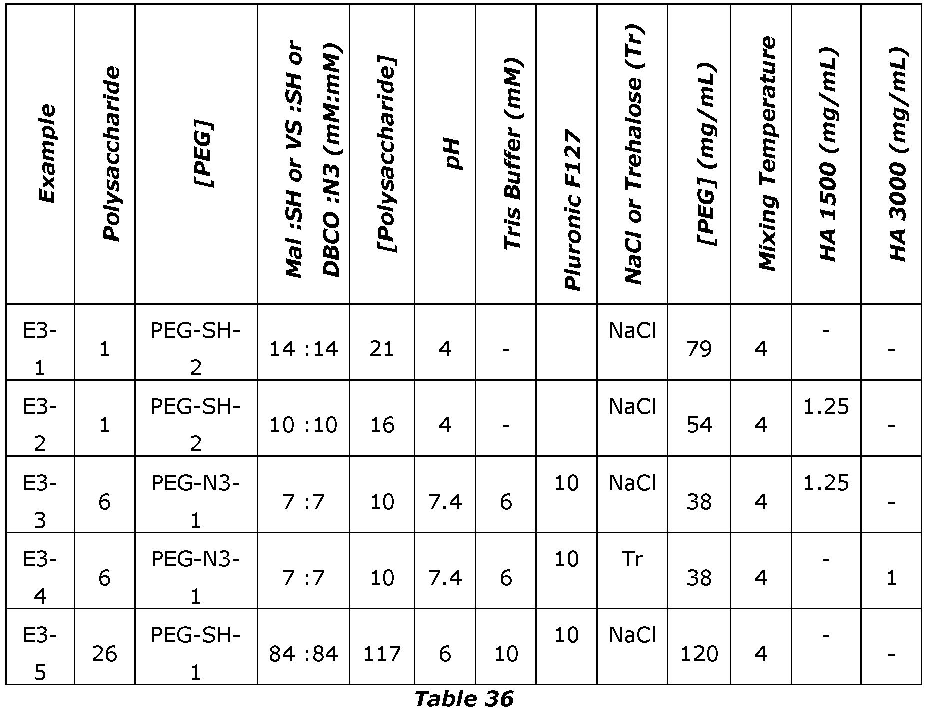

| **Hydrogels (concentration in reactive species (mM))** | **Structure** | | |
|---|---|---|---|
| E3-1 | 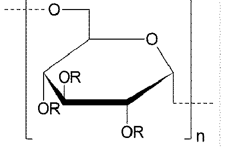 | | |
| E3-2 | | | |
| | Mw (Dextran) = 40 kg/mol | | |
| | n = 205 | | |
| | R = H or | 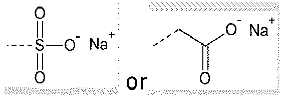 or | |
| | 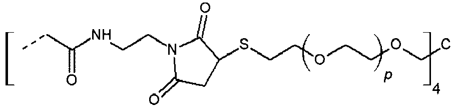 | | |
| | p = 114 | | |
| | DS₃ (sulfate) = 1.5 | | |
| | DS₂ (methylcarboxylate) = 0.8 | | |
| | DS₁ (succinimide derivative) = 0.25 | | |
| E3-3 | 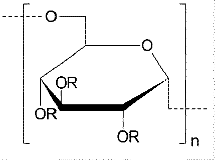 | | |
| E3-4 | | | |
| | Mw (Dextran) = 40 kg/mol | | |
| | n = 205 | | |
| | R = H or | 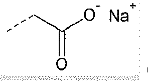 | or |
| | 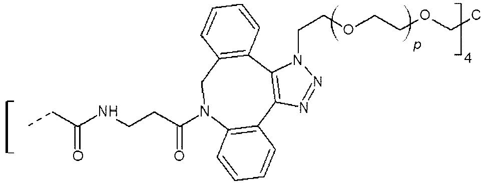 | | |
| | or | | |
| | 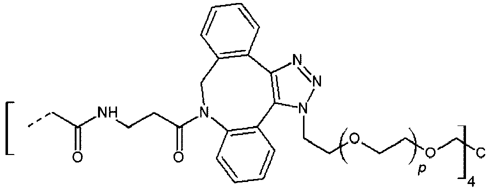 | | |
| | p = 114 | | |
| | DS₂ (methylcarboxylate) = 2.1 | | |
| | DS₁ (triazole derivatives (mixture of 2 isomers)) = 0.28 | | |
| E3-5 | 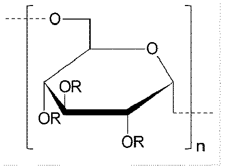 | | |
| | Mw (Dextran) = 40 kg/mol | | |
| | n = 205 | | |
| | R = H or | 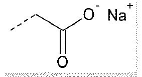 | or |
| | 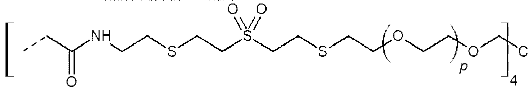 | | |
| | p = 30 | | |
| | DS₂ (methylcarboxylate) = 2 | | |
| | DS₁ (sulfone derivative) = 0.25 | | |

### Example E4: Peeling test

### Example E4-1: Peeling test description

To quantify adhesion, a peeling test is conducted on a swollen rectangular hydrogel piece as prepared as in example E2A. The strip sample is freshly glued to a non-mobile substrate. A section of the strip is kept glue-free to allow further clamping.

Substrates can be a fresh pig omentum outstretched with an embroidery frame to guarantee immobility.

Substrates can be a chicken breast piece 80x30x10 mm deposited on aluminium foil. Chicken piece is glued to a rigid paper sheet to guarantee immobility.

Mechanical peeling test consists in stretching the sample uniaxially in air at 20-25°C by using a universal mechanical tester apparatus (Zwickroell). The initial position of the upper clamp is set at 1cm from the substrate surface. The sample strip adopts a 90° angle between the upper clamp and the planar substrate at rest before test. Peeling is initiated by controlling the displacement of the upper clamp at 5 mm/min speed.

### Example E4-2: Adhesion energy calculation

Adhesion is quantified from the peeling force recorded during the peeling test described in example E4-1.

The measured force obtained during peeling depends on the weight of the gel that increases with the coat stroke. However, we have considered this contribution to be negligible. Moreover, as the clamp rise, the angle between the peel strip and the omentum increases which implies that the stress is distributed over an increasing area. The measured force is therefore increasing as the displacement occurs. The influence of increasing angle with displacement implies therefore that the adhesion energy is overestimated compared to an angle peel constant 90°.

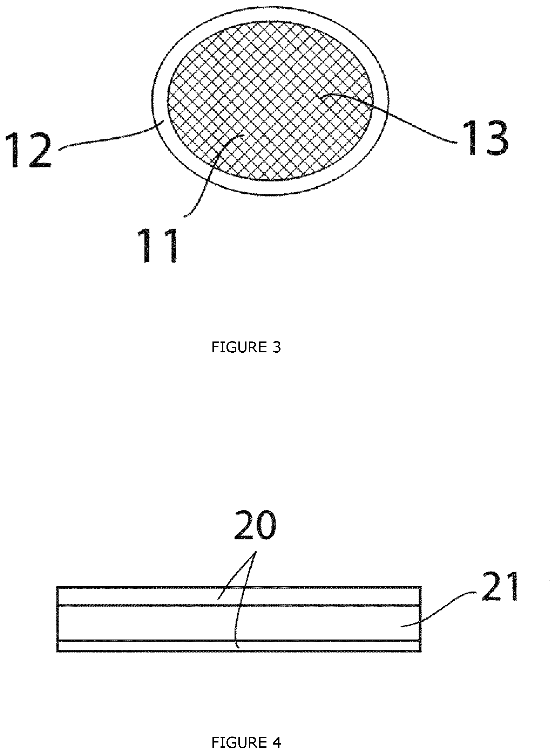

The beginning and the end of the test is considered as a transition regime where the peeling is not stable. It was therefore chosen to consider values energy between 5 and 25 mm of peel stroke as shown in Figure 3.

Adhesion energy (G) is derived from the integration of the force-displacement curve considering the 5 to 25 mm displacement.

The adhesion energy G per unit area, is obtained by the relationship: $G = \frac{{\int }_{x = 0.005}^{x = 0.025} F \left(x\right) dx}{Δ x . w}$

Where F(x) is the peel force at the displacement x and w is the width of the hydrogel strip.

The contact of reference samples (or controls) onto humid substrate (preformed samples E4-3-1 and E4-3-4) is considered non adherent. The adhesion energy without glue is measured at *G_{ref}* = 1.0 ± 0.1 J/m². Therefore, the relevant data to appreciate the improvement of adhesion is: $Δ G = G - {G}_{ref}$

### Example E4-3: Results

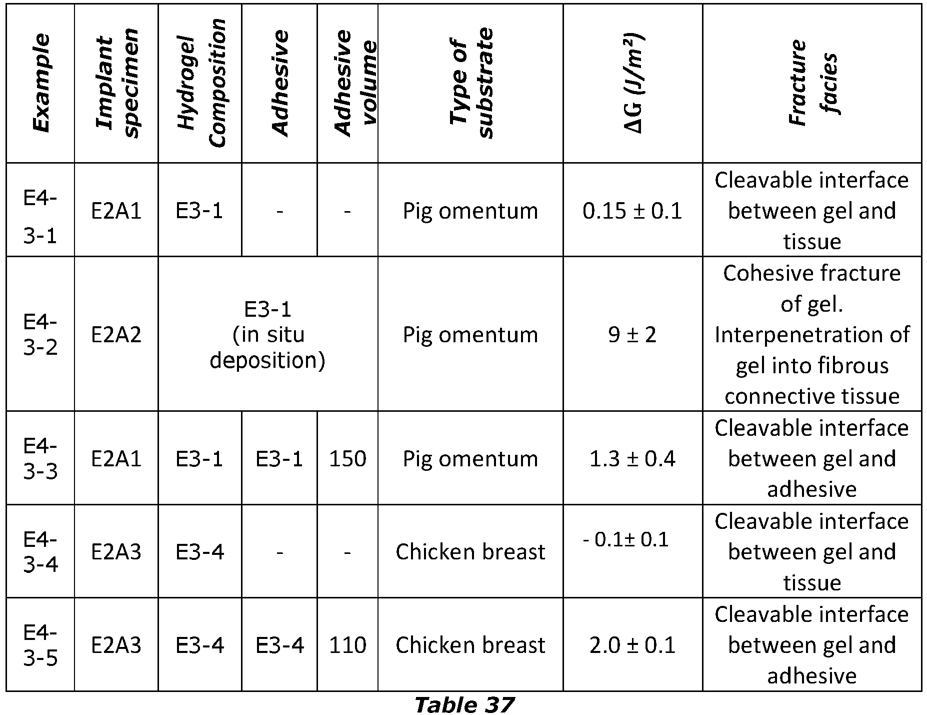

A cleavable interface between gel and tissue is observed without glue for preformed hydrogel (E4-3-1). On the contrary a cohesive fracture of gel for *in situ* formed implant (E4-3-2) is revealed at the end of the test. The adhesion energy significantly increases compared to preformed hydrogel without glue.

Therefore, the *in-situ* formation of hydrogel constitutes a performant glue which can be used to improve implant adhesion.

Regardless of the tissue types, the addition of glue hydrogel between tissue and preformed hydrogel (E4-3-3 and E4-3-5) permits to increase adhesion energy.

The hydrogel can thus be used as interpenetrating glue into tissue to improve hydrogel implant adhesion to tissue.

### Example E5: In vivo adhesion and biocompatibility

Hydrogel glues were also deposited on tissue *in vivo* in rat omentum, rat intraperitoneal membrane or in rat sub cutaneous site to evaluate the *in vivo* adhesion and biocompatibility.

The *in-situ* implants were not stitched and directly casted into the PDMS ring spacer, onto the animal tissue or injected in subcutaneous site.

The preformed implant can contain islets (rat or human primary islet).

The preformed implants including ring net and hydrogel as described in examples E2A4 and E2A6 are first stitched onto the animal tissue. Two up to four stitches are done with surgical sterile thread.

20µL of glue is then deposited in between the implant and the animal tissue directly *in situ* using a pipettor. A manual compression during dozen seconds allows the glue to crosslink and adhere to both surfaces.

The hydrogel implants were glued in different *in vivo* configurations as described in Table 38.

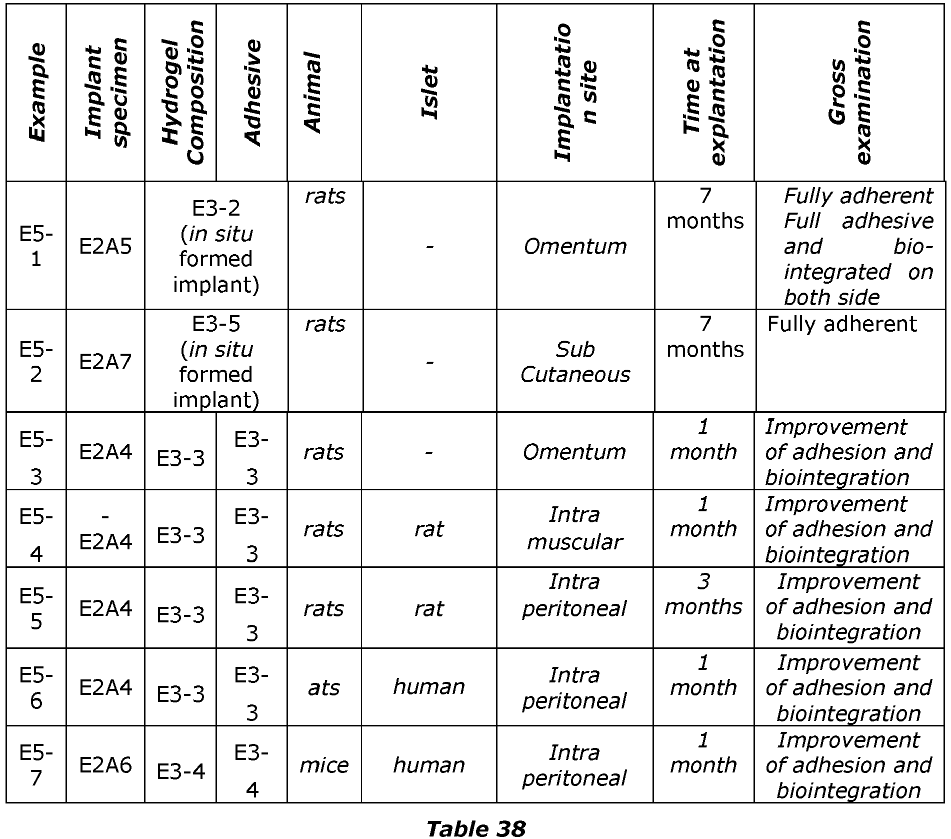

The hydrogels were carefully observed at the time of and after surgery.

Regardless of the hydrogel composition, site, or implant specimen (glued preformed hydrogel or *in situ* formed hydrogel) the hydrogels were well tolerated. No signs of inflammation were observed at the implant site or in the surrounding tissues.

Injected hydrogels in subcutaneous site were found to be adherent to tissues but extractable. The explant was associated with an adherent tissue, easily removable, and non-fibrous appearance.

Glueing the implant to tissue avoids primary and secondary implant folding. Glueing also enhances implant fixation thus promoting better biointegration.

Histological examination confirms biointegration or tissue integration of the glue and good local tolerance. Either the glue was used for implant-tissue adhesion (E5-3, E5-4, E5-5, E5-6, E5-7) or tissue-tissue adhesion (example E5-1 and E5-2).

### Part F - Multilayer hydrogel implants and 3D printing

Multilayered hydrogel structures can be obtained by casting consecutively the reactive solution mixtures onto the others. The first layer needs to be already at a pre gelified state (soft solid) before casting new layer onto it. The casting time between layers is chosen short enough between 2 and 15 minutes, preferably around 5 min, to allow a nice gluing, maybe via interpenetration of the neighbouring layers upon gelation.

Multilayered hydrogel is preferably constituted of one main hydrogel part and one sublayer. The sublayer is casted first and represents the smallest volume fraction of the total structure, typically less than half of the the total volume.

The formulation of each layer can be tuned to offer various properties. The sublayer is mainly developped to increase barrier properties and promoting enhanced immunoisolation. The main hydrogel part can contain living cells for cell therapy application in particularly islets for diabete.

### Example F1: Multilayered hydrogel preparation

The preparation of the hydrogels was made in an aseptic environment.

Polysaccharide and PEG derivatives concentrated sterile solutions prepared according to example C1B and example C2, respectively, were adjusted with a concentrated NaCl or trehalose solution to obtain isotonic stock solutions (300 mOsm/kg) and equilibrated either at room temperature (20-25°C).

A concentrated solution of PEG/Hyaluronate was prepared by mixing a concentrated solution of PEG prepared according to example C1B and a concentrated solution of sodium hyaluronate prepared according to example C3.

A concentrated solution of polysaccharide was supplemented by a tris buffer at pH 7,4. Optionally a poloxamer F127 or F68 at 1% was also added.

A concentrated solution of PEG was added to a concentrated solution of polysaccharide in a 2 mL Eppendorf. The volume ratio of the PEG solution to the polysaccharide solution was 80: 20 (%:%) or 50:50 (%:%). The solutions were mixed with a pipette and a controlled volume of the mixture was introduced in a silicone isolator. The sublayer volume was first casted and waited to gel between 5 to 15 min before a second volume of main hydrogel part was pipetted on the top. Optionally, the sublayer can be dried in air before applying a second humid hydrogel volume on top. The sublayer reabsorbs efficiently water and was found stable once immersed. A silicon ring spacer or a ring net construct can be used, adhering to a glass slide or a Petri dish respectively.

The ring net construct is made with two silicone rings surrounding a surgical mesh (PVDF, 230µm thickness, Dynamesh)

Biocompatible silicone sheets supplied by Grace Biolabs or Interstate Speciality Product were cut in a form of a square incorporating a circular empty disc using a stainless-steel punch. A part of the net was introduced in between two square silicone pieces. The two silicone pieces and the surgical net were glued together with biocompatible silicone glue (Silbione MED ADH 4200 supplied by Elkem). The circular empty discs were aligned, and the surgical net was kept tense during gluing. Finally, the square construct was cut with a stainless-steel punch to obtain the final object constituted by two PDMS rings sandwiching a surgical net and glued together. The pieces were washed with a solution of poloxamer F127 at 1% and rinsed with deionized water. Ring net constructs undergo either decontamination using a Surfa'Safe Premium (Anios) bath or ETO sterilization.

Different molded hydrogels geometries were prepared.

**Table 39: Molded multilayer hydrogel geometries conditions.**

| **Geometry** | **Type of silicone isolator** | **Silicone inner diameter (mm)** | **Silicone Isolator Thickness (mm)** | **Hydrogel volume (µL)** | **Sublayer Volume (µL)** |
|---|---|---|---|---|---|
| F1-1 | Disc | 4.5 | 0.5 | 10 | 0 |
| F1-2 | Disc | 9 | 1.7 | 70 | 30 |
| F1-3 | Ring-net | 12 | 0.5 | 100 | 0 |
| F1-4 | Ring-net | 10 | 0.5 | 60 | 0 |
| F1-5 | Ring-net | 10 | 0.5 | 50 | 20 |
| F1-6 | Ring-net | 12 | 0.5 | 70 | 30 |

Cross-linking process leading to gelation was carried out for 30min or 1h at 20°C. The hydrogel was unmolded and introduced in PBS at pH 7.4 for 1h at 37°C.

After gelation, a new geometry can be produced from the ring net multilayer implant (geometry F1-4 or F1-5) by cutting circular disc inside the implant using biopsy punch of 4 mm diameter. 3 to 4 punched discs can be cut and easily extracted from initial implant. The thickness is similar to initial ring-net implant.

**Table 40**

| **Geometry** | **Ininital silicon geometry** | **Diameter of punched disc (mm)** |
|---|---|---|
| F1-7 | F1-4 | 4 |
| F1-8 | F1-5 | 4 |

The hydrogel was rinsed with 20 mL of PBS solution and further immersed in 10 mL of the PBS solution overnight at 37°C.

Solid disc-shaped hydrogel pieces were obtained. The hydrogels pieces were easily unmoulded and handled with tweezers for characterization.

### Example F2: Hydrogel compositions

Different hydrogel compositions were prepared according to the protocol described in Example C1 and are shown in Table 41. Concentrations of the two reactive Alcyne groups (DBCO) reacting with Azide (N3) and polymers (polysaccharide and PEG derivatives) correspond to the final concentration upon mixing of the polymer solutions. Same reactive polymers are used for both hydrogel parts. Yet, the sublayer does not contain sodium hyaluronate to facilitate deposition with a minimal viscosity.

**Table 41**

| ***Exa mpl*** e | ***Polysac charide*** | ***[P EG ]*** | ***Mal: SH or VS:S H or DBC O:N* 3 *(mM :mM )*** | ***[Polysac charide] (mg*/*mL )*** | ***p H*** | ***Tr is Bu ff er ( m M )*** | ***[PE G] (mg* /*mL )*** | ***Sodiu m Hyalu ronat* e *3000 kDa* (*mg*/ *mL)**** | ***Mixin g Temp eratur* e *(°C)*** | ***Gellin g temp eratu re (°C)*** |
|---|---|---|---|---|---|---|---|---|---|---|
| F2-1 | 6 | PEG-N3-1 | 7- : 7 | 10.3 | 7.4 | 6 | 38.1 | 1 | 4 | 20-25 |
| F2-2 | 6 | PEG-N3-1 | 12-12 | 17.7 | 7.4 | 6 | 65.4 | 1 | 4 | 20-25 |

| | | | | | | | | | | |
|---|---|---|---|---|---|---|---|---|---|---|
| **sodium hyaluronate is not added in the sublayer.* | | | | | | | | | | |

| **Hydrogels (concentration in reactive species (mM))** | **Structure** | |
|---|---|---|
| F2-1 | 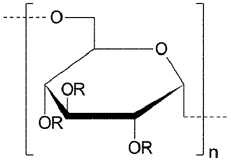 | |
| F2-2 | | |
| | Mw (Dextran) = 40 kg/mol | |
| | n = 205 | |
| | R = H or | 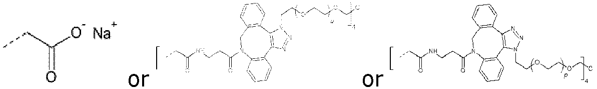 |
| | p = 114 | |
| | DS₂ (methylcarboxylate) = 2.1 | |
| | DS₁ (triazole derivatives (mixture of 2 isomers)) = 0.28 | |

### Example F3: Evaluation of the multilayered structure

Different multilayer hydrogels were prepared according to the protocol described in Example F1 and are shown in Table 40.

The sublayer was optionally supplemented with phenol red biocompatible dye at less than 0.01% to help visualization during casting. The visualization of the red color is satisfyingly well distributed over the bottom surface indicating a good covering.

The sublayer was optionally supplemented with a fluorescent 500kDa Dextran-FITC probe (1.5mg/mL). After swelling, the multilayer hydrogel was cut using a flat razor blade to produce a 2mm width strip. The section of such sample was observed with a fluorescent microscope) in air while keeping the strip humid. The thickness of the layer could be evaluated by measuring the fluorescence emitted by the probe located exclusively in the sublayer. The overall bottom part of the section is found fluorescent assessing again for a good covering of the sublayer. Such observation allowed to extract the overall relative thickness of the sublayer with respect to the total thickness of the gel.

The relative sublayer thickness is evaluated at several locations along the section by the ratio of the sublayer thickness over the total thickness of the gel. The average relative sublayer thicknesses are reported in table 41 for different hydrogel implants. The relative thickness is found lower in the absence of mesh present in the ring net construct. The sublayer is hence considered to represent in average less than 100µm thickness in discs and less than 200 µm in ring net constructs. Noticeably, the thickness of the sublayer is found to vary within the radial position in the section in particular near the external zone of the implant. This nonlinear thickness profile is inherent to wetting effect which varies in the presence of the mesh but does not impair the good covering of the sublayer. The sublayer is considered to protect the bottom surface of the hydrogel from a direct contact with surrounding environment (air or liquid).

All samples were stable in immersion in PBS or CMRL for several weeks and show no extra significant swelling (less than 15% increase of reference swollen state). The cohesion of the multilayered was verified: no defect was observed with magnifying glass after sample been twisted manually to force mechanical stress. The consecutive casting of two layered hydogels allows to produce stable and cohesives structures satsifying for implantation procedure.

**Table 42: Description of multilayered hydrogels samples structure and stability**

| **Configuration** | **Geometry** | **Hydrogel composition** | **Relative sublayer thickess** | **Stability** |
|---|---|---|---|---|
| F3-1 | F1-2 | F2-1 | 11 ± 3 % | Stable and cohesive |
| F3-2 | F1-2 | F2-1 | 13 ± 5 % | Stable and cohesive |
| F3-3 | F1-3 | F2-1 | 22 ± 8 % | Stable and cohesive |
| F3-4 | F1-3 | F2-1 | 27 ± 9 % | Stable and cohesive |
| F3-5 | F1-5 | F2-1 | 16 ± 9 % | Stable and cohesive |

### Example F4: Printability of the hydrogel

Hydrogel compositions are adapted to extrusion-based 3D printing. An ongoing gelation mixture from solutions prepared as described in Example F2, can be printed throughout a nozzle. This outflow mixture results from the covalent reaction of two reactive compouds as described in example F2 . The mixing time prior nozzle ouput has to be controlled by printing set up and parameters to form a stable and elastic deposit.

The reactive mixture has intermediate viscosity (80-100 mPa.s) and exhibits a shear thinning behavior allowing an easy extrusion for good printability.

Two solution precursors were loaded independently in a double syringe 4:1 system (K-system 504541 MedMix) at room temperature (20-25 °C). This process is adapted to sterile conditionning. The mixing was controlled using a static mixing chamber (502109 Medmix) representing 230µL dead volume. The mixer output was connected to a plastic flexible tubing of 1mm diameter (Venofix) and ended by a flat ended precision needle tip (23G-20G-18G). The flow was controlled using a syringe pump at a constant flow rate between 30-50mL/h, preferably at 33mL/h. The tubing length can be vary according to flow and needle geometries to reach a final premix time constant of 1min (in the present example with formulation F1-2).

The syringe pumping system was adapted on a printing machine at room temperature (20-25 °C). The printed head speed and hydrogel flow were independently monitored. The distance between nozzle tip and bottom substrate was set constant and low enough (not more than inner nozzle diameter). The printing head speed was adapted according to diameter size as described. Approximately 300µL were printed in an alternative rectangular pattern over 10 to 20 cm length. The printing was performed in air onto a large Petri Dish at room temperature. The quality of the deposit was evaluated by considering first, the stability of the printing (good cylindrical shape fidelity) and second, the continuity of the deposit. A magnifying glass was used to measure the filament diameter at several locations allowing to obtain an average filament thickness. Observations are described in different printing conditions listed in Table 43.

**Table 43: Description of hydrogel filament deposition by extrusion-based 3D printing**

| **Example** | **Hydrogel composition** | **Printing head speed (mm/min)** | **Inner needle diameter (µm)** | **Approximate deposited filament diameter (µm)** | **Observations** |
|---|---|---|---|---|---|
| F4-1 | F2-2 | 840 | 600 | 1000 | continuous and stable |
| F4-2 | F2-2 | 475 | 800 | 1200 | continuous and very stable |

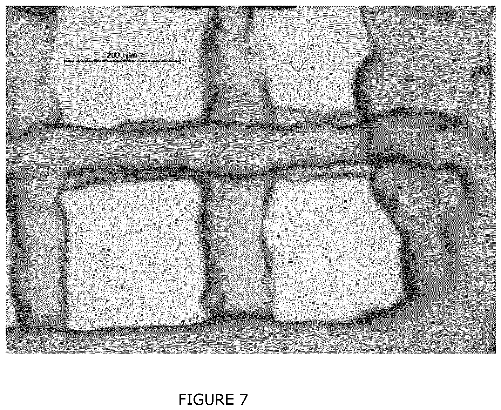

Using F4-1 printing parameters, a multilayered 3D structre is build by superimposing 3 layers of a continuous filament. A final cohesive and stable structure is obtained associated with an average porosity of 2 mm (see Figure 7). The superimposition of the filaments is well observed and maintained after immersion. This porous 3D scaffold can promote bio integration for vessels and enhance oxygenation thanks to high surface / volume ratio.

### Part G - Biology

### Example G1: Encapsulation of insulin producing cell islets in Hydrogels

Cell islets encapsulation was made in an aseptic environment.

Polysaccharide and PEG concentrated sterile solutions prepared according to examples C1 or C1A and C2, respectively, were adjusted with a concentrated NaCl or trehalose solution in order to obtain isotonic stock solutions (300 mOsm/kg). Solutions were equilibrated either at room temperature (20-25°C or at 4°C).

A concentrated mixture of PEG and islets was prepared by mixing equal volumes of isotonic PEG solution or PEG / Sodium hyaluronate solution (prepared according to example C2, C3 and C4A) and islets suspension (prepared according to example B1). Then the mixture containing PEG and islets was gently mixed with an isotonic concentrated solution of polysaccharide by a 80:20 (Volume:Volume) proportion of islets suspension/ PEG/Hyaluronate .: polysaccharide.

The solutions were gently mixed with a pipette and a controlled volume of the mixture was introduced in a circular silicone isolator or in a Ring Net construct as described in F1. Different hydrogel sizes and multilayered gel were made by using different silicone molds or ring net constructs. In the case of multilayered gels, only the mail hydrogel volume contained islet while the sublayer is empty.

Crosslinking leading to gelation was carried out from 30min in an oven at a controlled temperature such as 20°C. The hydrogel disc or hydrogel / ring net incorporating islets was introduced in culture medium for 15 min at room temperature (DBCO:N3 cross-linking). Hydrogels were then immersed in culture medium at 37°C. After 1 h the culture medium containing cysteine was removed and replaced by culture medium. Sterile hydrogels containing cells were stored at 37°C and 5% CO2 before further in vitro testing or in vivo implantation.

Different hydrogel / Islets compositions were prepared according to this process, see the following table.

**Table 44**

| **Ex am ple** | **Ge om etr y** | **Impla nt geome try** | **Hydro gel formu lation** | **Subl ayer** | **Polys accha ride** | **PEG** | **DBCO:N 3 (mM:mM )** | **Gelatio n Temp erature ( °C)** | **Isle t typ e** | **Islets density (iEQ/ mL)** |
|---|---|---|---|---|---|---|---|---|---|---|
| G1-3 | F1-1 | Disc | F2-1 | no | 6 | PEG-N3-1 | 7- : 7 | 20°C | Hu man | 15 000 |
| G1-4 | F1-1 | Disc | F2-1 | no | 6 | PEG-N3-1 | 7- : 7 | 20°C | Hu man | 15 000 |
| G1-5 | F1-1 | Disc | F2-1 | no | 6 | PEG-N3-1 | 7- : 7 | 20°C | Hu man | 15 000 |
| G1-6 | F1-1 | Disc | F2-1 | no | 6 | PEG-N3-1 | 7- : 7 | 20°C | Hu man | 15 000 |
| G1-7 | F1-1 | Disc | F2-1 | no | 6 | PEG-N3-1 | 7- : 7 | 20°C | Hu man | 15 000 |
| G1-8 | F1-6 | Punche d ring net | F2-1 | no | 6 | PEG-N3-1 | 7- : 7 | 20°C | Hu man | 25000 |
| G1-9 | F1-7 | Punche d ring net | F2-1 | yes | 6 | PEG-N3-1 | 7- : 7 | 20°C | Hu man | 25000 |

### Example B2-G2: Islet equivalent counting

To normalize the quantity of islets used in each experiment, islets were counted as a batch and in each sample to determine the islet equivalent count (IEQs). One IEQ corresponds to the volume of a perfectly spherical islet with a diameter of 150 µm. During counting, a multiplicative factor was applied to each islet depending on its size. This mathematical compensation for islet varying diameters allows for normalization between islet preparations (See NIH CIT Consortium Chemistry Manufacturing Controls Monitoring Committee; Purified Human Pancreatic Islet: Qualitative and Quantitative Assessment of Islets Using Dithizone (DTZ): Standard Operating Procedure of the NIH Clinical Islet Transplantation Consortium. CellR4 Repair Replace Regen Reprogram).

Naked and encapsulated islets were observed under a stereomicroscope (Leica, S9D). Images obtained were analyzed using ImageJ to determine the islet ferret's diameter. Islets were categorized by size according to Table 45.

**Table 45: Multiplication factor of islet size for islet equivalent determination**

| **Islet size** | **Multiplicative factor** |
|---|---|
| 50-100 µm | 1/6 |
| 100-150 µm | 1/1.5 |
| 150-200 µm | 1.7 |
| 200-250 µm | 3.5 |
| 250-300 µm | 6.3 |

### Example G3: Static GSIS (Glucose Stimulated Insulin secretion) experiments

To evaluate the *in vitro* functionality of encapsulated human primary islets, static GSIS (Glucose Stimulated Insulin Secretion) experiments were performed. Briefly, encapsulated islets were first washed 3 times in Krebs buffer containing 0.1% BSA and 3mM glucose (Solution G3) (Table 46). Insulin secretion was then equilibrated by incubating the encapsulated islets in Solution G3 for 4 × 30min at 37°C. After a washing step, basal insulin secretion was obtained by incubating encapsulated islets in Solution G3 for another 60min at 37°C. At the end of this incubation, media were collected (Basal insulin secretion samples). Encapsulated islets were then stimulated in Krebs buffer containing 0.1% BSA and 17mM of glucose (Solution G17) for 60min at 37°C. At the end of the incubation, media were collected (Stimulated insulin secretion samples).

**Table 46: Buffers and conditions for static GSIS experiments**

| **Step** | **Solution** | **Duration (min)** | **Insulin quantification** |
|---|---|---|---|
| Wash | Krebs buffer + 3mM Glucose (Solution G3) | 3 x 10 min | Not performed |
| Equilibration - G3 | | 4 x 30 min | |
| Basal secretion - G3 | | 60 min | Basal insulin secretion |
| Stimulation - G17 | Krebs buffer + 17mM Glucose (Solution G17) | 60min | Stimulated insulin secretion |

Insulin levels in these samples were then quantified using a qualified ELISA assay specific for insulin. Insulin levels were normalized by the IEQ content of the samples (see Example G2). Insulin secretion levels correspond to the quantity of insulin secreted during the G17 stimulation step normalized by IEQ content. Secretion indexes were calculated as the ratio of insulin concentrations measured between Stimulation and Basal secretion steps.

### Example G4: Dynamic GSIS experiments - Perifusion

To evaluate the *in vitro* functionality of encapsulated islets, perifusion experiments were performed. Encapsulated islets were introduced in different perifusion chambers. Chambers were then simultaneously perfused following the protocols described in Table 47.

**Table 47: Perifusion conditions**

| ***Step*** | ***Solution*** | ***Duration (min)*** | ***Flow Collection Frequency*** |
|---|---|---|---|
| *Equilibration* - *G3* | *Krebs buffer* + *3mM Glucose (G3)* | *48* | *100µL*/*min No collection* |
| *1^{st} Basal secretion* - *G3* | | *12* | *100µL*/*min* |
| *Stimulation* - *G17* | *Krebs buffer* + *17mM Glucose (G17)* | *36* | |
| *2^{nd} Basal secretion* - *G3* | *G3* | *12* | |
| *Stimulation* - *KCI* | *Krebs buffer* + *25mM KCI* | *6* | *1 well*/*2min* |
| *3^{rd} Basal secretion* - *G3* | *G3* | *6* | |

Flow-through buffer was collected, and insulin was quantified using a qualified ELISA assay specific for insulin.

The Insulin output for each sample was calculated as the sum of insulin concentrations measured over the 1^{st} Basal secretion, the Stimulation, and the 2^{nd} Basal secretion steps.

The Secretion Index for each sample was calculated as the ratio of the average insulin concentration measured during the stimulation step over the average insulin concentration measured during the 1^{st} basal secretion step.

### Example G5: Addition of an underlayer to the hydrogel maintains human islets functionality

Primary human islets were encapsulated in G1-8 and G1-9 as described in Example G1 and maintained in culture medium in a 37°C / 5% CO₂ incubator. One day after encapsulation (E1), G1-8 and G1-9 containing islets were punched to obtain discs of 4 mm of diameter and maintained in culture medium in a 37°C / 5% CO₂ incubator for 24h. Their functionality was evaluated 2 days after encapsulation (E2). See Table 48.

**Table 48: Functionality of encapsulated primary human islets (E2) in presence or absence of an underlayer. Errors are standard deviations. N=1 human islet preparation, n=1 replicate for encapsulated islets in G1-8 and n=2 replicates for encapsulated islets in G1-9, 2 punches per replicate were studied.**

| **Example** | **Insulin output (mUI/L)** | **Functionality Secretion index** | **Time to reach first G17 peak (minutes)** |
|---|---|---|---|
| Encapsulated islets in G1-8 | 3851 ± 884 | 2.4 ± 0.7 | 11 ± 1.4 |
| Encapsulated islets in G1-9 | 4569 ± 606 | 2.0 ± 0.2 | 12 ± 0 |

Addition of an underlayer maintained insulin output, secretion index and insulin secretion kinetics compared to encapsulated islets without underlayer.

In conclusion, the presence of an underlayer maintains the functionality of encapsulated primary human islets at least 2 days after encapsulation.

### Example G6: In vitro pre-treatment of encapsulated human primary islets with Kineret^{®} favors resistance to hypoxia

### a) Human primary islet encapsulation

Human primary islets from 3 independent preparations were isolated as described in Example B2A, and encapsulated in G1-3, Cxxx4-G1-4 and G1-5, at 7, 4 and 7 days after isolation, respectively.

### b) Pre-treatment of encapsulated human primary islets with Kineret^{®}

Kineret^{®} is a biopharmaceutical medication containing Anakinra, an interleukin 1 receptor antagonist recombinant protein, that therefore harbors antiinflammatory properties.

Encapsulated islets were pre-treated with Kineret^{®} diluted in culture medium for a final concentration of 10µg/mL of Anakinra, and incubated for 48h at 37°C under 5% CO₂ in a humidified atmosphere. After Kineret^{®} treatment, culture medium was changed with a fresh medium without Kineret^{®}.

### c) Incubation of encapsulated human primary islets in in vitro hypoxic conditions

Encapsulated islets were incubated for 48h at 37°C under 5% CO₂ and 5% O₂, in a humidified atmosphere.

### d) In vitro functionality evaluation of encapsulated human primary islets

The *in vitro* functionality of encapsulated human primary islets was evaluated as described in Example B3, 4 days after encapsulation. Hypoxic stress was induced *in vitro* by incubating the encapsulated human primary islets in hypoxic condition for 48h. Effect of Kineret^{®} on hypoxic stress reversal was tested by pretreating the encapsulated human primary islets 48h before hypoxia. For each sample, secretion indexes were normalized to the mean secretion index of their respective untreated control condition (normoxia). Results from Examples G1-3, G1-4 and G1-5 were pooled. See Table 49.

**Table 49: Functionality of encapsulated human primary islets in G1-3, G1-4 and G1-5. Data generated with N=3 independent human primary islets preparations were pooled. A total of n=12 samples were analyzed per condition. Statistical analyses = One-way ANOVA: Post test p-values vs. Hypoxia condition are reported. N.A. = Not Applicable. Sd = Standard deviation.**

| | **Untreated control (normoxia)** | **Hypoxia** | **Kineret^{®}** / **Hypoxia** |
|---|---|---|---|
| Normalized secretion indexes (Mean ± sd) (%) | 100 ± 32 | 44 ± 18 | 74 ± 30 |
| Post test p-values | 0.0004 | N.A. | 0.0153 |

Secretion indexes of encapsulated human primary islets were drastically decreased upon incubation in hypoxia for 48h (one-way ANOVA, p=0.0004 vs. Untreated control). Pretreatment with Kineret^{®} for 48h before hypoxia incubation, partially restored encapsulated islet functionality (one-way ANOVA, p=0.0153 vs. Hypoxia).

In conclusion, in vitro pretreatment of encapsulated human primary islets with 10µg/mL of Kineret^{®} for 48h improves islet resistance to hypoxia.

### Example G7: In vitro pretreatment of encapsulated human primary islets with Kineret^{®} restores islet functionality after cytokine induced inflammation.

### a) Human primary islet encapsulation

Human primary islets from 3 independent preparations were isolated as described in Example B2A, and encapsulated in G1-3, G1-5 and G1-6, at 7, 7 and 12 days after isolation, respectively.

### b) Pre-treatment of encapsulated human primary islets with Kineret^{®}

Encapsulated islets were pre-treated with Kineret^{®} for 48h as described in Example G6.

### c) Treatment of encapsulated human primary islets with a cocktail of proinflammatory cytokines

Encapsulated islets were treated with 20 ng/mL of TNFo, 10 ng/mL of IL1β and 50 ng/mL of IFNγ, diluted in culture medium, for 24h at 37°C under 5% CO₂ in a humidified atmosphere.

### d) In vitro functionality evaluation of encapsulated human primary islets

The *in vitro* functionality of encapsulated human primary islets was evaluated as described in Example G3, 4 days after encapsulation. Proinflammatory stress was induced *in* vitro by stimulating the encapsulated human primary islets with a cocktail of proinflammatory cytokines for 24h. The effect of Kineret^{®} on the impact of proinflammatory stress on islet functionality was tested by pretreating the encapsulated human primary islets for 48h before cytokine treatment. For each sample, secretion indexes were normalized to the mean secretion index of their respective untreated control condition. Results from Examples G1-3, G1-5 and G1-6 were pooled. See Table 50.

**Table 50: Functionality of encapsulated human primary islets in G1-3, G1-5 and G1-6. Data generated with N=3 independent human primary islet preparations were pooled. A total of n=12 samples were analyzed per condition. Statistical analyses = One-way ANOVA. N.A. = Not Applicable. Sd = Standard deviation.**

| | | **Untreated control condition** | **Cytokines** | **Kineret^{®}** / **Cytokines** |
|---|---|---|---|---|
| Normalized secretion indexes (Mean ± sd) (%) | | 100 ± 25 | 69 ± 19 | 101 ± 35 |
| Post test p-values | vs. Untreated control | N.A. | 0.0101 | 0.9985 |
| | vs. Cytokines condition | 0.0101 | N.A. | 0.0142 |

Secretion indexes of encapsulated human primary islets were drastically decreased upon 24h cytokine treatment (one-way ANOVA, p=0.0101 vs. Untreated control condition). Pretreatment with Kineret^{®} for 48h prior to cytokine treatment, led to a complete restoration of encapsulated islet functionality (one-way ANOVA, p=0.0142 vs. cytokines condition and p=0.9985 vs. untreated control condition).

In conclusion, *in vitro* pretreatment of encapsulated human primary islets with 10µg/mL of Kineret^{®} for 48h restores islet functionality after cytokine induced inflammation.

### Example G8: Enrichment of human primary islet preparations with small islet populations improves in vitro functionality upon encapsulation

### a) Method for specific enrichment of human primary islet preparations with smaller or larger islets

Human primary islets from 2 independent preparations were isolated as described in Example B2A. 7 days after isolation, specific enrichment of the preparation with smaller, intermediate or larger islets was performed using different filters (pore sizes of 150, 200 and 300 µm). Three populations were isolated: a population of islets with larger sizes (large islets) - not passing the 200 µm pore size filter-, a population of islets with intermediate sizes (intermediate islets) - passing through filter of 200 µm pore size - and a population of islets with smaller sizes (small islets) - passing through filter of 150 µm pore size. They were compared to the initial population of islets (pooled population).

### b) Islet encapsulation

The different populations of islets from 2 independent experiments were encapsulated in G1-3 and G1-4, 7 days after isolation.

### c) Islet size distribution quantification

The quantification of the islet size distribution was performed on each encapsulated islet populations, as described in Example B2-G2, 3^{rd} paragraph, in comparison with the encapsulated pool population. Objects with a diameter < 50µm were excluded for size distribution analyses. See Table 51.

**Table 51: Islet size distribution in the different encapsulated islet populations. Mean +/- sd are reported. Statistical analyses = One-way nonparametric ANOVA: Post test p-values vs. the pool population are reported. N.A. = Not Applicable. N.D. = Not Done. Sd = Standard deviation.**

| **Size distribution** | | **Pool Population** | **Small islets** | **Intermediate islets** | **Large islets** |
|---|---|---|---|---|---|
| G1-3 | Mean ± sd (µm) | 138 ± 72 | 105 ± 43 | 124 ± 56 | N.D. |
| | Post test p-values | N.A. | < 0.0001 | 0.0008 | N.D. |
| G1-4 | Mean ± sd (µm) | 144 ± 84 | 93 ± 33 | N.A. | 246 ± 99 |
| | Post test p-values | N.A. | < 0.0001 | N.A. | < 0.0001 |

The population of encapsulated small islets showed a drastic shift of the islet size distribution, towards smaller islets (One-way nonparametric ANOVA, Post test p-values vs. the encapsulated pool population p < 0.0001). On the opposite, the population of encapsulated large islets showed a drastic shift of the islet size distribution, towards larger islets (One-way nonparametric ANOVA, Post test p-value vs. the encapsulated pool population p < 0.0001). Even if the population of encapsulated intermediate islets showed a lower number of large islets (highlighted by the lower standard deviation compared to the encapsulated pool population), the average diameter of the objects was only slightly decreased (One-way nonparametric ANOVA, Post test p-value vs. the encapsulated pool population p < 0.0008).

In conclusion, the filtration procedure allows to enrich human primary islets with populations of islets with smaller or larger diameters. Enrichment with smaller islets, allows to obtain a population of islets with a more homogenous diameter, compared to the initial pool population.

### d) In vitro functionality evaluation of encapsulated human primary islets

The *in vitro* functionality of the different populations of encapsulated human primary islets was evaluated as described in Example B3-G3, 10 and 7 days after encapsulation, for G1-3 and G1-4, respectively. For each sample, secretion indexes were normalized to the mean secretion index of their respective Pool Population condition. Results from Examples G1-3, and G1-4 were pooled. See Table 52.

**Table 52: Functionality of encapsulated human primary islets in G1-3 and G1-4. Data generated with N=2 independent human primary islet preparations were pooled. A total of n=8 (Pool Population and Small islets) or n=4 (Intermediate and large islets) samples were analyzed. Statistical analyses = One-way ANOVA: Post test p-values vs. the pool population are reported. N.A. = Not Applicable. Sd = Standard deviation.**

| | **Pool Population** | **Small islets** | **Intermediate islets** | **Large islets** |
|---|---|---|---|---|
| Mean normalized secretion indexes (Mean ± sd)(%) | 100 ± 34 | 193 ± 55 | 226 ± 57 | 57 ± 8 |
| Post test p-values | N.A. | 0.0025 | 0.0623 | 0.1190 |

Secretion indexes of the encapsulated small islets were drastically increased compared to the encapsulated Pool Population (One-Way ANOVA, Post test p-value vs. the encapsulated pool population p=0.0025). Secretion indexes of the encapsulated intermediate islets were also increased compared to the encapsulated pool population, closed to the significativity (One-Way ANOVA, Post test p-values vs. the encapsulated pool population p=0.0623). On the opposite, secretion indexes of the encapsulated large islets were decreased compared to the encapsulated pool population, even if statistical significance was not reached (One-Way ANOVA, Post test p-values vs. the encapsulated pool population p=0.1190).

In conclusion, enrichment of human primary islet preparations with smaller islets improves *in vitro* functionality upon encapsulation.

### Example G9: Encapsulated controlled small size Human derived pseudo islets shows improved in vitro functionality as compared to encapsulated native human primary islets

### a) Method for the generation of pseudo islets

Human primary islets were isolated as described in Example B2A. Pseudo islets were generated 6 days after isolation. To do so, islets were first dissociated using TrypLE cell dissociation reagent, at 37°C for 10min. Dissociation was stopped by adding complete medium and the single cell preparation was filtrated through a 150µm filter to discard any non-disaggregated islet. Single cells were then seeded in Eplasia microwell plates at a cellular density of 800 or 1600 cells per microwell. To allow cell reaggregation, plates were incubated for 7 days at 37°C under 5% CO2 in a humidified atmosphere.

### b) Islet and pseudo islet encapsulation

The different populations of pseudo islets and islets were encapsulated in G1-7, 13 days after isolation.

### c) Quantification of the size distribution of encapsulated pseudo islets.

The quantification of the size distribution was performed on each encapsulated pseudo islet population as well as on encapsulated native islets, as described in Example B2-G2 3^{rd} paragraph. Objects with a diameter < 50µm were excluded for size distribution analyses. See Table 53.

**Table 53: Size distribution of pseudo islets encapsulated in G1-7, in comparison with encapsulated native islets. Mean +/- standard deviations are reported. Sd = Standard deviation.**

| **G1-7** | **Native islets** | **Pseudo islets (800 cells** / **micro well)** | **Pseudo islets (1600 cells** / **micro well)** |
|---|---|---|---|
| Size distribution (Mean ± sd) (µm) | 125 ± 65 | 120 ± 36 | 145 ± 44 |

Encapsulated pseudo islets had a more homogenous size compared to encapsulated native islets, as highlighted by the lower standard deviation of the size distribution. Even if encapsulated pseudo islets generated with 800 cells per microwell did not contain structures with large diameters, the mean diameter was not drastically decreased compared to encapsulated native islets.

### d) In vitro functionality evaluation of encapsulated pseudo islets

The *in vitro* functionality of the different populations of encapsulated pseudo islets was evaluated as described in Example B3, 4 days after encapsulation, in comparison with encapsulated native islets from the same islet preparation. For each sample, secretion indexes were normalized to the mean secretion index of encapsulated native islets condition. See Table 54.

**Table 54: Functionality of human primary islets and pseudo islets encapsulated in G1-7. Two conditions of pseudo islets were analyzed: pseudo islets generated either with 800 or 1600 cells per microwell. A total of n=4 samples per condition were analyzed. Statistical analyses = One-way nonparametric ANOVA: Post test p-values vs. native islets are reported. N.A. = Not Applicable. Sd = Standard deviation.**

| **G1-7** | **Native islets** | **Pseudo islets (800 cells** / **micro well)** | **Pseudo islets (1600 cells** / **micro well)** |
|---|---|---|---|
| Normalized secretion indexes (Mean ± sd)(%) | 100 ± 21 | 193 ± 37 | 71 ± 10 |
| Post test p-values | N.A. | 0.0011 | 0.2418 |

Secretion indexes of the encapsulated pseudo islets generated with 800 cells per microwell were drastically increased compared to encapsulated native islets (One-Way ANOVA, Post test p-values vs. encapsulated native islets p=0.0011). On the opposite, secretion indexes of the encapsulated pseudo islets generated with 1600 cells per microwell were slightly decreased compared to encapsulated native islets even if statistical significance was not reached (One-Way ANOVA, Post test p-values vs. encapsulated native islets p=0.2418).

In conclusion, generation of controlled small size pseudo islets from human primary islets preparation allows to improve *in vitro* functionality upon encapsulation.

## Claims

1. Bicomponent glue **characterized in that** it comprises precursors of a hydrogel, said hydrogel comprising:
a crosslinked dextran polymer Dx bearing anionic groups wherein the at least divalent radical L(-)_{I} is covalently bound to the dextran polymer backbone with I W radicals, wherein,
- L(-)_{I} is a linear or branched polyether
- I is the valence of L and the number of W radicals bound to the dextran polymer and is an integer comprised from 2 to 8 (2 ≤ I ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

2. Bicomponent glue according to claim 1, for therapeutical and/or surgical use.

3. Bicomponent glue for therapeutical and/or surgical use according to claim 2, for improving adhesion and/or immobilizing *in vivo* an implant onto biological tissue of the recipient patient.

4. Bicomponent glue for therapeutical and/or surgical use according to any of the claims 2 or 3, wherein the implant is an artificial organ.

5. Bicomponent glue for therapeutical and/or surgical use according to any of the claims 2 to 4, wherein the implant is an artificial pancreas.

6. Bicomponent glue for therapeutical and/or surgical use according to any of the claims 2 to 5, wherein the artificial pancreas is a hydrogel comprising active ingredients.

7. Bicomponent glue for therapeutical and/or surgical use according to any of the claims 2 to 6, wherein the artificial pancreas is a hydrogel comprising secreting cells.

8. Bicomponent glue for therapeutical and/or surgical use according to any of the claims 2 to 7, wherein the artificial pancreas is a hydrogel comprising insulin secreting cells.

9. Bicomponent glue for therapeutical and/or surgical use according to any of the claims 2 or 3, wherein the implant is a biological tissue.

10. Bicomponent glue for therapeutical and/or surgical use according to any of the claims 2 to 9, wherein the bicomponent glue is applied on the surface of the implant, on the surface of the biological tissue of the recipient patient and/or on the contact area.

11. Bicomponent glue for surgical use according to any of the claims 2 to 10, wherein the bicomponent glue is therapeutical and/or surgical on the contact area.

12. Bicomponent glue for therapeutical and/or surgical use according to any of the claims 10 or 11, wherein the quantity of bicomponent glue applied per square centimeters of contact area is from 0,01 µL/cm² to 500 µL/cm².

13. Bicomponent glue for therapeutical and/or surgical use according to any of the claims 10 to 12, wherein the quantity of bicomponent glue applied per square centimeters of contact area is from 10 µL/cm² to 50 µL/cm².

14. Bicomponent glue for therapeutical and/or surgical use according to any of the claims 10 to 13, wherein the quantity of bicomponent glue applied per square centimeters of contact area is from 20 µL/cm² to 30 µL/cm².

15. Process for improving adhesion and/or immobilizing in vivo an implant onto biological tissue of the recipient patient, **characterized in that** it comprises the step of applying the bicomponent glue according to claim 1 on the surface of the implant, on the surface of the biological tissue of the recipient patient and/or on the contact area.
